(19) Europäisches Patentamt
European Patent Office
Office européen des brevets

(11) EP 2 055 705 A1

(12) EUROPEAN PATENT APPLICATION
published in accordance with Art. 153(4) EPC

(43) Date of publication:
**06.05.2009 Bulletin 2009/19**

(21) Application number: **07791578.3**

(22) Date of filing: **30.07.2007**

(51) Int Cl.:
***C07D 487/10*** *(2006.01)* ***A61K 31/438*** *(2006.01)*
***A61K 45/00*** *(2006.01)* ***A61P 9/10*** *(2006.01)*
***A61P 11/00*** *(2006.01)* ***A61P 11/06*** *(2006.01)*
***A61P 19/02*** *(2006.01)* ***A61P 27/02*** *(2006.01)*
***A61P 29/00*** *(2006.01)* ***A61P 31/18*** *(2006.01)*
***A61P 35/00*** *(2006.01)* ***A61P 35/04*** *(2006.01)*
***A61P 37/02*** *(2006.01)* ***A61P 37/06*** *(2006.01)*
***A61P 43/00*** *(2006.01)*

(86) International application number:
**PCT/JP2007/064893**

(87) International publication number:
**WO 2008/016006 (07.02.2008 Gazette 2008/06)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **31.07.2006 JP 2006207718**

(71) Applicant: **Ono Pharmaceutical Co., Ltd.**
**Osaka-shi, Osaka 541-8526 (JP)**

(72) Inventors:
- **HANADA, Keisuke**
**Mishima-gun**
**Osaka 618-8585 (JP)**
- **KOKUBO, Masaya**
**Mishima-gun**
**Osaka 618-8585 (JP)**
- **OCHIAI, Hiroshi**
**Mishima-gun**
**Osaka 618-8585 (JP)**
- **TANI, Kousuke**
**Mishima-gun**
**Osaka 618-8585 (JP)**
- **SHIBAYAMA, Shiro**
**Tsukuba-shi**
**Ibaraki 300-4247 (JP)**

(74) Representative: **Henkel, Feiler & Hänzel**
**Patentanwälte**
**Maximiliansplatz 21**
**80333 München (DE)**

(54) **COMPOUND HAVING CYCLIC GROUP BOUND THERETO THROUGH SPIRO BINDING AND USE THEREOF**

(57) A compound represented by general formula (I):

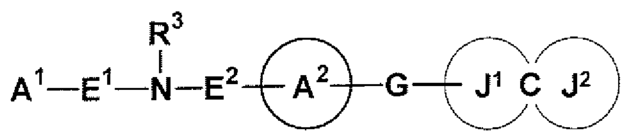

(I)

a salt thereof, a solvate thereof, or a prodrug thereof wherein all symbols are as defined in the specification has an antagonistic activity against CXCR4 and is therefore useful as a preventive and/or therapeutic agent for CXCR4-mediated diseases, for example, inflammatory and immune diseases (for example, rheumatoid arthritis, arthritis, retinopathy, macular degeneration, pulmonary fibrosis, transplanted organ rejection, etc.), allergic diseases, infections (for example, human immunodeficiency virus infection, acquired immunodeficiency syndrome, etc.), psychoneurotic diseases, cerebral diseases, cardiovascular disease, metabolic diseases, cancerous diseases (for example, cancer, cancer metastasis, etc.), a preventive and/or therapeutic agent for cancerous diseases or infections, or an agent for regeneration therapy.

EP 2 055 705 A1

**Description**

TECHNICAL FIELD

The present invention relates to compounds having a spiro-bound cyclic group and use thereof.
More particularly, the present invention relates to (1) compound represented by formula (I)

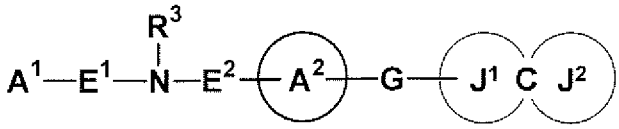 (I)

(wherein all symbols have the same meaning as described hereinafter), salts thereof, N-oxides thereof or solvates thereof, prodrugs thereof, (2) use thereof, and (3) a method for producing the same.

BACKGROUND ART

Chemokine is known as a basic protein which has chemotaxis and an activating activity against endogenous leucocytes and also has strong heparin-binding abilities. It is now considered that chemokine is associated with not only control of infiltration of specific leucocytes upon inflammatory and immune responses, but also development, homing of lymphocytes under physiological conditions and migration of hemocyte precursor cells and somatic cells.
Differentiation, proliferation and cell death of blood cells are controlled by various cytokines. Inflammation occurs at a local region in a living body. Differentiation and maturation of lymphocytes, and the like are carried out at a specific site. More particularly, required various cells migrate and accumulate in the specific site and a sequence of inflammatory and immune responses arise. Thus, in addition to differentiation, proliferation and death of cells, cell migration is also an essential phenomenon to an immune system.
In the living body, migration of blood cells start with sifting hemopoiesis that started at AGM (Aorta Gonad Mesonephros) region via fetal liver to permanent hematopoiesis at bone marrow in a development course. Moreover, precursors of T cells and thymus dendritic cells migrate from fetal liver into bone marrow and then into the thymus gland. They differentiate under thymus environment. The T cells are subjected to clonal selection migrates into secondary lymphoid tissues, where they contribute to immune responses in periphery. Skin Langerhans cells that caught antigen, thereby undergone activation and differentiation migrate to T cell region in a topical lymph node, where they activate naive T cells therein as dendritic cells. The memory T cells again perform its homing into the lymph node via lymphatic and blood vessels. In addition, B cells, T cells in intestinal epithelia, γδT cells, NKT cells, and dendritic cells migrate from bone marrow not via thymus, differentiate and contribute to immune responses.
Chemokine is closely associated with such a migration of the various cells. For example, SDF-1 (Stromal cell derived factor-1) and its receptor, CXCR4 also act on various immune- and inflammatory reactions. For example, they have been reported to be associated with accumulation and activation of CD4+T cells in a synovial membrane from a human patient suffering from rheumatoid arthritis (J. Immunol., 165, 6590-6598 (2000)). In addition, in a CIA model mouse, CXCR4 inhibitor inhibited accumulation of leucocytes in a joint and dramatically reduced arthritis score (J. Immunol., 167, 4648-4692 (2001)). In a mouse OVA-induced airway hypersensitive model, an anti-CXCR4 antibody reduced the number of eosinophiles accumulating in pulmonary interstitial tissues and prevented airway hypersensitivity (J. Immunol., 165, 499-508 (2000)). In a mouse bleomycin-created pulmonary disorder models, an anti-SDF-1 antibody inhibited invasion of fibrocytes to the lung and inhibited fibrosis of the lung (J. Clin. Invest., 114, 438-446 (2004)). In a mouse LPS-induced pneumonia model, it was observed that the number of neutrophiles was increased with an increase in an SDF-1 concentration in an alveolar lavage fluid, and the number of neutrophiles in the alveolar lavage fluid was prevented from being incrased by administration of anti-SDF-1 antibody. In a mouse retinopathy model, an anti-SDF-1 antibody inhibited vascular endothelial progenitor cell invasion to the retina and inhibited neovascularisation at the retina (J. Clin. Invest., 115, 86-93 (2005)).
There has been reported that SDF-1 and its receptor, CXCR4 play an important role in maintaining hemopoietic stem cells in bone marrow J. Exp. Med., 185, 111-120 (1997), Blood, 97, 3354-3360 (2001)). Accordingly, control of SDF-1 and CXCR4 is expected to modulate recruitment of hemopoietic stem cells to peripheral blood and are useful for peripheral blood stem cell transplantation and reproduction transplantation treatment.
SDF-1 and CXCR4 are associated with infiltration of various cancer cells such as breast cancer, prostate cancer, and ovarian cancer (Nature, 410, 50-56 (2001), Cancer Res., 62, 1832-1837 (2002), Cancer Res., 62, 5930-5938 (2002)). In a model of transferring a human breast cancer cell strain into a SCID mouse, an anti-CXCR4 antibody prevented metastasis of breast cancer cells to lung (Nature, 410, 50-56 (2001)). In human ovarian epithelial tumor, highly

expression of SDF-1 promotes accumulation of plasmacytoid dendritic cells and inhibits the act of bone marrow dendritic cells associated with tumor immune and suppresses tumor immune (Nat. Med., 12, 1339 (2001)). Moreover, SDF-1 is associated with proliferation and migration of non-Hodgkin's lymphoma cells, and in a model of transferring a human non-Hodgkin's lymphoma cells into a NOD/SCID mouse, an anti-CXCR4 antibody inhibited proliferation of the tumor cells and improved mouse mortality (Cancer Res., 62, 3106-3112 (2002)). A low molecular weight CXCR4 antagonist increased apoptosis of medulloblastoma transplantated in the mouse skull and inhibited tumor proliferation (Proc. Nat. Acad. Sci. USA, 100, 13513-13518 (2003)). In a lung metastasis model using malignant melanoma, the low molecular weight CXCR4 antagonist enhanced the antitumor effect of an immunostimulant and an anticancer drug (Mol Cancer Ther., 5, 2592-9 (2006)).

SDF-1 and CXCR4 play an important role for formation of hippocampus dentate gyrus granulocyte, that is essential for memory and learning and are associated with development of a disease associated with adult plasticity and pathology of hippocampus, for example Alzheimer's disease, stroke and epilepsy (Development, 129, 4249-4260 (2002), Trends in Neuroscience, 25, 548-549 (2002)).

SDF-1 and CXCR4 are essential for a function of self-reactive B cells associated with development of diabetes. In NOD mouse, an anti-SDF-1 antibody reduced blood glucose level and the number of mature IgM+B cells in a periphery tissue (Immunology, 107, 222-232 (2002)). In a human arteriosclerotic plaque, SDF-1 was highly expressed and activated blood platelets (Circ. Res., 86, 131-138 (2000)).

SDF-1 and CXCR4 are associated with residence of hematopoietic stem cells and hemopoietic precursor cells in bone marrow, and use of AMD3100 being CXCR4 antagonist in combination with G-CSF enabled an increase in the number of hemopoietic stem cells and hemopoietic precursor cells in peripheral blood (Journal Experimental Medicine, 2001, 1307-1318 (2005). It is known that the number of neutrophiles, lymphocytes and monocytes in peripheral blood are increased by administering a low molecular weight CXCR4 antagonist to human (Blood, 102, 2728-2730 (2003)). Therefore, the immunological enhancing effect is expected to the low molecular weight CXCR4 antagonist.

In addition, the results of SDF-1/CXCR4 knock-out mice showed that SDF-1 is essential for functions of central nervous system, heart and vessels of gastrointestinal tract in addition to lymphocytes (Nature, 382, 635-639 (1996), Nature, 393, 591-594 (1998), Nature, 393, 595-599 (1998)). Accordingly, it may be associated with a disease of these tissues.

Thus, chemokine receptors are expressed at various specific cells and at a specific time. They are largely associated with the control of inflammatory- and immune-responses through a mechanism by which their effector cells accumulate in a site where chemokine is produced.

Acquired immunodeficiency syndrome (also called AIDS) that caused by infection of human immunodeficiency virus (hereinafter abbreviated to HIV) is one of diseases for which therapies are the most eagerly desired lately. Once HIV infection has been established in a main target cell, CD4+ cell, HIV repetitively proliferates in a patient's body and in the event deathly destroys T cells responsible for immunological functions by necrosis. In this process, immunological functions are gradually deteriorated, various immunocompromised states become to develop such as fever, diarrhea and swelling of a lymph node, and various opportunistic infections such as carinii pneumonia are easily complicated. It is well known that such a state is the onset of AIDS and induces malignant tumors such as Kaposi's sarcoma and becomes severe.

Currently, there are tried various preventive and therapeutic treatments for AIDS as follows: for example, (1) inhibition of HIV proliferation by administration of reverse transcriptase inhibitors and protease inhibitors, and (2) prevention or alleviation of opportunistic infections by administration of an immunostimulant, etc.

HIV mainly infects helper T cells which play a key role in the immune system. Since 1985, it has been known that in this process HIV utilizes a membrane protein CD4 that is expressed on the membrane of T cells (Cell, 52, 631 (1985)). CD4 molecule consists of 433 amino acid residues and is expressed in macrophages, some B cells, vascular endothelial cells, Langerhans cells in skin tissues, dendritic cells located in lymphatic tissues, glia cells of central nervous system and the like in addition to mature helper T cells. However, as it becomes obvious that HIV infection cannot be established with only CD4 molecule, the possible presence of some factor that is responsible for infection of cell with HIV, other than CD4 molecule, has been suggested.

In 1996, a cell membrane protein called Fusin has been identified as a factor responsible for HIV infection other than a CD4 molecule (Science, 272, 872 (1996)). This Fusin molecule has been demonstrated to be a receptor for SDF-1, namely, CXCR4. In addition, it has been shown that SDF-1 specifically inhibits infection of T cell-directed (X4) HIV in vitro (Nature, 382, 829 (1996), Nature, 382, 833 (1996)). This may be considered that SDF-1 binds to CXCR4 prior to HIV, thereby taking away a scaffold for infecting a cell from HIV resulting in inhibition of HIV infection.

Also, at the same period, there has been found that another chemokine receptor CCR5, that is a receptor for RANTES, MIP-1$\alpha$, and MIP-1$\beta$, is utilized at infection of macrophage-directed (R5) HIV (Science, 272, 1955 (1996)).

Accordingly, those which can compete with HIV for CXCR4 and CCR5 or those which bind to a HIV virus and prevent for said virus from binding to CXCR4 and CCR5 may be a HIV infection inhibitor. In addition, there is a case where a low molecular weight compound discovered as a HIV infection inhibitor was showed to be indeed an antagonist

of CXCR4 (Nature Medicine, 4, 72 (1998)).

As described above, compounds having an antagonistic activity against CXCR4 are effective, such as, for prevention and/or treatment of inflammatory and immune diseases, allergic diseases, infections, particularly HIV infection, and diseases associated with the infection, psychoneurotic diseases, cerebral diseases, cardiovascular diseases, metabolic diseases, cancerous diseases and the like. Also, the compounds are useful for cell therapy and regeneration therapy.

Heretofore, the following compound has been reported. For example, it is disclosed that a compound represented by formula (R):

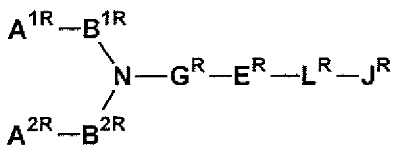

(R)

(wherein $A^{1R}$ and $A^{2R}$ each independently represents a nitrogen-containing heterocyclic ring group which may have a substituent(s); $B^{1R}$ and $B^{2R}$ each independently represents - CO-, $-SO_2-$, or $-CH_2-$; $G^R$ represents a bond, -CO-, $-SO_2-$, or $-CH_2-$; $E^R$ represents a cyclic group; $L^R$ represents a bond or a spacer having a main chain of 1 to 4 atom(s); $J^R$ represents (1) a cyclic group which is substituted with a group having a basic group, and also may have a substituent (s) or (2) a spiro-bound cyclic group which may be substituted with a group having a basic group, and also may have a substituent(s); and only required portions were extracted with respect to definition of each group), a salt thereof, an N-oxide thereof or a solvate thereof, a prodrug thereof has antagonistic activity against CXCR4 (see WO 2007/058322 pamphlet).

It is disclosed that a compound represented by formula (S):

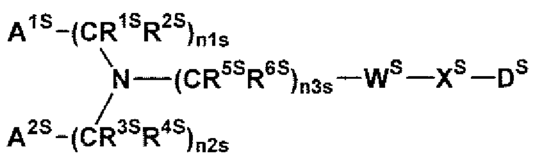

(S)

(wherein n1s, n2s and n3s represent 0 to 3; $R^{1S}$, $R^{2S}$, $R^{3S}$, $R^{4S}$, $R^{5S}$ and $R^{6S}$ each independently represents a hydrogen atom or an optionally substituted C 1 to 15 alkyl group; $A^{1S}$ and $A^{2S}$ each independently represents an optionally substituted monocyclic or polycyclic heteroaromatic ring; $W^S$ represents an optionally substituted C1 to 15 alkylene group, $X^S$ represents O, $CH_2$, or $NR^{11S}$; $D^S$ represents $-Q^S-Y^S-B^S$; $Q^S$ represents a bond or - CO- when $X^S$ is $NR^{11S}$; $Y^S$ represents $-(CR^{18S}R^{19S})_{m3Y}-$; $R^{18S}$ and $R^{19S}$ each independently represents a hydrogen atom or an optionally substituted C1 to 15 alkyl group; m3s represents 0 to 6; $B^S$ represents $-NR^{25S}R^{26S}$; $R^{25S}$ and $R^{26S}$ represents a hydrogen atom or an optionally substituted C1 to 15 alkyl group when $X^S$ is not $CH_2$; and only required portions were extracted with respect to definition of each group), a pharmaceutically acceptable salt thereof, or a prodrug thereof has an antagonistic activity against CXCR4 (see WO 2004/024697 pamphlet).

It is disclosed that a compound represented by formula (T):

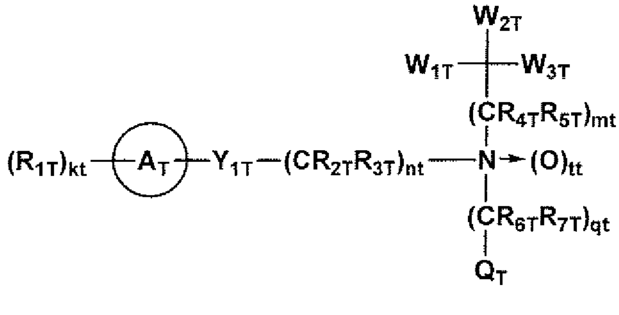

(wherein $W_{1T}$ represents a heterocyclic ring ring which may have a substituent(s); $W_{2T}$ represents a hydrogen atom,

a heterocyclic ring ring which may have a substituent(s) or a substituent(s); $W_{3T}$ represents a hydrogen atom or a substituent(s); $R_{1T}$ represents an alkyl group which may have a substituent(s); $R_{2T}$, $R_{3T}$, $R_{4T}$, $R_{5T}$, $R_{6T}$ and $R_{7T}$ each independently represents a hydrogen atom or an alkyl group which may have a substituent(s); $Y_{1T}$ represents -C($R_{2T}$)($R_{3T}$)-; $A_T$ represents a heterocyclic ring which may have a substituent(s); $Q_T$ represents a heterocyclic ring which may have a substituent(s) or an aryl group which may have a substituent(s); nt represents an integer of 2 to 8; mt, qt and tt represent 0 or 1; kt represents an integer of 0 to 2; and only required portions were extracted with respect to definition of each group), a pharmaceutically acceptable salt thereof, or a pharmaceutically acceptable solvate thereof has a Liver X receptor (LXR) antagonistic activity (see WO 2005/023247 pamphlet).

It is disclosed that a compound represented by formula (U):

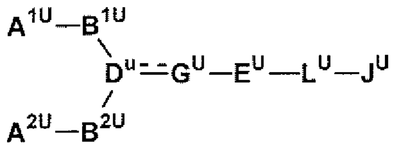

(U)

(wherein $A^{1U}$ and $A^{2U}$ each independently represents a nitrogen-containing heterocyclic ring group which may have a substituent(s); $B^{1U}$, $B^{2U}$ and $G^U$ each independently represents -CO-, $-SO_2-$, or $-CH_2-$; $D^U$ represents a carbon atom or a nitrogen atom; $E^U$ represents a cyclic group; $L^U$ represent a bond or a spacer having a main chain of 1 to 4 atom (s); $J^U$ represent (1) a cyclic group which is substituted with a group having a basic group, and also may have a substituent(s)) or (2) a spiro-bound cyclic group which may be substituted with a group having a basic group, and also may have a substituent(s), provided that any one of $B^{1U}$, $B^{2U}$ and $G^U$ represents -CO- or $-SO_2-$; and only required portions were extracted with respect to definition of each group), a salt thereof, an N-oxide thereof or a solvate thereof, a prodrug thereof has an antagonistic activity against CXCR4 (see WO 2006/022454 pamphlet).

It is disclosed that a compound represented by formula (V):

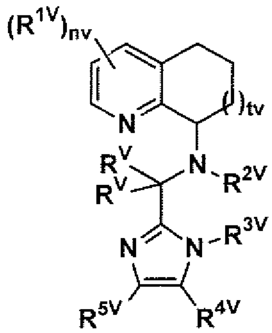

(V)

(wherein $R^V$ represents a hydrogen atom, alkyl, or alkenyl; $R^{1V}$ represents alkyl or alkenyl; tv represents 0, 1, or 2; $R^{2V}$ represents a hydrogen atom, alkyl, or $-R^{aY}NR^{6V}R^{7V}$; $R^{3V}$ represents Het which may be substituted with $-R^{aV}NR^{6V}R^{7V}$, or $-R^{aV}$Het; $R^{aV}$ represents alkylene or alkynylene; $R^{6V}$ and $R^{7V}$ each independently represents a hydrogen atom or alkyl; Het represents a heterocyclic ring which may have a substituent(s); and only required portions were extracted with respect to definition of each group), a salt thereof, a solvate thereof, or physiologically functionable derivative thereof has an antagonistic activity against CXCR4 (see WO 2006/020415 pamphlet).

It is disclosed that a compound represented by formula (W):

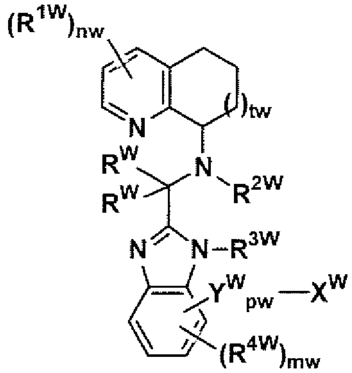

(W)

(wherein $R^W$ represents a hydrogen atom, alkyl, or alkenyl; $R^{1W}$ represents alkyl or alkenyl; tw, mw and nw each independently represents 0, 1, or 2; pw represents 0 or 1; $R^{2W}$ represents a hydrogen atom, alkyl which may have a substituent(s), or $-R^{aW}NR^{6W}R^{7W}$; $R^{3W}$ represents alkyl which may have a substituent(s) or $-R^{aW}Ay$; $R^{4W}$ represents alkyl, -Ay, or - $R^{aW}NR^{6W}R^{7W}$; $X^W$ represents $-N(R^{10W})_2$, $-R^{aW}N(R^{10W})_2$, or $-R^{aW}AyR^{aW}N(R^{10W})_2$; $Y^W$ represents $-NR^{10W}-$, $-C(O)NR^{10W}-$, or -C(O)-; $R^{aW}$ represents alkylene or alkynylene; $R^{6W}$ and $R^{7W}$ each independently represents a hydrogen atom or alkyl; Ay represents aryl which may have a substituent(s);, Het represents a heterocyclic ring which may have a substituent(s); and only required portions were extracted with respect to definition of each group), or a pharmaceutically acceptable salt thereof, or an ester thereof has an antagonistic activity against CXCR4 (see WO 2006/023400 pamphlet).

It is disclosed that a compound represented by formula (X):

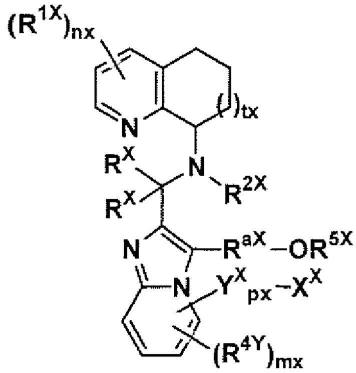

(X)

(wherein $R^X$ represents a hydrogen atom, alkyl, or alkenyl; $R^{1X}$ represents alkyl or alkenyl; tx, mx and nx each independently represents 0, 1, or 2; px represents 0 or 1; $R^{2X}$ represents a hydrogen atom, alkyl which may have a substituent(s), or $-R^{aX}NR^{6X}R^{7X}$; $R^{3X}$ represents alkyl which may have a substituent(s) or $-R^{aX}Ay$; $R^{4X}$ represents alkyl, -Ay, or - $R^{aX}NR^{6X}R^{7X}$; $R^{5X}$ represents a hydrogen atom, alkyl, or -Ay; $X^X$ represents $-N(R^{10X})_2$, - $R^{aX}N(R^{10X})_2$, or $-R^{aX}AyR^{aX}N(R^{10X})_2$; $Y^X$ represents $-NR^{10X}-$, $-C(O)NR^{10X}-$, or -C(O)-; $R^{aX}$ represents alkylene or alkynylene; $R^{6X}$ and $R^{7X}$ each independently represents a hydrogen atom or alkyl; $R^{10X}$ represents a hydrogen atom or alkyl; Ay represents aryl which may have a substituent(s); Het represents a heterocyclic ring which may have a substituent(s); and only required portions were extracted with respect to definition of each group), a pharmaceutically acceptable salt thereof, or an ester thereof has antagonistic activity against CXCR4 (see WO 2006/026703 pamphlet).

It is disclosed that a compound represented by formula (Y):

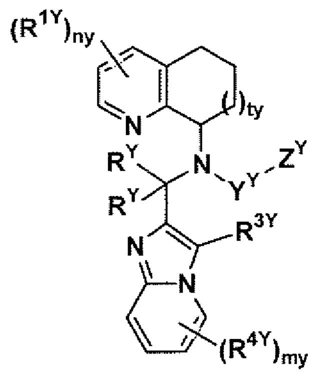

(Y)

(wherein $R^Y$ represents a hydrogen atom, alkyl, or alkenyl; $R^{1Y}$ represents alkyl or alkenyl; ty, my and ny each independently represents 0, 1, or 2; $R^{2Y}$ represents a hydrogen atom, alkyl which may have a substituent(s), or $-R^{aY}NR^{6Y}R^{7Y}$; $R^{3Y}$ represents alkyl which may have a substituent(s) or $-R^{aY}Ay$; $R^{4X}$ represents alkyl, -Ay, or $-R^{aY}NR^{6Y}R^{7Y}$; $R^{5Y}$ represents a hydrogen atom, alkyl, or -Ay; $X^Y$ represents $-N(R^{10Y})_2$, $-R^{aY}N(R^{10Y})_2$, or - $R^{aY}AyR^{aY}N(R^{10Y})_2$; $Y^Y$ represents alkyl, hydroxy, or alkylene which may be substituted with oxo; $Z^Y$ represents $-N(R^{10Y})_2$, -HetN$(R^{10Y})_2$, or $-AyR^{aY}N(R^{10Y})_2$; $R^{aY}$ represents alkylene or alkynylene; $R^{bY}$ and $R^{7Y}$ each independently represents a hydrogen atom or alkyl; $R^{10Y}$ represents a hydrogen atom, or alkyl; Ay represents aryl which may have a substituent (s); Het represents a heterocyclic ring which may have a substituent(s); and only required portions were extracted with respect to definition of each group), a pharmaceutically acceptable salt thereof, or an ester thereof has an antagonistic activity against CXCR4 (see WO 2006/036816 pamphlet).

It is disclosed that a compound represented by formula (Z):

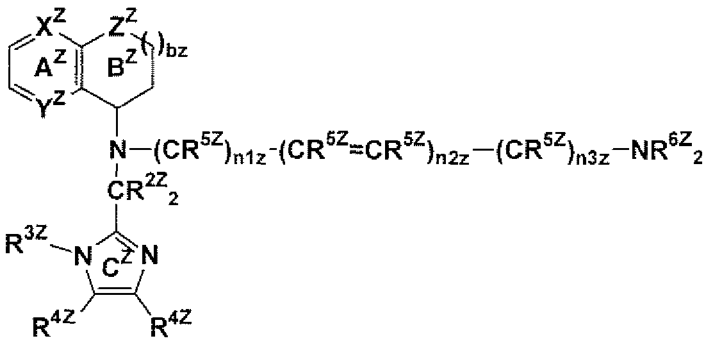

(Z)

(wherein $X^Z$ and $Y^Z$ each independently represents a nitrogen atom or $CR^{1Z}$; $Z^Z$ represent a sulfur atom, an oxygen atom, $NR^{1Z}$, or $CR^{1Z}_2$; $R^{1Z}$ to $R^{6Z}$ each independently represents a hydrogen atom or a substituent(s); n1z and n3z each independently represents 0 or an integer of 1 to 4; n2z represents 0 or 1; the sum of n1z, n2z and n3z represents 2 or more; bz represents 0, 1, or 2; two $R^{2Z}$(s), two $R^{4Z}$(s), two $R^{5Z}$(s) and two $R^{6Z}$(s) may form a ring, and $R^{2Z}$ and $R^{3Z}$, $R^{3Z}$ and $R^{4Z}$, and $R^{5Z}$ and $R^{6Z}$ may be combined to form a ring; and only required portions were extracted with respect to definition of each group), a salt thereof, or a prodrug thereof has an antagonistic activity against CXCR4 (see WO 2003/055876 pamphlet).

It is also known that a drug, which is a basic drug and has high lipophilicity, causes various side effects associated with accumulation of phospholipid and a basic drug generally has a very large distribution volume (YAKUGAKU ZASSHI (Journal of The Pharmaceutical Society of Japan), 121, 557-565 (2001)).

Patent Literature 1 WO 2007/058322 pamphlet
Patent Literature 2 WO 2004/024697 pamphlet
Patent Literature 3 WO 2005/023247 pamphlet
Patent Literature 4 WO 2006/022454 pamphlet
Patent Literature 5 WO 2006/020415 pamphlet
Patent Literature 6 WO 2006/023400 pamphlet
Patent Literature 7 WO 2006/026703 pamphlet
Patent Literature 8 WO 2006/036816 pamphlet

Patent Literature 9 WO 2003/055876 pamphlet

DISCLOSURE OF THE INVENTION

PROBLEMS TO BE SOLVED BY THE INVENTION

It is earnestly desired to develop an antagonist of CXCR4, which is useful as a preventive and/or therapeutic agent for inflammatory and immune diseases (for example, rheumatoid arthritis, arthritis, systemic erythematosus, retinopathy, macular degeneration, pulmonary fibrosis, rejection of transplanted organ, etc.), allergic diseases, infections (for example, human immunodeficiency virus infection, acquired immunodeficiency syndrome, etc.), cancerous diseases (for example, cancer, cancer metastasis, etc.), cardiac/vascular diseases (for example, arteriosclerosis, myocardial infarction, stenocardia, cerebral infarction, chronic arterial occlusive disease, etc.), or an agent for regeneration therapy, and is also safe with less side effects.

MEANS FOR SOLVING THE PROBLEMS

The present inventors have found that the group of specific chemical compounds can be used as a safe CXCR4 antagonist with less side effects. Particularly found that the group of specific chemical compounds can be used as the group of compounds, which has low basicity and also has decreased risk of side effects such as phospholipidosis and a small distribution volume, when compared with the compound described in WO 2006/022454 pamphlet (Patent Document 3), namely, the group of compounds, which has higher safety, and that anti-CXCR4 antagonic activity is retained. Thus, the present invention has been completed.
Thus, the present invention relates to:

[1] A compound represented by formula (I):

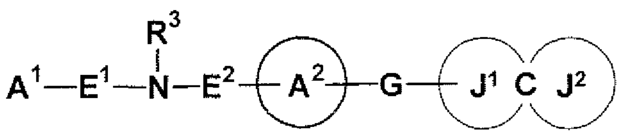

(I)

wherein $A^1$ represent a nitrogen-containing heterocyclic ring which may have a substituent(s) or $-NR^1R^2$ wherein $R^1$ and $R^2$ each independently represents a hydrogen atom or a substituent(s);
ring $A^2$ represents a divalent monocyclic cyclic group which may have a substituent(s); $E^1$ represents a divalent C1-4 aliphatic hydrocarbon group which may have a substituent(s);
$E^2$ represents a methylene group or a carbonyl group;
$R^3$ represents (1) a hydrogen atom, (2) a C1-4 aliphatic hydrocarbon group which may be substituted with a hydroxyl group which may be protected by a protective group, a carboxyl group which may be protected by a protective group, or a sulfo group which may be protected by a protective group, and also may have a substituent(s), (3) a carbocyclic ring group which may be substituted with a hydroxyl group which may be protected by a protective group, a carboxyl group which may be protected by a protective group, or a sulfo group which may be protected by a protective group, and also may have a substituent(s), (4) a heterocyclic group which may be substituted with a hydroxyl group which may be protected by a protective group, a carboxyl group which may be protected by a protective group, or a sulfo group which may be protected by a protective group, and also may have a substituent(s), (5) a C1-4 aliphatic hydrocarbon group which may be substituted with a hydroxyl group which may be protected by a protective group, a carboxyl group which may be protected by a protective group, or a sulfo group which may be protected by a protective group, and is also substituted with a carbocyclic ring group which may have a substituent(s) and may have a substituent(s), (6) a C1-4 aliphatic hydrocarbon group which may be substituted with a hydroxyl group which may be protected by a protective group, a carboxyl group which may be protected by a protective group, or a sulfo group which may be protected by a protective group, and is also substituted with a heterocyclic group which may have a substituent(s) and may have a substituent(s), (7) -(a divalent C1-4 aliphatic hydrocarbon group which may have a substituent(s))-(a divalent carbocyclic ring which may have a substituent(s))-$(Y^3)_t$-Z, or (8)-(a divalent C1-4 aliphatic hydrocarbon group which may have a substituent(s))-(a divalent heterocyclic ring which may have a substituent(s))-$(Y^3)_t$-Z wherein $Y^3$ represent a methylene group which may have a substituent(s), an ethenylene group which may have a substituent(s), an ethynylene group, a divalent nitrogen atom which may have a substituent(s), -C(O)-, -O-, -S-, -S(O)-, or $-SO_2-$, Z represents a hydroxyl group which may be protected by a protective group, a carboxyl group which may be protected by a protective group, or a sulfo group which may be protected by a protective

group, t represents an integer of 1 to 4, provided that when t represents an integer of 2 or more, a plurality of $Y^3$ may be the same or different,
G represents

**$-(G^1)_p-$** , or (a)

(b) 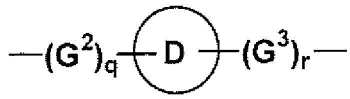

wherein $G^1$, $G^2$ and $G^3$ each independently represents a methylene group which may have a substituent(s), an ethenylene group which may have a substituent(s), an ethynylene group, a divalent nitrogen atom which may have a substituent(s), -C(O)-, -O-, -S-, -S(O)-, or-$SO_2$-, ring D represents a divalent monocyclic cyclic group which may have a substituent(s), p represents an integer of 1 to 8, q and r each independently represents 0 or an integer of 1 to 6, provided that when p represents an integer of 2 or more, a plurality of $G^1$ may be the same or different, when q represents an integer of 2 or more, a plurality of $G^2$ may be the same or different, and when r represents an integer of 2 or more, a plurality of $G^3$ may be the same or different, and the sum of q and r represents 6 or less,
ring $J^1$ represents a 3- to 10-membered monocyclic or bicyclic heterocyclic ring which has at least one nitrogen atom and also may have an oxygen atom and/or an optionally oxidized sulfur atom; ring $J^2$ represents (i) a C3-10 monocyclic or bicyclic carbocyclic ring substituted with a group having a basic group, (ii) a 3- to 10-membered monocyclic or bicyclic heterocyclic ring consisting of a carbon atom, an oxygen atom and/or an optionally oxidized sulfur atom which is substituted with a group having a basic group, or (iii) a 3- to 10-membered monocyclic or bicyclic heterocyclic ring which may be substituted with a group having a basic group, and also has at least one nitrogen atom and may have an oxygen atom and/or an optionally oxidized sulfur atom,
ring $J^1$ and ring $J^2$ may have 1 to 8 substituent(s) on the substitutable position and, when the number of substituents is 2 or more, a plurality of substituents may be the same or different,
a salt thereof, an N-oxide thereof or a solvate thereof, or a prodrug thereof;
[2] The compound according to the above [1], wherein $R^3$ is:

(a) $-Y^1-Z$ , or

(b) 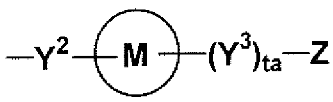

wherein $Y^1$ represent a divalent C1-4 aliphatic hydrocarbon group which may have a substituent(s);
Z represents (1) a hydroxyl group which may be protected by a protective group, (2) a carboxyl group which may be protected by a protective group, or (3) a sulfo group which may be protected by a protective group;
ring M represents a divalent C3-15 carbocyclic ring which may have a substituent(s), or a divalent 5- to 6-membered heterocyclic ring consisting of carbon atom, oxygen atom and/or an optionally oxidized sulfur atom which may have a substituent(s);
$Y^2$ represents a bond or a divalent C1-4 aliphatic hydrocarbon group which may have a substituent(s);
$Y^3$ has the same meaning as described in the above [1]; and
ta represents 0 or an integer of 1 to 4 and, provided that when ta represents an integer of 2 or more, a plurality of $Y^3$ may be the same or different and, when ta represents an integer of 1 or more, $Y^2$ represent a divalent C1-4 aliphatic hydrocarbon group which may have a substituent(s));
[3] The compound according to the above [2], wherein Z is (1) a hydroxyl group protected by a protective group, (2) a carboxyl group protected by a protective group, or (3) a sulfo group protected by a protective group.;
[4] The compound according to the above [2], wherein (1) the hydroxyl group which may be protected by a protective group, (2) the carboxyl group which may be protected by a protective group, or (3) the sulfo group which may be protected by a protective group are respectively (1) a hydroxyl group in a prodrug modification, (2) a carboxyl group in a prodrug modification, or (3) a sulfo group in a prodrug modification;
[5] The compound according to the above [1], wherein $A^1$ is a nitrogen-containing heterocyclic ring which may have a substituent(s);

[6] The compound according to the above [5], wherein the nitrogen-containing heterocyclic ring is imidazole, benzoimidazole, 6,7-dihydro-5H-cyclopenta[b]pyridine, 5,6,7,8-tetrahydroquinoline, 6,7,8,9-tetrahydro-5H-cyclohepta [b]pyridine, or imidazo[1,2-a]pyridine ring;
[7] The compound according to the above [1], wherein the ring $A^2$ is a divalent 3- to 8-membered monocyclic heterocyclic ring which may have a substituent(s), and also have 1 to 3 nitrogen atom(s) and may have an oxygen atom and/or an optionally oxidized sulfur atom;
[8] The compound according to the above [7], wherein the 3- to 8-membered monocyclic heterocyclic ring is a 5- or 6-membered monocyclic aromatic heterocyclic ring;
[9] The compound according to the above [8], wherein 5- or 6-membered monocyclic aromatic heterocyclic ring is

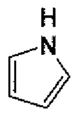 , 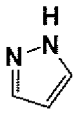 , 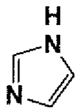 , 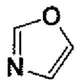 , 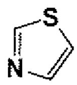 , 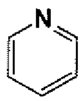 ,

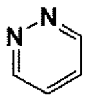 , 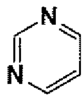 , 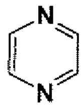 or 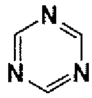

wherein a nitrogen atom of -NH- may be combined with $E^2$ or G, and also a nitrogen atom of -NH- may have a substituent(s));
[10] The compound according to the above [9], wherein the 5- or 6-membered monocyclic aromatic heterocyclic ring is:

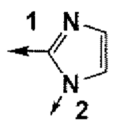 or 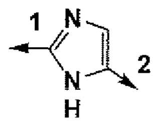

wherein arrow 1 is bonded to $E^2$, arrow 2 is bonded to G, and a nitrogen atom of -NH-represents a substituent(s);
[11] The compound according to the above [1], wherein $E^2$ is a methylene group;
[12] The compound according to the above [1], wherein $G^1$ represents a methylene group which may have a substituent(s) and p is an integer of 1 to 4;
[13] The compound according to the above [1], wherein

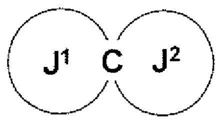 is 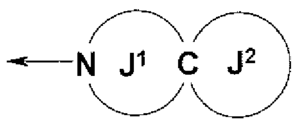

wherein arrow is bonded to G and other symbols have the same meanings as described in the above [1];
[14] The compound according to the above [13], wherein

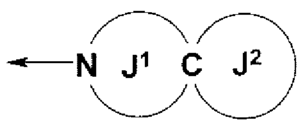

is

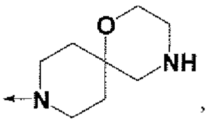, 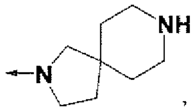, 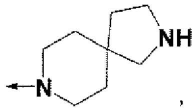, 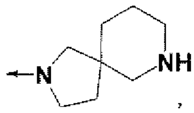, 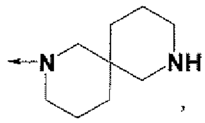,

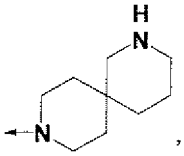, 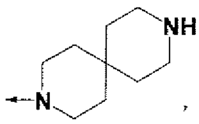, 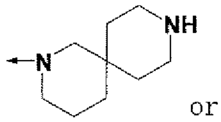 or 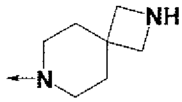

which may have a substituent(s) (wherein nitrogen atom of -NH- may have a substituent(s));

[15] The compound according to the above [1], which is represented by formula (I-1):

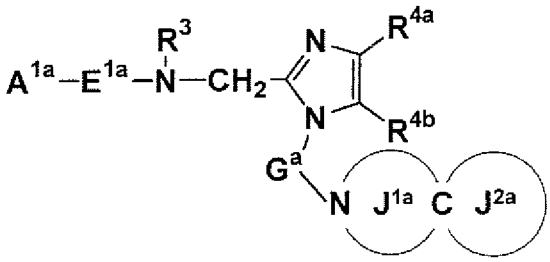

(I-1)

wherein $A^{1a}$ represent an imidazole ring which may have a substituent(s), a benzoimidazole ring which may have a substituent(s), a 6,7-dihydro-5H-cyclopenta[b]pyridine ring which may have a substituent(s), a 5,6,7,8-tetrahydroquinoline ring which may have a substituent(s), a 6,7,8,9-tetrahydro-5H-cyclohepta[b]pyridine ring which may have a substituent(s), or an imidazo[1,2-a]pyridine ring which may have a substituent(s);

$E^{1a}$ represents a methylene group which may have a substituent(s);

$R^{4a}$ and $R^{4b}$ each independently represents a hydrogen atom or a substituent(s);

$G^a$ represents:

**(a)** $-(G^1)_{pa}-$ , or

**(b)** 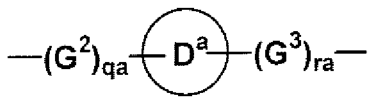

wherein ring $D^a$ represents a divalent C3-8 monocyclic carbocyclic ring which may have a substituent(s), pa represents an integer of 1 to 4, qa and ra each independently represents 0

or an integer of 1 to 4, and other symbols have the same meanings as described in the above [1], provided that when pa represents an integer of 2 or more, a plurality of $G^1$ may be the same or different, when qa represents an integer of 2 or more, a plurality of $G^2$ may be the same or different, and when ra represents an integer of 2 or more, a plurality of $G^3$ may be the same or different, and the sum of qa and ra represents 4 or less; and

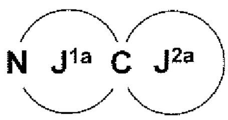

represents:

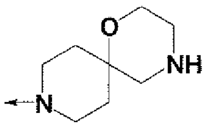, 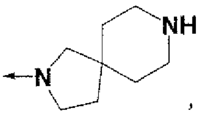, 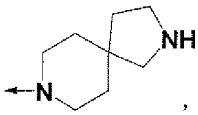, 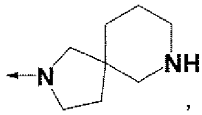, 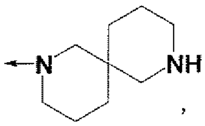,

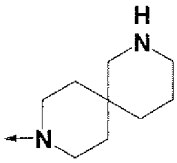, 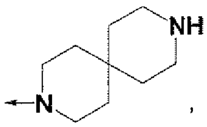, 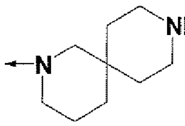 or 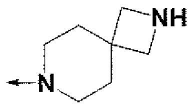

which may have a substituent(s) wherein arrow is bonded to $G^a$, a nitrogen atom of -NH-may have a substituent(s); and other symbols have the same meanings as described in the above [1];

[16] The compound according to the above [15], which is represented by formula (I-1-1):

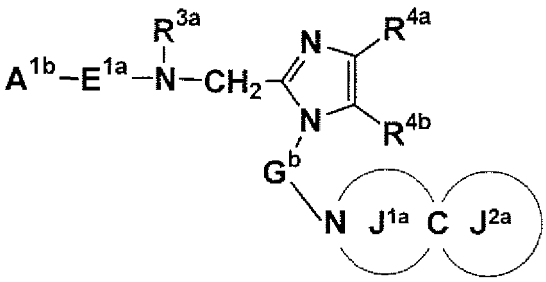

(I-1-1)

wherein $A^{1b}$ represents an imidazole ring which may have a substituent(s);

$G^b$ represents a divalent C1-4 aliphatic hydrocarbon group which may have a substituent(s);

$R^{3a}$ represent:

(a) $-Y^1-Z^a$ , or

(b) 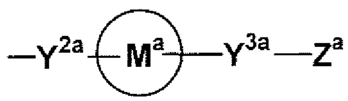

wherein $Z^a$ represents a carboxyl group which may be protected by a protective group, ring $M^a$ represents a divalent C5-10 aromatic carbocyclic ring which may have a substituent(s), a thiophene ring which may have a substituent (s), or a furan ring which may have a substituent(s), $Y^{2a}$ represents a divalent C1-4 aliphatic hydrocarbon group which may have a substituent(s), $Y^{3a}$ represents a bond, a divalent C1-4 aliphatic hydrocarbon group which may have a substituent(s), or ←O- (a divalent C1-3 alkylene group which may have a substituent(s)) → wherein arrow directing the left is bonded to ring $M^a$, and other symbols have the same meanings as described in the above [2]; and other symbols have the same meanings as described in the above [15];

[17] The compound according to the above [16], wherein the protective group is a C1-4 aliphatic hydrocarbon group, or a C1-4 aliphatic hydrocarbon group substituted with a mono- or di-substituted carbamoyl group which may have a have a substituent(s);

[18] The compound according to the above [1], which is represented by formula (I-2):

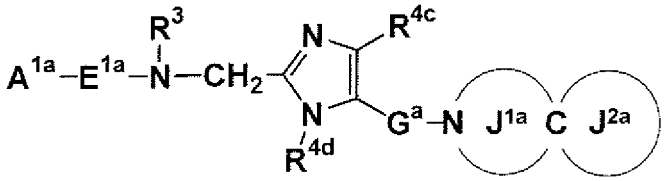

(I-2)

wherein $R^{4c}$ and $R^{4d}$ each independently represents a hydrogen atom or a substituent(s) and other symbols have the same meanings as described in the above [1] or [15];

[19] The compound according to the above [16], which is 4-{[(1-{3-[8-(1-ethylpropyl)-2,8-diazaspiro[4.5]deca-2-yl]propyl}-1H-imidazol-2-yl)methyl](1H-imidazol-2-ylmethyl) amino}butanoic acid,

ethyl [4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenoxy]acetate,

[4-({[(1-{3-[8-(1-ethylpropyl)-2,8-diazaspiro[4.5]deca-2-yl]propyl}-1H-imidazol-2-yl)methyl](1H-imidazol-2-ylmethyl) amino}methyl)phenoxy]acetic acid,

ethyl [3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenoxy]acetate,

[3-({[(1-{3-[8-(1-ethylpropyl)-2,8-diazaspiro[4.5]deca-2-yl]propyl}-1H-imidazol-2-yl)methyl](1H-imidazol-2-ylmethyl) amino}methyl)phenoxy]acetic acid,

2-(dimethylamino)-2-oxoethyl 4-{[(1-{3-[8-(1-ethylpropyl)-2,8-diazaspiro[4.5]deca-2-yl]propyl}-1H-imidazol-2-yl) methyl](1H-imidazol-2-ylmethyl)amino}butanoate, or

2-(diethylamino)-2-oxoethyl 4-{[(1-{3-[8-(1-ethylpropyl)-2,8-diazaspiro[4.5]deca-2-yl]propyl}-1H-imidazol-2-yl)methyl](1H-imidazol-2-ylmethyl)amino}butanoate;

[20] A pharmaceutical composition comprising the compound represented by formula (I) according to the above [1], a salt thereof, an N-oxide thereof or a solvate thereof, or a prodrug thereof;

[21] The pharmaceutical composition according to the above [20], which is a CXCR4 antagonist;

[22] The pharmaceutical composition according to the above [20], which is preventive and/or therapeutic agent for CXCR4-mediated diseases or cancerous diseases, or an agent for regeneration therapy;

[23] The pharmaceutical composition according to the above [22], wherein CXCR4-mediated disease is asthma, human immunodeficiency virus infection, acquired immunological deficiency syndrome, cancer, cancer metastasis, rheumatoid arthritis, arthritis, retinopathy, macular degeneration, pulmonary fibrosis, acute lung injury, ischemic diseases, systemic lupus erythematosus, or transplanted organ rejection, or the agent for regeneration therapy is an agent for a transplantation medical treatment, or an agent for a mobilization to peripheral blood of hematopoietic stem cells;

[24] The pharmaceutical composition according to the above [22], wherein the CXCR4-mediated disease is human immunodeficiency virus infection;

[25] A pharmaceutical comprising the compound represented by formula (I) according to the above [1], a salt thereof, an N-oxide thereof or a solvate thereof, or a prodrug thereof, and one or more kinds selected from reverse transcriptase inhibitor, protease inhibitor, CCR2 antagonist, CCR3 antagonist, CCR4 antagonist, CCR5 antagonist, CXCR4 antagonist, HIV integrase inhibitor, fusion inhibitor, CD4 antagonist, antibody against surface antigen of HIV, short interfering RNA of HIV, and vaccine of HIV;

[26] A pharmaceutical comprising a compound represented by formula (I) according to the above [1], a salt thereof, an N-oxide thereof or a solvate thereof, or a prodrug thereof, and one or more kinds selected from anticancer drug, antimetabolite, antibiotic, antimitotic drug, platinum complex, plant-derived antineoplastic drug, anticancerous hormone, immunopotentiator, interferon, biologics capable of performing T cell activation, and neovascularisation inhibitor;

[27] A method for antagonizing CXCR4 in a mammal, which comprises administering an effective dosage of a compound represented by formula (I) according to the above [1], a salt thereof, an N-oxide thereof or a solvate thereof, or a prodrug thereof to the mammal;

[28] A method for preventing and/or treating CXCR4-mediated diseases in a mammal, which comprises administering an effective dosage of a compound represented by formula (I) according to the above [1], a salt thereof, an N-oxide thereof or a solvate thereof, or a prodrug thereof to the mammal;

[29] Use of a compound represented by formula (I) according to the above [1], a salt thereof, an N-oxide thereof or a solvate thereof, or a prodrug thereof for the preparation of a CXCR4 antagonist;

[30] Use of a compound represented by formula (I) according to the above [1], a salt thereof, an N-oxide thereof or a solvate thereof, or a prodrug thereof for the preparation of preventive and/or therapeutic agent for CXCR4-mediated

diseases; and

[31] A compound represented by formula (I-0):

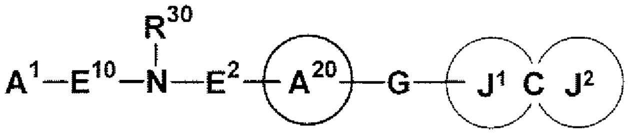

(I-0)

wherein $A^1$ represents a nitrogen-containing heterocyclic ring which may have a substituent(s) or $-NR^1R^2$;
$R^1$ and $R^2$ each independently represents a hydrogen atom or a substituent(s);
ring $A^{20}$ represents a divalent cyclic group which may have a substituent(s);
$E^{10}$ represents a bond or a divalent C1-4 aliphatic hydrocarbon group which may have a substituent(s);
$E^2$ represents a methylene group or a carbonyl group;
$R^{30}$ represents (1) a hydrogen atom, (2) a C1-4 aliphatic hydrocarbon group which may be substituted with a hydroxyl group which may be protected by a protective group, a carboxyl group which may be protected by a protective group or a sulfo group which may be protected by a protective group, and also may have a substituent(s), (3) a carbocyclic ring group which may be substituted with a hydroxyl group which may be protected by a protective group, a carboxyl group which may be protected by a protective group, a sulfo group which may be protected by a protective group, or a halogen atom, and also may have a substituent(s), or (4) a C1-4 aliphatic hydrocarbon group which may be substituted with a hydroxyl group which may be protected by a protective group, a carboxyl group which may be protected by a protective group, a sulfo group which may be protected by a protective group, or a halogen atom, and is also substituted with a carbocyclic ring group which may have a substituent(s) and may have a substituent(s);
G represents:

$-(G^1)_p-$ , or (a)

(b) 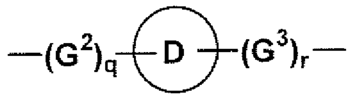

wherein $G^1$ represent a methylene group which may have a substituent(s), an ethenylene group which may have a substituent(s), an ethynylene group, a divalent nitrogen atom which may have a substituent(s), -C(O)-, -O-, -S-, -S(O)- or $-SO_2-$, $G^2$ and $G^3$ each independently represents a methylene group which may have a substituent(s), an ethenylene group which may have a substituent(s), an ethynylene group, a divalent nitrogen atom which may have a substituent (s), -C(O)-, -O-, -S-, -S(O)- or $-SO_2-$, ring D represents a divalent monocyclic cyclic group which may have a substituent (s), p represents an integer of 1 to 8, and q and r each independently represents 0 or an integer of 1 to 6, provide that a plurality of $G^1$, $G^2$ and $G^3$ may be the same or different and the sum of q and r is 6 or less;
ring $J^1$ represents a 3- to 10-membered monocyclic or bicyclic heterocyclic ring which has at least one nitrogen atom, and also may have an oxygen atom and/or an optionally oxidized sulfur atom;
ring $J^2$ represent (i) a C3-10 monocyclic or bicyclic carbocyclic ring substituted with a group having a basic group, (ii) a 3- to 10-membered monocyclic or bicyclic heterocyclic ring consisting of a carbon atom, an oxygen atom and/or an optionally oxidized sulfur atom which is substituted with a group having a basic group, or (iii) a 3- to 10-membered monocyclic or bicyclic heterocyclic ring which may be substituted with a group having a basic group, and also has at least one nitrogen atom and may have an oxygen atom and/or an optionally oxidized sulfur atom; and
ring $J^1$ and ring $J^2$ may have 1 to 8 substituent(s) on the substitutable position and, when the number of substituents is 2 or more, a plurality of substituents may be the same or different),
a salt thereof, an N-oxide thereof or a solvate thereof, or a prodrug thereof.

In the present specification, the "cyclic group" includes, for example, a monocyclic or fused cyclic group, a bridged cyclic group, or a spiro-bound cyclic group. The "monocyclic or fused cyclic group" includes, for example, a C3-15 monocyclic or fused carbocyclic ring, or a 3- to 15-membered monocyclic or fused heterocyclic ring having 1 to 4 nitrogen atom(s), 1 to 2 oxygen atom(s) and/or 1 to 2 optionally oxidized sulfur atom(s) as heteroatoms. In the "C3-15 monocyclic or fused carbocyclic ring", a C3-15 monocyclic or unsaturated fused carbocyclic ring, and a partially or completely saturated fused carbocyclic ring thereof are included. Examples of the "C3-15 monocyclic or fused unsaturated carbocyclic ring, and partially or completely saturated carbocyclic ring" include cyclopropane, cyclobutane, cyclopentane,

cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, phenanthrene, anthracene, and 1,2,3,5,6,7-hexahydro-s-indacene rings. Among these rings, examples of the "C3-15 monocyclic or fused aromatic carbocyclic ring" include benzene, azulene, naphthalene, phenanthrene, and anthracene rings.

In the "3- to 15-membered monocyclic or fused heterocyclic ring having 1 to 4 nitrogen atom(s), 1 to 2 oxygen atom(s) and/or 1 to 2 optionally oxidized sulfur atom(s) as heteroatoms", 3- to 15-membered monocyclic or fused unsaturated heterocyclic ring having 1 to 4 nitrogen atom(s), 1 to 2 oxygen atom(s) and/or 1 to 2 optionally oxidized sulfur atom(s) as heteroatoms, and a partially or completely saturated heterocyclic ring thereof are included. Examples of the "3- to 15-membered monocyclic or fused unsaturated heterocyclic ring having 1 to 4 nitrogen atom(s), 1 to 2 oxygen atom(s) and/or 1 to 2 optionally oxidized sulfur atom(s) as heteroatoms, and a partially or completely saturated heterocyclic ring thereof" include pyrrole, imidazole, triazole, tetrazole, pyrazole, pyridine, pyrazine, pyrimidine, pyridazine, azepine, diazepine, furan, pyran, oxepin, thiophene, thiopyran, thiepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolidine, purine, phthalazine, pteridine, naphthylidine, quinoxaline, quinazoline, cinnoline, benzoxazole, benzothiazole, benzoimidazole, chromene, benzoxepin, benzoxazepine, benzoxadiazepine, benzothiepine, benzothiazepine, benzothiadiazepine, benzoazepine, benzodiazepine, benzofurazane, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepin, tetrahydrooxepin, perhydrooxepin, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazane, tetrahydrofurazane, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisohenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthylidine, tetrahydronaphthylidine, perhydronaphthylidine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzoimidazole, perhydrobenzoimidazole, dihydrobenzoazepine, tetrahydrobenzoazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindane, benzodioxane, chroman, benzodithiolane, benzodithiane, 6,7-dihydro-5H-cyclopenta[b]pyrazine, 5H-cyclopenta[b]pyrazine, imidazo[2,1-b][1,3]thiazole, pyrid[2,3-b]pyrazine, pyrid[3,4-b]pyrazine, [1,3]thiazolo[4,5-b]pyrazine, thieno[2,3-b]pyrazine, 3,4-dihydro-2H-pyrazino[2,3-b][1,4]oxazine, 6,7-dihydro-5H-cyclopenta[b]pyrazine, imidazo[1,2-a]pyrazine, 6,7-dihydro-5H-cyclopenta[b]pyridine, furo[3,2-b]pyridine, pyrid[2,3-d]pyrimidine, 2,3-dihydro-1H-pyrrolo[2,3-b]pyridine, 5,6,7,8-tetrahydro-1,6-naphthylidine, 6,7,8,9-tetrahydro-5H-pyrid[2,3-d]azepine, 3,4-dihydro-2H-pyrano[3,2-c]pyridine, 2,3-dihydrofuro[3,2-c]pyridine, hexahydro-1H-pyrrolidine, octahydrocyclopenta[c]pyrrole, octahydrocyclopenta[b]pyrrole, octahydropyrrolo[3,2-b]pyrrole, octahydropyrrolo[3,4-c]pyrrole, hexahydro-2H-furo[3,2-b]pyrrole, hexahydro-2H-thieno[3,2-b]pyrrole, decahydroquinoline, decahydro-2,6-naphthylidine, octahydro-2H-quinolidine, octahydro-1H-pyrid[1,2-c]pyrimidine, octahydro-2H-1,4-benzoxazine, decahydro-1,5-naphthylidine, octahydro-1H-pyrrolo[3,4-b]pyridine, octahydro-1H-pyrrolo[3,4-c]pyridine,,7-dihydro-5H-cyclopenta[b]pyridine, 5,6,7,8-tetrahydroquinoline, 6,7,8,9-tetrahydro-5H-cyclohepta[b]pyridine, 6,7-dihydro-5H-cyclopenta[c]pyridine, 5,6,7,8-tetrahydroisoquinoline, 6,7,8,9-tetrahydro-5H-cyclohepta[c]pyridine, 1,3-benzoxazole, 1,3-benzothiazole, pyrazolo[1,5-a]pyridine, imidazo[1,5-a]pyridine, 1,5-naphthylidine, and 1,6-naphthylidine rings. Among these rings, examples of the "3- to 15-membered monocyclic or fused aromatic heterocyclic ring having 1 to 4 nitrogen atom(s), 1 to 2 oxygen atom(s) and/or 1 to 2 optionally oxidized sulfur atom(s) as heteroatoms" include pyrrole, imidazole, triazole, tetrazole, pyrazole, pyridine, pyrazine, pyrimidine, pyridazine, furan, thiophene, oxazole, isoxazole, thiazole, isothiazole, furazane, oxadiazole, thiadiazole, indole, isoindole, benzofuran, isobenzofuran, benzothiophene, isobenzothiophene, indazole, quinoline, isoquinoline, purine, phthalazine, pteridine, naphthylidine, quinoxaline, quinazoline, cinnoline, benzoxazole, benzothiazole, benzoimidazole, benzofurazane, benzothiadiazole, benzotriazole, carbazole, β-carboline, acridine, phenazine, dibenzofuran, dibenzothiophene, phenanthridine, phenanthroline, perimidine, 1,3-benzoxazole, 1,3-benzothiazole, pyrazolo[1,5-a]pyridine, imidazo[1,5-a]pyridine, 1,5-naphthylidine, and 1,6-naphthylidine rings.

In the "bridged cyclic group", a bridged polycyclic carbocyclic ring and a bridged polycyclic heterocyclic ring are included. The "bridged polycyclic carbocyclic ring" includes, for example, a C5-15 bridged polycyclic carbocyclic ring. Examples of the "C5-15 bridged polycyclic carbocyclic ring" include bicyclo[2.2.1]heptane, bicyclo[2.2.1]hept-2-ene, bicyclo[3.1.1]heptane, bicyclo[3.1.1]hept-2-ene, bicyclo[3.2.1]octane, bicyclo[2.2.2]octane, bicyclo[2.2.2]oct-2-ene, adamantane, noradamantane, bicyclo[2.1.1]hexane, bicyclo[3.3.1]nonane, bicyclo[3.2.1]octane, and bicyclo[3.3.2]decane rings.

The "bridged polycyclic heterocyclic ring" includes, for example, a bridged polycyclic heterocyclic ring having 1 to 4 nitrogen atom(s), 1 to 2 oxygen atom(s) and/or 1 to 2 optionally oxidized sulfur atom(s) as heteroatoms. In the "bridged polycyclic heterocyclic ring having 1 to 4 nitrogen atom(s), 1 to 2 oxygen atom(s) and/or 1 to 2 optionally oxidized sulfur atom(s) as the heteroatoms", a "5- to 15-membered bridged polycyclic heterocyclic ring having 1 to 4 nitrogen atom(s), 1 to 2 oxygen atom(s) and/or 1 to 2 optionally oxidized sulfur atom(s) as heteroatoms" is included. Examples of the "5-to 15-membered bridged polycyclic heterocyclic ring having 1 to 4 nitrogen atom(s), 1 to 2 oxygen atom(s) and/or 1 to 2 optionally oxidized sulfur atom(s) as heteroatoms" include azabicyclo[2.2.1]heptane, oxabicyclo[2.2.1]heptane, azabicyclo[3.1.1]heptane, azabicyclo[3.2.1]octane, oxabicyclo[3.2.1]octane, azabicyclo[2.2.2]octane, diazabicyclo[2.2.2]octane, 1-azatricyclo[3.3.1.1$^{3,7}$]decane, 3-azabicyclo[3.3.1]nonane, and 3,7-diazabicyclo[3.3.1]nonane rings.

In the "spiro-bound cyclic group", a spiro-bound polycyclic carbocyclic ring and a spiro-bound polycyclic heterocyclic ring are included. The "spiro-bound polycyclic carbocyclic ring" includes, for example, a C7-15 spiro-bound polycyclic carbocyclic ring. Examples of the "C7-15 spiro-bound polycyclic carbocyclic ring" include spiro[4.4]nonane, spiro[4.5]decane, spiro[5.5]ondecane, spiro[3.4]octane, and spiro[3.5]nonane ring.

The "spiro-bound polycyclic heterocyclic ring" includes, for example, a spiro-bound polycyclic heterocyclic ring having 1 to 4 nitrogen atom(s), 1 to 2 oxygen atom(s) and/or 1 to 2 optionally oxidized sulfur atom(s) as heteroatoms. In the "spiro-bound polycyclic heterocyclic ring having 1 to 4 nitrogen atom(s), 1 to 2 oxygen atom(s) and/or 1 to 2 optionally oxidized sulfur atom(s) as heteroatoms", a spiro-bound 7- to 15-membered polycyclic heterocyclic ring having 1 to 4 nitrogen atom(s), 1 to 2 oxygen atom(s) and/or 1 to 2 optionally oxidized sulfur atom(s) as heteroatoms is included. Examples of the "7- to 15-membered spiro-bound polycyclic heterocyclic ring having 1 to 4 nitrogen atom(s), 1 to 2 oxygen atom(s) and/or 1 to 2 optionally oxidized sulfur atom(s) as heteroatoms" include azaspiro[4.4]nonane, oxazaspiro[4.4]nonane, dioxaspiro[4.4]nonane, azaspiro[4.5]decane, thiaspiro[4.5]decane, dithiaspiro[4.5]decane, dioxaspiro[4.5]decane, oxazaspiro[4.5]decane, azaspiro[5.5]undecane, oxaspiro[5.5]undecane, dioxaspiro[5.5]undecane, 2,7-diazaspiro[3.5]nonane, 2,8-diazaspiro[4.5]decane, 2,7-diazaspiro[4.5]decane, 2,9-diazaspiro[5.5]undecane, 2,8-diazaspiro[5.5]undecane, 3,9-diazaspiro[5.5]undecane, 2,7-diazaspiro[4.4]nonane, 1,2-dihydrospiro[indole -3,4'-piperidine], 2,3-dihydro-1H-spiro[isoquinoline-4,4'-piperidine], 1',4'-dihydro-2'H-spiro[piperidine -4,3'-quinoline], 2',3'-dihydro-1'H-spiro[piperidine -4,4'-quinoline], 8-azaspiro[4.5]decane, 7-azaspiro[4.5]decane, 3-azaspiro[5.5]undecane, 2-azaspiro[5.5]undecane, 1-oxa-4,8-diazaspiro[5.5]undecane, 1-oxa-4,9-diazaspiro[5.5]undecane, 3,4-dihydrospiro[chromene-2,4'-piperidine], 2-azaspiro[4.4]nonane, 7-azaspiro[3.5]nonane, 2,3-dihydrospiro[indene-1,4'-piperidine], 3,4-dihydro-2H-spiro[naphthalene-1,4'-piperidine], 3,4-dihydro-1H-spiro[naphthalene-2,4'-piperidine], 2-azaspiro[4.5]decane, 2-azaspiro[3.5]nonane, 1',2'-dihydrospiro [cyclohexane-1,3'-indole], 2',3'-dihydro-1'H-spiro[cyclohexane-1,4'-isoquinoline], 1',4'-dihydro-2'H-spiro[cyclohexane-1,3'-quinoline], 1,6-diazaspiro[3.4]octane, 1,5-diazaspiro[3.4]octane, 1,7-diazaspiro[3.5]nonane, 1,6-diazaspiro[3.5]nonane, 1,5-diazaspiro[3.5]nonane, 1,7-diazaspiro[4.4]nonane, 1,6-diazaspiro[4.4]nonane, 1,8-diazaspiro[4.5]decane, 1,7-diazaspiro[4.5]decane, 2,6-diazaspiro[3.4]octane, 1,6-diazaspiro[4.5]decane, 2,6-diazaspiro[3.5]nonane, 1,9-diazaspiro[5.5]undecane, 1,8-diazaspiro[5.5]undecane, 6-azaspiro[3.5]nonane, 6-azaspiro[3.4]octane, 2-azaspiro[3.4]octane, 1,7-diazaspiro[5.5]undecane, 1,4,9-triazaspiro[5.5]undecane, 1,3,8-triazaspiro[4.5]decane, 1-thia-4,9-diazaspiro[5.5]undecane, and 1-thia-4,8-diazaspiro[5.5]undecane rings.

In the present specification, "bond" means to directly bind without mediating the other atom therebetween.

In the present specification, "halogen atom" includes, for example, a fluorine atom, a chlorine atom, a bromine atom, or an iodine atom.

In the present specification, the "aliphatic hydrocarbon group" includes, for example, a "straight-chain or

branched aliphatic hydrocarbon group". The "straight-chain or branched aliphatic hydrocarbon group" includes, for example, a "C1-8 aliphatic hydrocarbon group", and the "C1-8 aliphatic hydrocarbon group" includes, for example, a C1-8 alkyl group, a C2-8 alkenyl group, or a C2-8 alkynyl group.

Examples of the C1-8 alkyl group include methyl, ethyl, propyl, isopropyl, butyl, sec-butyl, tert-butyl, pentyl, hexyl, heptyl, and octyl groups, and isomer groups thereof.

Examples of the C2-8 alkenyl group include vinyl, propenyl, butenyl, pentenyl, hexenyl, heptenyl, octenyl, butadienyl, pentadienyl, hexadienyl, heptadienyl, octadienyl, hexatrienyl, heptatrienyl, and octatrienyl groups, and isomer groups thereof.

Examples of the C2-8 alkynyl group include ethynyl, propynyl, butynyl, pentynyl, hexynyl, heptynyl, octynyl, butadiynyl, pentadiynyl, hexadiynyl, heptadiynyl, octadiynyl, hexatriynyl, heptatriynyl and octatriynyl groups, and isomer groups thereof.

In the present specification, the "C1-4 aliphatic hydrocarbon group" includes, for example, a "straight-chain or branched C1-4 aliphatic hydrocarbon group". Examples of the "straight-chain or branched C1-4 aliphatic hydrocarbon group" include a C1-4 alkyl group (for example, methyl, ethyl, propyl, isopropyl, butyl, sec-butyl, tert-butyl, and isobutyl group), a C2-4 alkenyl group (for example, vinyl, propenyl, butenyl group, and isomer group thereof), and a C2-4 alkynyl group (for example, ethynyl, propynyl, butynyl group, and isomer group thereof).

In the present specification,

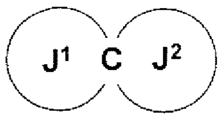

represents that ring $J^1$ and ring $J^2$ share only one carbon atom to form a spiro-bound cyclic group, or represents:

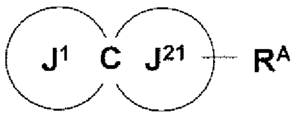

(wherein ring $J^{21}$ represents (i) a C3-10 monocyclic or bicyclic carbocyclic ring, or (ii) a 3-to 10-membered monocyclic or bicyclic heterocyclic ring consisting of a carbon atom, an oxygen atom and/or an optionally oxidized sulfur atom, $R^A$ represent a group having a basic group, ring $J^{21}$ may have 1 to 8 substituent(s) on the substitutable position and, when the number of substituents is 2 or more, a plurality of substituents may be the same or different, wherein the "substituent" has the same meaning as that in ring $J^2$, and ring $J^1$ has the same meaning as described above), or

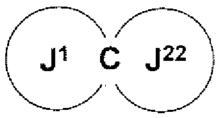

(wherein ring $J^{32}$ represents a 3- to 10-membered monocyclic or bicyclic heterocyclic ring which may be substituted with a group having a basic group, and also has at least one nitrogen atom and may have an oxygen atom and/or an optionally oxidized sulfur atom, ring $J^{22}$ may have 1 to 8 substituent(s) on the substitutable positions and, when the number of substituents is 2 or more, a plurality of substituents may be the same or different, wherein the "substituent" has the meaning as that in ring $J^2$, and ring $J^1$ has the same meaning as described above).

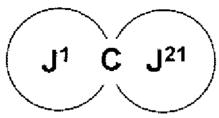

includes, for example,

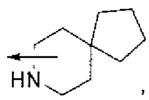, 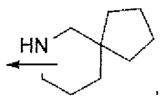, 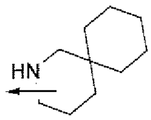, 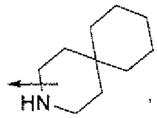, 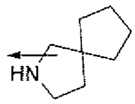, 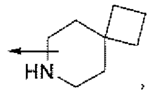,

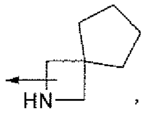, 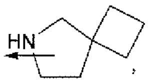, 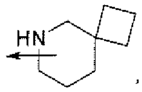, 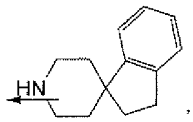, 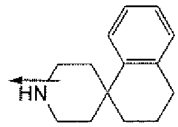,

or

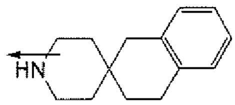

(wherein an arrow is bonded to G, G may be bonded to a nitrogen atom of -NH-, and the nitrogen atom of -NH- may have a substituent(s), wherein the "substituent" has the same meaning as that in ring $J^{21}$) and

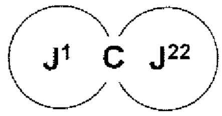

includes, for example,

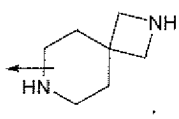, 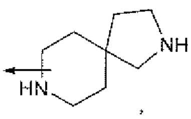, 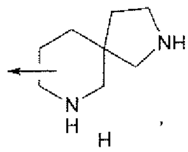, 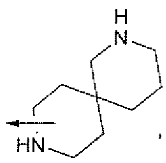, 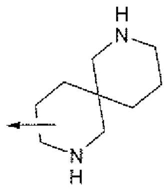,

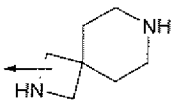, 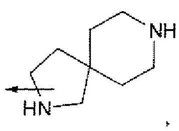, 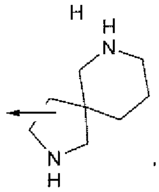, 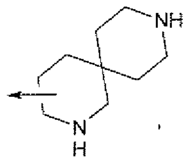, 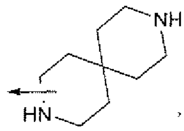,

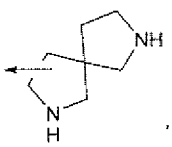, 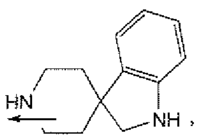, 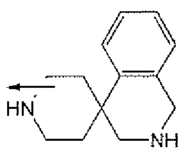, 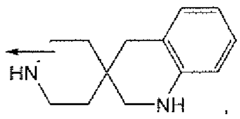,

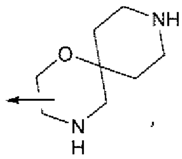 , 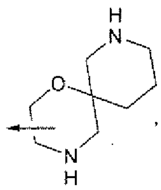 , 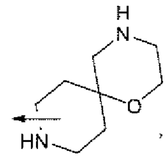 , 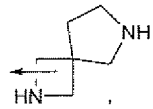 , 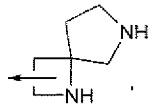 ,

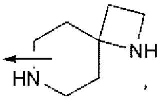 , 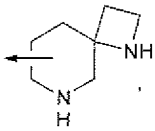 , 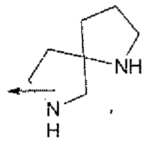 , 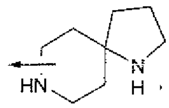 , 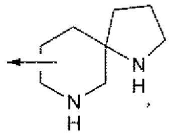 ,

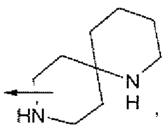 , 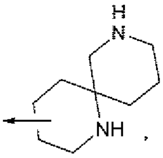 , 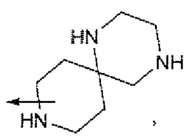 , 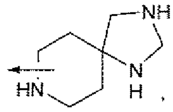 , 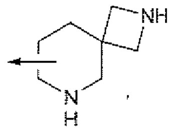 ,

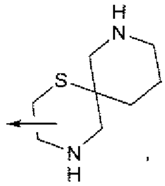 , or 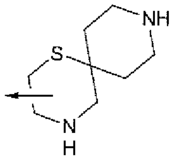

(wherein an arrow is bonded to G, G may be bonded to a nitrogen atom of -NH-, and the nitrogen atom of -NH- may have a substituent(s), wherein the "substituent" has the same meaning as that in ring $J^{22}$).

In the present specification, in the "3- to 10-membered monocyclic or bicyclic heterocyclic ring which has at least one nitrogen atom, and also may have an oxygen atom and/or an optionally oxidized sulfur atom" represented by ring $J^1$, a 3- to 10-membered monocyclic or bicyclic unsaturated heterocyclic ring which has at least one nitrogen atom, and also may have an oxygen atom and/or an optionally oxidized sulfur atom, and a partially or completely saturated 3- to 10-membered monocyclic or bicyclic heterocyclic ring which has at least one nitrogen atom, and also may have an oxygen atom and/or an optionally oxidized sulfur atom are included. Examples thereof include aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazane, tetrahydrofurazane, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, indoline, isoindoline, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthylidine, tetrahydronaphthylidine, perhydronaphthylidine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzoimidazole, perhydrobenzoimidazole, benzodioxepane, pyrrole, imidazole, triazole, tetrazole, pyrazole, pyridine, pyrazine, pyrimidine, pyridazine, azepine, diazepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine, indole, isoindole, indolizine, indazole, quinoline, isoquinoline, quinolidine, purine, phthalazine, pteridine, naphthylidine, quinoxaline, quinazoline, cinnoline, benzoxazole, benzothiazole, benzoimidazole, benzoxazepine, benzothiepine, benzothiazepine, benzodiazepine, benzofurazane, benzothiadiazole, and benzotriazole ring.

In the present specification, in the "C3-10 monocyclic or bicyclic carbocyclic ring" in the "C3-10 monocyclic or

bicyclic carbocyclic ring substituted with a group having a basic group" represented by ring $J^2$, a C3-10 monocyclic or bicyclic unsaturated carbocyclic ring, and a C3-10 partially or completely saturated monocyclic or bicyclic carbocyclic ring are included. Examples thereof include benzene, azulene, naphthalene, cyclopropane, cyclobutane, cyclopentane, cyclohexane, cycloheptane, cyclooctane, cyclononane, cyclodecane, cyclohexene, cycloheptene, cyclooctene, cyclopentadiene, cyclohexadiene, cycloheptadiene, cyclooctadiene, pentalene, perhydropentalene, perhydroazulene, indene, perhydroindene, indane, dihydronaphthalene, tetrahydronaphthalene, perhydronaphthalene, heptalene, and perhydroheptalene rings.

In the present specification, in the "3- to 10-membered monocyclic or bicyclic heterocyclic ring consisting of a carbon atom, an oxygen atom and/or an optionally oxidized sulfur atom" in the "3- to 10-membered monocyclic or bicyclic heterocyclic ring consisting of a carbon atom, an oxygen atom and/or an optionally oxidized sulfur atom substituted with a group having a basic group" represented by ring $J^2$, a 3- to 10-membered monocyclic or bicyclic unsaturated heterocyclic ring consisting of a carbon atom, an oxygen atom and/or an optionally oxidized sulfur atom, and a partially or completely saturated 3- to 10-membered monocyclic or bicyclic heterocyclic ring consisting of a carbon atom, an oxygen atom and/or an optionally oxidized sulfur atom are included. Examples of the "3- to 10-membered monocyclic or bicyclic unsaturated heterocyclic ring consisting of a carbon atom, an oxygen atom and/or an optionally oxidized sulfur atom, and partially or completely saturated 3- to 10-membered monocyclic or bicyclic heterocyclic ring consisting of a carbon atom, an oxygen atom and/or an optionally oxidized sulfur atom" include furan, pyran, oxepin, thiophene, thiopyran, thiepine, benzofuran, isobenzofuran, benzothiophene, isobenzothiophene, dithianaphthalene, chromene, benzoxepin, benzothiepine, oxirane, oxetane, dihydrofuran, tetrahydrofuran, dihydropyran, tetrahydropyran, dihydrooxepin, tetrahydrooxepin, perhydrooxepin, thiirane, thietane, dihydrothiophene, tetrahydrothiophene, dihydrothiopyran, tetrahydrothiopyran, dihydrothiepine, tetrahydrothiepine, perhydrothiepine, oxathiane, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, benzoxathiane, benzodioxepane, dioxolane, dioxane, dithiolane, dithiane, dioxaindane, benzodioxane, chroman, benzodithiolane, and benzodithiane rings.

In the present specification, "3- to 10-membered monocyclic or bicyclic heterocyclic ring which has at least one nitrogen atom, and also may have an oxygen atom and/or an optionally oxidized sulfur atom" in the "3- to 10-membered monocyclic or bicyclic heterocyclic ring which may be substituted with a group having a basic group, and also has at least one nitrogen atom and may have an oxygen atom and/or an optionally oxidized sulfur atom" represented by ring $J^2$ has the same meaning as that of "3- to 10-membered monocyclic or bicyclic heterocyclic ring which has at least one nitrogen atom, and also may have an oxygen atom and/or an optionally oxidized sulfur atom" represented by ring $J^1$.

The "C3-10 monocyclic or bicyclic carbocyclic ring" and "3- to 10-membered monocyclic or bicyclic heterocyclic ring consisting of a carbon atom, an oxygen atom and/or an optionally oxidized sulfur atom" represented by ring $J^{21}$ have the same meanings as in the "C3-10 monocyclic or bicyclic carbocyclic ring" in the "C3-10 monocyclic or bicyclic carbocyclic ring substituted with a group having a basic group", and the "3- to 10-membered monocyclic or bicyclic heterocyclic ring consisting of a carbon atom, an oxygen atom and/or an optionally oxidized sulfur atom" in the "3- to 10-membered monocyclic or bicyclic heterocyclic ring consisting of a carbon atom, an oxygen atom and/or an optionally oxidized sulfur atom substituted with a group having a basic group", each being represented by ring $J^2$. The "3- to 10-membered monocyclic or bicyclic heterocyclic ring which has at least one nitrogen atom, and also may have an oxygen atom and/or an optionally oxidized sulfur atom" in the "3- to 10-membered monocyclic or bicyclic heterocyclic ring which may be substituted with a group having a basic group, and also has at least one nitrogen atom and may have an oxygen atom and/or an optionally oxidized sulfur atom" represented by ring $J^{22}$ has the same meaning as that of the "3- to 10-membered monocyclic or bicyclic heterocyclic ring which has at least one nitrogen atom, and also may have an oxygen atom and/or an optionally oxidized sulfur atom" represented by ring $J^1$.

In the present specification, the "substituent" of "may have 1 to 8 substituent(s) on the substitutable position" represented by ring $J^1$, ring $J^2$, ring $J^{21}$, and ring $J^{22}$ is not particularly limited. Examples thereof include those described as for (1) aliphatic hydrocarbon group which may have a substituent(s) (2) cyclic group which may have a substituent (s), (3) aliphatic hydrocarbon group substituted with a cyclic group which may have a substituent(s), and (4) L described hereinafter. In the present specification, the "aliphatic hydrocarbon group" and "cyclic group" in the "aliphatic hydrocarbon group which may have a substituent(s)", "cyclic group which may have a substituent(s)" and "aliphatic hydrocarbon group substituted with a cyclic group which may have a substituent(s)" have the same meaning as described above. The "substituent" in (1) to (3) includes, for example, substituents exemplified hereinafter as for L and these optional substituents may be substituted on the substitutable position in the number of 1 to 5.

The "group having a basic group" in $R^A$, the "group having a basic group" in "(i) C3-10 monocyclic or bicyclic carbocyclic ring substituted with a group having a basic group, (ii) 3- to 10-membered monocyclic or bicyclic heterocyclic ring consisting of a carbon atom, an oxygen atom and/or an optionally oxidized sulfur atom substituted with a group having a basic group, or (iii) 3- to 10-membered monocyclic or bicyclic heterocyclic ring which may be substituted with group having a basic group, and also has at least one nitrogen atom and may have an oxygen atom and/or an optionally oxidized sulfur atom" in ring $J^2$, and the "group having a basic group" of the "3- to 10-membered monocyclic or bicyclic

heterocyclic ring which may be substituted with a group having a basic group, and also has at least one nitrogen atom and may have an oxygen atom and/or an optionally oxidized sulfur atom" in ring $J^{22}$ are not particularly limited as long as they have a basic group. Examples thereof include (1) basic group, (2) aliphatic hydrocarbon group which is substituted with a basic group, and also may have a substituent(s), and (3) cyclic group which is substituted with a basic group, and also may have a substituent(s). The "aliphatic hydrocarbon group" in the "aliphatic hydrocarbon group which is substituted with a basic group, and also may have a substituent(s)" has the same meaning as described above. The "cyclic group" in the "cyclic group which is substituted with a basic group, and also may have a substituent(s)" has the same meaning as described above. The "substituent" in the "aliphatic hydrocarbon group which is substituted with a basic group, and also may have a substituent(s)" and "cyclic group which is substituted with a basic group, and also may have a substituent (s)" is not particularly limited as long as it is a substituent. Examples of the substituent include the following substituents defined as L.

Examples of L include:

(1) aliphatic hydrocarbon group,
(2) C1-8 alkylidene group (for example, methylidene, ethylidene, propylidene, butylidene, pentylidene, hexylidene, heptylidene, octylidene group, isomer thereof, etc.),
(3) cyclic group,
(4) aliphatic hydrocarbon group substituted with a cyclic group (for example, cyclopropylmethyl, cyclopentylmethyl, cyclohexylmethyl, phenylmethyl, naphthylmethyl, pyridinylmethyl, cyclopropylethyl, cyclopentylethyl, cyclohexylethyl, phenylethyl, naphthylethyl, pyridinylethyl, cyclopropylpropyl, cyclopentylpropyl, cyclohexylpropyl, phenylmethyl, phenylpropyl, naphthylpropyl, pyridinylpropyl, etc.),
(5) hydroxyl group,
(6) -O-aliphatic hydrocarbon group (for example, methoxy, ethoxy, propoxy, isopropoxy, butoxy, isobutoxy, tert-butoxy, pentyloxy, hexyloxy, heptyloxy, octyloxy, propenyloxy, butenyloxy, pentenyloxy, hexenyloxy, propynyloxy, butynyloxy, pentynyloxy, hexynyloxy, etc.),
(7) -O-cyclic group (for example, cyclopropyloxy, cyclopentyloxy, cyclohexyloxy, phenoxy, naphthyloxy, pyridinyloxy, etc.),
(8) -O-(aliphatic hydrocarbon group)-cyclic group (for example, cyclopentylmethoxy, cyclohexylmethoxy, benzyloxy, etc.),
(9) mercapto group,
(10) -S-aliphatic hydrocarbon group (for example, methylthio, ethylthio, propylthio, isopropylthio, butylthio, isobutylthio, tert-butylthio, pentylthio, hexylthio, heptylthio, octylthio, propenylthio, butenylthio, pentenylthio, hexenylthio, propynylthio, butynylthio, pentynylthio, hexynylthio, etc.),
(11) -S-cyclic group (for example, cyclopropylthio, cyclopentylthio, cyclohexylthio, phenylthio, naphthylthio, pyridinylthio, etc.),
(12) -S-(aliphatic hydrocarbon group)-cyclic group (for example, cyclopentylmethylthio, cyclohexylmethylthio, benzylthio, etc.),
(13) -S(O)-aliphatic hydrocarbon group (for example, methylsulfinyl, ethylsulfinyl, propylsulfinyl, isopropylsulfinyl, butylsulfinyl, isobutylsulfinyl, tert-butylsulfinyl, pentylsulfinyl, hexylsulfinyl, heptylsulfinyl, octylsulfinyl, propenylsulfinyl, butenylsulfinyl, pentenylsulfinyl, hexenylsulfinyl, propynylsulfinyl, butynylsulfinyl, pentynylsulfinyl, hexynylsulfinyl, etc.),
(14) -S(O)-cyclic group (for example, cyclopropylsulfinyl, cyclopentylsulfinyl, cyclohexylsulfinyl, phenylsulfinyl, naphthylsulfinyl, pyridinylsulfinyl, etc.),
(15) -S(O)-(aliphatic hydrocarbon group)-cyclic group (for example, cyclopentylmethylsulfinyl, cyclohexylmethylsulfinyl, benzylsulfinyl, etc.),
(16) -$SO_2$-aliphatic hydrocarbon group (for example, methylsulfonyl, ethylsulfonyl, propylsulfonyl, isopropylsulfonyl, butylsulfonyl, isobutylsulfonyl, tert-butylsulfonyl, pentylsulfonyl, hexylsulfonyl, heptylsulfonyl, octylsulfonyl, propenylsulfonyl, butenylsulfonyl, pentenylsulfonyl, hexenylsulfonyl, propynylsulfonyl, butynylsulfonyl, pentynylsulfonyl, hexynylsulfonyl, etc.),
(17) -$SO_2$-cyclic group (for example, cyclopropylsulfonyl, cyclopentylsulfonyl, cyclohexylsulfonyl, phenylsulfonyl, naphthylsulfonyl, pyridinylsulfonyl, etc.),
(18) -$SO_2$-(aliphatic hydrocarbon group)-cyclic group (for example, cyclopentylmethylsulfonyl, cyclohexylmethylsulfonyl, benzylsulfonyl, etc.),
(19) -O-CO-aliphatic hydrocarbon group (for example, acetyloxy, propionyloxy, propanoyloxy, isopropanoyloxy, butanoyloxy, isobutanoyloxy, tert-butanoyloxy, pentanoyloxy, hexanoyloxy, heptanoyloxy, octanoyloxy, propenoyloxy, butenoyloxy, pentenoyloxy, hexenoyloxy, propynoyloxy, butynoyloxy, pentynoyloxy, hexynoyloxy, etc.),
(20) -O-CO-cyclic group (for example, cyclopropylcarbonyloxy, cyclopentylcarbonyloxy, cyclohexylcarbonyloxy, benzoyloxy, naphthylcarbonyloxy, pyridinylcarbonyloxy, pyrrolidinylcarbonyl, morpholinylcarbonyl, etc.),

(21) -O-CO-(aliphatic hydrocarbon group)-cyclic group (for example, cyclopentylmethanoyloxy, cyclohexylmethanoyloxy, phenylmethanoyloxy, etc.),
(22) -CO-aliphatic hydrocarbon group (for example, acetyl, propionyl, propanoyl, isopropanoyl, butanoyl, isobutanoyl, tert-butanoyl, pentanoyl, hexanoyl, heptanoyl, octanoyl, propenoyl, butenoyl, pentenoyl, hexenoyl, propynoyl, butynoyl, pentynoyl, hexynoyl, etc.),
(23) -CO-cyclic group (for example, cyclopropylcarbonyl, cyclopentylcarbonyl, cyclohexylcarbonyl, benzoylcarbonyl, naphthylcarbonyl, pyridinylcarbonyl, etc.),
(24) -CO-(aliphatic hydrocarbon group)-cyclic group (for example, cyclopentylmethanoyl, cyclohexylmethanoyl, phenylmethanoyl, etc.),
(25) oxo group,
(26) thioxo group,
(27) sulfino group,
(28) sulfo group,
(29) amino group,
(30) mono- or di-substituted amino group (the "substituent"in the "mono- or di-substituted amino group" includes , for example, (1) aliphatic hydrocarbon group, (2) cyclic group, or (3) aliphatic hydrocarbon group substituted with a cyclic group, and examples of the "mono- or di-substituted amino group" include methylamino, ethylamino, propylamino, isopropylamino, butylamino, isobutylamino, tert-butylamino, pentylamino, hexylamino, heptylamino, octylamino, dimethylamino, diethylamino, dipropylamino, dibutylamino, dipentylamino, dihexylamino, diheptylamino, dioctylamino, N-methyl-N-ethylamino, cyclopropylamino, cyclopentylamino, cyclohexylamino, phenylamino, diphenylamino, dibenzylamino, N-phenyl-N-methylamino, N-phenyl-N-ethylamino, N-benzyl-N-methylamino, N-benzyl-N-ethylamino, N-cyclohexyl-N-propylamino, etc.),
(31) sulfamoyl group,
(32) mono- or di-substitutedsulfamoyl group (the "substituent" in the "mono- or di-substitutedsulfamoyl group" includes, for example, (1) aliphatic hydrocarbon group, (2) cyclic group, or (3) aliphatic hydrocarbon group substituted with a cyclic group, and examples of the "mono- or di-substitutedsulfamoyl group" include N-methylsulfamoyl, N-ethylsulfamoyl, N-propylsulfamoyl, N-isopropylsulfamoyl, N-butylsulfamoyl, N-isobutylsulfamoyl, N-(tert-butyl)sulfamoyl, N-pentylsulfamoyl, N-hexylsulfamoyl, N-heptylsulfamoyl, N-octylsulfamoyl, N,N-dimethylsulfamoyl, N,N-diethylsulfamoyl, N,N-dipropylsulfamoyl, N,N-dibutylsulfamoyl, N,N-dipentylsulfamoyl, N,N-dihexylsulfamoyl, N-methyl-N-ethylsulfamoyl, N-cyclopropylsulfamoyl, N-cyclopentylsulfamoyl, N-cyclohexylsulfamoyl, N-phenylsulfamoyl, N,N-diphenylsulfamoyl, N,N-dibenzylsulfamoyl, N-phenyl-N-methylsulfamoyl, N-phenyl-N-ethylsulfamoyl, N-benzyl-N-methylsulfamoyl, N-benzyl-N-ethylsulfamoyl, N-cyclohexyl-N-propylamino, etc.),
(33) carboxyl group,
(34) -COO- aliphatic hydrocarbon group (for example, methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, hexyloxycarbonyl, heptyloxycarbonyl, octyloxycarbonyl, propenyloxycarbonyl, butenyloxycarbonyl, pentenyloxycarbonyl, hexenyloxycarbonyl, propynyloxycarbonyl, butynyloxycarbonyl, pentynyloxycarbonyl, hexynyloxycarbonyl, etc.),
(35) -COO-cyclic group (for example, cyclopropyloxycarbonyl, cyclopentyloxycarbonyl, cyclohexyloxycarbonyl, phenoxycarbonyl, naphthyloxycarbonyl, pyridinyloxycarbonyl, etc.),
(36) -COO-(aliphatic hydrocarbon group)-cyclic group (for example, cyclopentylmethoxycarbonyl, cyclohexylmethoxycarbonyl, benzyloxycarbonyl, etc.),
(37) carbamoyl group,
(38) mono- or di-substituted carbamoyl group (the "substituent" in the "mono- or di-substituted carbamoyl group" includes, for example, (1) aliphatic hydrocarbon group, (2) cyclic group, or (3) aliphatic hydrocarbon group substituted with a cyclic group, and examples of the "mono- or di-substituted carbamoyl group" include N-methylcarbamoyl, N-ethylcarbamoyl, N-propylcarbamoyl, N-isopropylcarbamoyl, N-butylcarbamoyl, N-isobutylcarbamoyl, N-(tert-butyl) carbamoyl, N-pentylcarbamoyl, N-hexylcarbamoyl, N-heptylcarbamoyl, N-octylcarbamoyl, N-cyclopropylcarbamoyl, N-cyclopentylcarbamoyl, N-cyclohexylcarbamoyl, N-phenylcarbamoyl, N,N-dimethylcarbamoyl, N,N-diethylcarbamoyl, N,N-dipropylcarbamoyl, N,N-dibutylcarbamoyl, N,N-dipentylcarbamoyl, N,N-dihexylcarbamoyl, N-methyl-N-ethylcarbamoyl, N,N-diphenylcarbamoyl, N,N-dibenzylcarbamoyl, N-phenyl-N-methylcarbamoyl, N-phenyl-N-ethylcarbamoyl, N-benzyl-N-methylcarbamoyl, N-benzyl-N-ethylcarbamoyl, etc.),
(39) -NH-CO-aliphatic hydrocarbon group (for example, acetylamino, propionylamino, propanoylamino, isopropanoylamino, butanoylamino, isobutanoylamino, tert-butanoylamino, pentanoylamino, hexanoylamino, heptanoylamino, octanoylamino, propenoylamino, butenoylamino, pentenoylamino, hexenoylamino, propynoylamino, butynoylamino, pentynoylamino, hexynoylamino, etc.),
(40) -NH-CO-cyclic group (for example, cyclopropylcarbonylamino, cyclopentylcarbonylamino, cyclohexylcarbonylamino, benzoylcarbonylamino, naphthylcarbonylamino, pyridinylcarbonylamino, etc.),
(41) -NH-CO-(aliphatic hydrocarbon group)-cyclic group (for example, cyclopentylmethanoylamino, cyclohexylmethanoylamino, phenylmethanoylamino, etc.),
(42) -NH-$SO_2$-aliphatic hydrocarbon group (for example, methylsulfonylamino, ethylsulfonylamino, propylsulfonylamino, isopropylsulfonylamino, butylsulfonylamino, isobutylsulfonylamino, tert-butylsulfonylamino, pentylsulfonylamino, hexylsulfonylamino, heptylsulfonylamino, octylsulfonylamino, propenylsulfonylamino, butenylsulfonylamino, pentenylsulfonylamino, hexenylsulfonylamino, propynylsulfonylamino, butynylsulfonylamino, pentynylsulfonylamino, hexynylsulfonyl, etc.),
(43) -NH-$SO_2$-cyclic group (for example, cyclopropylsulfonylamino, cyclopentylsulfonylamino, cyclohexylsulfonylamino, phenylsulfonylamino, naphthylsulfonylamino, pyridinylsulfonyl, etc.),
(44) -NH-$SO_2$-(aliphatic hydrocarbon group)-cyclic group (for example, cyclopentylmethylsulfonylamino, cyclohexylmethylsulfonylamino, benzylsulfonyl, etc.),
(45) cyano group,
(46) hydazino group,
(47) nitro group,
(48) nitroso group,
(49) imino group,
(50) mono-substituted imino group (the "substituent" of the "mono-substituted imino group" includes , for example, (1) aliphatic hydrocarbon group, (2) cyclic group, (3) aliphatic hydrocarbon group substituted with a cyclic group, (4)hydroxyl group, (5)-O-aliphatic hydrocarbon group, (6) -O-cyclic group, or (7)-O-(aliphatic hydrocarbon group)-cyclic group, and examples of the "mono-substituted imino group" include methylimino, ethylimino, propylimino, isopropylimino, butylimino, isobutylimino, (tert-butyl)imino, pentylimino, hexylimino, heptylimino, octylimino, cyclopropylimino, cyclopentylimino, cyclohexylimino, phenylimino, benzylimino, hydroxyimino, ethoxyimino, propoxyimino, isopropoxyimino, butoxyimino, cyclopentyloxyimino, cyclohexyloxyimino, phenoxyimino, benzyloxyimino, etc.),
(51) halogen atom (for example, fluorine atom, chlorine atom, bromine atom, iodine atom, etc.),
(52) methyl group substituted with 1 to 3 halogen atom(s) (for example, fluoromethyl, difluoromethyl, trifluoromethyl, trichloromethyl, etc.),
(53) methoxy group substituted with 1 to 3 halogen atom(s) (for example, fluoromethoxy, difluoromethoxy, trifluoromethoxy, trichloromethoxy, etc.),
(54) formyloxy group, and
(55) formyl group. These optional substituents may be substituted on the optional substitutable position in optional substitutable number. The number of substituents is preferably from 1 to 8, and more preferably from 1 to 5. The "aliphatic hydrocarbon group" and "cyclic group" in L have the same meanings as described above, and the "-(aliphatic hydrocarbon group)-" includes, for example, a divalent group in which one optional hydrogen atom is further removed from the "aliphatic hydrocarbon group". Optional two hydrogen atoms may be combined with the same carbon atom or different carbon atoms, and are preferably combined with different carbon atoms.

The "basic group" in the "group having a basic group" is not particularly limited as long as it has a basic nitrogen atom and examples thereof include (a) amino group, (b) amidino group, (c) guanidino group, (d) hydazino group, (e) mono- or di-substituted amino group, (f) mono-, di- or tri-substituted amidino group, (g) mono-, di-, tri- or tetra-substituted guanidino group, (h) mono-, di- or tri-substituted hydazino group, and (i) a nitrogen-containing heterocyclic ring which may have a substituent(s).

In the present specification, the "substituent" in the "mono- or di-substituted amino group" includes, for example, (1) aliphatic hydrocarbon group (which has the same meaning as described above), (2) cyclic group (which has the same meaning as described above), or (3) aliphatic hydrocarbon group substituted with a cyclic group (aliphatic hydrocarbon and cyclic group have the same meaning as described above). Examples of the "mono- or di-substituted amino group" include methylamino, ethylamino, propylamino, isopropylamino, butylamino, isobutylamino, tert-butylamino, pentylamino, hexylamino, heptylamino, octylamino, dimethylamino, diethylamino, dipropylamino, dibutylamino, dipentylamino, dihexylamino, diheptylamino, dioctylamino, N-methyl-N-ethylamino, cyclopropylamino, cyclopentylamino, cyclohexylamino, phenylamino, diphenylamino, dibenzylamino, N-phenyl-N-methylamino, N-phenyl-N-ethylamino, N-benzyl-N-methylamino, N-benzyl-N-ethylamino, N-benzyl-N-cyclohexylamino, N-cyclohexyl-N-propylamino, N-cyclohexyl-N-(3-hydroxypropyl)amino, N-(4-hydroxycyclohexyl)-N-propylamino, N-(4-hydroxycyclohexyl)-N-(3-hydroxypropyl)amino, N-(4-hydroxycyclohexyl)methyl-N-propylamino, N-cyclohexyl-N-acetylamino, N-(3-methoxypropyl)-N-propylamino, N-(2-carboxyethyl)-N-propylamino, N-(2-ethylpropyl)-N-propylamino, N-cyclohexyl-N-(methylsulfonyl) amino, N-(tetrahydropyran-4-yl)-N-propylamino, and N-(indane-2-yl)-N-propylamino.

The "substituent" in the "mono-, di- or tri-substituted amidino group" includes, for example, (1) aliphatic hydrocarbon group (which has the same meaning as described above), (2) cyclic group (which has the same meaning as described above), or (3) aliphatic hydrocarbon group substituted with a cyclic group (aliphatic hydrocarbon and cyclic group have the same meaning as described above). Examples of the "mono-, di- or tri-substituted amidino group" include methylamidino, ethylamidino, propylamidino, isopropylamidino, butylamidino, isobutylamidino, tert-butylamidino, pentylamidino, hexylamidino, heptylamidino, octylamidino, N,N-dimethylamidino, N,N'-dimethylamidino, N,N,N'-trimethylamidino, N,N-diethylamidino, N,N'-diethylamidino, N,N,N'-triethylamidino, N,N-dipropylamidino, N,N'-dipropylamidino, N,N,N'-tripropylamidino, N,N-dibutylamidino, N,N'-dibutylamidino, N,N,N'-tributylamidino, N,N-dipentylamidino, N,N'-dipentylamidino, N,N,N'-tripentylamidino, N,N-dihexylamidino, N,N'-dihexylamidino, N,N,N'-trihexylamidino, N,N-diheptylamidino, N,N'-diheptylamidino, N,N,N'-triheptylamidino, N,N-dioctylamidino, N,N'-dioctylamidino, N,N,N'-trioctylamidino, N-methyl-N-ethylamidino, N-methyl-N'-ethylamidino, cyclopropylamidino, cylopentylamidino, cyclohexylamidino, phenylamidino, N,N-diphenylamidino, N,N'-diphenylamidino, N,N,N'-triphenylamidino, N,N-dibenzylamidino, N,N'-dibenzylamidino, N,N,N'-tribenzylamidino, N-phenyl-N'-methylamidino, N-phenyl-N'-ethylamidino, N-benzyl-N-methylamidino, and N-benzyl-N-ethylamidino.

The "substituent" in the "mono-, di-, tri- or tetra-substituted guanidino group" includes, for example, (1) aliphatic hydrocarbon group (which has the same meaning as described above), (2) cyclic group (which has the same meaning as described above), or (3) aliphatic hydrocarbon group substituted with a cyclic group (aliphatic hydrocarbon and cyclic group have the same meaning as described above). Examples of the "mono-, di-, tri- or tetra-substituted guanidino group" include methylguanidino, ethylguanidino, propylguanidino, isopropylguanidino, butylguanidino, isobutylguanidino, tert-butylguanidino, pentylguanidino, hexylguanidino, heptylguanidino, octylguanidino, N,N-dimethylguanidino, N,N'-dimethylguanidino, N,N,N'-trimethylguanidino, N,N,N',N"-tetramethylguanidino, N,N-diethylguanidino, N,N'-diethylguanidino, N,N,N'-triethylguanidino, N,N,N',N"-tetraethylguanidino, N,N-dipropylguanidino, N,N'-dipropylguanidino, N,N,N'-tripropylguanidino, N,N,N',N"-tetrapropylguanidino, N,N-dibutylguanidino, N,N'-dibutylguanidino, N,N,N'-tributylguanidino, N,N,N',N"-tetrabutylguanidino, N,N-dipentylguanidino, N,N'-dipentylguanidino, N,N,N'-tripentylguanidino, N,N,N',N"-tetrapentylguanidino, N,N-dihexylguanidino, N,N'-dihexylguanidino, N,N,N'-trihexylguanidino, N,N,N',N"-tetrahexylguanidino, N,N-diheptylguanidino, N,N'-diheptylguanidino, N,N,N'-triheptylguanidino, N,N,N',N"-tetraheptylguanidino, N,N-dioctylguanidino, N,N'-dioctylguanidino, N,N,N'-trioctylguanidino, N,N,N',N"-tetraoctylguanidino, N-methyl-N-ethylguanidino, N-methyl-N'-ethylguanidino, cyclopropylguanidino, cyclopentylguanidino, cyclohexylguanidino, phenylguanidino, N,N-diphenylguanidino, N,N'-diphenylguanidino, N,N,N'-triphenylguanidino, N,N,N',N"-tetraphenylguanidino, N,N-dibenzylguanidino, N,N'-dibenzylguanidino, N,N,N'-tribenzylguanidino, N,N,N',N"-tetrabenzylguanidino, N-phenyl-N'-methylguanidino, N-phenyl-N'-ethylguanidino, N-benzyl-N-methylguanidino, and N-benzyl-N-ethylguanidino.

The "substituent" in the "mono-, di- or tri-substituted hydazino group" includes, for example, (1) aliphatic hydrocarbon group (which has the same meaning as described above), (2) cyclic group (which has the same meaning as described above), or (3) aliphatic hydrocarbon group substituted with a cyclic group (aliphatic hydrocarbon and cyclic group have the same meaning as described above). Examples of the "mono-, di- or tri-substituted hydazino group" include methylhydazino, ethylhydazino, propylhydazino, isopropylhydazino, butylhydazino, isobutylhydazino, tert-butylhydazino, pentylhydazino, hexylhydazino, heptylhydazino, octylhydazino, N,N-dimethylhydazino, N,N'-dimethylhydazino, N,N,N'-trimethylhydazino, N,N-diethylhydazino, N,N'-diethylhydazino, N,N,N'-triethylhydazino, N,N-dipropylhydazino, N,N'-dipropylhydazino, N,N,N'-tripropylhydazino, N,N-dibutylhydazino, N,N'-dibutylhydazino, N,N,N'-tributylhydazino, N,N-dipentylhydazino, N,N'-dipentylhydazino, N,N,N'-tripentylhydazino, N,N-dihexylhydazino, N,N'-dihexylhydazino, N,N,N'-trihexylhydazino, N,N-diheptylhydazino, N,N'-diheptylhydazino, N,N,N'-triheptylhydazino, N,N-dioctylhydazino, N,N'-dioctylhydazino, N,N,N'-trioctylhydazino, N-methyl-N-ethylhydazino, N-methyl-N'-ethylhydazino, cyclopropylhydazino, cyclopentylhydazino, cyclohexylhydazino, phenylhydazino, N,N-diphenylhydazino, N,N'-diphenylhydazino, N,N,N'-triphenylhydazino, N,N-dibenzylhydazino, N,N'-dibenzylhydazino, N,N,N'-tribenzylhydazino, N-phenyl-N'-methylhydazino, N-phenyl-N'-ethylhydazino, N-benzyl-N-methylhydazino, and N-benzyl-N-ethylhydazino.

The "nitrogen-containing heterocyclic ring" in the "nitrogen-containing heterocyclic ring which may have a substituent(s)" includes 3- to 15-membered monocyclic or fused heterocyclic ring having at least one nitrogen atom, bridged polycyclic heterocyclic ring, or spiro-bound polycyclic heterocyclic ring, and examples thereof include pyridine, azepine, oxazole, isoxazole, thiazole, isothiazole, oxazine, oxazepine, thiazine, thiazepine, isoindole, indolizine, quinoline, isoquinoline, quinolidine, benzoxazole, benzothiazole, benzoxazepine, benzothiazepine, benzoazepine, carbazole, acridine, phenothiazine, phenoxazine, phenanthridine, aziridine, azetidine, pyrrolidine, dihydropyridine, tetrahydropyridine, dihydroazepine, tetrahydroazepine, perhydroazepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrooxazine, tetrahydrooxazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, indoline, isoindoline, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthylidine, tetrahydronaphthylidine, perhydronaphthylidine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzoimidazole, perhydrobenzoimidazole, dihydrobenzoazepine, tetrahydrobenzoazepine, dihydrobenzodiazepine, tetrahydrobenzodiazepine, dihydrobenzoxazepine, tetrahydrobenzoxazepine, dihydrocarbazole, tetrahydrocarbazole, perhydrocarbazole, dihydroacridine, tetrahydroacridine, perhydroacridine, azaspiro[4.4]nonane, oxazaspiro[4.4]nonane, azaspiro[4.5]decane, oxazaspiro[4.5]decane, azaspiro[5.5]undecane, azabicyclo[2.2.1]heptane, azabicyclo[3-1.1]heptane, azabicycio[3.2.1]octane, azabicyclo[2.2.2]octane, pyrazole, pyrazine, pyrimidine, pyridazine, diazepine, furazane, oxadiazole, oxadiazine, oxadiazepine, thiadiazole, thiadiazine, thiadiazepine, indazole, phthalazine, naphthylidine, quinoxaline, quinazoline, cinnoline, benzoxadiazepine, benzothiadiazepine, benzodiazepine, benzofurazane, benzothiadiazole, β-carboline, phenazine, phenanthroline, perimidine, pyrazoline, pyrazolidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrodiazepine, tetrahydrodiazepine, perhydrodiazepine, dihydrofurazane, tetrahydrofurazane, dihydrooxadiazole, tetrahydrooxadiazole (oxadiazolidine), dihydrooxadiazine, diazabicyclo[2.2.2]octane, triazole, benzotriazole, triazoline, triazolidine, tetrazole, pteridine, tetrazoline, tetrazolidine, octahydrocyclopenta[b]pyrrole, octahydropyrrolo[2,3-b]pyrrole, octahydropyrrolo[3,4-b]pyrrole, octahydropyrrolo[3,2-b]pyrrole, hexahydro-2H-furo[2,3-b]pyrrole, hexahydro-1H-furo[3,4-b]pyrrole, hexahydro-2H-furo[3,2-b]pyrrole, octahydro-1H-indole, octahydro-1H-pyrrolo[2,3-b]pyridine, octahydro-1H-pyrrolo[2,3-c]pyridine, octahydro-1H-pyrrolo[3,2-c]pyridine, octahydro-1H-pyrrolo[3,2-b]pyridine, octahydropyrano[2,3-b]pyrrole, octahydropyrano[3,4-b]pyrrole, octahydropyrano [4,3-b]pyrrole, octahydropyrano[3,2-b]pyrrole, 2,3-dihydro-1H-pyrrolo[2,3-b]pyridine, 2,3-dihydro-1H-pyrrolo[2,3-c]pyridine, 2,3-dihydro-1H-pyrrolo[3,2-c]pyridine, 2,3-dihydro-1H-pyrrolo[3,2-b]pyridine, decahydrocyclohepta[b]pyrrole, decahydropyrrolo[2,3-b]azepine, decahydropyrrolo[2,3-c]azepine, decahydropyrrolo[2,3-d]azepine, decahydropyrrolo[3,2-c]azepine, decahydropyrrolo[3,2-b]azepine, octahydro-2H-oxepino[2,3-b]pyrrole, octahydro-1H-oxepino[3,4-b]pyrrole, octahydro-1H-oxepino[4,5-b]pyrrole, octahydro-1H-oxepino[4,3-b]pyrrole, octahydro-1H-oxepino[3,2-b]pyrrole, 2,5-dihydro-1H-pyrrole, 2,3-dihydro-1H-pyrrole, 1H-pyrrole, 1,4,5,6-tetrahydrocyclopenta[b]pyrrole, 4,5,6,7-tetrahydro-1H-indole, 1,4,5,6,7,8-hexahydrocyclohepta[b]pyrrole, 1H-indole, 1H-pyrrolo[2,3-b]pyridine, 1H-pyrrolo[2,3-c]pyridine, 1H-pyrrolo[3,2-c]pyridine, 1H-pyrrolo[3,2-b]pyridine, imidazolidine, 4,5-dihydro-1H-imidazole, 1,3a,4,5,6,6a-hexahydrocyclopenta[d]imidazole, 3a,4,5,6,7,7a-hexahydro-1H-benzoimidazole, 1,3a,4,5,6,7,8,8a-octahydrocyclohepta[d]imidazole, 1H-imidazole, 1,4,5,6-tetrahydrocyclopenta[d]imidazole, 4,5,6,7-tetrahydro-1H-benzoimidazole, 1,4,5,6,7,8-hexahydrocyclohepta[d]imidazole, 3,4-dihydropyrrolo[2,3-d]imidazole, 3H-thieno[2,3-d]imidazole, 1H-benzoimidazole, 3H-imidazo[4,5-b]pyridine, 3H-imidazo[4,5-c]pyridine, 9H-purine, 1H-imidazo[4,5-b]pyrazine, 7H-imidazo[4,5-c]pyridazine, 1H-imidazo[4,5-d]pyridazine, hexahydro-1H-pyrrolo[1,2-a]imidazole, hexahydro-1H-imidazo[1,2-b]pyrazole, hexahydroimidazo[1,2-b]isoxazole, hexahydro-1H-imidazo[1,2-a]imidazole, octahydroimidazo[1,2-a]pyridine, octahydroimidazo[1,2-b]pyridazine, hexahydro-1H-imidazo[1,2-b][1,2]oxazine, octahydroimidazo[1,2-a]pyrimidine, octahydroimidazo [1,2-a]pyrazine, hexahydro-1H-imidazo[2,1-c][1,4]oxazine, octahydro-1H-imidazo[1,2-a]azepine, octahydro-1H-imidazo [1,2-b][1,2]diazepine, octahydroimidazo[1,2-b][1,2]oxazepine, octahydro-1H-imidazo[1,2-a][1,3]diazepine, octahydro-1H-imidazo[1,2-a][1,4]diazepine, octahydro-1H-imidazo[1,2-d][1,4]diazepine, hexahydro-1H,5H-imidazo[2,1-c][1,4]oxazepine, octahydroimidazo[1,2-d][1,4]oxazepine, 6,7-dihydro-5H-pyrrolo[1,2-a]imidazole, 6,7-dihydro-5H-imidazo[1,5-a]imidazole, 7H-imidazo[1,2-c][1,3]oxazole, 7H-imidazo[1,2-c][1,3]thiazole, 5H-imidazo[1,2-b]pyrazole, 2,3-dihydro-1H-imidazo[1,2-a]imidazole, 2,3-dihydroimidazo[2,1-b][1,3]oxazole, 2,3-dihydroimidazo[2,1-b][1,3]thiazole, 5,6,7,8-tetrahydroimidazo[1,2-a]pyridine, 5,6,7,8-tetrahydroimidazo[1,2-c]pyrimidine, 7,8-dihydroimidazo[1,2-c][1,3]oxazine, 7,8-dihydroimidazo[1,2-c][1,3]thiazine, 5,6,7,8-tetrahydroimidazo[1,2-a]pyrazine, 5,6-dihydro-8H-imidazo[2,1-c][1,4]oxazine, 5,6-dihydro-8H-imidazo[2,1-c][1,4]thiazine, 5,6,7,8-tetrahydroimidazo[1,2-a]pyrimidine, 6,7-dihydro-5H-imidazo[2,1-b] [1,3]oxazine, 6,7-dihydro-5H-imidazo[2,1-b][1,3]thiazine, imidazo[1,2-a]pyridine, imidazo[1,2-b]pyridazine, imidazo[1,2-c]pyrimidine, imidazo[1,2-a]pyrazine, imidazo[1,2-a]pyrimidine, imidazo[1,2-c][1,2,3]triazine, imidazo[2,1-f][1,2,4]triazine, imidazo[1,2-b][1,2,4]triazine, imidazo[1,2-d][1,2,4]triazine, imidazo[1,2-a][1,3,5]triazine, imidazo[2,1-c][1,2,4]triazine, 6,7,8,9-tetrahydro-5H-imidazo[1,2-a]azepine, 6,7,8,9-tetrahydro-5H-imidazo[1,2-c][1,3]diazepine, 6,7,8,9-tetrahydro-5H-imidazo[1,2-d][1,4]diazepine, 6,7,8,9-tetrahydro-5H-imidazo[1,2-a][1,4]diazepine, 6,7,8,9-tetrahydro-5H-imidazo[1,2-a][1,3]diazepine, 8,9-dihydro-7H-imidazo[1,2-c][1,3]diazepine, 6,7,8,9-tetrahydro-5H-imidazo[1,2-e][1,3,5] triazepine, 1H-1,2,4-triazole, 6,7-dihydro-5H-imidazo[1,5-b][1,2,4]triazole, 7H-[1,3]oxazolo[3,4-b][1,2,4]triazole, 7H-[1,3]thiazolo[3,4-b][1,2,4]triazole, 5H-pyrazolo[1,5-b][1,2,4]triazole, 6,7-dihydro-5H-pyrrolo[1,2-b][1,2,4]triazole, 5,6-dihydro-4H-imidazo[1,2-b][1,2,4]triazole, 5,6-dihydro[1,3]oxazolo[3,2-b][1,2,4]triazole, 5,6-dihydro[1,3]thiazolo[3,2-b] [1,2,4]triazole, 5,6,7,8-tetrahydro[1,2,4]triazolo[1,5-a]pyridine, 5,6,7,8-tetrahydro[1,2,4]triazolo[1,5-c]pyrimidine, 7,8-dihydro[1,2,4]triazolo[1,5-c][1,3]oxazine, 7,8-dihydro[1,2,4]triazolo[1,5-c][1,3]thiazine, 5,6,7,8-tetrahydro[1,2,4]triazolo [1,5-a]pyrazine, 5,6-dihydro-8H-[1,2,4]triazolo[5,1-c][1,4]oxazine, 5,6-dihydro-8H-[1,2,4]triazolo[5,1-c][1,4]thiazine, 4,5,6,7-tetrahydro[1,2,4]triazolo[1,5-a]pyrimidine, 6,7-dihydro-5H-[1,2,4]triazalo[5,1-b][1,3]oxazine, 6,7-dihydro-5H-[1,2,4]triazolo[5,1-b][1,3]thiazine, [1,2,4]triazolo[1,5-a]pyridine, [1,2,4]triazolo[1,5-b]pyridazine, [1,2,4]triazolo[1,5-c] pyrimidine, [1,2,4]triazolo[1,5-a]pyrazine, [1,2,4]triazolo[1,5-a]pyrimidine, [1,2,4]triazolo[1,5-c][1,2,3]triazine, [1,2,4]triazolo[5,1-f][1,2,4]triazine, [1,2,4]triazolo[1,5-b][1,2,4]triazine, [1,2,4]triazolo[1,5-d][1,2,4]triazine, [1,2,4]triazolo[1,5-a] [1,3,5]triazine,[1,2,4]triazolo[5,1-c][1,2,4]triazine, 6,7,8,9-tetrahydro-5H-[1,2,4]triazolo[1,5-a]azepine, 6,7,8,9-tetrahydro-5H-[1,2,4]triazolo[1,5-c][1,3]diazepine, 6,7,8,9-tetrahydro-5H-[1,2,4]triazolo[1,5-d][1,4]diazepine, 6,7,8,9-tetrahydro-5H-[1,2,4]triazolo[1,5-a][1,4]diazepine, piperidine, 8,9-dihydro-7H-[1,2,4]triazolo[1,5-c][1,3]diazepine, 6,7,8,9-tetrahydro-5H-[1,2,4]triazolo[1,5-e][1,3,5]triazepine, 6,7-dihydro-5H-cyclopenta[b]pyridine, 5,6,7,8-tetrahydroquinoline, 6,7,8,9-tetrahydro-5H-cyclohepta[b]pyridine, 6,7-dihydro-5H-cyclopenta[c]pyridine, 5,6,7,8-tetrahydroisoquinoline, 6,7,8,9-tetrahydro-5H-cyclohepta[c]pyridine, 1,3-benzoxazole, 1,3-benzothiazole, pyrazolo[1,5-a]pyridine, imidazo[1,5-a]pyridine, 1,5-naphthylidine, and 1,6-naphthylidine rings.

The "substituent" in the "nitrogen-containing heterocyclic ring which may have a substituent(s)" is not particularly limited and includes, for example, (1) aliphatic hydrocarbon group which may have a substituent(s) (2) cyclic group which may have a substituent(s), (3) aliphatic hydrocarbon group substituted with a cyclic group which may have a substituent (s), or (4) substituents exemplified as for L. In the present specificaion, the "aliphatic hydrocarbon group" and "cyclic group" in the "aliphatic hydrocarbon group which may have a substituent(s)", "cyclic group which may have a substituent (s)", or "aliphatic hydrocarbon group substituted with a cyclic group which may have a substituent(s)" have the same meanings as described above and include the "substituents" in (1) to (3) and substituents exemplified as for L. These optional substituent(s) may be substituted on the substitutable position in the number of 1 to 5.

In the present specification, "nitrogen-containing heterocyclic ring which may have a substituent(s)" represented by $A^1$ has the same meaning as that of the "nitrogen-containing heterocyclic ring which may have a substituent(s)" as the "basic group" in the "group having a basic group".

In the present specification, the "substituent" represented by $R^1$ and $R^2$ includes, for example, substituents exemplified as the "substituent" of the "mono- or di-substituted amino group" in the "basic group".

In the present specification, "$-NR^1R^2$" represented by $A^1$ includes, for example, groups exemplified as the "mono- or di-substituted amino group" in the "basic group".

In the present specification, the "divalent monocyclic cyclic group" in the "divalent monocyclic cyclic group which may have a substituent(s)" represented by ring D includes, for example, a divalent group in which optional two hydrogen atoms are removed from the "3- to 8-membered monocyclic cyclic group". Optional two hydrogen atoms may be combined with the same carbon atom or different carbon atoms, and are preferably combined with different carbon atoms. In the present specification, the "3- to 8-membered monocyclic cyclic group" includes, for example, "C3-8 monocyclic carbocyclic ring" or "3- to 8-membered monocyclic heterocyclic ring". The "C3-8 monocyclic carbocyclic ring" includes a C3-8 monocyclic unsaturated carbocyclic ring, and a partially or completely saturated carbocyclic ring thereof. Examples of the "C3-8 monocyclic unsaturated carbocyclic ring, and partially or completely saturated carbocyclic ring thereof" include cyclopropane, cyclobutane, cyclopentane, cyclohexane, cycloheptane, cyclooctane, cyclopentene, cyclohexene, cycloheptene, cyclooctene, cyclopentadiene, cyclohexadiene, cycloheptadiene, cyclooctadiene, and benzene ring. Among these groups, the "C3-8 monocyclic aromatic carbocyclic ring" includes benzene ring.

The "3- to 8-membered monocyclic heterocyclic ring" includes, for example, "3-to 8-membered monocyclic heterocyclic ring having 1 to 4 nitrogen atom(s), 1 to 2 oxygen atom(s) and/or 1 to 2 sulfur atom(s) as heteroatoms". In the present specification, the "3-to 8-membered monocyclic heterocyclic ring having 1 to 4 nitrogen atom(s), 1 to 2 oxygen atom(s) and/or 1 to 2 sulfur atom(s) as heteroatoms" includes 3- to 8-membered monocyclic unsaturated heterocyclic ring having 1 to 4 nitrogen atom(s), 1 to 2 oxygen atom(s) and/or 1 to 2 sulfur atom(s) as heteroatoms, and a partially or completely saturated heterocyclic ring thereof. Examples of the "3- to 8-membered monocyclic unsaturated heterocyclic ring having 1 to 4 nitrogen atom(s), 1 to 2 oxygen atom(s) and/or 1 to 2 sulfur atom(s) as heteroatoms, and partially or completely saturated heterocyclic ring thereof" include pyrrole, imidazole, triazole, tetrazole, pyrazole, pyridine, pyrazine, pyrimidine, pyridazine, azepine, diazepine, furan, pyran, oxepin, thiophene, thiopyran, thiepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepin, tetrahydrooxepin, perhydrooxepin, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazane, tetrahydrofurazane, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, dioxolane, dioxane, dithiolane, and dithiane rings. Among them, examples of the "3- to 8-membered monocyclic aromatic heterocyclic ring having 1 to 4 nitrogen atom(s), 1 to 2 oxygen atom(s) and/or 1 to 2 optionally oxidized sulfur atom(s) as heteroatoms" include pyrrole, imidazole, triazole, tetrazole, pyrazole, pyridine, pyrazine, pyrimidine, pyridazine, furan, thiophene, oxazole, isoxazole, thiazole, isothiazole, furazane, oxadiazole, and thiadiazole rings.

In the present specification, the "substituent" of the "divalent monocyclic cyclic group which may have a substituent(s)" represented by ring D is not particularly limited and includes, for example, (1) aliphatic hydrocarbon group which may have a substituent(s) (2) cyclic group which may have a substituent(s), (3) aliphatic hydrocarbon group substituted with a cyclic group which may have a substituent(s), or (4) substituents exemplified as for L. In the present specification, the "aliphatic hydrocarbon group" and "cyclic group" in the "aliphatic hydrocarbon group which may have a substituent(s)", "cyclic group which may have a substituent(s)" and "aliphatic hydrocarbon group substituted with a cyclic group which may have a substituent(s)" have the same meanings as described above. In the present specification, the "substituent" in (1) to (3) includes, for example, substituents exemplified as for L. These optional substituent(s) may be substituted in the substitutable position in the number of 1 to 5.

In the present specification, the "divalent cyclic group" of the "divalent monocyclic cyclic group which may have a substituent(s)" represented by ring $A^2$ has the same meaning as that of the "divalent monocyclic cyclic group" in the "divalent monocyclic cyclic group which may have a substituent(s)" defined as ring D. Examples of the "substituent" of the "divalent monocyclic cyclic group which may have a substituent(s)" include substituents exemplified as for L.

In the present specification, the "divalent 3- to 8-membered monocyclic heterocyclic ring which has 1 to 3 nitrogen atom(s), and may also have an oxygen atom and/or an optionally oxidized sulfur atom" of the "divalent 3- to 8-membered monocyclic heterocyclic ring which may have a substituent(s), and also have 1 to 3 nitrogen atom(s) and may have an oxygen atom and/or an optionally oxidized sulfur atom" represented by ring $A^2$ includes, for example, divalent group obtained by removing optional two hydrogen atoms from the "3- to 8-membered monocyclic heterocyclic ring which has 1 to 3 nitrogen atom(s), and also may have an oxygen atom and/or an optionally oxidized sulfur atom. Optional two hydrogen atoms may be combined with the same carbon atom or different carbon atoms, and are preferably combined with different carbon atoms.

Examples of the "3- to 8-membered monocyclic heterocyclic ring which has 1 to 3 nitrogen atom(s), and also may have an oxygen atom and/or an optionally oxidized sulfur atom" include pyrrolidine, 2,5-dihydro-1H-pyrrole, 2,3-dihydro-IH-pyrrole, pyrrole, imidazolidine, 4,5-dihydro-1H-imidazole, imidazole, triazole, pyrazole, pyridine, pyrazine, pyrimidine, pyridazine, azepine, diazepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine, aziridine, azetidine, pyrroline, pyrrolidine, imidazoline, triazoline, triazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazane, tetrahydrofurazane, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, and thiomorpholine rings. Among them, examples of the 5- or 6-membered monocyclic aromatic heterocyclic ring include pyrrole, imidazole, triazole, pyrazole, pyridine, pyrazine, pyrimidine, pyridazine, oxazole, isoxazole, thiazole, isothiazole, furazane, oxadiazole, and thiadiazole rings.

The "substituent" in the "divalent 3- to 8-membered monocyclic heterocyclic ring which may have a substituent (s), and also has 1 to 3 nitrogen atom(s) and may have an oxygen atom and/or an optionally oxidized sulfur atom" represented by ring $A^2$ has the same meaning as that of the "substituent" in the "divalent monocyclic cyclic group which may have a substituent(s)" represented by ring $A^2$.

In the present specification, the "divalent cyclic group" in the "divalent cyclic group which may have a substituent (s)" represented by ring $A^{20}$ includes a divalent group obtained by removing optional two hydrogen atoms from the "cyclic group". Optional two hydrogen atom may be combined with the same carbon atom or different carbon atoms, and are preferably combined with different carbon atoms.

In the present specification, the "substituent" in the "divalent cyclic group which may have a substituent(s)" represented by ring $A^{20}$ has the same meaning as that of the "substituent" of the "divalent monocyclic cyclic group which may have a substituent(s)" represented by ring $A^2$.

In the present specification, the "divalent C1-4 aliphatic hydrocarbon group" in the "divalent C1-4 aliphatic hydrocarbon group which may have a substituent(s)" represented by $E^1$ or $E^{10}$ includes, for example, C1-4 alkylene group (for example, $-CH_2-$, - $(CH_2)_2-$, $-(CH_2)_3-$, $-(-CH_2)_4-$, etc.), C2-4 alkenylene group (for example, -CH=CH-, $-CH_2-CH=CH-$, $-CH=CH-CH_2-$, $-(CH_2)_2-CH=CH-$, $-CH=CH-(CH_2)_2-$, $-CH_2-CH=CH-CH_2-$, etc.), or C2-4 alkynylene group (for example, -C≡C-, $-CH_2-C≡C-$, $-C≡C-CH_2-$, $-(CH_2)_2-C≡C-$, - $C≡C-(CH_2)_2-$, $-CH_2-C≡C-CH_2-$, etc.). In the present specification, the "substituent" in the "divalent C1-4 aliphatic hydrocarbon group which may have a substituent(s)" is not particularly limited. The substituent includes, for example, substituents exemplified as for L. These optional substituents

may be substituted on the optional substitutable position in optional substitutable number. The number of substituents is from 1 to 5, and preferably from 1 to 2.

In the present specification, the "substituent" in the "methylene group which may have a substituent(s)" and "ethenylene group which may have a substituent(s)" represented by $G^1$, $G^2$ and $G^3$ is not particularly limited. The substituent includes, for example, (1) aliphatic hydrocarbon group which may have a substituent(s) (2) cyclic group which may have a substituent(s), (3) aliphatic hydrocarbon group substituted with a cyclic group which may have a substituent (s), or (4) substituents exemplified as for L. In the present specification, the "aliphatic hydrocarbon group" and "cyclic group" in the "aliphatic hydrocarbon group which may have a substituent(s)", "cyclic group which may have a substituent (s)", or "aliphatic hydrocarbon group substituted with a cyclic group which may have a substituent(s)" have the same meanings as described above and the "substituent" in (1) to (3) includes, for example, substituents exemplified as for L. These optional substituents may be substituted on the optional substitutable position in optional substitutable number. The number of substituents is from 1 to 2, and preferably 1.

In the present specification, the "divalent nitrogen atom which may have a substituent(s)" represented by $G^1$, $G^2$ and $G^3$ represents, in addition to -NH-, those in which hydrogen atoms in an "-NH-" group are optionally substituted with a substituent such as aliphatic hydrocarbon group, aliphatic hydrocarbon group substituted with a cyclic group, -O-aliphatic hydrocarbon group, -$SO_2$-aliphatic hydrocarbon group, -CO-aliphatic hydrocarbon group, -COO- aliphatic hydrocarbon group, nitro group, or methyl group substituted with 1 to 3 halogen atom(s) among substituent defined as L.

In the present specification, the "protective group" in the "C1-4 aliphatic hydrocarbon group which may be substituted with a hydroxyl group which may be protected by a protective group, a carboxyl group which may be protected by a protective group, or a sulfo group which may be protected by a protective group, and also may have a substituent (s)", "carbocyclic ring group which may be substituted with a hydroxyl group which may be protected by a protective group, a carboxyl group which may be protected by a protective group, or a sulfo group which may be protected by a protective group, and also may have a substituent(s)", "heterocyclic ring group which may be substituted with a hydroxyl group which may be protected by a protective group, a carboxyl group which may be protected by a protective group, or a sulfo group which may be protected by a protective group, and also may have a substituent(s)", "C1-4 aliphatic hydrocarbon group which may be substituted with a hydroxyl group which may be protected by a protective group, a carboxyl group which may be protected by a protective group, or a sulfo group which may be protected by a protective group, and is also substituted with a carbocyclic ring group which may have a substituent(s) and may have a substituent (s)", or "C1-4 aliphatic hydrocarbon group which may be substituted with a hydroxyl group which may be protected by a protective group, a carboxyl group which may be protected by a protective group, or a sulfo group which may be protected by a protective group, and is also substituted with a heterocyclic ring group which may have a substituent(s) and may have a substituent(s)" represented by $R^3$ is not particularly limited as long as it protects hydroxyl group, carboxyl group and sulfo group. The "protective group" includes, for example, a hydrocarbon group which may have a substituent (s), an amino group which may have a substituent(s), or a group represented by formula (P):

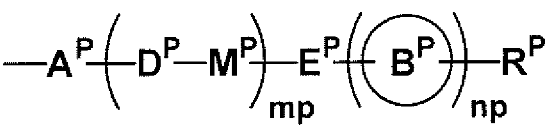

(P)

(wherein $A^P$ represents an oxygen atom or an optionally substituted nitrogen atom, ring $B^P$ represents a cyclic group which may have a substituent(s), $D^P$ and $E^P$ each independently represents an optionally substituted C1-8 alkylene group, an optionally substituted C2-8 alkenylene group, an optionally substituted C2-8 alkynylene group or a bond, $M^P$ represents a spacer having an oxygen atom, a nitrogen atom, a sulfur atom and/or a phosphorus atom in its main chain or a bond, $R^P$ represents a hydrogen atom or a substituent(s), mp represents an integer of 1 to 3 and, when mp is 2 or more, a plurality of $D^P$ and a plurality of $M^P$ may be the same or different, np represents 0 or an integer of 1 to 2 and, when np is 2, a plurality of rings $B^P$ may be the same or different.

For example, when the "carboxyl group protected by a protective group" in $R^3$ is a carboxyl group protected by a group represented by formula (P), it represents a group represented by the following formula (P-1):

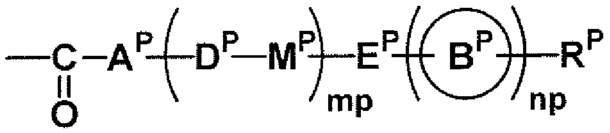

(P-1)

In the present specification, examples of the "hydrocarbon group" in the "hydrocarbon group which may have a substituent(s)" include C1-15 alkyl group such as methyl, ethyl, propyl, isopropyl, butyl, isobutyl, sec-butyl, tert-butyl, pentyl, hexyl, heptyl, octyl, nonyl, decyl, undecyl, dodecyl, tridecyl, tetradecyl, and pentadecyl group; C3-8 cycloalkyl group such as cyclopropyl, cyclobutyl, cyclopentyl, and cyclohexyl group; C2-10 alkenyl group such as vinyl, allyl, 2-methylallyl, 2-butenyl, 3-butenyl, and 3-octenyl group; C2-10 alkynyl group such as ethynyl, 2-propynyl, and 3-hexynyl group; C3-10 cycloalkenyl group such as cyclopropenyl, cyclopentenyl, and cyclohexenyl group; C6-14 aryl group such as phenyl, and naphthyl group; C7-16 aralkyl group such as benzyl, and phenylethyl group; and (C3-8 cycloalkyl group)-(C1-4 alkyl group) such as cyclohexylmethyl, cyclohexylethyl, cyclohexylpropyl, 1-methyl-1-cyclohexylmethyl, and cyclopropylethyl group. Examples of the "substituent" in the "hydrocarbon group which may have a substituent(s)" include (1) nitro group, (2) hydroxyl group, (3) oxo group, (4) thioxo group, (5) cyano group, (6) carbamoyl group, (7) aminocarbonyl group substituted with C1-8 hydrocarbon, such as N-butylaminocarbonyl, N-cyclohexylmethylaminocarbonyl, N-butyl-N-cyclohexylmethylaminocarbonyl, N-cyclohexylaminocarbonyl, and phenylaminocarbonyl group, (8) carboxyl group, (9) C1-4 alkoxycarbonyl group such as methoxycarbonyl, and ethoxycarbonyl group, (10) sulfo group, (11) halogen atom such as fluorine atom, chlorine atom, bromine atom, and iodine atom, (12) C1-4 lower alkoxy group which may be substituted with a halogen atom, such as methoxy, ethoxy, propoxy, isopropoxy, butoxy, isobutoxy, sec-butoxy, tert-butoxy, difluoromethoxy, and trifluoromethoxy group, (13) phenoxy group, (14) halogenophenoxy group such as o-, m- or p-chlorophenoxy group, and o-, m- or p-bromophenoxy, (15) C1-4 lower alkylthio group such as methylthio, ethylthio, n-propylthio, isopropylthio, n-butylthio, and tert-butylthio group, (16) phenylthio group, (17) C1-4 lower alkylsulfinyl group such as methylsulfinyl, and ethylsulfinyl group, (18) C1-4 lower alkylsulfonyl group such as methylsulfonyl, and ethyl sulfonyl group, (19) C1-4 lower acyl group such as formyl, and acetyl group, (20) benzoyl group, (21) 5- or 6-membered heterocyclic ring group having, in addition to a carbon atom, 1 to 4 heteroatom(s) selected from oxygen atom, sulfur atom and/or nitrogen atom, which may have 1 to 4 substituents selected from (a) halogen atom such as bromine atom, chlorine atom, and fluorine atom, (b) hydrocarbon group which may be substituted with oxo or hydroxy group, such as methyl, ethyl, propyl, isopropyl, benzyl, cyclohexyl, cyclohexylmethyl, and cyclohexylethyl group, (the "hydrocarbon group" has the same meaning as that of the above "hydrocarbon group"), (c) halogenophenoxy group such as o-, m- or p-chlorophenoxy group, and o-, m- or p-bromophenoxy, and (d) oxo group, for example, 2- or 3-thienyl, 2- or 3-furyl, 3-, 4- or 5-pyrazolyl, 4-tetrahydropyranyl, 2-, 4- or 5-thiazolyl, 3-, 4- or 5-isothiazolyl, 2-, 4- or 5-oxazolyl, 3-, 4- or 5-isoxazolyl, 2-, 4- or 5-imidazolyl, 1,2,3- or 1,2,4-triazolyl, 1H or 2H-tetrazolyl, 2-, 3- or 4-pyridyl, 2-, 4- or 5-pyrimidyl, 3- or 4-pyridazinyl, quinolyl, isoquinolyl, and indolyl group, and (22) C1-10 haloalkyl group such as difluoromethyl, trifluoromethyl, trifluoroethyl, and trichloroethyl group. The "hydrocarbon group which may have a substituent(s)" may have 1 to 10 substituent(s) selected from (1) to (22) in the "substituents" in the "hydrocarbon group which may have a substituent(s)", and may also have 1 to 4 C1-4 lower alkyls such as methyl, ethyl, propyl, isopropyl, and butyl as substituent (s) when the "hydrocarbon group" is cycloalkyl, cycloalkenyl, aryl or aralkyl group. When the number of substituent is 2 or more, the substituent may be the same or different.

In the present specification, the "substituent" of the amino group in the "amino group which may have a substituent(s)" includes, for example, those exemplified in the "hydrocarbon group which may have a substituent(s)".

In the present specification, "cyclic group" in the "cyclic group which may further have a substituent(s)" represented by ring $B^P$ includes, for example, divalent groups obtained by removing optional two hydrogen atoms from "carbocyclic ring", "heterocyclic ring", or "steroid framework". Optional two hydrogen atoms may be combined with the same carbon atom or different carbon atoms, and are preferably combined with different carbon atoms.

The "carbocyclic ring" as the "cyclic group" in the "cyclic group which may further have a substituent(s)" represented by ring $B^P$ includes, for example, "C3-15 carbocyclic ring". In the "C3-15 carbocyclic ring", "C3-15 monocyclic, bicyclic or tricyclic carbocyclic ring" and "C3-15 spiro-bound bicyclic carbocyclic ring and bridged bicyclic carbocyclic ring" are included. Examples of the "C3-15 monocyclic, bicyclic or tricyclic carbocyclic ring" include cyclopropane, cyclobutane, cyclopentane, cyclohexane, cycloheptane, cyclooctane, cyclononane, cyclodecane, cycloundecane, cyclododecane, cyclotridodecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, 6,7-dihydro-5H-benzo[7]annulene, 5H-benzo[7]annulene, heptalene, perhydroheptalene, biphenylene, as-indacene, s-indacene, acenaphthylene, acenaphthene, fluorene, phenalene, phenanthrene, and anthracene

rings. Examples of the "C3-15 spiro-bound bicyclic carbocyclic ring and bridged bicyclic carbocyclic ring" include spiro [4.4]nonane, spiro[4.5]decane, spiro[5.5]undecane, bicyclo[2.2.1]heptane, bicyclo[2.2.1]hept-2-ene, bicyclo[3.1.1]heptane, bicyclo[3.1.1]hept-2-ene, bicyclo[3.2.1]octane, bicyclo[2.2.2]octane, bicyclo[2.2.2]oct-2-ene, adamantane, and noradamantane rings.

The "heterocyclic ring" as the "cyclic group" in the "cyclic group which may further have a substituent(s)" represented by ring $B^P$ includes, for example, "3- to 15-membered heterocyclic ring having 1 to 5 nitrogen atom(s), 1 to 2 oxygen atom(s) and/or 1 sulfur atom". In the "3- to 15-membered heterocyclic ring having 1 to 5 nitrogen atom(s), 1 to 2 oxygen atom(s) and/or 1 sulfur atom", "3- to 15-membered monocyclic, bicyclic or tricyclic heterocyclic ring having 1 to 5 nitrogen atom(s), 1 to 2 oxygen atom(s) and/or 1 sulfur atom" and "3- to 15-membered spiro-bound bicyclic heterocyclic ring and bridged bicyclic heterocyclic ring having 1 to 5 nitrogen atom(s), 1 to 2 oxygen atom(s) and/or 1 sulfur atom" are included.

Examples of the "3- to 15-membered monocyclic, bicyclic or tricyclic heterocyclic ring having 1 to 5 nitrogen atom(s), 1 to 2 oxygen atom(s) and/or 1 sulfur atom" include pyrrole, imidazole, triazole, tetrazole, pyrazole, pyridine, pyrazine, pyrimidine, pyridazine, azepine, diazepine, furan, pyran, oxepin, thiophene, thiopyran, thiepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolidine, purine, phthalazine, pteridine, naphthylidine, quinoxaline, quinazoline, cinnoline, benzoxazole, benzothiazole, benzoimidazole, chromene, benzoxepin, benzoxazepine, benzoxadiazepine, benzothiepine, benzothiazepine, benzothiadiazepine, benzoazepine, benzodiazepine, benzofurazane, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepin, tetrahydrooxepin, perhydrooxepin, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazane, tetrahydrofurazane, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthylidine, tetrahydronaphthylidine, perhydronaphthylidine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzoimidazole, perhydrobenzoimidazole, dihydrobenzoazepine, tetrahydrobenzoazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindane, benzodioxane, chromene, chroman, benzodithiolane, and benzodithiane rings.

Examples of the "3- to 15-membered spiro-bound bicyclic heterocyclic ring and bridged bicyclic heterocyclic ring having 1 to 5 nitrogen atom(s), 1 to 2 oxygen atom(s) and/or 1 sulfur atom" include azaspiro[4.4]nonane, oxazaspiro [4.4]nonane, oxaazaspiro[2.5]octane, azaspiro[4.5]decane, 1,3,8-triazaspiro[4.5]decane, 2,7-diazaspiro[4.5]decane, 1,4,9-triazaspiro[5.5]undecane, oxazaspiro[4.5]decane, azaspiro[5.5]undecane, azabicyclo[2.2.1]heptane, azabicyclo [3.1.1]heptane, azabicyclo[3.2.1]octane (8-azabicyclo[3.2.1]octane ring, etc.), azabicyclo[2.2.2]octane (2-azabicyclo [2.2.2]octane ring, etc.), and azabicyclo[2.1.1]hexane (5-azabicyclo[2.1.1]hexane ring, etc.) rings.

In the present specification, the "steroid framework" in the "steroid framework which may have a substituent (s)" represented by ring $B^P$ may be any one as long as it is generally called a steroid framework, and usually means a perhydro-1H-cyclopenta[a]phenanthrene framework. The "steroid framework which may have a substituent(s)" includes, for example, structures derived from cholic acid, deoxycholic acid, chenodeoxycholic acid, ursocholic acid, and ursodeoxycholic acid represented by formula ($S^P$).

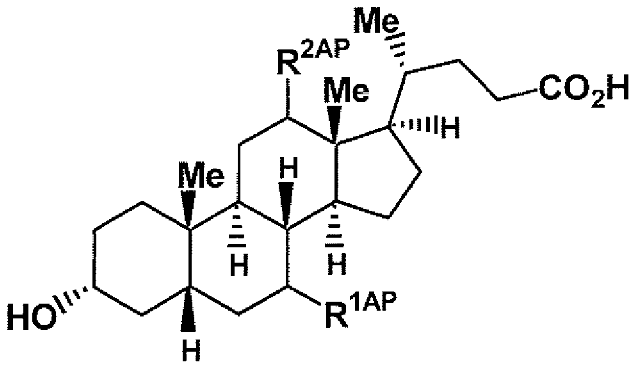

($S^P$)

$R^{1AP}$ = $\alpha$-OH, $R^{2AP}$ = $\alpha$-OH : cholic acid
$R^{1AP}$ = H, $R^{2AP}$ = $\alpha$-OH : deoxycholic acid
$R^{1AP}$ = $\alpha$-OH, $R^{2AP}$ = H : chenodeoxycholic acid
$R^{1AP}$ = $\beta$-OH, $R^{2AP}$ = $\alpha$-OH : ursocholic acid
$R^{1AP}$ = $\beta$-OH, $R^{2AP}$ = H : ursodeoxycholic acid

In the present specification, the "substituent" in the "cyclic group which may further have a substituent(s)" represented by ring $B^P$ is not particularly limited as long as it is a substituent. Examples of the "substituent" include (1) optionally substituted C1-20 alkyl group, (2) optionally substituted C2-20 alkenyl group, (3) optionally substituted C2-20 alkynyl group, (4) optionally substituted C1-20 alkylidene group, (5) optionally substituted cyclic group, (6) oxo group, (7) hydroxyl group, (8) optionally substituted C1-20 alkyloxy group, (9) optionally substituted C2-20 alkenyloxy group, (10) optionally substituted C2-20 alkynyloxy group, (11) hydroxyl group which may be protected by an optionally substituted cyclic group, (12) optionally substituted C1-20 acyloxy group, (13) thioxo group, (14) mercapto group, (15) optionally substituted C1-20 alkylthio group, (16) optionally substituted C2-20 alkenylthio group, (17) optionally substituted C2-20 alkynylthio group, (18) mercapto group substituted with an optionally substituted cyclic group, (19) optionally substituted C1-20 alkylsulfinyl group (for example, methylsulfinyl group, ethylsulfinyl group, etc.), (20) optionally substituted C2-20 alkenylsulfinyl group, (21) optionally substituted C2-20 alkynylsulfinyl group, (22) sulfinyl group substituted with an optionally substituted cyclic group (for example, phenylsulfinyl group, etc.), (23) optionally substituted C1-20 alkylsulfonyl group (for example, methylsulfonyl group, ethylsulfonyl group, etc.), (24) optionally substituted C2-20 alkenylsulfonyl group, (25) optionally substituted C2-20 alkynylsulfonyl group, (26) sulfonyl group substituted with an optionally substituted cyclic group (for example, phenylsulfonyl group, etc.), (27) optionally substituted sulfino group, (28) optionally substituted sulfo group, (29) optionally substituted sulfamoyl group (for example, non-substituted sulfamoyl group, N-mono- or di- (optionally substituted C1-20 alkyl) sulfamoyl group (for example, N-mono-C1-6 alkylsulfamoyl group (for example, N-methylsulfamoyl group, N-ethylsulfamoyl group, N-propylsulfamoyl group, N-isopropylsulfamoyl group, N-butylsulfamoyl group, N-isobutylsulfamoyl group, N-(tert-butyl) sulfamoyl group, N-pentylsulfamoyl group, N-hexylsulfamoyl group, etc.), N,N-di-C1-6 alkylsulfamoyl group (for example, N,N-dimethylsulfamoyl group, N,N-diethylsulfamoyl group, N,N-dipropylsulfamoyl group, N,N-dibutylsulfamoyl group, N,N-dipentylsulfamoyl group, N,N-dihexylsulfamoyl group, N-methylN-ethylsulfamoyl group, etc.), etc.), etc.), (30) optionally substituted carbonyl group (for example, C1-6 alkoxycarbonyl group such as methoxycarbonyl group, ethoxycarbonyl group, t-butoxycarbonyl group, etc.; C3-8 cycloalkanoyl group such as cyclopentylcarbonyl group, cyclohexylcarbonyl group, etc.; C6-10 allylcarbonyl group such as benzoyl group, etc.; heterocyclic ringcarbonyl group which may have a substituent(s), such as morpholin-4-ylcarbonyl group, piperidin-1-ylcarbonyl group, 1-methylpiperazin-4-ylcarbonyl group, etc.), (31) optionally substituted C1-20 acyl group (for example, formyl group, acetyl group, propanoyl group, pivaloyl group, etc.), (32) optionally substituted carbamoyl group (for example, non-substituted carbamoyl group, N-mono- or di- (optionally substituted C1-20 alkyl)carbamoyl group (for example, N-mono-C1-6 alkylcarbamoyl group (for example, N-methylcarbamoyl group, N-ethylcarbamoyl group, N-propylcarbamoyl group, N-isopropylcarbamoyl group, N-butylcarbamoyl group, N-isobutylcarbamoyl group, N-(tert-butyl)carbamoyl group, N-pentylcarbamoyl group, N-hexylcarbamoyl group, etc.), N-mono-Cl-6 alkylcarbamoyl group substituted with a hydroxyl group (for example, N-hydroxymethylcarbamoyl group, N-(2-hydroxyethyl) carbamoyl group, N-(3-hydroxypropyl)carbamoyl group, N-(4-hydroxybutyl)carbamoyl group, etc.), N-mono-C1-6 alkylcarbamoyl group substituted with an amino group or a dimethylamino group (for example, N-aminomethylcarbamoyl group, N-(2-aminoethyl)carbamoyl group, N-(3-aminopropyl)carbamoyl group, N-(4-aminobutyl)carbamoyl group, N-(dimethylamino)methylcarbamoyl group, N-(2-dimethylaminoethyl)carbamoyl group, N-(3-dimethylaminopropyl)carbamoyl group, N-(4-dimethylaminobutyl)carbamoyl group, etc.), N,N-di-C1-6 alkylcarbamoyl group (for example, N,N-dimethylcarbamoyl group, N,N-diethylcarbamoyl group, N,N-dipropylcarbamoyl group, N,N-dibutylcarbamoyl group, N.N-dipentylcarbamoyl group, N,N-dihexylcarbamoyl group, N-methyl-N-ethylcarbamoyl group, etc.), etc.), N-mono- or di- (optionally substituted carbocyclic ring or heterocyclic ring)carbamoyl group (for example, N-mono(an optionally substituted carbocyclic ring)carbamoyl group (for example, N-cyclopropylcarbamoyl group, N-cyclopentylcarbamoyl group, N-cyclohexylcarbamoyl group, N-phenylcarbamoyl group, etc.), etc.), etc.), (33) cyano group, (34) optionally substituted amidino group, (35) nitro group, (36) nitroso group, (37) optionally substituted imino group (for example, non-substituted imino group, imino group substituted with C1-6 alkyl group (for example, methylimino group, ethylimino group, etc.), imino group substituted with a C6-10 allyl group which may have a substituent(s) (for example, phenylimino group, p-fluorophenylimino group, p-chlorophenylimino group, etc.), imino group substituted with a hydroxyl group such as hydroxyimino group, etc.), (38)an optionally substituted amino group (for example, mono- or di-C1-6 alkylamino group (for example, methylamino, group, ethylamino group, propylamino group, dimethylamino group, diethylamino group, etc.), mono- or di-C6-10 allylamino group (for example, phenylamino group, diphenylamino group, etc.), mono-C1-6 alkylmono-C6-10 allylamino group (for example, N-phenyl-N-methylamino group, N-phenyl-N-ethylamino group, etc.), etc.). (39) halogen atom, (40) carboxyl group, (41) phosphono group ($-PO(OH)_2$), (42) dihydroxyboryl group ($-B(OH)_2$), (43) C1-20 alkylcarbonylhydazino group (for example, methylcarbonylhydazino group, ethylcarbonylhydazino group, etc.), and (44) C6-10 allylhydrazone group which may have a substituent(s), such as benzoaldehydehydrazone group, p-methoxybenzoaldehydehydrazone group, etc. These optional substituents may be substituted on the optional substitutable position in optional substitutable number. When the number of substituents is 2, the "optionally substituted sulfamoyl group", "optionally substituted carbamoyl group", "optionally substituted amidino group" and "optionally substituted amino group" exemplified as for (29), (32), (34) and (3 8) of the "substituents" in the "cyclic group which may further have a substituent(s)" represented by ring $B^P$ are combined together with nitrogen atoms to which they are attached to form a 5- to 7-membered monocyclic heterocyclic ring having 1 to 5 nitrogen atom(s), one oxygen atom and/or one sulfur atom, and the heterocyclic ring thus formed may be substituted with a C1-8 alkyl group, hydroxyl group or amino group.

The "cyclic group" in substituents exemplified in (1) to (44) as the substituents of ring $B^P$ have the same meaning as the "cyclic group" in the ring $B^P$.

The description "optionally substituted" in substituents exemplified in (1) to (44) as the substituents of the ring $B^P$ means to be optionally substituted with a substituent(s). Examples of the "substituent" here include (1) C1-20 alkyl group, (2) C2-20 alkenyl group, (3) C2-20 alkynyl group, (4) C1-20 alkylidene group, (5) cyclic group, (6) C1-20 alkyl group substituted with a cyclic group, (7) oxo group, (8) hydroxyl group, (9) C1-20 alkyloxy group, (10) C2-20 alkenyloxy group, (11) C2-20 alkynyloxy group, (12) hydroxyl group which may be protected by a cyclic group, (13) C1-20 acyloxy group, (14) thioxo group, (15) mercapto group, (16) C1-20 alkylthio group, (17) C2-20 alkenylthio group, (18) C2-20 alkynylthio group, (19) mercapto group substituted with a cyclic group, (20) C1-20 alkylsulfinyl group, (21) C2-20 alkenylsulfinyl group, (22) C2-20 alkynylsulfinyl group, (23) sulfinyl group substituted with a cyclic group, (24) C1-20 alkylsulfonyl group, (25) C2-20 alkenylsulfonyl group, (26) C2-20 alkynylsulfonyl group, (27) sulfonyl group substituted with a cyclic group, (28) C1-20 alkylsulfonyl group substituted with a cyclic group, (29) sulfino group, (30) sulfo group, (31) sulfamoyl group, (32) C1-20 acyl group, (33) C1-20 acyl group substituted with a cyclic group, (34) carbonyl group substituted with a cyclic group, (35) carbamoyl group, (36) cyano group, (37) amidino group, (38) nitro group, (39) nitroso group, (40) imino group, (41) amino group, (42) halogen atom, (43) carboxyl group and the like. These optional substituents may be substituted on the optional substitutable position in optional substitutable number. The "cyclic group" in these substituents has the same meaning as the "cyclic group" in the ring $B^P$.

In the present specification, the "C1-20 alkyl group" means methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl and icosyl groups, and isomer groups thereof.

In the present specification, the "C2-20 alkenyl group" means ethenyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, nonadecenyl and icosenyl groups, and isomer groups thereof.

In the present specification, "C2-20 alkynyl group" means ethynyl, propynyl, butynyl, pentynyl, hexynyl, heptynyl, octynyl, nonynyl, decynyl, undecynyl, dodecynyl, tridecynyl, tetradecynyl, pentadecynyl, hexadecynyl, heptadecynyl, octadecynyl, nonadecynyl and icosynyl group, and isomer groups thereof.

In the present specification, the "C1-20 alkylidene group" means methylidene, ethylidene, propylidene, butylidene, pentylidene, hexylidene, heptylidene, octylidene, nonylidene, decylidene, undecylidene, dodecylidene, tridecylidene, tetradecylidene, pentadecylidene, hexadecylidene, heptadecylidene, octadecylidene, nonadecylidene and icosylidene groups, and isomer groups thereof.

In the present specification, the "C1-20 alkyloxy group" means methoxy, ethoxy, propoxy, butoxy, pentyloxy, hexyloxy, heptyloxy, octyloxy, nonyloxy, decyloxy, undecyloxy, dodecyloxy, tridecyloxy, tetradecyloxy, pentadecyloxy, hexadecyloxy, heptadecyloxy, octadecyloxy, nonadecyloxy and icosyloxy groups, and isomer groups thereof.

In the present specification, the "C2-20 alkenyloxy group" means ethenyloxy, propenyloxy, butenyloxy, pentenyloxy, hexenyloxy, heptenyloxy, octenyloxy, nonenyloxy, decenyloxy, undecenyloxy, dodecenyloxy, tridecenyloxy, tetradecenyloxy, pentadecenyloxy, hexadecenyloxy, heptadecenyloxy, octadecenyloxy, nonadecenyloxy and icosenyloxy groups, and isomer groups thereof.

In the present specification, the "C2-20 alkynyloxy group" means ethynyloxy, propynyloxy, butynyloxy, pentynyloxy, hexynyloxy, heptynyloxy, octynyloxy, nonyloxy, decynyloxy, undecynyloxy, dodecynyloxy, tridecynyloxy, tetradecynyloxy, pentadecynyloxy, hexadecynyloxy, heptadecynyloxy, octadecynyloxy, nonadecynyloxy and icosynyloxy groups, and isomer groups thereof.

In the present specification, the "C1-20 alkylthio group" means methylthio, ethylthio, propylthio, butylthio, pentylthio, hexylthio, heptylthio, octylthio, nonylthio, decylthio, undecylthio, dodecylthio, tridecylthio, tetradecylthio, pentadecylthio, hexadecylthio, heptadecylthio, octadecylthio, nonadecylthio and icosylthio groups, and isomer groups thereof.

In the present specification, the "C2-20 alkenylthio group" means ethenylthio, propenylthio, butenylthio, pentenylthio, hexenylthio, heptenylthio, octenylthio, nonenylthio, decenylthio, undecenylthio, dodecenylthio, tridecenylthio, tetradecenylthio, pentadecenylthio, hexadecenylthio, heptadecenylthio, octadecenylthio, nonadecenylthio and icosenylthio groups, and isomer groups thereof.

In the present specification, the "C2-20 alkynylthio group" means ethynylthio, propynylthio, butynylthio, pentynylthio, hexynylthio, heptynylthio, octynylthio, nonylthio, decynylthio, undecynylthio, dodecynylthio, tridecynylthio, tetradecynylthio, pentadecynylthio, hexadecynylthio, heptadecynylthio, octadecynylthio, nonadecynylthio and icosynylthio groups, and isomer groups thereof.

In the present specification, the "C 1-20 alkylsulfinyl group" means ethylsulfinyl, ethylsulfinyl, propylsulfinyl, butylsulfinyl, pentylsulfinyl, hexylsulfinyl, heptylsulfinyl, octylsulfinyl, nonylsulfinyl, decylsulfinyl, undecylsulfinyl, dodecylsulfinyl, tridecylsulfinyl, tetradecylsulfinyl, pentadecylsulfinyl, hexadecylsulfinyl, heptadecylsulfinyl, octadecylsulfinyl, nonadecylsulfinyl and icosylsulfinyl groups, and isomer groups thereof.

In the present specification, the "C2-20 alkenylsulfinyl group" means ethenylsulfinyl, propenylsulfinyl, butenylsulfinyl, pentenylsulfnyl, hexenylsulfinyl, heptenylsulfinyl, octenylsulfinyl, nonenylsulfinyl, decenylsulfinyl, undecenylsulfinyl, dodecenylsulfinyl, tridecenylsulfinyl, tetradecenylsulfinyl, pentadecenylsulfinyl, hexadecenylsulfinyl, heptadecenylsulfinyl, octadecenylsulfinyl, nonadecenylsulfinyl and icosenylsulfinyl groups, and isomer groups thereof.

In the present specification, the "C2-20 alkynylsulfinyl group" means ethynylsulfinyl, propynylsulfnyl, butynylsulfinyl, pentynylsulfinyl, hexynylsulfinyl, heptynylsulfinyl, octynylsulfinyl, nonylsulfinyl, decynylsulfinyl, undecynylsulfinyl, dodecynylsulfinyl, tridecynylsulfinyl, tetradecynylsulfinyl, pentadecynylsulfinyl, hexadecynylsulfinyl, heptadecynylsulfinyl, octadecynylsulfinyl, nonadecynylsulfinyl and icosynylsulfinyl groups, and isomer groups thereof.

In the present specification, the "C1-20 alkylsulfonyl group" means methylsulfonyl, ethylsulfonyl, propylsulfonyl, butylsulfonyl, pentylsulfonyl, hexylsulfonyl, heptylsulfonyl, octylsulfonyl, nonylsulfonyl, decylsulfonyl, undecylsulfonyl, dodecylsulfonyl, tridecylsulfonyl, tetradecylsulfonyl, pentadecylsulfonyl, hexadecylsulfonyl, heptadecylsulfonyl, octadecylsulfonyl, nonadecylsulfonyl and icosylsulfonyl groups, and isomer groups thereof.

In the present specification, the "C2-20 alkenylsulfonyl group" means ethenylsulfonyl, propenylsulfonyl, butenylsulfonyl, pentenylsulfonyl, hexenylsulfonyl, heptenylsulfonyl, octenylsulfonyl, nonenylsulfonyl, decenylsulfonyl, undecenylsulfonyl, dodecenylsulfonyl, tridecenylsulfonyl, tetradecenylsulfonyl, pentadecenylsulfonyl, hexadecenylsulfonyl, heptadecenylsulfonyl, octadecenylsulfonyl, nonadecenylsulfonyl and icosenylsulfonyl groups, and isomer groups thereof.

In the present specification, the "C2-20 alkynylsulfonyl group" means ethynylsulfonyl, propynylsulfonyl, butynylsulfonyl, pentynylsulfonyl, hexynylsulfonyl, heptynylsulfonyl, octynylsulfonyl, nonylsulfonyl, decynylsulfonyl, undecynylsulfonyl, dodecynylsulfonyl, tridecynylsulfonyl, tetradecynylsulfonyl, pentadecynylsulfonyl, hexadecynylsulfonyl, heptadecynylsulfonyl, octadecynylsulfonyl, nonadecynylsulfonyl and icosynylsulfonyl groups, and isomer groups thereof.

In the present specification, the "C1-20 acyl group" means methanoyl, ethanoyl, propanoyl, butanoyl, pentanoyl, hexanoyl, heptanoyl, octanoyl, nonanoyl, decanoyl, undecanoyl, dodecanoyl, tridecanoyl, tetradecanoyl, pentadecanoyl, hexadecanoyl, heptadecanoyl, octadecanoyl, nonadecanoyl and icosanoyl groups, and isomer groups thereof.

In the present specification, the "C1-20 acyloxy group" means methanoyloxy, ethanoyloxy, propanoyloxy, butanoyloxy, pentanoyloxy, hexanoyloxy, heptanoyloxy, octanoyloxy, nonanoyloxy, decanoyloxy, undecanoyloxy, dodecanoyloxy, tridecanoyloxy, tetradecanoyloxy, pentadecanoyloxy, hexadecanoyloxy, heptadecanoyloxy, octadecanoyloxy, nonadecanoyloxy and icosanoyloxy groups, and isomer groups thereof.

In the present specification, the "optionally substituted nitrogen atom" represented by $A^P$ has the same meaning

as the "divalent nitrogen atom which may have a substituent(s)" in the "spacer having a main chain of 1 to 4 atom(s)" represented by $B^1$ and $B^2$ described hereinafter.

In the present specification, the "optionally substituted C1-8 alkylene group" represented by $D^P$ and $E^P$ means those in which optional substituents are combined with optional carbon atoms of a C1-8 alkylene group. The "substituent" includes, for example, substituents exemplified as "substituents" in the "cyclic group which may further have a substituent (s)" represented by ring $B^P$.

In the present specification, the "optionally substituted C2-8 alkenylene group" represented by $D^P$ and $E^P$ means those in which optional substituents are combined with optional carbon atoms of a C2-8 alkylene group. The "substituent" includes, for example, substituents exemplified as "substituents" in the "cyclic group which may further have a substituent(s)" represented by ring $B^P$.

In the present specification, the "optionally substituted C2-8 alkynylene group" represented by $D^P$ and $E^P$ means those in which optional substituents are combined with optional carbon atoms of a C2-8 alkylene group. The "substituent" includes, for example, substituents exemplified as "substituents" in the "cyclic group which may further have a substituent (s)" represented by ring $B^P$.

In the present specification, the "spacer having an oxygen atom, a nitrogen atom, a sulfur atom and/or a phosphorus atom in its main chain" represented by $M^P$ means a spacer having at least one of an oxygen atom, a nitrogen atom, a sulfur atom and/or a phosphorus atom in its main chain, and the kind and number of other constituent atoms are not limited. Examples of the spacer having an oxygen atom, a nitrogen atom, a sulfur atom and/or a phosphorus atom in its main chain include -COO-, -COOCO-, $-CONR^{XP}-$, $-CONR^{XP}CO-$, - O-, -OCO-, -OCOO-, $-OCONR^{XP}-$, $-NR^{XP}-$, $-NR^{XP}CO-$, $-NR^{XP}COO-$, $-NR^{XP}CONR^{YP}-$, -S-, -S(O)-, $-SO_2-$ $-SO_2NR^{XP}-$, $-NR^{XP}SO_2-$, $-OSO_3-$, and $-OP(=O)(R^{ZP})O-$ [wherein $R^{XP}$ and $R^{YP}$ each independently represents a hydrogen atom or a substituent, and $R^{ZP}$ represents an optionally protected hydroxyl group or "$O^-$"].

In the present specification, the "substituent" represented by $R^P$, $R^{XP}$ and $R^{YP}$ are not particularly limited as long as it is a substituent. Examples of the "substituent" in the "cyclic group which may further have a substituent(s)" represented by ring $B^P$ include substituents exemplified above.

In the present specification, examples of the "protecting" group in the "optionally protected hydroxyl group" represented by $R^{ZP}$ include the same substituents as those exemplified as the "substituents" in the "cyclic group which may further have a substituent(s)" represented by ring $B^P$.

In the present specification, "$O^-$" represented by $R^{ZP}$ means an oxygen anion. When $R^{ZP}$ represents "$O^-$", a cation part (for example, quaternized nitrogen atom, etc.) necessarily exists in the molecule.

In the present specification, the "C1-8 alkylene group" means methylene, ethylene, trimethylene, tetramethylene, pentamethylene, hexamethylene, heptamethylene and octamethylene groups, and isomer groups thereof.

In the present specification, the "C2-8 alkenylene group" means ethenylene, propenylene, butenylene, butadienylene, pentenylene, pentadienylene, hexenylene, hexadienylene, heptenylene, heptadienylene, octenylene, and octadienylene groups, and isomer groups thereof.

In the present specification, the "C2-8 alkynylene group" means ethynylene, propynylene, butynylene, butadiynylene, pentynylene, pentadiynylene, hexynylene, hexadiynylene, heptynylene, heptadiynylene, octynylene and octadiynylene groups, and isomer groups thereof.

In the present specification, the "C1-4 aliphatic hydrocarbon group" in the "C1-4 aliphatic hydrocarbon group which may be substituted with a hydroxyl group which may be protected by a protective group, a carboxyl group which may be protected by a protective group, or a sulfo group which may be protected by a protective group, and also may have a substituent(s)", "carbocyclic ring group which may be substituted with a hydroxyl group which may be protected by a protective group, a carboxyl group which may be protected by a protective group, or a sulfo group which may be protected by a protective group, and also may have a substituent(s)", "heterocyclic ring group which may be substituted with a hydroxyl group which may be protected by a protective group, a carboxyl group which may be protected by a protective group, or a sulfo group which may be protected by a protective group, and also may have a substituent(s)", "C1-4 aliphatic hydrocarbon group which may be substituted with a hydroxyl group which may be protected by a protective group, a carboxyl group which may be protected by a protective group, or a sulfo group which may be protected by a protective group, and is also substituted with a carbocyclic ring group which may have a substituent(s) and may have a substituent(s)" and "C1-4 aliphatic hydrocarbon group which may be substituted with a hydroxyl group which may be protected by a protective group, a carboxyl group which may be protected by a protective group, or a sulfo group which may be protected by a protective group, and is also substituted with a heterocyclic ring which may have a substituent (s) and may have a substituent(s)" represented by $R^3$ has the same meaning as that of the "1-4 aliphatic hydrocarbon group".

In the present specification, the "carbocyclic ring group" in the "carbocyclic ring group which may be substituted with a hydroxyl group which may be protected by a protective group, a carboxyl group which may be protected by a protective group, or a sulfo group which may be protected by a protective group, and also may have a substituent(s)" or "C1-4 aliphatic hydrocarbon group which may be substituted with a hydroxyl group which may be protected by a

protective group, a carboxyl group which may be protected by a protective group, or a sulfo group which may be protected by a protective group, and is also substituted with a carbocyclic ring group which may have a substituent(s) and may have a substituent(s)" represented by $R^3$ means "C3-15 monocyclic or fused carbocyclic ring", "bridged polycyclic carbocyclic ring" and "spiro-bound polycyclic carbocyclic ring" of the above "cyclic group".

In the present specification, the "heterocyclic ring group" in the "heterocyclic ring group which may be substituted with a hydroxyl group which may be protected by a protective group, a carboxyl group which may be protected by a protective group, or a sulfo group which may be protected by a protective group, and also may have a substituent(s)" or "C1-4 aliphatic hydrocarbon group which may be substituted with a hydroxyl group which may be protected by a protective group, a carboxyl group which may be protected by a protective group, or a sulfo group which may be protected by a protective group, and is also substituted with a heterocyclic ring group which may have a substituent(s) and may have a substituent(s)" represented by $R^3$ means "3- to 15-membered monocyclic or fused heterocyclic ring having 1 to 4 nitrogen atom(s), 1 to 2 oxygen atom(s) and/or 1 to 2 optionally oxidized sulfur atom(s) as heteroatoms", "bridged polycyclic heterocyclic ring" and "spiro-bound polycyclic heterocyclic ring" of the above "cyclic group".

In the present specification, the "substituent" in the "C1-4 aliphatic hydrocarbon group which may be substituted with a hydroxyl group which may be protected by a protective group, a carboxyl group which may be protected by a protective group or an optionally substituted sulfo group, and also may have a substituent(s)", "carbocyclic ring group which may be substituted with a hydroxyl group which may be protected by a protective group, a carboxyl group which may be protected by a protective group, or a sulfo group which may be protected by a protective group, and also may have a substituent(s)", "heterocyclic ring group which may be substituted with a hydroxyl group which may be protected by a protective group, a carboxyl group which may be protected by a protective group, or a sulfo group which may be protected by a protective group, and also may have a substituent(s)", "C1-4 aliphatic hydrocarbon group which may be substituted with a hydroxyl group which may be protected by a protective group, a carboxyl group which may be protected by a protective group, or a sulfo group which may be protected by a protective group, and is also substituted with a carbocyclic ring group which may have a substituent(s) and may have a substituent(s)", "C1-4 aliphatic hydrocarbon group which may be substituted with a hydroxyl group which may be protected by a protective group, a carboxyl group which may be protected by a protective group, or a sulfo group which may be protected by a protective group, and is also substituted with a heterocyclic ring which may have a substituent(s) and may have a substituent(s)", "-(divalent C1-4 aliphatic hydrocarbon group which may have a substituent(s))-(divalent carbocyclic ring which may have a substituent(s))-$(Y^3)_t$-Z", or "-(divalent C1-4 aliphatic hydrocarbon group which may have a substituent(s))-(divalent heterocyclic ring which may have a substituent(s)-$(Y^3)_t$-Z" represented by $R^3$ means (1) to (8), (22) to (25), (33) to (36) and (51) to (53) of substituents exemplified as for L. These optional substituent(s) may be substituted on the substitutable position in the number of 1 to 4.

In the present specification, the "divalent C1-4 aliphatic hydrocarbon group" of the "divalent C1-4 aliphatic hydrocarbon group which may have a substituent(s)" in the "-(divalent C1-4 aliphatic hydrocarbon group which may have a substituent(s))-(divalent carbocyclic ring which may have a substituent(s))-$(Y^3)_t$-Z", or "-(divalent C1-4 aliphatic hydrocarbon group which may have a substituent(s))-(divalent heterocyclic ring which may have a substituent(s))-$(Y^3)_t$-Z" represented by $R^3$ has the same meaning as that of the "divalent C1-4 aliphatic hydrocarbon group" in the "divalent C1-4 aliphatic hydrocarbon group which may have a substituent(s)" in $E^1$.

In the present specification, the "divalent carbocyclic ring" of the "divalent carbocyclic ring which may have a substituent(s)" in the "-(divalent C1-4 aliphatic hydrocarbon group which may have a substituent(s))-(divalent carbocyclic ring which may have a substituent(s))-$(Y^3)_t$-Z" represented by $R^3$ includes, for example, divalent group which can be obtained by removing optional two hydrogen atoms from "C3-15 monocyclic or fused carbocyclic ring", "bridged polycyclic carbocyclic ring", or "spiro-bound polycyclic carbocyclic ring" of the above "cyclic group". These optional two hydrogen atoms may be combined with the same carbon atom or different carbon atoms, and are preferably combined with different carbon atoms.

In the present specification, the "divalent heterocyclic ring" of the "divalent heterocyclic ring which may have a substituent(s)" in the "-(divalent C1-4 aliphatic hydrocarbon group which may have a substituent(s))-(a divalent heterocyclic ring which may have a substituent(s))-$(Y^3)_t$-Z" represented by $R^3$ includes, for example, divalent group which can be obtained by removing optional two hydrogen atoms from "3- to 15-membered monocyclic or fused heterocyclic ring having 1 to 4 nitrogen atom(s), I to 2 oxygen atom(s) and/or 1 to 2 optionally oxidized sulfur atom(s) as heteroatoms", "bridged polycyclic heterocyclic ring", or "spiro-bound polycyclic heterocyclic ring" of the above "cyclic group". These optional two hydrogen atoms may be combined with the same carbon atom or different carbon atoms, and are preferably combined with different carbon atoms.

In the present specification, the "protective group", "C1-4 aliphatic hydrocarbon group", "carbocyclic ring group", "substituent(s)" in the "C1-4 aliphatic hydrocarbon group which may be substituted with a hydroxyl group which may be protected by a protective group, a carboxyl group which may be protected by a protective group, or a sulfo group which may be protected by a protective group, and also may have a substituent(s)", "a carbocyclic ring group which may be substituted with a hydroxyl group which may be protected by a protective group, a carboxyl group which may be protected

by a protective group, a sulfo group which may be protected by a protective group, or halogen atom, and also may have a substituent(s)", or "C1-4 aliphatic hydrocarbon group which may be substituted with a hydroxyl group which may be protected by a protective group, a carboxyl group which may be protected by a protective group, a sulfo group which may be protected by a protective group, or a halogen atom, and is also substituted with a carbocyclic ring group which may have a substituent(s) and may have a substituent(s)" represented by $R^{30}$ have the same meanings as those of "protective group", "C1-4 aliphatic hydrocarbon group", "carbocyclic ring group" and "substituent(s)" defined in $R^3$.

In the present specification, the "hydroxyl group which may be protected by a protective group", "carboxyl group which may be protected by a protective group", or "sulfo group which may be protected by a protective group" represented by Z have the same meanings as those of "hydroxy group which may be protected by a protective group", "carboxyl group which may be protected by a protective group" and "sulfo group which may be protected by a protective group" defined in $R^3$.

In the present specification, "in a prodrug modification" in the "hydroxyl group in a prodrug modification", "carboxyl group in a prodrug modification", or "sulfo group in a prodrug modification" represented by Z means that the structure is chemically modified for the purpose of improving solubility, alimentary canal mucosa permeability, migration in blood, and tissue migration of the compound, thereby improving bioavailability as a drug, or improving properties as problems caused upon oral administration, such as bitterness and irritation of the compound.

In the present specification, the "hydroxyl group in a prodrug modification", "carboxyl group in a prodrug modification", or "sulfo group in a prodrug modification" may have any structure as long as it is converted into an original hydroxyl group, carboxyl group or sulfo group by the reaction with an enzyme such as hydrolase or oxidoreductase, or gastric acid in vivo, or under physiological conditions.

In the present specification, the "divalent C1-4 aliphatic hydrocarbon group which may have a substituent(s)" represented by $Y^1$ and $Y^2$ has the same meaning as that of the "divalent C1-4 aliphatic hydrocarbon group which may have a substituent(s)" defined in $R^3$.

In the present specification, the "substituent" in the "methylene group which may have a substituent(s)" and "ethenylene group which may have a substituent(s)" represented by $Y^3$ includes, for example, (1) to (8), (22) to (25), (33) to (36) and (51) to (53) among substituents exemplified as for L. These optional substituents may be substituted on the substitutable position in the number of 1 to 2. When $Y^3$ represents "methylene group which may have a substituent (s)" and two substituents are aliphatic hydrocarbon groups, two substituents may be combined with carbon atoms to which they are attached to form a C3-8 saturated monocyclic carbocyclic ring (for example, cyclopropane, cyclobutane, cyclopentane, cyclohexane, cycloheptane, cyclooctane, etc.).

In the present specification, the "divalent nitrogen atom which may have a substituent(s)" represented by $Y^3$ has the same meaning as that of the "divalent nitrogen atom which may have a substituent(s)" defined as for $G^1$, $G^2$ and $G^3$.

In the present specification, the "divalent C3-15 carbocyclic ring which may have a substituent(s)" represented by M has the same meaning as that of the "divalent carbocyclic ring which may have a substituent(s)" defined in $R^3$.

In the present specification, the "divalent 5- to 6-membered heterocyclic ring consisting of carbon atom, oxygen atom and/or an optionally oxidized sulfur atom" of the "divalent 5- to 6-membered heterocyclic ring consisting of carbon atom, oxygen atom and/or an optionally oxidized sulfur atom which may have a substituent(s)" represented by M includes, for example, divalent group which can be obtained by removing optional two hydrogen atoms from a 5- to 6-membered heterocyclic ring consisting of a carbon atom, an oxygen atom and/or an optionally oxidized sulfur atom. These optional two hydrogen atoms may be combined with the same carbon atom or different carbon atoms, and are preferably combined with different carbon atom. Examples of the "5- to 6-membered heterocyclic ring consisting of a carbon atom, an oxygen atom and/or an optionally oxidized sulfur atom" include furan, thiophene, dihydrofuran, tetrahydrofuran, dihydrothiophene, tetrahydrothiophene, dioxolane, dithiolane, dihydropyran, tetrahydropyran, dihydrothiopyran, tetrahydrothiopyran, oxathiane, dioxane, and dithiane rings.

In the present specification, the "substituent" of the "divalent 5- to 6-membered heterocyclic ring consisting of carbon atom, oxygen atom and/or an optionally oxidized sulfur atom which may have a substituent(s)" represented by M includes, for example, (1) to (8), (22) to (25), (33) to (36) and (51) to (53) among substituents exemplified as for L. These optional substituents may be substituted on the substitutable position in the number of 1 to 4.

In the present specification, the "substituent" of the "imidazole ring which may have a substituent(s)", "benzoimidazole ring which may have a substituent(s)", "6,7-dihydro-5H-cyclopenta[b]pyridine ring which may have a substituent(s)", "5,6,7,8-tetrahydroquinoline ring which may have a substituent(s)", "6,7,8,9-tetrahydro-5H-cyclohepta[b] pyridine ring which may have a substituent(s)", or "imidazo[1,2-a]pyridine ring which may have a substituent(s)" represented by $A^{1a}$ is not particularly limited. The substituents includes, for example, (1) aliphatic hydrocarbon group which may have a substituent(s) (2) cyclic group which may have a substituent(s), (3) aliphatic hydrocarbon group substituted with a cyclic group which may have a substituent(s), and (4) substituents exemplified as for L. The "aliphatic hydrocarbon group" and "cyclic group" in the "aliphatic hydrocarbon group which may have a substituent(s)", "cyclic group which may have a substituent(s)" and "aliphatic hydrocarbon group substituted with a cyclic group which may have a substituent (s)" have the same meanings as described above and the "substituent" in (1) to (3) includes, for example, substituents

exemplified as for L. These optional substituent(s) may be substituted on the substitutable position in the number of 1 to 5.

In the present specification, the "substituent" of the "methylene group which may have a substituent(s)" represented by $E^{1a}$ has the same meaning as that in the "substituent" of the "divalent C1-4 aliphatic hydrocarbon group which may have a substituent(s)" represented by $E^1$. These optional substituent(s) may be substituted on the substitutable position in the number of 1 to 2.

In the present specification, the "divalent C3-8 monocyclic carbocyclic ring" in the "divalent C3-8 monocyclic carbocyclic ring which may have a substituent(s)" represented by ring $D^a$ includes, for example, divalent group which can be obtained by removing optional two hydrogen atoms from the "C3-8 monocyclic carbocyclic ring" in the "divalent monocyclic cyclic group which may have a substituent(s)" represented by ring D. Optional two hydrogen atoms may be combined with the same carbon atom or different carbon atoms, and are preferably combined with different carbon atoms. The "substituent" here has the same meaning as that in the "substituent" of the "divalent monocyclic cyclic group which may have a substituent(s)" represented by ring D.

In the present specification, the "imidazole ring which may have a substituent(s)" represented by $A^{1b}$ has the same meaning as that of the "imidazole ring which may have a substituent(s)" in $A^{1a}$.

In the present specification, the "divalent C1-4 aliphatic hydrocarbon group" in the "divalent C1-4 aliphatic hydrocarbon group which may have a substituent(s)" represented by $G^b$ has the same meaning as that of the "divalent C1-4 aliphatic hydrocarbon group" in the "divalent C1-4 aliphatic hydrocarbon group which may have a substituent(s)" represented by $E^1$. The "substituent" here has the same meaning as that of the "substituent" in the "methylene group which may have a substituent(s)" and "ethenylene group which may have a substituent(s)" represented by $G^1$, $G^2$ and $G^3$. These optional substituent(s) may be substituted on the substitutable position in the number of 1 to 5.

In the present specification, the "substituent" in

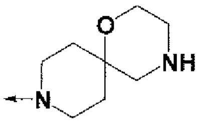 , 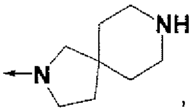 , 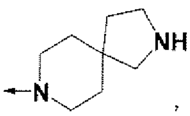 , 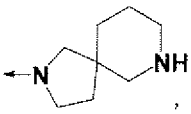 , 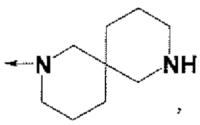 ,

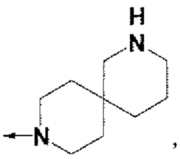 , 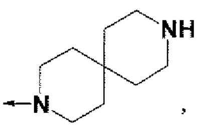 , 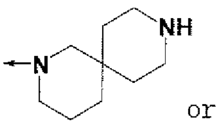 or 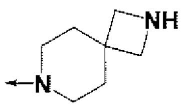

(wherein a nitrogen atom of -NH- may have a substituent(s)) which may have a substituent represented by formula:

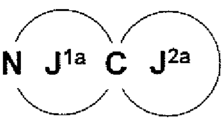

is not particularly limited. Examples of the substituent include (1) aliphatic hydrocarbon group which may have a substituent(s) (2) cyclic group which may have a substituent(s), (3) aliphatic hydrocarbon group substituted with a cyclic group which may have a substituent(s), and (4) substituents exemplified as for L. The "aliphatic hydrocarbon group" and "cyclic group" in the "aliphatic hydrocarbon group which may have a substituent(s)", "cyclic group which may have a substituent(s)" and "aliphatic hydrocarbon group substituted with a cyclic group which may have a substituent(s)" have the same meanings as described above, and the "substituent" in (1) to (3) includes, for example, substituents exemplified as for L. These optional substituents may be substituted on the substitutable position. The number of substituents is preferably from 1 to 5, and more preferably from 1 to 2.

In the present specification, the "divalent C5-10 aromatic carbocyclic ring" in the "divalent C5-10 aromatic carbocyclic ring which may have a substituent(s)" represented by ring $M^a$ includes, for example, divalent group which can be obtained by removing optional two hydrogen atoms from the "C5-10 aromatic carbocyclic ring". Optional two hydrogen atoms may be combined with the same atom or different atoms, and are preferably combined with different atoms. Examples of the "C5-10 aromatic carbocyclic ring" here include benzene, azulene, and naphthalene.

In the present specification, the "divalent C1-4 aliphatic hydrocarbon group" in the "divalent C1-4 aliphatic hydrocarbon group which may have a substituent(s)" represented by $Y^{3a}$ has the same meaning as that of the "divalent C1-4 aliphatic hydrocarbon group" in the "divalent C1-4 aliphatic hydrocarbon group which may have a substituent(s)" represented by $E^1$.

In the present specification, the "substituent" in the "divalent C1-4 aliphatic hydrocarbon group which may have a substituent(s)" represented by $Y^{3a}$ has the same meaning as that of the "substituent" in the "ethylene group which may have a substituent(s)" and "ethenylene group which may have a substituent(s)" represented by $Y^3$. These optional substituent(s) may be substituted on the substitutable position in the number of 1 to 5.

In the present specification, the "divalent C1-3 aliphatic hydrocarbon group" of the "divalent C1-3 aliphatic hydrocarbon group which may have a substituent(s)" in the "← O-(divalent C1-3 aliphatic hydrocarbon group which may have a substituent(s) →" represented by $Y^{3a}$ includes, for example, C1-3 alkylene group (for example, $-CH_2-$, $-(CH_2)_2-$, $-(CH_2)_3-$, etc.), C2-3 alkenylene group (for example, $-CH=CH-$, $-CH_2-CH=CH-$, $-CH=CH-CH_2-$, etc.), C2-3 alkynylene group (for example, $-C\equiv C-$, $-CH_2-C\equiv C-$, $-C\equiv C-CH_2-$, etc.). The "substituent" of the "divalent C1-3 aliphatic hydrocarbon group which may have a substituent(s)" has the same meaning as that of the "substituent" in the "ethylene group which may have a substituent(s)" and "ethenylene group which may have a substituent(s)" represented by $Y^3$. These optional substituents may be substituted on the substitutable position in the number of 1 to 5.

In the present specification, the "carboxyl group which may be protected by a protective group" represented by $Z^a$ has the same meaning as that of the "carboxyl group which may be protected by a protective group" represented by Z.

In the present specification, the "C1-4 aliphatic hydrocarbon group" as the "protective group" in the "carboxyl group which may be protected by a protective group" represented by $Z^a$ and the "C1-4 aliphatic hydrocarbon group" in the "C1-4 aliphatic hydrocarbon group substituted with a mono- or di-substituted carbamoyl group" have the same meaning as that of the "C1-4 aliphatic hydrocarbon group". The "mono- or di-substituted carbamoyl group" in the "C1-4 aliphatic hydrocarbon group substituted with a mono- or di-substituted carbamoyl group" has the same meaning as that of the "mono- or di-substituted carbamoyl group" defined in L.

In the present specification, the "substituent" represented by $R^{4a}$, $R^{4b}$, $R^{4c}$, or $R^{4d}$ is not particularly limited. The "substituent" here includes, for example, substituents exemplified as for L.

In the present invention, all isomers are included unless otherwise specified. For example, alkyl group, alkenyl group, alkynyl group, alkylene group, alkenylene group, alkynylene group, alkylidene group and the like include those which are linear and branched. Furthermore, all of isomers (E-, Z-, cis-, and trans-isomers) on the double bond, ring and fused ring, isomers (R-isomer, S-isomer, α, β configuration, enantiomer, and diastereomer) due to the presence of a symmetric carbon, optically active substances with optical rotation (D-, L-, d-, and 1-compounds), polar compounds (high polar compound and low polar compound) generated by chromatographic separation, equilibrium compounds, rotational isomers, mixtures in an optional mixing ratio and racemic mixtures are included in the present invention.

[Salts and Solvates]

Salts of the compound represented by formula (I) include all of nontoxic salts and pharmaceutically acceptable salts. The pharmaceutically acceptable salt is preferably a water soluble salt which shows less toxicity. Examples of the suitable salt of the compound represented by formula (I) include salts of alkali metal (potassium, sodium, lithium, etc.), salts of alkali earth metal (calcium, magnesium, etc.), ammonium salts (tetramethylammonium salt, tetrabutylammonium salt, etc.), salts of organic amine (triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, etc.), acid addition salts [inorganic acid salts (hydrochloride, hydrobromide, hydroiodide, sulfate, phosphate, nitrate, etc.), and organic acid salts (acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate, etc.)] and the like.

Furthermore, salts include quaternary ammonium salts. The quaternary ammonium salt is obtained by quaternizing a nitrogen atom of the compound represented by formula (I) with $R^0$ group ($R^0$ group represents a C1-8 alkyl group, or a C1-8 alkyl group substituted with a phenyl group).

The compound of the present invention can be converted into N-oxide by an optional method. N-oxide is obtained by oxidizing a nitrogen atom of the compound represented by formula (I).

Examples of suitable solvate of the compound represented by formula (I) include solvates such as water, alcoholic solvent (for example, methanol, ethanol, etc.) and the like. The solvate is preferably nontoxic and water soluble. The solvate of the compound of the present invention also includes solvates of alkali metal salts, alkali earth metal salts, ammonium salts, salts of organic amine, and acid addition salts of the compound of the present invention.

The compound of the present invention can be converted into the above salts, N-oxides and solvates by a known method.

[Prodrug]

A prodrug in the compound represented by formula (I), a salt thereof, an N-oxide thereof or a solvate thereof, or a prodrug thereof means a compound which is converted into the compound represented by formula (II):

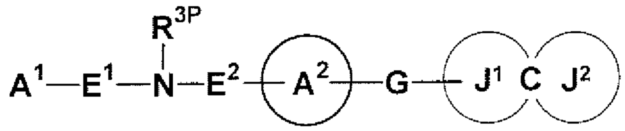

(II)

(wherein $R^{3P}$ represents (1) a C1-4 aliphatic hydrocarbon group which is substituted with a hydroxyl group, a carboxyl group or sulfo group, and may also have a substituent(s), (2) a carbocyclic ring group which is substituted with a hydroxyl group, a carboxyl group or a sulfo group, and also may have a substituent(s), (3) a heterocyclic ring group which is substituted with a hydroxyl group, a carboxyl group or a sulfo group, and also may have a substituent(s), (4) a C1-4 aliphatic hydrocarbon group which is substituted with a hydroxyl group, a carboxyl group or a sulfo group, and is also substituted with a carbocyclic ring group and may have a substituent(s), (5) a C1-4 aliphatic hydrocarbon group which is substituted with a hydroxyl group, a carboxyl group, or a sulfo group, and is also substituted with a heterocyclic ring which may have a substituent(s) and may have a substituent(s), (6) -(divalent C1-4 aliphatic hydrocarbon group which may have a substituent(s))-(divalent carbocyclic ring which may have a substituent(s))-$(Y^3)_t$-$Z^P$, or (7) -(divalent C1-4 aliphatic hydrocarbon group which may have a substituent(s))-(divalent heterocyclic ring which may have a substituent(s))-$(Y^3)_t$-$Z^P$, $Z^P$ represents a hydroxyl group, a carboxyl group, or sulfo group, and other symbols have the same meanings as described above), by the reaction with an enzyme or gastric acid in vivo. The prodrug of the compound represented by formula (I) includes, for example, a compound of formula (I)
wherein the end of $R^3$ is substituted with a hydroxyl group protected by a protective group, a carboxyl group protected by a protective group, or a sulfo group protected by a protective group. In the present invention, examples of the prodrug of the compound represented by formula (I) include compound wherein an amino group is acylated, alkylated, or phosphorylated (for example, compound wherein an amino group of the compound represented by formula (I) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, tert-butylated, etc.) when the compound represented by formula (I) has an amino group; compound wherein a hydroxyl group is acylated, alkylated, phosphorylated, or boricated (for example, compound wherein a hydroxyl group of the compound represented by formula (I) is acetylated, palmitoylated, propanoylated, pivaloylated, succinylated, fumarylated, alanylated, dimethylaminomethylcarbonylated, etc.) when the compound represented by formula (I) has a hydroxyl group; and compound wherein a carboxy group is esterificated, or amidated (for example, compound wherein a carboxy group of the compound represented by formula (I) is ethylesterificated, phenylesterificated, carboxymethylesterificated, dimethylaminomethylesterificated, pivaloyloxymethylesterificated, 1-{(ethoxycarbonyl)oxy}ethylesterificated, phthalidylesterificated, (5-methyl-2-oxo-1,3-dioxolen-4-yl)methylesterificated, 1-{[(cyclohexyloxy)carbonyl]oxy}ethylesterificated, methylamidated, etc.) when the compound represented by formula (I) has a carboxy group. These compounds can be prepared by a per se known method. Also, the prodrug of the compound represented by formula (I) may be converted into the compound represented by formula (I) under physiological conditions described in "Development of Drug" published in 1990 by Hirokawa Shoten, Vol. 7, "Molecular Design", pp.163-198. Furthermore, the compound represented by formula (I) may be labelled with isotope (for example, $^3H$, $^{14}C$, $^{35}S$, $^{125}I$, etc.) and the like.

Among the compound represented by formula (I) of the present invention, a salt thereof, an N-oxide thereof, a solvate thereof, or a prodrug thereof (hereinafter sometimes abbreviated to a compound of the present invention), a compound having a hydroxyl group protected by a protective group, a carboxyl group protected by a protective group or a sulfo group protected by a protective group of the present invention is excellent in solubility and oral absorption. Also a compound of the present invention obtained by converting the compound by an enzyme or gastric acid in vivo is a compound which maintains its pharmacological activity for a long period of time, and is also less likely to be inhibited by a drug metabolizing enzyme and has low toxicity. These properties are most important properties required when preparations are developed, described in "The Merck Manual of Diagnosis and Therapy (17th Ed.), Merck & Co.". The compound of the present invention satisfies these conditions and is expected to be useful for developing extremely excellent.

In the formula (I) of the present invention, any of each definition by $A^1$, ring $A^2$, $E^1$, $E^2$, G, $J^1$, $J^2$ and $R^3$ is preferred. In the following, preferable groups will be listed. The symbols used herein have the same meaning as described above.

In the present specification, $A^1$ is preferably a nitrogen-containing heterocyclic ring which may have a substituent(s). The "nitrogen-containing heterocyclic ring" of the "nitrogen-containing heterocyclic ring which may have a substituent (s)" is preferably a monocyclic or fused nitrogen-containing heterocyclic ring, more preferably a nitrogen-containing aromatic heterocyclic ring, still more preferably an imidazole, benzoimidazole, pyridine or pyrimidine ring, and most preferably an imidazole ring.

In the present specification, $A^{1a}$ is preferably an imidazole ring which may have a substituent(s) or a benzoimidazole ring which may have a substituent(s), more preferably an imidazole ring which may have a substituent(s), and particularly preferably:

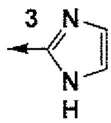

(wherein arrow 3 is bonded to $E^{1a}$ and a nitrogen atom of -NH- may have a substituent(s)), which may have a substituent (s).

In the present specification, the "substituent" of the "imidazole ring which may have a substituent(s)", "benzoimidazole ring which may have a substituent(s)", "6,7-dihydro-5H-cyclopenta[b]pyridine ring which may have a substituent(s)", "5,6,7,8-tetrahydroquinoline ring which may have a substituent(s)", "6,7,8,9-tetrahydro-5H-cyclohepta[b] pyridine which may have a substituent(s)", or "imidazo[1,2-a]pyridine ring which may have a substituent(s)" represented by $A^{1a}$ preferably, for example, an aliphatic hydrocarbon group which is not substituted or may have a substituent(s), an aliphatic hydrocarbon group substituted with a cyclic group which may have a substituent(s), or substituents exemplified as for L. The "substituent" in the "aliphatic hydrocarbon group which may have a substituent(s)" and "cyclic group which may have a substituent(s)" is preferably, for example, a carboxyl group or a -COO- aliphatic hydrocarbon group. The "cyclic group" in the "cyclic group which may have a substituent(s)" is preferably, for example, a benzene, furan or thiophene ring. The "substituent" exemplified as for L is preferably, for example, an aliphatic hydrocarbon group, a carboxyl group, or a -COO-aliphatic hydrocarbon group. The "substituent" is more preferably, for example, non-substitution, an aliphatic hydrocarbon group, a $-(CH_2)_{nA1}$-COOH, $-(CH_2)_{nA1}$-COO-aliphatic hydrocarbon group, $-(CH_2)_{nA1}$-(cyclic group)-COOH or $-(CH_2)_{nA1}$-(cyclic group)-COO- aliphatic hydrocarbon group, and most preferably non-substitution. The "aliphatic hydrocarbon group" and "-COO- aliphatic hydrocarbon group" have the same meanings as those defined in L and nA1 represents an integer of 1 to 4. nA1 is preferably 3 or 4. The "cyclic group" is preferably, for example, a benzene, furan or thiophene ring, and more preferably a benzene ring.

In the present specification, $A^{1b}$ is preferably:

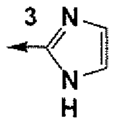

(wherein arrow 3 is bonded to $E^{1a}$ and a nitrogen atom of -NH- may have a substituent(s)), which may have a substituent (s).

In the present specification, the "substituent" in the "imidazole ring which may have a substituent(s)" represented by $A^{1b}$ is preferably, for example, non-substitution, an aliphatic hydrocarbon group which may have a substituent(s), an aliphatic hydrocarbon group substituted with a cyclic group which may have a substituent(s), or substituents exemplified as for L. The "substituent" in the "aliphatic hydrocarbon group which may have a substituent(s)" and "cyclic group which may have a substituent(s)" preferably include, for example, a carboxyl group, or an aliphatic hydrocarbon group substituted with a -COO- aliphatic hydrocarbon group. The "cyclic group" in the "cyclic group which may have a substituent (s)" is preferably, for example, a benzene, furan or thiophene ring. The "substituent" in the "imidazole ring which may have a substituent(s)" represented by $A^{1b}$ is more preferably, for example, non-substitution, $-(CH_2)_{nA1}$-COOH, $-(CH_2)_{nA1}$-COO-aliphatic hydrocarbon group, $-(CH_2)_{nA1}$-(cyclic group)-COOH, or $-(CH_2)_{nA1}$-(cyclic group)-COO- aliphatic hydrocarbon group, and most preferably non-substitution. The "aliphatic hydrocarbon group" and "-COO- aliphatic hydrocarbon group" have the same meaning as those defined in L. nA1 has the same meaning as described above. The "cyclic group" is preferably, for example, a benzene, furan or thiophene ring, and more preferably a benzene ring.

In the present specification, $R^1$ and $R^2$ include preferably, for example, a hydrogen atom and an aliphatic hydrocarbon group which may have a substituent(s). $R^1$ and $R^2$ may be the same or different.

In the present specification, ring $A^2$ is preferably a divalent 3- to 8-membered monocyclic heterocyclic ring which may have a substituent(s) and has 1 to 3 nitrogen atom(s), and also may have an oxygen atom and/or an optionally

oxidized sulfur atom. The "3- to 8-membered monocyclic heterocyclic ring" in the "3- to 8-membered monocyclic heterocyclic ring which has 1 to 3 nitrogen atom(s), and also may have an oxygen atom and/or an optionally oxidized sulfur atom" is preferably a 5- or 6-membered monocyclic aromatic heterocyclic ring, and more preferably an imidazole ring.

In the present specification, the "substituent" in the "divalent monocyclic cyclic group which may have a substituent(s)" represented by ring $A^2$ is preferably, for example, non-substitution or an aliphatic hydrocarbon group, and more preferably non-substitution.

In the present specification, $E^1$ is preferably, for example, methylene group which may have a substituent(s) or an ethylene group which may have a substituent(s), and more preferably a methylene group which may have a substituent(s). The "substituent" is preferably, for example, non-substitution or a methyl group, and more preferably non-substitution.

In the present specification, the "substituent" of the "methylene group which may have a substituent(s)" represented by $E^{1a}$ is preferably, for example, non-substitution or a methyl group, and more preferably non-substitution.

In the present specification, $E^2$ is preferably a methylene group.

In the present specification, the "monocyclic cyclic group" in the "divalent monocyclic cyclic group which may have a substituent(s)" represented by ring D is preferably a C3-8 monocyclic carbocyclic ring group, and more preferably benzene or cyclohexane. The "substituent" in the "divalent monocyclic cyclic group which may have a substituent(s)" is preferably, for example, non-substitution, an aliphatic hydrocarbon group which may have a substituent(s) or substituents exemplified as for L. The "substituent" in the "aliphatic hydrocarbon group which may have a substituent(s)" is preferably, for example, a carboxyl group and the substituent exemplified as for L is preferably, for example, an aliphatic hydrocarbon group, a carboxyl group or a hydroxyl group. The "substituent" in the "divalent monocyclic cyclic group which may have a substituent(s)" is preferably, for example, non-substitution, an aliphatic hydrocarbon group, a carboxyl group or a hydroxyl group, and most preferably, for example, non-substitution.

In the present specification, the "C3-8 monocyclic carbocyclic ring group" in the "divalent C3-8 monocyclic carbocyclic ring which may have a substituent(s)" represented by ring $D^a$ is preferably, for example, benzene or cyclohexane. The "substituent" in the "divalent C3-8 monocyclic carbocyclic ring which may have a substituent(s)" is preferably, for example, non-substitution, an aliphatic hydrocarbon group, a hydroxyl group, a carboxyl group or a hydroxyl group, and more preferably non-substitution.

In the present specification, $G^1$ in $-(G^1)_p-$ represented by G is preferably, for example, a methylene group which may have a substituent(s) or a nitrogen atom which may have a substituent(s), and more preferably a methylene group which may have a substituent(s). p is preferably an integer of 1 to 4. The "substituent" in the "methylene group which may have a substituent(s)" is preferably, for example, non-substitution, an aliphatic hydrocarbon group, an oxo group or a hydroxyl group, and more preferably non-substitution.

In the present specification, $G^2$ and $G^3$ in formula:

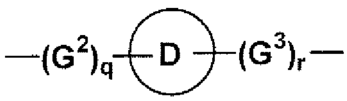

which is represented by G is preferably, for example, a methylene group which may have a substituent(s), a nitrogen atom which may have a substituent(s) or an oxygen atom, and more preferably, for example, a methylene group which may have a substituent(s) or a nitrogen atom which may have a substituent(s). The "substituent" is preferably, for example, non-substitution, an aliphatic hydrocarbon group which may have a substituent(s) or substituents exemplified as for L, and more preferably, for example, non-substitution, an aliphatic hydrocarbon group, an oxo group, a -COO-aliphatic hydrocarbon group or a hydroxyl group. q is preferably 0 or an integer of 1 to 2 and r is preferably an integer of 1 to 4. The sum of q and r is preferably 0 or an integer of 1 to 4. In the present specification,

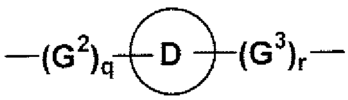

which is represented by G is preferably:

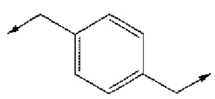 , 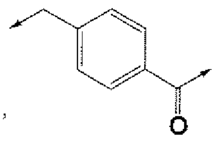 or 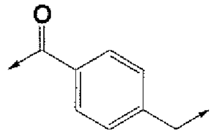

(wherein arrow directing the left is bonded to ring $A^2$ and arrow directing the right is bonded to

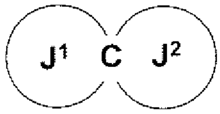 )

In the present specification, G is preferably $-(G^1)_p-$, and more preferably, for example, -(methylene group which may have a substituent(s))$_{pa}$-, wherein the "substituent" in the "methylene group which may have a substituent(s)" is preferably, for example, non-substitution or an aliphatic hydrocarbon group, and more preferably non-substitution.

In the present specification, $G^a$ is preferably, for example, $-(CH_2)_2-$, $-(CH_2)_3-$, - $(CH_2)_4-$, $-CH_2-C(O)-$, $-(CH_2)_2-C(O)-$, $-(CH_2)_3-C(O)-$, $-C(O)-CH_2-$, $-C(O)-(CH_2)_2-$, $-C(O)-(-CH_2)_3-$,

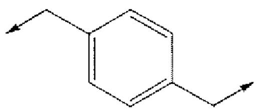 , 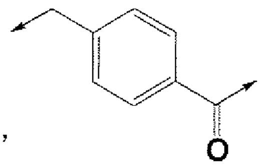 or 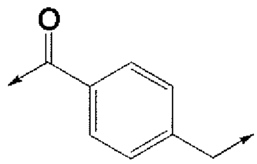

(wherein arrow directing the left is bonded to ring $A^2$, and arrow directing the right is bonded to

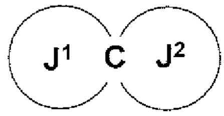 )

more preferably, for example, $-(CH_2)_2-$, $-(CH_2)_3-$ or $-(CH_2)_4-$, and most preferably $-(CH_2)_3-$.

In the present specification, pa is preferably an integer of 2 to 4, and more preferably 3.

In the present specification, qa is preferably, 1 or 2 and ra is preferably 1 or 2.

In the present specification, the "substituent" in the "divalent C1-4 aliphatic hydrocarbon group which may have a substituent(s)" represented by $G^b$ is preferably, for example, non-substitution, an aliphatic hydrocarbon group or an oxo group, and more preferably non-substitution.

In the present specification, $R^{4a}$ and $R^{4b}$ are preferably, for example, a hydrogen atom or an aliphatic hydrocarbon group, wherein the aliphatic hydrocarbon group is preferably, for example, methyl, ethyl, propyl or butyl.

In the present specification, $R^{4c}$ and $R^{4d}$ are preferably, for example, a hydrogen atom or an aliphatic hydrocarbon group, wherein the aliphatic hydrocarbon group is preferably, for example, methyl, ethyl, propyl or butyl. $R^{4c}$ and $R^{4d}$ are more preferably a hydrogen atom.

In the present specification,

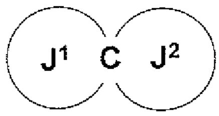

is preferably:

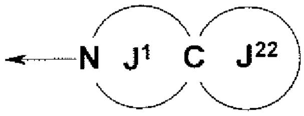

(wherein arrow directing the left is bonded to G and other symbols have the same meaning as described above), and more preferably:

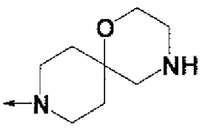 , 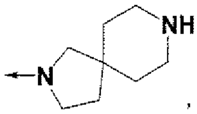 , 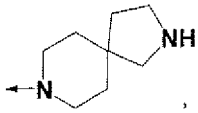 , 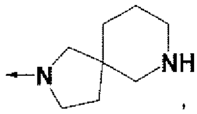 , 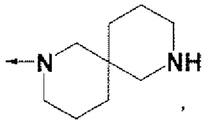 ,

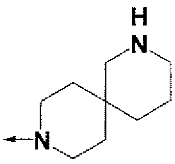 , 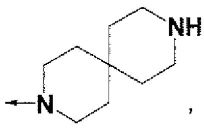 , 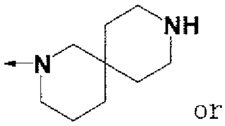 or 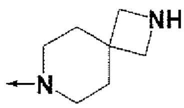

(wherein arrow is bonded to G and a nitrogen atom of -NH- may have a substituent(s)), which may have a substituent (s). The "substituent" is preferably, for example, non-substitution, an aliphatic hydrocarbon group which may have a substituent(s) or substituents exemplified as for L. The "substituent" in the "aliphatic hydrocarbon group which may have a substituent(s)" is preferably, for example, a carboxyl group or a -COO-aliphatic hydrocarbon group, and the "substituent exemplified as for L" is preferably, for example, an aliphatic hydrocarbon group, a carboxyl group or a hydroxyl group. The "substituent" is more preferably, for example, non-substitution, an aliphatic hydrocarbon group, a cyclic group, a carboxyl group or a hydroxyl group, more preferably, for example, an aliphatic hydrocarbon group or a cyclic group, and most preferably, for example, isobutyl, tert-butyl, 2,2-dimethylpropyl, cyclobutane, cyclopentane, cyclohexane or cycloheptane.

In the present specification,

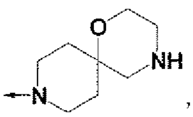 , 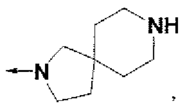 , 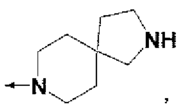 , 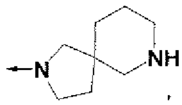 , 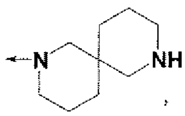 ,

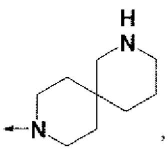 , 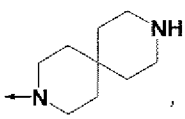 , 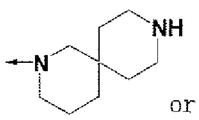 or 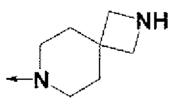

(wherein arrow is bonded to G and a nitrogen atom of -NH- represents a substituent(s)), which may have a substituent (s) represented by formula:

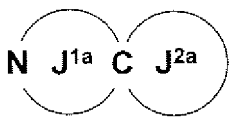

is preferably, for example,

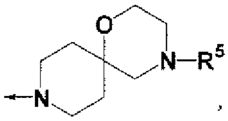 , 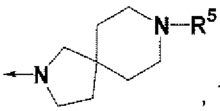 , 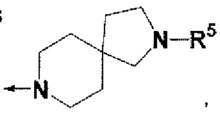 , 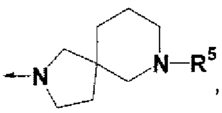 , 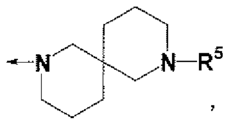 ,

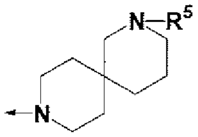 , 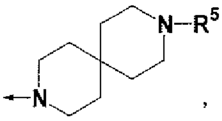 , 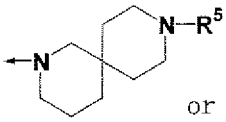 or 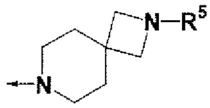

(wherein arrow is bonded to G and $R^5$ represent a substituent(s)), and more preferably:

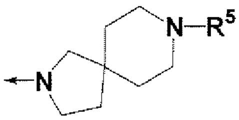

(wherein all symbols have the same meanings as described above). The "substituent" represented by $R^5$ includes, for example, an aliphatic hydrocarbon group which may have a substituent(s), an aliphatic hydrocarbon group substituted with a cyclic group which may have a substituent(s) or substituents exemplified as for L. The "aliphatic hydrocarbon group" and "cyclic group" in the "aliphatic hydrocarbon group which may have a substituent(s)" and "aliphatic hydrocarbon group substituted with a cyclic group which may have a substituent(s)" have the same meaning as described above and the "substituent" in (1) to (3) includes, for example, substituents exemplified as for L.

In the present specification, the "substituent" represented by $R^5$ is preferably, for example, substituents exemplified as for L. The "substituent exemplified as for L" is preferably, for example, an aliphatic hydrocarbon group or a cyclic group, and more preferably, for example, isobutyl, tert-butyl, 2,2-dimethylpropyl, cyclobutane, cyclopentane, cyclohexane or cycloheptane.

In the present specification, $R^3$ includes preferably, for example, a hydrogen atom, a C1-4 aliphatic hydrocarbon group which may have a substituent(s), a C1-4 aliphatic hydrocarbon group which is substituted with an optionally protected hydroxyl group, and also may have a substituent(s), or a C1-4 aliphatic hydrocarbon group which is substituted with an optionally protected carboxyl group, and also may have a substituent(s). $R^3$ is also preferably:

(a) $-Y^1-Z$ or

(b) 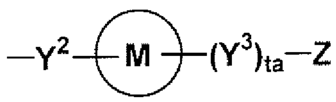

(wherein all symbols have the same meanings as described above).

In the present specification, the "substituent" in the "divalent C1-4 aliphatic hydrocarbon group which may have a substituent(s)" represented by $Y^1$ is preferably, for example, non-substitution or an aliphatic hydrocarbon group, and more preferably non-substitution.

In the present specification, $Y^2$ is preferably a divalent C1-4 aliphatic hydrocarbon group which may have a substituent(s). The "C1-4 aliphatic hydrocarbon group" is preferably, for example, methyl or ethyl. The "substituent" is preferably, for example, non-substitution, an oxo group or an aliphatic hydrocarbon group, and more preferably non-substitution.

In the present specification, ring M is preferably, for example, a benzene ring which may have a substituent (s), naphthalene ring which may have a substituent(s), a thiophene ring which may have a substituent(s) or a furan ring which may have a substituent(s), and more preferably a benzene ring which may have a substituent(s). The "substituent" in the "benzene ring which may have a substituent(s)", "naphthalene ring which may have a substituent(s)", "thiophene

ring which may have a substituent(s)" and "furan ring which may have a substituent(s)" is preferably, for example, non-substitution, an aliphatic hydrocarbon group, a halogen atom or an -O-aliphatic hydrocarbon group, and more preferably non-substitution or a halogen atom.

In the present specification, $Y^3$ is preferably, for example, a methylene group which may have a substituent (s), an ethenylene group which may have a substituent(s), -S-or -O-.

In the present specification, $-(Y^3)_{ta}-$ is preferably, for example, -S-(divalent C1-3 aliphatic hydrocarbon group which may have a substituent(s))-, -O-(divalent C1-3 aliphatic hydrocarbon group which may have a substituent(s))- or a divalent C1-4 aliphatic hydrocarbon group which may have a substituent(s), more preferably, for example, -O-(a divalent C1-3 aliphatic hydrocarbon group which may have a substituent(s))- or a divalent C1-4 aliphatic hydrocarbon group which may have a substituent(s), and most preferably a divalent C1-4 aliphatic hydrocarbon group which may have a substituent(s).

In the present specification, Z is preferably, for example, a carboxyl group which may be protected by a protective group or a hydroxyl group which may be protected by a protective group, and more preferably a carboxyl group which may be protected by a protective group.

In the present specification, the "C1-4 aliphatic hydrocarbon group" in the "C1-4 aliphatic hydrocarbon group which may have a substituent(s)" represented by $Y^{2a}$ is preferably $-CH_2-$. The substituent is preferably, for example, non-substitution or an oxo group, and more preferably non-substitution.

In the present specification, the "C5-10 aromatic carbocyclic ring" of the "divalent C5-10 aromatic carbocyclic ring which may have a substituent(s)" represented by ring $M^a$ is preferably, for example, a benzene or naphthalene ring, and more preferably a benzene ring. The "substituent" in the "divalent C5-10 aromatic carbocyclic ring which may have a substituent(s)" is preferably, for example, non-substitution, an aliphatic hydrocarbon group or a halogen atom, and more preferably non-substitution or a halogen atom.

In the present specification, ring $M^a$ is preferably, for example, a thiophene ring which may have a substituent (s), a benzene ring which may have a substituent(s) or a naphthalene ring which may have a substituent(s), and more preferably a benzene ring which may have a substituent(s). The "substituent" in the "thiophene ring which may have a substituent(s)", "benzene ring which may have a substituent(s)" and "naphthalene ring which may have a substituent (s)" is preferably, for example, non-substitution, an aliphatic hydrocarbon group or a halogen atom, more preferably non-substitution or a halogen atom, and most preferably non-substitution.

In the present specification, the "C1-4 aliphatic hydrocarbon group" in the "C1-4 aliphatic hydrocarbon group which may have a substituent(s)" represented by $Y^{3a}$ is preferably, for example, $-CH_2-$, $-(CH_2)_2-$, $-(CH_2)_3-$ or -CH=CH-, and more preferably, for example, $-CH_2-$ or $-(CH_2)_2-$. The "substituent" is preferably, for example, non-substitution, an aliphatic hydrocarbon group or a C3-8 saturated monocyclic, carbocyclic ring, more preferably, for example, non-substitution, methyl, ethyl or cyclopropane, and most preferably non-substitution.

In the present specification, the "substituent" of the "divalent C1-3 aliphatic hydrocarbon group which may have a substituent(s)" in the "←O-(a divalent C1-3 aliphatic hydrocarbon group which may have a substituent(s)) →" represented by $Y^{3a}$ is preferably, for example, non-substitution, an aliphatic hydrocarbon group or a C3-8 saturated monocyclic carbocyclic ring, and more preferably, for example, non-substitution or cyclopropane.

In the present specification, as the "carboxyl group which may be protected by a protective group" represented by $Z^a$, both a carboxyl group and a carboxyl group protected by a protective group are preferable.

In the present specification, the "protective group" in the "carboxyl group which may be protected by a protective group" represented by $Z^a$ is preferably a C1-4 aliphatic hydrocarbon group, a C1-4 aliphatic hydrocarbon group substituted with an optionally substituted mono- or di-substituted carbamoyl group, a -(divalent C1-4 aliphatic hydrocarbon group)-CO- cyclic group, 1-{[(cyclohexyloxy)carbonyl]oxy}ethyl or a pivaloyloxymethyl group. The "C1-4 aliphatic hydrocarbon group" in the "C1-4 aliphatic hydrocarbon group" and "-(divalent C1-4 aliphatic hydrocarbon group)-CO-cyclic group" has the same meaning as that of the "C1-4 aliphatic hydrocarbon group". The "divalent C1-4 aliphatic hydrocarbon group" in the "-(divalent C1-4 aliphatic hydrocarbon group)-CO-cyclic group" has the same meaning as that of the "divalent C1-4 aliphatic hydrocarbon group" in the "divalent C1-4 aliphatic hydrocarbon group which may have a substituent(s)" represented by $E^1$. The "-CO-cyclic group" in the "-(divalent C1-4 aliphatic hydrocarbon group)-CO-cyclic group" has the same meaning as that of the "-CO-cyclic group" in L. The "-(divalent C1-4 aliphatic hydrocarbon group)-CO-cyclic group" is preferably, for example, a 2-oxo-2-pyrrolidin-1-ylethyl or 2-(morpholin-4-yl)-ethyl group. The "C1-4 aliphatic hydrocarbon group substituted with an optionally substituted mono- or di-substituted carbamoyl group" has the same meaning as that of the "C1-4 aliphatic hydrocarbon group substituted with an optionally substituted mono- or di-substituted carbamoyl group" as the "protective group" in the "carboxyl group which may be protected by a protective group" represented by $Z^a$. The "C1-4 aliphatic hydrocarbon group substituted with an optionally substituted mono- or di-substituted carbamoyl group" is preferably $-CH_2-C(O)-$(mono- or di-C1-6 alkylamino group). The "protective group" in the "carboxyl group which may be protected by a protective group" represented by $Z^a$ is preferably, for example, a C1-4 aliphatic hydrocarbon group or a C1-4 aliphatic hydrocarbon group substituted with an optionally substituted mono- or di-substituted carbamoyl group, and most preferably, for example, a C1-4 aliphatic hydrocarbon group or $-CH_2-C(O)-$(mo-

no- or di-C1-6 alkylamino group) (for example, 2-(diethylamino)-2-oxoethyl, 2-(dimethylamino)-2-oxoethyl, etc.).

In the present invention, a compound of formula (I), which has a combination of preferred groups described above, is preferred.

Among the compound represented by formula (I), preferred compound is a compound represented by formula (I-1):

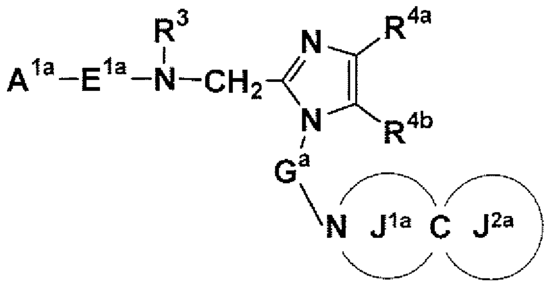

**(I-1)**

(wherein all symbols have the same meanings as described above), or formula (I-2):

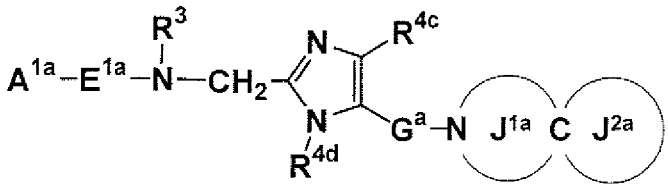

**(I-2)**

(wherein all symbols have the same meanings as described above), a salt thereof, an N-oxide thereof or a solvate thereof, and a prodrug thereof.

In the present specification, among the compound represented by formula (I), more preferred compound is a compound represented by formula (I-1-1):

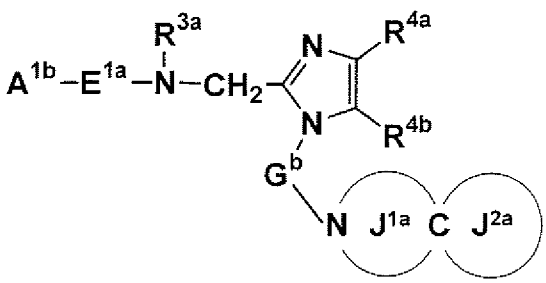

**(I-1-1)**

(wherein all symbols have the same meanings as described above), a salt thereof, an N-oxide thereof or a solvate thereof, or a prodrug thereof.

In the present specification, among the compound represented by formula (I), still more preferred compound is a compound represented by formula (I-1-1a):

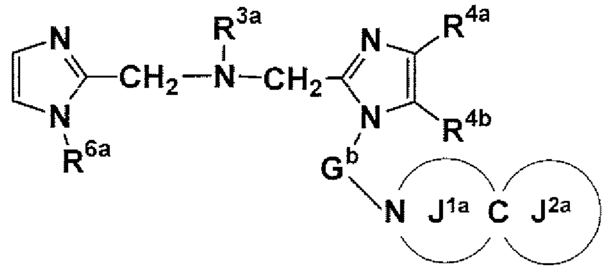

(I-1-1a)

(wherein $R^{6a}$ represents hydrogen atom or aliphatic hydrocarbon group and other symbols have the same meanings as described above), or formula (I-1-1b)

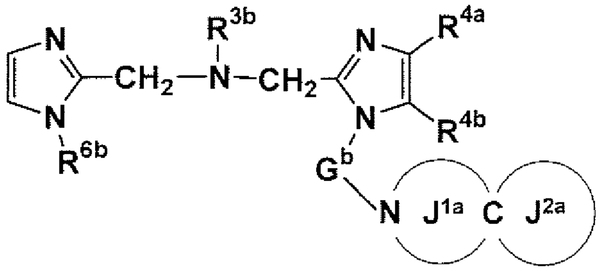

(I-1-1b)

(wherein $R^{3b}$ represents an aliphatic hydrocarbon group, $R^{6b}$ represents $-(CH_2)_{nA1}-COOH$, a $-(CH_2)_{nA1}-COO-$ aliphatic hydrocarbon group, $-(CH_2)_{nA1}$-(cyclic group)-COOH, or a-$(CH_2)_{nA1}$-(cyclic group)-COO- aliphatic hydrocarbon group, and other symbols have the same meaning as described above), a salt thereof, an N-oxide thereof or a solvate thereof, or a prodrug thereof.

In the present specification, the "aliphatic hydrocarbon group" represented by $R^{3b}$ has the same meaning as that of the "aliphatic hydrocarbon group".

In the present specification, the "aliphatic hydrocarbon group" in the "$-(CH_2)_{nA1}$-COO- aliphatic hydrocarbon group" and "$-(CH_2)_{nA1}$-(cyclic group)-COO- aliphatic hydrocarbon group" represented by $R^{6b}$ has the same meaning as that of "aliphatic hydrocarbon group".

In the present specification, the "COO- aliphatic hydrocarbon group," in the "$-(CH_2)_{nA1}$-COO- aliphatic hydrocarbon group" and "$-(CH_2)_{nA1}$-(cyclic group)-COO-aliphatic hydrocarbon group" represented by $R^{6b}$ has the same meaning as that of the "-COO- aliphatic hydrocarbon group" in L.

In the present specification, the "cyclic group" in the "$-(CH_2)_{nA1}$-(cyclic group)-COOH" and "$-(CH_2)_{nA1}$-(cyclic group)-COO- aliphatic hydrocarbon group" represented by $R^{6b}$ represents a benzene, thiophene or furan ring.

The compound of the present invention is the group of compounds which have strong antagonistic activity against CXCR4 and also have reduced basicity. Particularly, the group of compounds having a hydroxyl group, a sulfo group or a carboxyl group has reduced basicity. The group of compounds having a hydroxyl group protected by a protective group, a sulfo group protected by a protective group and a carboxyl group protected by a protective group is converted into the group having reduced basicity by an enzyme or gastric acid in vivo, which is the group of compounds having a low risk of side effects such as phospholipidosis, small distribution volume and high safety.

Examples of preferred compound of the present invention include compounds described in Examples, salts thereof, N-oxides thereof or solvates thereof, and prodrugs thereof.

Examples of more preferred compound of the present invention include: 4-{[(1-{3-[8-(1-ethylpropyl)-2,8-diazaspiro[4.5]deca-2-yl]propyl}-1H-imidazol-2-yl)methyl](1H-imidazol-2-ylmethyl)amino}butanoic acid, ethyl [4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenoxy]acetate, [4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenoxy]acetic acid, ethyl [3-({[(1-{3-[8-(1-ethylpropyl)-2,8-diazaspiro[4.5]deca-2-yl]propyl}-1H-imidazol-2-yl)methyl](1H-imidazo1-2-ylmethyl)amino}methyl)phenoxy]acetate, [3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenoxy]acetic acid, 2-(dimethylamino)-2-oxoethyl 4-{[(1-{3-[8-(1-ethylpropyl)-2,8-diazaspiro[4.5]deca-2-yl]propyl}-1H-imidazol-2-yl)methyl](1H-imidazol-2-ylmethyl)amino}butanoate, and 2-(diethylamino)-2-oxoethyl 4-{[(1-{3-[8-(1-ethylpropyl)-2,8-diazaspiro[4.5]deca-2-yl]propyl}-1H-imidazol-2-yl)methyl](1H-imidazol-2-ylmethyl)amino}butanoate, salts thereof, N-oxides thereof or solvates thereof, and prodrugs thereof.

[Method for Producing Compound of the Present Invention]

The compound of the present invention represented by formula (I) can be prepared by appropriately improving a known method, for example, methods shown below, methods described in Examples, and a method described in Comprehensive Organic Transformations: A Guide to Functional Group Preparations, Second edition (written by Richard C. Larock, John Wiley & Sons Inc, 1999) and using improved methods in combination. In the following production methods, starting compounds may be used in the form of a salt. As the salt, those described as a salt of the above described formula (I) are used.

Among the compound of the present invention represented by formula (I), a compound wherein $E^1$ represents a methylene group substituted with $R^{B1}$, $E^2$ represents a methylene group and $R^3$ represents $-CH_2-R^{B2}$, namely, a compound represented by formula (I-A):

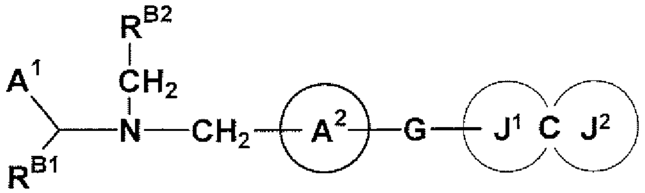
(I-A)

(wherein $R^{B1}$ represents a hydrogen atom, an aliphatic hydrocarbon group which may have a substituent(s), a cyclic group which may have a substituent(s), an aliphatic hydrocarbon group substituted with a cyclic group which may have a substituent(s), or substituents exemplified as for L; $R^{B2}-CH_2-$ represents a substituent which is combined with a nitrogen atom to which $R^3$ is attached via a methylene group among substituents represented by $R^3$; and other symbols have the same meanings as described above), can be prepared by subjecting a compound represented by formula (2):

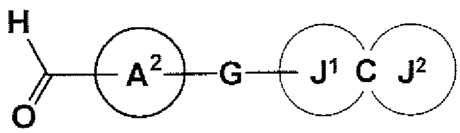
(2)

(wherein all symbols have the same meanings as described above), and a compound represented by formula (3):

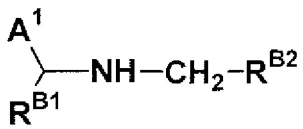
(3)

(wherein all symbols have the same meanings as described above), to the reductive amination reaction and optionally subjecting to the deprotection reaction of a protective group and/or the cleavage reaction from a resin; or subjecting a compound represented by formula (4):

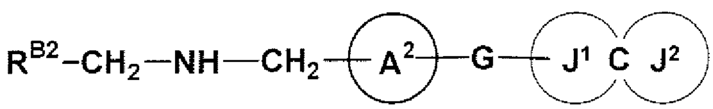
(4)

(wherein all symbols have the same meanings as described above), and a compound represented by formula (5):

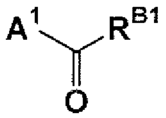

(5)

(wherein all symbols have the same meanings as described above), to the reductive amination reaction and optionally subjecting to the deprotection reaction of a protective group and/or the cleavage reaction from a resin; or subjecting a compound represented by formula (6):

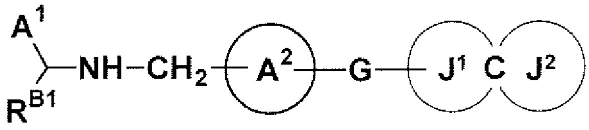

(6)

(wherein all symbols have the same meanings as described above), and a compound represented by formula (7):

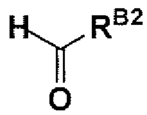

(7)

(wherein all symbols have the same meanings as described above), to the reductive amination reaction and optionally subjecting to the deprotection reaction of a protective group and/or the cleavage reaction from a resin.

This reductive amination reaction represent is known and is carried out, for example, in an organic solvent (tetrahydrofuran, diethylether, dichloroethane, dichloromethane, dimethylformamide, acetic acid, methanol, ethanol, or a mixture thereof) at a temperature of 0 to 40°C in the presence of a reducing agent (sodium triacetoxyborohydride, sodium cyanoborohydride, sodium borohydride, zinc borohydride, diisobutylalminium hydride, etc.), or carried out in a solvent (ether-based solvent (tetrahydrofuran, dioxane, dimethoxyethane, diethylether, etc.), alcohol-based solvent (methanol, ethanol, etc.), benzene-based solvent (benzene, toluene, etc.), nitrile-based solvent (acetonitrile, etc.), amide-based solvent (dimethylformamide, etc.), water, ethyl acetate, acetic acid, or a solvent mixture of two or more kinds of them) under a normal pressure or under a pressure in a hydrogen atmosphere at a temperature of 0 to 200°C in the presence of a catalyst (palladium-carbon, palladium black, palladium hydroxide, platinum oxide, Raney nickel, etc.).

The deprotection reaction of a protective group can be carried out by the following method.

Examples of the protective group of the carboxyl group include methyl group, ethyl group, allyl group, tert-butyl group, trichloroethyl group, benzyl (Bn) group, and phenacyl group.

Examples of the protective group of the hydroxyl group include methyl group, trityl group, methoxymethyl (MOM) group, 1-ethoxyethyl (EE) group, methoxyethoxymethyl (MEM) group, 2-tetrahydropyranyl (THP) group, trimethylsilyl (TMS) group, triethylsilyl (TES) group, tert-butyldimethylsilyl (TBDMS) group, tert-butyldiphenylsilyl (TBDPS) group, acetyl(Ac) group, pivaloyl group, benzoyl group, benzyl (Bn) group, p-methoxybenzyl group, allyloxycarbonyl (Alloc) group, and 2,2,2-trichloroethoxycarbonyl (Troc) group.

Examples of the protective group of the amino group include benzyloxycarbonyl group, t-butoxycarbonyl group, allyloxycarbonyl (Alloc) group, 1-methyl-1-(4-biphenyl)ethoxycarbonyl (Bpoc) group, trifluoroacetyl group, 9-fluorenylmethoxycarbonyl group, N,N-dimethylaminosulfamoyl group, benzyl(Bn) group, p-methoxybenzyl group, benzyloxymethyl (BOM) group, and 2-(trimethylsilyl)ethoxymethyl (SEM) group.

Examples of the protective group of the mercapto group include benzyl group, methoxybenzyl group, methoxymethyl (MOM) group, 2-tetrahydropyranyl (THP) group, diphenylmethyl group, and acetyl (Ac) group.

Other protective groups of the carboxyl group, hydroxyl group, amino group or mercapto group can be used without any limitation as long as they are groups capable of being eliminated easily and selectively. For example, those described in T. W. Greene, Protective Groups in Organic Synthesis, Wiley, New York, 1999 can be used.

The deprotection reaction of the protective group of the carboxyl group, hydroxyl group, amino group or mercapto group is well known and includes:

(1) deprotection reaction through alkali hydrolysis,
(2) deprotection reaction under acidic conditions,
(3) deprotection reaction through hydrolysis,

(4) deprotection reaction of silyl group,
(5) deprotection reaction using metal, and
(6) deprotection reaction using metal complex.

These methods are described in detail below.

(1) The deprotection reaction through alkali hydrolysis is carried out, for example, in an organic solvent (methanol, tetrahydrofuran, dioxane, etc.) at 0°C to a reflux temperature of the solvent by using a hydroxide (sodium hydroxide, potassium hydroxide, lithium hydroxide, etc.) of alkali metal, a hydroxide (barium hydroxide, calcium hydroxide, etc.) of alkali earth metal or a carbonate (sodium carbonate, potassium carbonate, etc.), or an aqueous solution thereof or a mixture of them.
(2) The deprotection reaction under acidic conditions is carried out, for example, in an organic solvent (dichloromethane, chloroform, dioxane, ethyl acetate, anisole, etc.), an organic acid (acetic acid, trifluoroacetic acid, methanesulfonic acid, p-tosilic acid, etc.), or an inorganic acid (hydrochloric acid, sulfuric acid, etc.) or a mixture (hydrogen bromide/acetic acid, etc.) thereof at 0°C to a reflux temperature of the solvent.
(3) The deprotection reaction through hydrolysis is carried out, for example, in a solvent (ether-based solvent (tetrahydrofuran, dioxane, dimethoxyethane, diethylether, etc.), alcohol-based solvent (methanol, ethanol, etc.), benzene-based solvent (benzene, toluene, etc.), ketone-based solvent (acetone, methyl ethyl ketone, etc.), nitrile-based solvent (acetonitrile, etc.), amide-based solvent (dimethylformamide, etc.), water, ethyl acetate, acetic acid, or a solvent mixture of two or more kinds of them, etc.) under normal pressure or under a pressure in a hydrogen atmosphere in the presence of a catalyst (palladium-carbon, palladium black, palladium hydroxide, platinum oxide, Raney nickel, etc.), or in the presence of ammonium formate at 0°C to a reflux temperature of the solvent.
(4) The deprotection reaction of a silyl group is carried out, for example, in an organic solvent (tetrahydrofuran, acetonitrile, etc.) which is miscible with water at a temperature of 0 to 40°C by using tetrabutylammonium fluoride or hydrogen fluoride.
(5) The deprotection reaction using metal is carried out, for example, in an acidic solvent (acetic acid, buffer of pH 4.2 to 7.2, or a mixed solution of a solution thereof and an organic solvent such as tetrahydrofuran) at a temperature of 0 to 40°C in the presence of a zinc powder while optionally applying ultrasonic wave.
(6) The deprotection reaction using a metal complex is carried out, for example, in an organic solvent (dichloromethane, dimethylformamide, tetrahydrofuran, ethyl acetate, acetonitrile, dioxane, ethanol, etc.), water or a solvent mixture thereof at a temperature of 0 to 40°C in the presence of a trapping reagent (tributyltin hydride, triethylsilane, dimedone, morpholine, diethylamine, pyrrolidine, etc.), an organic acid (acetic acid, formic acid, 2-ethylhexanoic acid, etc.) and/or an organic acid salt (sodium 2-ethylhexanoate, potassium 2-ethylhexanoate, etc.) in the presence or absence of a phosphine-based reagent (triphenylphosphine, etc.) by using a metal complex (tetrakistriphenylphosphine palladium(0), bis(triphenylphosphine)palladium(II) dichloride, palladium(II) acetate, tris(triphenylphosphine)rhodium(I) chloride, etc.).

The deprotection reaction can also be carried out, for example, by the method described in T. W. Greene, Protective Groups in Organic Synthesis, Wiley, New York, 1999.
As is apparent to those skilled in the art, the objective compound of the present invention can be easily prepared by selecting these deprotection reactions.
If the compound has a moiety to bind to a resin in the molecule and the resin is a polystyrene resin, the compound of the present invention can be cleaved from the resin by the following method. The reaction for cleavage from the resin is known and can be carried out, for example, by reacting in an organic solvent (dichloromethane, 1,2-dichloroethane, toluene, etc.) at 0 to 100°C by using an acid (acetic acid, trifluoroacetic acid, hydrochloric acid, etc.).
The compound represented by formula (I-A) can be prepared by subjecting a compound represented by formula (6) and a compound represented by formula (8):

$$X^1\text{-}CH_2\text{-}R^{B2} \qquad (8)$$

(wherein $X^1$ represents halogen atom and other symbols have the same meanings as described above), to the alkylation reaction.
This alkylation reaction is known and is carried out, for example, in an organic solvent (for example, dimethylformamide, dimethyl sulfoxide, etc.) at a temperature of about 0 to 150°C in the presence or absence of an alkali (potassium carbonate, sodium carbonate, triethylamine, etc.) and sodium iodide or potassium iodide.
This reaction is preferably carried out under an inert gas (argon, nitrogen, etc.) atmosphere under anhydrous conditions.
Among the compound of the present invention represented by formula (I), a compound wherein $R^3$ is represented

by -$Y^A$-$COOR^{D1}$, namely, a compound represented by formula (I-B):

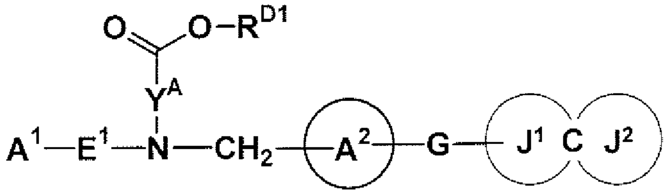

(I-B)

(wherein $R^{D1}$ represents a protective group of a carboxyl group, $Y^A$ represents a group having the which may be substituted with a carboxyl group protected by a protective group among substituents represented by $R^3$, and other symbols have the same meanings as described above), can be prepared by subjecting a compound represented by formula (9):

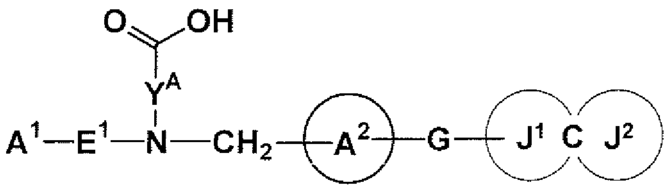

(9)

(wherein all symbols have the same meanings as described above), and a compound represented by formula (10):

$R^{D1}$-OH (10)

(wherein all symbols have the same meanings as described above), to the esterification reaction and optionally subjecting to the deprotection reaction of a protective group and/or the cleavage reaction from a resin.

This esterification reaction is known and examples thereof include:

(1) a method using an acid halide,
(2) a method using a mixed acid anhydride, and
(3) a method using a condensing agent.

These methods are described in detail below.

(1) The method using an acyl halide is carried out, for example, by reacting carboxylic acid with an acid halogenating agent (oxalyl chloride, thionyl chloride, etc.) in an organic solvent (chloroform, dichloromethane, diethylether, tetrahydrofuran, etc.) or in the absence of the solvent at -20°C to reflux temperature. Then the obtained acyl halide derivative may be reacted with amine in the presence of a base (pyridine, triethylamine, N,N-dimethylaniline, 4-dimethylaminopyridine, diisopropylethylamine, etc.) in an alcohol and an organic solvent (chloroform, dichloromethane, diethylether, tetrahydrofuran, etc.) at 0 to 40°C.
(2) The method using a mixed acid anhydride is carried out, for example, by reacting carboxylic acid with an acyl halide (pivaloyl chloride, tosyl chloride, mesyl chloride, etc.) or an acid derivative (ethyl chloroformate, butyl chloroformate, etc.) in the presence of a base (pyridine, triethylamine, dimethylaniline, dimethylaminopyridine, diisopropylethylamine, etc.) in an organic solvent (chloroform, dichloromethane, diethylether, tetrahydrofuran, etc.) or in the absence of the solvent at 0 to 40°C, and reacting the resulting mixed acid anhydride with alcohol in an organic solvent (chloroform, dichloromethane, diethylether, tetrahydrofuran, etc.) at 0 to 40°C.
(3) The method using a condensing agent is carried out, for example, by reacting carboxylic acid with alcohol in an organic solvent (chloroform, dichloromethane, dimethylformamide, diethylether, tetrahydrofuran, etc.) or in the absence of the solvent at 0 to 40°C in the presence or absence of a base (pyridine, triethylamine, diisopropylethylamine, N,N-dimethylaniline, N-methylmorpholine, 4-dimethylaminopyridine, etc.), using a condensing agent (1,3-dicyclohexylcarbodiimide (DCC), 1-ethyl-3-[3-(dimethylamino)propyl]carbodiimide (EDC), 1,1'-carbonyldiimidazole (CDI), O-(7-azabenzotriazol-1-yl)N,N,N',N'-tetramethyluronium hexafluorophosphate (HATU), 2-chloro-1-methylpyridiniumiodine, 1-propylphosphonic acid cyclic anhydride (1-propanephosphonic acid cyclic anhydride, PPA), etc.) and using, or not using, 1-hydroxybenztriazole (HOBt) or 1-hydroxy-7-azabenzotriazole (HOAt).

The reactions described in (1), (2) and (3) are preferably carried out under an inert gas (argon, nitrogen, etc.) atmosphere under anhydrous conditions.

The compound represented by formula (I-B) can be prepared, for example, by subjecting a compound represented by formula (9) and a compound represented by formula (11):

$$R^{D1}\text{-}X^{1} \qquad (11)$$

(wherein all symbols have the same meanings as described above), to the alkylation reaction.

This alkylation reaction is known and is carried out, for example, in an organic solvent (for example, dimethylformamide, dimethyl sulfoxide, etc.) at a temperature of about 0 to 150°C in the presence or absence of an alkali (potassium carbonate, sodium carbonate, triethylamine, etc.) and sodium iodide or potassium iodide.

This reaction is preferably carried out under an inert gas (argon, nitrogen, etc.) atmosphere under anhydrous conditions.

Among the compound of the present invention represented by formula (I), a compound wherein ring $A^2$ represents an imidazole ring and $E^2$ represents a methylene group, namely, a compound represented by formula (I-D):

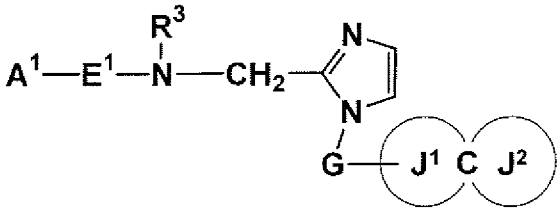

(I-D)

(wherein all symbols have the same meanings as described above), can be prepared by subjecting a compound represented by formula (12):

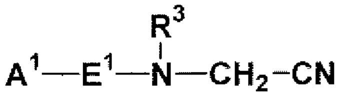

(12)

(wherein all symbols have the same meanings as described above), to the ring formation reaction by using acetal ([2,2-bis(methyloxy)ethyl]amine, [2,2-bis(ethyloxy)ethyl]amine, etc.) of glycinal, optionally subjecting to the deprotection reaction of a protective group and/or the cleavage reaction from a resin, and reacting the resultant compound (13) of the formula:

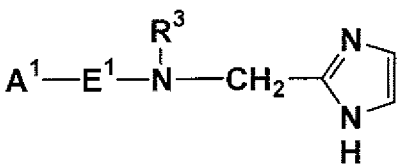

(13)

with a compound represented by formula (14):

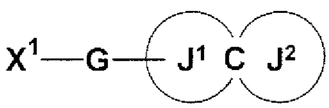

(14)

(wherein all symbols have the same meanings as described above) in an organic solvent (methanol, ethanol, N,N-dimethylformamide, N-methylpiperidone, etc.) at room temperature to 150°C in the presence of a base (sodium carbonate, potassium carbonate, sodium hydrogencarbonate, etc.).

This ring formation reaction is known and can be carried out by improving the method described in Synthesis, 2001, (10), 1546-1550. For example, a nitrile compound is reacted in an organic solvent (methanol, ethanol, etc.) at 0 to 40°C in the presence of a base (sodium methoxide, sodium ethoxide, etc.) and then the resultant solution is reacted at 40 to 150°C in the presence of acetal or glycinal and a dehydrating agent (acetic acid, etc.). The deprotection reaction of a protective group or the cleavage reaction from a resin can be carried out by the same manner as that described above.
The compounds represented by formulas (2) to (8) and (10) to (14) used as other starting materials or reagents can be easily prepared by using per se known methods or known methods, for example, methods described in Comprehensive Organic Transformations: A Guide to Functional Group Preparations, Second edition (written by Richard C. Larock, John Wiley & Sons Inc, 1999) in combination. The compound represented by formula (9) can be prepared by the method described above.
In the respective reactions in the present specification, as is apparent to those skilled in the art, the reaction with heating can be carried out using a water bath, an oil bath, a sand bath, or microwave.
In the respective reactions in the present specification, a solid phase supported reagent obtained by supporting on a polymer (for example, polystyrene, polyacrylamide, polypropylene, polyethylene glycol, etc.) may be used.
In the respective reactions in the present specification, the reaction product can be purified by conventional purification means, for example, distillation under normal pressure or reduced pressure, high performance liquid chromatography using a silica gel or magnesium silicate, thin layer chromatography, ion-exchange resin, scavenger resin or column chromatography or washing, or recrystallization. The purification may be carried out for every reaction, or may be carried out after the completion of some reactions.
In the reaction using a polystyrene resin in the present specification, the reaction product can be purified by conventional purification methods, for example, washing plural times with a solvent (N,N-dimethylformamide, dichloromethane, methanol, tetrahydrofuran, toluene, acetic acid/toluene, etc.).

[Toxicity]

The compound of the present invention has very low toxicity and is considered to be safe enough for pharmaceutical use. Although the compound having basicity has a large distribution volume, the compound of the present invention also has a distribution volume and therefore has high safety since there is very low risk of side effects such as phospholipidosis.

[Application to Pharmaceuticals]

The compound of the present invention has CXCR4 antagonistic activity in an animal including human, particularly human, and is therefore effective, for example, for a prevention and/or treatment for inflammatory and immune diseases, allergic diseases, infections, particularly HIV infection, and diseases associated with the infection, psychoneurotic diseases, cerebral diseases, cardiovascular diseases, metabolic diseases, and cancerous diseases. Also, the compound is useful as an agent for regeneration therapy for the purpose of in vitro or in vivo amplification of stem cells for gene therapy as well as peripheral blood stem cells mobilization and tissue repair. The compound is particularly useful as an agent for transplantation medical treatment used in organ transplantation including bone marrow transplantation, peripheral blood stem cell transplantation and tissue repair among in the regeneration therapy. Furthermore, the compound is useful as an antiangiogenic agent which is effective for prevention and/or treatment of diseases associated with neoangiogenesis, such as retinopathy (diabetic retinopathy etc.), macular degeneration, and cancer proliferation.
Examples of the inflammatory and immune disease include rheumatoid arthritis, arthritis, retinopathy, gout, rejection of transplanted organ, graft-versus-host disease (GVHD), pneumonia, nephritis, psoriasis, rhinitis, conjunctivitis, multiple sclerosis, ulcerative colitis, Crohn's disease, shock associated with bacterial infection, pulmonary fibrosis, systemic inflammatory response syndrome (SIRS), acute lung injury, and diabetes.
Examples of the allergic disease include asthma, atopic dermatitis, rhinitis, and conjunctivitis.
Examples of infections include various infections caused by streptococcus (Group A-hemolytic streptococcus, Streptococcus pneumoniae, etc.), staphylococcus aureus (MSSA, MRSA), Staphylococcus epidermidis, enterococcus, Listeria, meningococcus, gonococcus, E. coli bacteria (0157:H7, etc.), klebsiella (Klebsiella pneumoniae), Proteus, tussis convulsiva, Pseudomonas aeruginosa, Serratia marcescens, Shitorobactar, Ashinetobactar, Enterobactar, mycoplasma, chlamydia, and Crostorigeum, cholera, diphtheria, dysentery, scarlet fever, anthrax, trachoma, syphilis, tetanus, Hansen's disease, legionella, Reptospira, Lyme disease, tularaemia, Q fever, meningitis, encephalitis, rhinitis, sinusitis, pharyngitis, laryngitis, orbital cellulitis, thyroiditis, Lemierre syndrome, pneumonia, bronchitis, tuberculosis, infectious endocarditis, pericarditis, myocarditis, infectious aortitis, septicemia, cholecystitis, cholangitis, hepatitis, liver abscess, acute pancreatitis, splenic abscess, enteritis, iliopsoas abscess, pyelonephritis, cystitis, prostatitis, colpitis, Pelvic inflammatory disease, cellulitis, panniculitis, gas gangrene, furuncle, carbuncle, contagious impetigo, staphylococcal scalded skin syndrome, herpes zoster, varicella, measles, rubella, impetigo, scabies, infectious arthritis, osteomyelitis, fasciitis, myositis,

and lymphadenitis.

Examples of the disease associated with HIV infection, particularly HIV infection, include acquired immunodeficiency syndrome (AIDS), candidiasis, Pneumocystis carinii pneumonia, Cytomegalovirus retinitis, Kaposi's sarcoma, malignant lymphoma, AIDS encephalopathy, and bacterial sepsis.

Examples of the psychoneurotic disease and cerebral disease include dementia including Alzheimer's disease, Parkinson's disease, stroke, cerebral infarction, cerebral hemorrhage, epilepsia, schizophrenia, and peripheral nerve disorder.

Examples of the cardiovascular disease include arteriosclerosis, ischemia reperfusion, hypertension, myocardial infarction, stenocardia, and heart failure.

Examples of the metabolic diseases include diabetes, osteoporosis, enlarged prostate, and frequent micturition.

Macular degeneration is a disease wherein progressive disorder arises in macula lutea that is present in the center of retina and controls visual acuity, and age-related one is referred to as age-related macular degeneration. Macular degeneration includes atrophy type (dry type) macular degeneration wherein macula lutea tissue causes atrophy and exudation type (wet type) macular degeneration wherein new blood vessel are formed in the chorioidea of the macula lutea site.

Examples of the cancerous disease include malignant tumor such as breast cancer, brain cancer, or malignant lymphoma, cancer metastasis, and myelosuppression or thrombocytopenia after radiation therapy/chemotherapy.

The compound of the present invention is effective for prevention and/or treatment of cancerous diseases or infections in animals including human, particulary human, and preferably cancerous diseases.

The fact that the compound of the present invention is useful as pharmaceuticals can be evaluated by methods described in various tests and biological examples described hereinafter, and methods which can be carried out by appropriately improving the above methods. The fact that the compound of the present invention is kinetically excellent can be easily evaluated by a known method, for example, a method described in "Drug Bioavailability (Science of Evaluation and Improvement)", Gendai Iryosha, published on July 6, 1998. The fact that the compound of the present invention having a hydroxyl group protected by a protective group, a carboxyl group protected by a protective group, or a sulfo group protected by a protective group is excellent in solubility, and oral absorption, and that the compound of the present invention obtained by converting the compound by an enzyme or gastric acid in vivo, namely, the compound of the present invention having a hydroxyl group, a carboxyl group, or a sulfo group is excellent in half-life in blood, stability in digestive tract and bioavilability by various tests or known methods.

(1) Evaluation Experiment of Inhibitory Activity of Drug Metabolizing Enzyme of Compound of the Present Invention

(i) Inhibitory Activity against Human CYP2A9

An inhibitory activity against CYP2C9 of the compound of the present invention can be evaluated by improving accuracy and/or sensitivity of the measurement in accordance with the method of Sato et al. (Pharmacokinetic, Xenobio. Metabol. and Dispos., 16(2), pp.115-126 (2001)).

(ii) Inhibitory Activity against Human CYP3A4

Inhibitory activity against CYP3A4 of the compound of the present invention can be evaluated by an improved method described in Drug Metabolism and Disposition, Vol. 28 (12), 1440-1448 (2000).

(2) Evaluation Experiment of Toxicity of Compound of Present Invention

(i) Single Acute Toxicity Test in Rat

The test compound is administered to six-week Crj:CD (SD) rat by single intravenous dose or single oral administration. Toxicity can be evaluated by contrast with value at no addition of the test compound. Basic evaluation of toxicity can be done by, for example, observation of performance status or locomotor activity, etc.

(ii) Evaluation of Activity of Compound of Present Invention against hERG $I_{Kr}$ Current

According to the report by Zou et al. (Biophys. J., Vol. 74, 230-241 (1998)), using HEK293 cell overexpressed of human ether-a-go-go-related gene (hERG), max tale current of hERG $I_{Kr}$ current induced by depolarization pulse followed by repolarization pulse is measured by patch-clamp recording. Rate of change (inhibition rate) is calculated by comparison max tale current between before addition of the test compound and 10 minutes after. The influence of the test compound against hERG $I_{Kr}$ current can be evaluated by the inhibition rate.

(iii) Evaluation of Action of Compound of Present Invention against Phospholipidosis

It is possible to easily evaluate in accordance with the report by Kasahara et al. (Toxicol. Sci., Vol. 90, pp. 1330-141 (2006)) and the report by Narita et al. (document "in vitro Phospholipidosis Detection System using Fluorescent-Labeled Phospholipids Analogue" distributed in presentation of results of research of Human Science Synthetic Research Promotion Business focusing on Drug Innovation in fisical year 2003).

(iv) Evaluation of Influence of Compound of the Present Invention on Blood Pressure and Heart Rate

A rat was anesthetized with urethane (1.2 g/kg subcutaneous administration). After neck midline dissection, a catheter for measuring blood pressure was inserted into a right common carotid artery. Then, after dissecting inguinal region, a catheter for chemical injection was inserted into a femoral vein and fixed. A catheter for measurement of blood pressure was connected to a pressure transducer and then the pressure waveform was recorded on a thermal writing pen recorder through an amplifier for strain compression (AP-641G (manufactured by NIHON KOHDEN CORPORATION)). In this case, regarding a heart rate, a value through a cardiotachometer (AT-601G (manufactured by NIHON KOHDEN CORPORATION)) using the pressure waveform obtained from the amplifier for strain compression as a trigger was recorded on a thermal writing pen recorder. The test compound was dissolved in a 10% WellSolve (trade name; manufactured by Celeste B Corporation) so as to adjust the concentration to 0.1, 0.3, 1,3 or 10 mg/mL to prepare solutions. Each solution was intravenous administered at 1 mL/kg through the caudal vein over about 10 seconds. Accumulative administration of stepwise increasing of a dosage was carried out to an individual to evaluate a reduction in blood pressure and an increasing in heart rate.

The measuring methods (1) to (2) are not limited to the above methods and conventional methods can be utilized based on the basic technology. The measuring methods of the present invention can be modified to improve accuracy and/or sensitivity of the measurement for evaluating the compound of the present invention.

The compound of the present invention may be administered as a concomitant drug by using in combination with other drugs for the purpose of:

1) complementation and/or enhancement of the preventive and/or therapeutic effects of the compound,
2) improvement of pharmacokinetics and absorption of the compound and reduction of the dosage, and/or
3) reduction of side effects of the compound.

Also, the compound of the present invention may be administered as a concomitant drug by using in combination with other drugs the purpose of (1) complementation and/or enhancement of preventive and/or therapeutic effects, (2) improvement of pharmacokinetics and absorption of the compound and reduction of the dosage, and/or (3) reduction of side effects.

The concomitant drug of the compound of the present invention and other drugs may be administered in the form of a compounding agent(s) comprising both these components, or may be in the form of separately. In case of separately administering a preparation, simultaneous administration and administration with time-lag are included. In case of administration with time-lag, other drugs may be administered after the compound of the present invention is administered, or the compound of the present invention may be administered after other drugs may be administered. The administration method may be the same or different.

The disease, on which the preventive and/or therapeutic effects are exerted by the concomitant drug, is not particularly limited, and may be any disease which complements and/or enhances the preventive and/or therapeutic effects of the compound of the present invention.

A mass ratio of the compound of the present invention drug to other drugs is not particularly limited.

A combination of any two or more kinds other drugs may be administered.

The other drugs, which complements and/or enhances the preventive and/or therapeutic effects of the compound of the present invention, includes not only those which have ever been found based on the above described mechanism, but also those which may be found in future.

Examples of the preventive and/or therapeutic agents for HIV infection and acquired immunodeficiency syndrome, which is used in combination of the compound of the present invention, include reverse transcriptase inhibitors, protease inhibitors, chemokine (for example, CCR2, CCR3, CCR4, CCR5, CXCR4, etc.) antagonists, CD4 antagonists, antibody against surface antigen of HIV (for example, HIV-1, HIV-2, etc.), vaccine of HIV (for example, HIV-1, HIV-2, etc.) and HIV-associated short interfering RNA.

Examples of the reverse transcriptase inhibitors include (1) nucleoside reverse transcriptase inhibitors such as zidovudine (trade name: Retrovir), didanosine (trade name: Videx), zalcitabine (trade name: Hivid), stavudine (trade name: Zerit), lamivudine (trade name: Epivir), abacavir (trade name: Ziagen), didanosine (trade name: videx), adefovir, dipivoxil, emtricitabine (trade name: coviracil), tenofovir (trade name: viread), Combivir, Trizivir, truvada, or epzicom, (2) non-nucleoside reverse transcriptase inhibitors such as nevirapine (trade name: viramune), delavirdine (trade name: Rescriptor), efavirenz (trade name: Sustiva, Stocrin), or capravirine (AG1549).

Examples of the protease inhibitors include indinavir (trade name: Kurikisiban), ritonavir (trade name: norvir), nelfinavir (trade name: Viracept), saquinavir (trade name: Invirase, Fortovase), amprenavir (trade name: agenerase), lopinavir (trade name: Kaletra), atazanavir (trade name: Reyataz), fosamprenavir (trade name: lexiva), tipranavir and the like.

Examples of the chemokine antagonist include an intrinsic ligand of a chemokine receptor, or a derivative thereof and a nonpeptidic low molecular weight compound, or an antibody against the chemokine receptor.

Examples of the intrinsic ligand of the chemokine receptor include MIP-1α, MIP-1β, RANTES, SDF-1α, SDF-1β, MCP-1, MCP-2, MCP-4, Eotaxin, and MDC.

Examples of the derivative of the intrinsic ligand include AOP-RANTES, Met-SDF-1α, and Met-SDF-1β.

Examples of the antibody of the chemokine receptor include Pro-140.

Examples of the CCR2 antagonist include compounds described in WO 99/07351, WO 99/40913, WO 00/46195, WO 00/46196, WO 00/46197, WO 00/46198, WO 00/46199, WO 00/69432, WO 00/69815, and Bioorg. Med. Chem. Lett., 10, 1803 (2000).

Examples of the CCR3 antagonist include compounds described in DE19837386, WO 99/55324, WO 99/55330, WO 00/04003, WO 00/27800, WO 00/27835, WO 00/27843, WO 00/29377, WO 00/31032, WO 00/31033, WO 00/34278, WO 00/35449, WO 00/35451, WO 00/35452 , WO 00/35453 , WO 00/35454, WO 00/35876, WO 00/35877, WO 00/41685, WO 00/51607, WO 00/51608, WO 00/51609, WO 00/51610, WO 00/53172, WO 00/53600, WO 00/58305, WO 00/59497, WO 00/59498, WO 00/59502, WO 00/59503, WO 00/62814, WO 00/73327, and WO 01/09088.

Examples of the CCR4 antagonist include compounds described in WO 02/030357, and WO 02/030358.

Examples of the CCR5 antagonist include compounds TAK-779, SCH-351125(SCH-C), SCH-417690(SCH-D), UK-427857, GW 873140A(ONO-4128), TAK-220 and TAK-652 described in WO 99/17773, WO 99/32100, WO 00/06085, WO 00/06146, WO 00/10965, WO 00/06153, WO 00/21916, WO 00/37455, EP1013276, WO 00/38680, WO 00/39125, WO 00/40239, WO 00/42045, WO 00/53175, WO 00/42852, WO 00/66551, WO 00/66558, WO 00/66559, WO 00/66141, WO 00/68203, JP2000309598, WO 00/51607, WO 00/51608, WO 00/51609, WO 00/51610, WO 00/56729, WO 00/59497, WO 00/59498, WO 00/59502, WO 00/59503, WO 00/76933, WO 98/25605, WO 99/04794, WO 99/38514, Bioorg. Med. Chem. Lett., 10, 1803 (2000).

Examples of the CD4 antagonist include curdlansulfuric acid, TNX-355, BT-061, CD4 antagonist 802-2, 4162W94, PP-0102, anti-CD4 antibody, AD-519, TRX-1, and CD4-IgG.

Examples of the CXCR3 antagonist include compounds described in WO 01/16114, WO 02/083143, WO 02/085862, US 6469002 and WO 03/101970.

Examples of the CXCR4 antagonist include MD-3100, AMD-070, T-22, KRH-1120, KRH-1636, KRH-2731, CS-3955, compounds described in WO 00/66112, WO 2003/055876, WO 2004/024697, WO 2004/052862, WO 2006/022454, WO 2006/023400, WO 2006/020415, WO 2006/020891, WO 2006/036816, US 2006/069122, WO 2006/034001, WO 2006/028896, WO 2006/048862, WO 2006/074426, US 2006/160860, WO 2006/076131, WO 2006/026703, JP 2006-188445, WO 2006/090853, WO 2006/096444, US 2006/281712, WO 2007/008539, US 2006/0293324, WO 2006117011, WO 2007/022385 and WO 2007027999.

Examples of the fusion inhibitor include T-20 (pentafuside, Enfuvirtide, Fuseon (trade names)), and T-1249.

Examples of the HIV integrase inhibitor include Equisetin, Temacrazine), MK0518 (Raltegravir), PL-2500, V-165, NSC-618929, L-870810, and L-708906 analog, S-1360, and 1838.

HIV-associated short interfering RNA is short interfering RNA with the gene of a HIV-associated factor as a target. Examples of the HIV-associated factor include reverse transcriptadse, protease, chemokine (for example, CCR2, CCR3, CCR4, CCR5, CXCR4, etc.), CD4, and HIV (HIV-1, HIV-2, etc.). Examples of the HIV-associated short interfering RNA include GPs-0193, HGTV-43, GEM-132, GEM-92, GEM-93, HYB-0184, GEM-91, UL36ANTI, ISIS-2922, ISIS-14803, GPI-2A, R-95288, and VRX-496.

Examples of the vaccine of HIV include Inflexal V, Vacc-4x, Vacc-5q, Typhim Vi, HBV-ISS, EP-1043, Tat Toxoid, IR-103, Remune, Flumist, AIDSVAX, and Therapore-P24.

The conventional clinical dosage of typical reverse transcriptase inhibitors and protease inhibitors is, for example, as described below, but is not limited thereto in the present invention.

Zidovudine: 100 mg capsule, three times per day in a dosage of 200 mg; 300 mg tablet, twice per day in a dosage of 300 mg;
Didanosine: 25 to 200 mg tablet, twice per day in a dosage of 125 to 200 mg;
Zalcitabine: 0.375 mg to 0.75 mg tablet, three times per day in a dosage of 0.75 mg; Stavudine: 15 to 40 mg capsule, twice per day in a dosage of 30 to 40 mg;
Lamivudine: 150 mg tablet, twice per day in a dosage of 150 mg;
Abacavir: 300 mg tablet, twice per day in a dosage of 300 mg;
Nevirapine: 200 mg tablet, once per day for 14 days in a dosage of 200 mg, followed by twice per day;
Delavirdine: 100 mg tablet, three times per day in a dosage of 400 mg;
Efavirenz: 50 to 200 mg capsule, once per day in a dosage of 600 mg;
Indinavir: 200 to 400 mg capsule, three times per day in a dosage of 800 mg;
Ritonavir: 100 mg capsule, twice per day in a dosage of 600 mg;
Nelfinavir: 250 mg tablet, three times per day in a dosage of 750 mg;
Saquinavir: 200 mg capsule, three times per day in a dosage of 1,200 mg;
Amprenavir: 50 to 150 mg tablet, twice per day in a dosage of 1,200 mg.

Examples of the other drugs for complementation and/or enhancement of the preventive and/or therapeutic effects of the compound of the present invention against asthma include antihistaminic agents, antiallergic agents (chemical mediator release inhibitors, histamine antagonists, thromboxane synthetase inhibitors, thromboxane antagonists,

Th2 cytokine inhibitors), steroids, bronchodilator agents (xanthine derivatives, sympathomimetic agents, parasympathomimetic agents), vaccinotherapeutic agents, gold preparations, Chinese medicines, basic nonsteroidal anti-inflammatory drugs, 5-lipoxygenase inhibitors, 5-lipoxygenase activation protein antagonists, leukotriene synthesis inhibitors, prostaglandins, cannabinoid-2 receptor stimulants, antitussive drugs, expectorants and the like.

Examples of the antihistaminic agents include diphenhydramine, diphenylpyraline hydrochloride, diphenylpyraline chlorotheophyllinate, clemastine fumarate, dimenhydrinate, dl-chlorpheniramine maleate, d-chlorpheniramine maleate, triprolidine hydrochloride, promethazine hydrochloride, alimemazine tartrate, isothipendyl hydrochloride, homochlorcyclizine hydrochloride, hydroxyzine, cyproheptadine hydrochloride, levocabastine hydrochloride, astemizole, bepotastine, desloratadine, TAK-427, ZCR-2060, NIP-530, mometasone furoate, mizolastine, BP-294, andolast, auranofin, acrivastine and the like.

Examples of the chemical mediator release inhibitors include disodium cromoglycate, tranilast, amlexanox, repirinast, ibudilast, pemirolast potassium, tazanolast, nedocromil, cromoglicate, israpafant and the like.

Examples of the histamine antagonists include ketotifen fumarate, azelastine hydrochloride, oxatomide, mequitazine, terfenadine, emedastine fumarate, epinastine hydrochloride, ebastine, cetirizine hydrochloride, olopatadine hydrochloride, loratadine, fexofenadine and the like.

Examples of the thromboxane synthetase inhibitors include ozagrel hydrochloride, imitrodast sodium and the like.

Examples of the thromboxane antagonists include seratrodast, ramatroban, domitroban calcium hydrate, KT-2-962 and the like.

Examples of the Th2 cytokine inhibitors include suplatast tosilate and the like.

Examples of the steroids include, for example, external medicine such as clobetasol propionate, diflorasone diacetate, fluocinonide, mometasone furoate, betamethasone dipropionate, betamethasone butyrate propionate, betamethasone valerate, difluprednate, budesonide, diflucortolone valerate, amcinonide, halcinonide, dexamethasone, dexamethasone propionate, dexamethasone valerate, dexamethasone acetate, hydrocortisone acetate, hydrocortisone butyrate, hydrocortisone butyrate propionate, deprodone propionate, prednisolone valerate acetate, fluocinolone acetonide, beclometasone dipropionate, triamcinolone acetonide, flumetasone pivalate, alclometasone propionate, clobetasone butyrate, prednisolone, beclomethasone propionate, fludroxycortide and the like. Examples of drugs for internal use and injections include cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredone acetate, methyl prednisolone, methylprednisolone acetate, methylprednisolone sodium succinate, triamcinolone, triamcinolone acetate, triamcinolone acetonide, dexamethasone, dexamethasone acetate, dexamethasone sodium phosphate, dexamethasone palmitate, paramethasone acetate, betamethasone and the like. Examples of the inhalations include beclometasone dipropionate, fluticasone propionate, budesonide, flunisolide, triamcinolone, ST-126P, ciclesonide, dexamethasone palmitate, mometasone furoate, prasterone sulfonate, deflazacort, methylprednisolone suleptanate, methylprednisolone sodium succinate and the like.

Examples of the xanthine derivative include aminophylline, theophylline, doxophylline, cipamfylline, diprophylline, proxyphylline, and choline theophylline.

Examples of the sympathomimetic agents include epinephrine, ephedrine hydrochloride, dl-methylephedrine hydrochloride, methoxyphenamine hydrochloride, isoproterenol sulfate, isoproterenol hydrochloride, orciprenaline sulfate, chloroprenaline hydrochloride, trimetoquinol hydrochloride, salbutamol sulfate, terbutaline sulfate, hexoprenaline sulfate, tulobuterol hydrochloride, procaterol hydrochloride, fenoterol hydrobromate, formoterol fumarate, clenbuterol hydrochloride, mabuterol hydrochloride, salmeterol xinafoate, R,R-formoterol, tulobuterol, pirbuterol hydrochloride, ritodrine hydrochloride, bambuterol, dopexamine hydrochloride, meluadrine tartrate, AR-C68397, levosalbutamol, KUR-1246, KUL-7211, AR-C89855, S-1319 and the like.

Examples of the parasympathomimetic agents include ipratropium bromide, flutropium bromide, oxitropium bromide, cimetropium bromide, temiverine, tiotropium bromide, revatropate (UK-112166) and the like.

Examples of the vaccinotherapeutic agents include paspat, asthremedin, Broncasma Bema, CS-560 and the like.

Examples of the gold preparations include gold sodium thiomalate and the like.

Examples of the basic nonsteroidal anti-inflammatory drugs include tiaramide hydrochloride, tinoridine hydrochloride, epirizole, emorfazone and the like.

Examples of the 5-lipoxygenase inhibitors include zyleuton, docebenone, piriprost, SCH-40120, WY-50295, E-6700, ML-3000, TMK-688, ZD-2138, dalbufelone mesilate, R-68151, E-6080, DuP-654, SC-45662, CV-6504, NE-11740, CMI-977, NC-2000, E-3040, PD-136095, CMI-392, TZI-41078, Orf-20485, IDB-18024, BF-389, A-78773, TA-270, FLM-5011, CGS-23885, A-79175, ETH-615 and the like.

Examples of the 5-lipoxygenase activation protein antagonists include MK-591, MK-886 and the like.

Examples of the leukotriene synthesis inhibitors include auranofin, proglumetacin maleate, L-674636, A-81834, UPA-780, A-93178, MK-886, REV-5901A, SCH-40120, M-591, Bay-x-1005, Bay-y-1015, DTI-0026, Amlexanox, E-6700

and the like.

Examples of the prostaglandins (hereinafter abbreviated to as PG) include PG receptor agonists, PG receptor antagonists and the like.

Examples of the PG receptor include PGD receptor (EP1, EP2, EP3, and EP4), PGD receptor (DP, CRTH2), PGF receptor (FP), PGI receptor (IP), TX receptor (TP) and the like.

Examples of the antitussive drugs include codeine phosphate, dihydrocodeine phosphate, oxymetebanol, dextromethorphan hydrobromate, pentoxyverine citrate, dimemorfan phosphate, oxeladin citrate, cloperastine, benproperine phosphate, clofedanol hydrochloride, fominoben hydrochloride, noscapine, tipepidine hibenzate, eprazinone hydrochloride, plantago herb extract and the like.

Examples of the expectorants include foeniculated ammonia spirit, sodium hydrogencarbonate, potassium iodide, bromhexine hydrochloride, cherry bark extract, carbocysteine, fudosteine, ambroxol hydrochloride, ambroxol hydrochloride sustained-release tablet, methylcysteine hydrochloride, acetylcysteine, L-ethylcysteine hydrochloride, tyloxapol and the like.

Examples of the other drugs for complementation and/or enhancement of the preventive and/or therapeutic effects against atopic dermatitis (urticaria, etc.) of the compound of the present invention include steroids, non-steroid anti-infilammatory drug (NSAID), immune inhibitor, prostaglandins, antiallergic agent, mediator release inhibitor, antihistaminic agent, forskolin preparation, phosphodiesterase inhibitor, and cannabinoid-2 receptor stimulant.

Examples of the other drugs for complementation and/or enhancement of the preventive and/or therapeutic effects against allergic diseases (allergic bronchopulmonary aspergillosis, allergic eoisinophilic gastroenteritis, etc.) of the compound of the present invention include antiasthmatic drug, inhaled steroid drug, inhaled β2 stimulant, methylxanthine-based stimulant, antiallergic agent, anti-inflammatory agent, anticholinergic agent, thromboxane antagonist, leukotriene antagonist, LTD4 antagonist, PAF antagonist, phosphodiesterase inhibitor, β2 agonist, steroid drug, mediator release inhibitor, eosinophile leukocytechemotaxis inhibitor, macrolide-based antibiotic, immune inhibitor, hyposensitization (allergen) injection and the like.

Examples of the antiasthmatic drug include theophylline, procaterol, ketotifen, azelastine and the like.

Examples of the inhaled steroid drug include beclomethasone, fluticasone, budesonide and the like.

Examples of the inhaled β2 stimulant include fenoterol, salbutamol, formoterol, salmeterol and the like.

Examples of the methylxanthine-based stimulant include theophylline and the like.

Examples of the antiallergic agent include ketotifen, terfenadine, azelastine, epinastine, suplatast, disodium cromoglycate and the like.

Examples of the anti-inflammatory agent include dichlofenac sodium, ibuprofen, indomethacin and the like.

Examples of the anticholinergic agent include ipratropium bromide, flutropium bromide, oxitropium bromide, tiotropium bromide and the like.

Examples of the thromboxane antagonist include ozagrel, seratrodast and the like.

Examples of the leukotriene antagonist include pranlukast, montelukast, zafirlukast, zyleuton and the like.

Examples of the macrolide-based antibiotic include erythromycin, roxithromycin and the like.

Examples of the immune inhibitor include tacrolimus (FK506), cyclosporine, sirolimus (rapamycin), corticosteroid, azathioprine, mycophenolate mophetyl, FTY720, cyclophosphamide and the like.

Examples of the other drug for complementation and/or enhancement of the preventive and/or therapeutic effects against hepatitis of the compound of the present invention include liver hydrolysate preparation, polyenephosphatidylcholine, glycyrrhizin preparation, protoporphyrin sodium, ursodeoxycholic acid, steroids, anticholinergic agent, gastric antiacid, propagermanium, lipid peroxidase inhibitor, and mitochondrial benzodiazepine receptor antagonist.

Examples of the other drug for complementation and/or enhancement of the preventive and/or therapeutic effects against arthritis and rheumatoid arthritis of the compound of the present invention include metalloproteinase inhibitor, immune inhibitor, non-steroid anti-inflammatory drug (UNSAID), steroid drug, prostaglandins, phosphodiesterase inhibitor, cannabinoid-2 receptor stimulant, disease modifying anti-rheumatic drug (slow-acting anti-rheumatic drug), anti-inflammatory enzyme preparation, cartilage protective agent, T cell inhibitor, TNF[alpha] inhibitor, prostaglandin synthetase inhibitor, L-6 inhibitor, interferon γ agonist, IL-1 inhibitor and the like.

Examples of the other drug for complementation and/or enhancement of the preventive and/or therapeutic effects against psoriasis of the compound of the present invention include steroid drug, vitamin D derivative and the like.

Examples of the other drug for complementation and/or enhancement of the preventive and/or therapeutic effects against rhinitis of the compound of the present invention include antihistaminic agent, mediator release inhibitor, thromboxane synthetase inhibitor, thromboxane $A_2$ receptor antagonist, leukotriene receptor antagonist, steroids, α adrenalin receptor stimulant, xanthine derivative, anticholinergic agent, prostaglandins, nitrogen monoxide synthetase inhibitor, $\beta_2$ adrenalin receptor stimulant, phosphodiesterase inhibitor, cannabinoid-2 receptor stimulant and the like.

Examples of the other drug for complementation and/or enhancement of the preventive and/or therapeutic effects against conjunctivitis of the compound of the present invention include leukotriene receptor antagonist, antihistaminic agent, mediator release inhibitor, non-steroid anti-inflammatory drug, prostaglandins, steroid drug, nitrogen

monoxide synthetase inhibitor, cannabinoid-2 receptor stimulant and the like.

Examples of the other drug for complementation and/or enhancement of the preventive and/or therapeutic effects against multiple sclerosis of the compound of the present invention include immune inhibitor, cannabinoid-2 receptor stimulant and the like.

Examples of the other drug for complementation and/or enhancement of the preventive and/or therapeutic effects against ulcerative colitis of the compound of the present invention include mesalazine, salazosulfapyridine, digestive tract ulcer therapeutic substance, anticholinergic agent, steroid drug, 5-lipoxygenase inhibitor, antioxidant, LTB4 antagonist, local anesthetic, immune inhibitor, protection factor enhancer, MMP inhibitor, and mitochondrial benzodiazepine receptor antagonist.

Examples of the other drug for complementation and/or enhancement of the preventive and/or therapeutic effects against diabetic complication of the compound of the present invention include sulfonyl urea-based hypoglycemic agent (for example, acetohexamide, glibenclamide, gliclazide, glyclopyramide, chlorpropamide, tolazamide, tolbutamide, Glimepiride, etc.), biguanide-based drug (for example, buformin hydrochloride, metformin hydrochloride, etc.), α-glucosidase inhibitor (for example, acarbose, voglibose, etc.), ultrashort-acting insulinotropic agent (for example, nateglinide, repaglinide, etc.), insulin drug, PPAR agonist (for example, pioglitazone, troglitazone, rosiglitazone, JTT-501, etc.), insulin sensitive enhancer having no PPAR antagonism (for example, ONO-5816, YM-440, etc.), β3 adrenalin receptor agonist (for example, AJ9677, L750355, CP331648, etc.), aldose reductase inhibitor (for example, epalrestat, fidarestat, zenarestat, etc.), dipeptidyl peptidase IV inhibitor and the like.

Examples of the other drug for complementation and/or enhancement of the preventive and/or therapeutic effects against cancer (malignant tumor) and cancer metastasis of the compound of the present invention include anticancer agent (for example, MMP inhibitor, alkylation agent (for example, cyclophosphamide, melphalan, thiotepa, mytomycin C, busulfan, procarbazine hydrochloride, etc.), antimetabolite (for example, methotrexate, mercaptpurine, azathiopurine, fluorouracil, tegafur, cytarabine, azaserine, etc.), antibiotic (for example, mytomycin C, bleomycin, Peplomycin, doxorubicin hydrochloride, aclarubicin, daunorubicin, actinomycin D, etc.), mitosis inhibitor, platinum complex (for example, Cisplatin), plant-derived antineoplastic agent (for example, vincristine sulfate, vinblastine sulfate, etc.), anticancerous hormone (for example, methyltestosterone, testosterone propionate, testosterone enanthate, mepitiostane, fosfestrol, chlormadinone acetate, etc.), immunopotentiator (for example, picibanil, krestin, etc.), and interferon (for example, IPN[α], IFN[α]-2a, IFN[α]-2b, IFN[β], IFN[γ]-1a, etc.). Examples thereof include biologics capable of conducting T cell activation (for example, anti-CTLA-4 antibody, anti-PD-1 antibody, etc.), antiangiogenic agent (for example, bevacizumab, pegaptanib, SU-6668, vatalanib, ranibizumab, sorafenib, SU-11248, neovastat), etc.), and the like.

Examples of the other drug for complementation and/or enhancement of the preventive and/or therapeutic effects against immune disease (for example, autoimmune disease, transplanted organ rejection, etc.) of the compound of the present invention include immune inhibitor. Examples of the immune inhibitor include tacrolimus (FK506), cyclosporine, sirolimus (rapamycin), corticosteroid, azathioprine, mycophenolate mophetyl, FTY720, cyclophosphamide and the like.

Examples of the other drug for complementation and/or enhancement of the preventive and/or therapeutic effects against dementia such as Senile dementia with Alzheimer's type of the compound of the present invention include acetylcholine esterase inhibitor, nicotinic receptor modifier, cerebral ameliorator, monoamineoxidase inhibitor, vitamin E, aldose reductase inhibitor and the like.

Examples of the other drug for complementation and/or enhancement of the preventive and/or therapeutic effects against epilepsia of the compound of the present invention include phenyloin, trimethadione, ethosuximide, carbamazepine, phenobarbitone, primidone, acetazolamide, sultiame, sodium valproate, clonazepam, diazepam, nitrazepam and the like.

Examples of the other drug for complementation and/or enhancement of the preventive and/or therapeutic effects against arteriosclerosis of the compound of the present invention include HMG-CoA reductase inhibitor, fibrates, probucol preparation, anion-exchange resin, EPA preparation, nicotinic acid preparation, MTP inhibitor, other anti-high cholesterol agent, EDG-2 antagonist and the like.

Examples of the other drug for complementation and/or enhancement of the effects when the compound of the present invention is used in a regeneration therapy include cytokines and various growth factors, for example, various CSFs (for example, G-CSF, GM-CSF, etc.), various interleukins (for example, IL-3, 6, 7, 11, 12, etc.), EPO, TPO, SCF, FLT3 ligand, MIP-1α and the like.

Examples of the other drug for complementation and/or enhancement of the preventive and/or therapeutic effects against retinopathy of the compound of the present invention include antiangiogenic agent (for example, bevacizumab, pegaptanib, SU-6668, vatalanib, ranibizumab, sorafenib, SU-11248, neovastat, etc.) and the like.

The compound of the present invention is safe and has low toxicity and therefore can be administered to human and mammal other than human (for example, rat, mouse, rabbit, sheep, pig, cow, cat, dog, monkey, etc.).

In order to use a pharmaceutical composition comprising the compound of the present invention or a concomitant drug of the compound of the present invention and other drugs, it is commonly administered, systematically or locally,

in an oral or parenteral dosage form.

The dosage of the pharmaceutical preparation varies depending on the age, body weight, symptom, desired therapeutic effect, route of administration and duration of treatment. For the human adult, the dosage per person is between 0.1 ng and 5,000 mg, by oral administration, up to several times per day, between 0.1 ng and 500 mg, by parenteral administration, or continuous administration 1 hour to 24 hours per day from vein.

As a matter of course, since the dosage varies under various conditions as is described above, the dosage may be sometimes sufficient which is smaller than the above range, or sometimes the dosage must be more than the above range.

In case of administering a pharmaceutical composition comprising the compound of the present invention, or a concomitant drug of the compound of the present invention and other drugs, it is used as solid preparations for internal use and solutions for internal use for oral administration, and injections, external preparations, suppositories, ophthalmic solutions, nasal drops, inhalants and the like for parenteral administration.

Examples of the solid preparation for internal use for oral administration include tablets, pills, capsules, powders, and granules. Capsules include hard capsules and soft capsules.

In such a solid preparation for internal use, one or more active substances are used as they are, or used after mixing with excipients (lactose, mannitol, glucose, microcrystalline cellulose, starch, etc.), binders (hydroxypropyl cellulose, polyvinyl pyrrolidone, magnesium aluminometasilicate, etc.), disintegrants (calcium carboxymethyl cellulose, etc.), lubricants (magnesium stearate, etc.), stabilizers and solubilizing agents (glutamic acid, aspartic acid, etc.) and forming into a preparation according to a conventional method. If necessary, the preparation may be coated with a coating agent (saccharose, gelatin, hydroxypropyl cellulose, hydroxypropylmethyl cellulosephthalate, etc.) or may be coated with two or more layers. Furthermore, capsules made of an absorbable substance such as gelatin is included.

The solutions for internal use for oral administration include pharmaceutically acceptable water, suspensions, emulsions, syrups, and elixirs. In such a solution, one or more active substances are dissolved, suspended or emulsified in a diluent used commonly (purified water, ethanol, mixed solution thereof, etc.). Furthermore, this solution may contain humectants, suspending agents, emulsifiers, sweeteners, flavors, aromatics, preservatives, buffers, and the like.

The dosage form of the external preparation for parenteral administration includes, for example, ointment, gel, cream, fomentation, patch, liniment, propellant, inhalant, spray, aerosol, ophthalmic solution, and nasal drop. These products contain one or more active substances and are prepared according to the formulation which is known or commonly used.

An ointment is prepared in accordance with a well known formulation or a commonly employed formulation. For example, it is prepared by triturating or dissolving one or more active substances in a base. An ointment base is selected from well known ones or those commonly employed. For example, those selected from higher fatty acids or higher fatty acid esters (adipic acid, myristic acid, palmitic acid, stearic acid, oleic acid, adipate ester, myristate ester, palmitate ester, stearate ester, oleate ester, etc.), waxes (beeswax, whale wax, ceresin, etc.), surfactants (polyoxyethylene alkyl ether phosphate ester, etc.), higher alcohols (cetanol, stearyl alcohol, cetostearyl alcohol, etc.), silicone oils (dimethylpolysiloxane, etc.), hydrocarbons (hydrophilic petrolatum, white petrolatum, purified lanolin, liquid paraffin, etc.), glycols (ethylene glycol, diethylene glycol, propylene glycol, polyethylene glycol, macrogol, etc.), vegetable oils (castor oil, olive oil, sesame oil, turpentine oil, etc.), animal oils (mink oil, egg yolk oil, squalane, squalene, etc.), water, absorption accelerators, agents for preventing contact dermatitis are used alone or in combination. Furthermore, it may contain humectants, preservatives, stabilizers, antioxidizing agents, flavors, and the like.

A gel is prepared according to the formulation which is known or commonly used. For example, it is prepared by dissolving one or more active substances in a base. A gel base is selected from a base which is known or commonly used. For example, those selected from lower alcohols (ethanol, isopropyl alcohol, etc.), gelling agents (carboxymethyl cellulose, hydroxyethyl cellulose, hydroxypropyl cellulose, ethyl cellulose, etc.), neutralizers (triethanolamine, diisopropanolamine, etc.), surfactants (monostearic acid polyethylene glycol, etc.), gums, water, absorption accelerator, and agent for preventing contact dermatitis are used alone or in combination. Furthermore, it may contain preservatives, antioxidizing agents, and flavoring agent.

A cream is prepared according to the formulation which is known or commonly used. For example, it is prepared by dissolving or emulsifying one or more active substances in a base. A cream base is selected from a base which is known or commonly used. For example, those selected from higher fatty acid esters, lower alcohols, hydrocarbons, polyhydric alcohols (propylene glycol, 1,3-butylene glycol, etc.), higher alcohols (2-hexyl decanol, cetanol, etc.), emulsifiers (polyoxyethylene alkyl ethers, fatty acid esters, etc.). water, absorption accelerators, and agents for preventing contact dermatitis are used alone or in combination. Furthermore, it may contain preservatives, antioxidizing agents, and flavoring agents.

A fomentation is prepared according to the formulation which is known or commonly used. For example, it is prepared by dissolving one or more active substances in a base to obtain a kneaded mixture and spreading the kneaded mixture over a substrate. A fomentation base is selected from a base which is known or commonly used. For example, those selected from thickeners (polyacrylic acid, polyvinyl pyrrolidone, gum arabic, starch, gelatin, methyl cellulose,

etc.), humectants (urea, glycerin, propylene glycol, etc.), fillers (kaolin, zinc oxide, talc, calcium, magnesium, etc.), water, solubilizing agents, tackifiers, and agents for preventing contact dermatitis are used alone or in combination. Furthermore, it may contain preservatives, antioxidizing agents, and flavoring agent.

A patch is prepared according to the formulation which is known or commonly used. For example, it is prepared by dissolving one or more active substances in a base, and spreading the solution over a substrate. A patch base is selected from a base which is known or commonly used. For example, those selected from polymer bases, fats and oils, higher fatty acids, tackifiers, and agents for preventing contact dermatitis are used alone or in combination. Furthermore, it may contain preservatives, antioxidizing agents, and flavoring agent.

A liniment is prepared according to the formulation which is known or commonly used. For example, it is prepared by dissolving, suspending or emulsifying one or more active substances in one or more kinds selected from water, alcohol (ethanol, polyethylene glycol, etc.), higher fatty acid, glycerin, soap, emulsifier, and suspending agent. Furthermore, it may contain preservatives, antioxidizing agents, and flavoring agent.

A propellant, an inhalant, and a spray may contain, in addition to a diluent used commonly, a stabilizer such as sodium hydrogensulfite and a buffer capable of imparting isotonicity, for example, an isotonicity such as sodium chloride, sodium citrate or citric acid.

An injection for parenteral administration includes all injections and also includes a drop. For example, it includes intramuscular injection, subcutaneous injection, endodermic injection, intraarterial injection, intravenous injection, intraperitoneal injection, intraspinal injection, intravitreous injection, and intravenous drop.

The injection for parenteral administration includes solutions, suspensions, emulsions, and solid injections used by dissolving or suspending in a solvent before use. The injection is used after dissolving, suspending, or emulsifying one or more active substances in a solvent. As the solvent, for example, distilled water for injection, physiological saline, vegetable oil, and alcohols such as propylene glycol, polyethylene glycol or ethanol are used alone or in combination. Furthermore, the injection may contain stabilizers, solubilizing agents (glutamic acid, aspartic acid, polysolvate 80®, etc.), suspending agents, emulsifiers, soothing agents, buffers, and preservatives. These injections are prepared by sterilizing in the final process, or prepared by an aseptic treatment. Also, a sterile solid, for example, a freeze-dried product is prepared and can be used after dissolving in sterilized distilled water or distilled water for sterile injection, or the other solvent before use.

An ophthalmic solution for parenteral administration includes ophthalmic solution, suspension type ophthalmic solution, emulsion type ophthalmic solution, ophthalmic solution soluble when used, and eye ointment.

These ophthalmic solutions are prepared according to a known method. For example, one or more active substances are dissolved, suspended or emulsified in a solvent before use. As the solvent for ophthalmic solution, for example, sterilized purified water, physiological saline, and other aqueous solvent or non-aqueous agent for injection (for example, vegetable oil, etc.) are used alone or in combination. If necessary, the ophthalmic solution may contain appropriately selected isotonizing agents (sodium chloride, concentrated glycerin, etc.), buffering agents (sodium phosphoate, sodium acetate, etc.), surfactants (polysolvate 80 (trade name), polyoxyl 40 stearate, polyoxyethylene hardened castor oil, etc.), stabilizers (sodium citrate, sodium edetate, etc.), and antiseptics (benzalkonium chloride, paraben, etc.). These ophthalmic solutions are prepared by sterilizing in the final process, or prepared by an aseptic treatment. Also, a sterile solid, for example, a freeze-dried product is prepared and can be used after dissolving in sterilized distilled water or distilled water for sterile injection, or the other solvent before use.

An inhalant for parenteral administration includes aerozol, inhalation powder, and inhalation solution, and the inhalation solution may be such a configuration that it is used after dissolving in water or other suitable medium at the point of use.

These inhalants are prepared according to a known method.

For example, an inhalation solution is prepared by appropriately selecting antiseptics (benzalkonium chloride, paraben, etc.), colorants, buffering agents (sodium phosphate, sodium acetate, etc.), isotonizing agents (sodium chloride, concentrated glycerin, etc.), thickeners (carboxyvinyl polymer, etc.), and absorption accelerator, if necessary.

An inhalation powder is prepared by appropriately selecting lubricants (stearic acid and a salt thereof, etc.), binders (starch, dextrin, etc.), excipients (lactose, cellulose, etc.), colorants, antiseptics (benzalkonium chloride, paraben, etc.), and absorption accelerator, if necessary.

In case of administering the inhalation solution, a spraying apparatus (atomizer, nebulizer) is commonly used. In case of administering the inhalation powder, an inhalation administration apparatus for powder is commonly used.

The other composition for parenteral administration includes suppositories for intrarectal injection and pessaries for vaginal administration, which contain one or more active substances and are formulate by a conventional method.

Designation of the compound of the present invention is described below.

The compounds used in the present invention were commonly designated using a computer program ACD/Name Batch® (manufactured by Advanced Chemistry Development Inc.) which designates according to the regulation of IUPAC, or commonly designated according to IUPAC Nomenclature. For example, a compound of formula (I) wherein $A^1$ is an imidazole ring, ring $A^2$ is an imidazole ring, $E^1$ is a methylene group, $E^2$ is a methylene group, G is

$-CH_2-CO-$ (wherein ring $A^2$ is combined with the left side), $R^3$ is a methyl group,

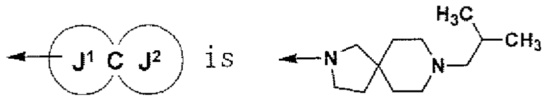

(wherein arrow directing the left is bonded to G), namely, a compound represented by the following formula:

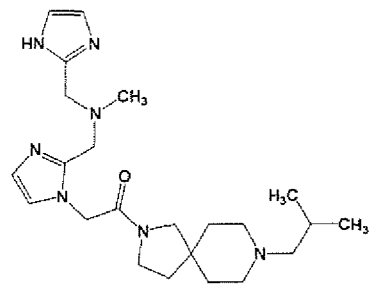

was designated as 1-(1H-imidazol-2-yl)-N-({1-[2-(8-isobutyl-2,8-diazaspiro[4.5]deca-2-yl)-2-oxoethyl]-1H-imidazol-2-yl}methyl)-N-methylmethaneamine.

The compound of the present invention has an antagonistic activity against CXCR4 and is therefore useful as a preventive and/or therapeutic agent for diseases involving CXCR4, namely, CXCR4-mediated diseases.

Since the compound of the present invention has a low risk of side effects (for example, phospholipidosis) and also has a small distribution volume, a risk of side effects is reduced extremely effectively. The compound of the present invention obtained by converting the compound into a prodrug has improved oral absorption and is also surely converted into the objective compound by an enzyme or gastric acid in vivo.

BEST MODE FOR CARRYING OUT THE INVENTION

The present invention is described in detail based on Examples, but the present invention is not limited thereto.

The point of separation by chromatography and the solvent in the parentheses shown in TLC indicate a dissolution medium or an eluent used, and the proportion indicates a volume ratio.

NMR is a measured value of $^1$HNMR at 300 MHz and the solvent shown in the parentheses of NMR indicates a solvent used in the measurement.

Example 1: Ethyl (2-formyl-1H-imidazol-1-yl)acetate

To an N-methylpyrrolidone (25 mL) solution of 1H-imidazole-2-carbaldehyde (2.0 g), potassium carbonate (2.9 g) and ethyl chloroacetate (6.7 mL) were added. The resultant solution was stirred at room temperature for 6 hours. To the reaction solution, water (50 mL) was added. The aqueous layer was extracted twice with ethyl acetate. The organic layers were combined, washed with saturated brine and then dried over anhydrous sodium sulfate. The anhydrous sodium sulfate was removed by filtration and then the organic solvent was conentrated under reduced pressure. The residue was purified by silica gel chromatography (n-hexane : ethyl acetate = 60 : 40 → 0 : 100) to obtain the title compound having the following physical properties (2.9 g).

TLC: Rf 0.54 (dichloromethane: methanol : 28% ammonia water = 90 : 10 : 1); NMR($CDCl_3$): δ 1.30(t, J = 7.2 Hz, 3H), 4.25(q, J = 7.2 Hz, 2H), 5.14(s, 2H), 7.15(d, J = 0.9 Hz, 1H), 7.33(d, J = 0.9 Hz, 1H), 9.79(s, 1H).

Example 2: N,N-dimethyl-2-[(methylamino)methyl]-1H-imidazole-1-sulfonamide

To a 1% acetic acid-dichloromethane solution (16 mL) of 2-formyl-N,N-dimethyl-1H-imidazole-1-sulfonamide (1.0 g) and methylamine hydrochloride (665 mg), triethylamine (1.4 mL) and sodium triacetoxyborohydride (1.3 g) were added. The reaction solution was stirred at room temperature for 5 hours. To the reaction solution, an aqeuous sodium hydrogencarbonate solution (50 mL) was added. The aqueous layer was extracted twice with dichloromethane. The organic layers were combined, washed with saturated brine and then dried over anhydrous sodium sulfate. Anhydrous

sodium sulfate was removed by filtration and then the organic solvent was conentrated under reduced pressure. The residue was purified by silica gel chromatography (ethyl acetate : methanol = 1 : 0 → 7 : 3) to obtain the title compound (500 mg) having the following physical properties.
TLC: Rf 0.42 (dichloromethane : methanol : 28% ammonia water = 90 : 10 : 1); NMR($CDCl_3$): δ 2.44(s, 3H), 2.90(s, 6H), 4.02(s, 2H), 6.99(d, J = 1.2 Hz, 1H), 7.24(d, J = 1.2 Hz, 1H).

Example 3: Ethyl (2-{[({(1-[(dimethylamino)sulfonyl]-1H-imidazol-2-yl}methyl)(methyl)amino]methyl}-1H-imidazol-1-yl) acetate

To a 1% acetic acid-N,N-dimethylformamide (5 mL) solution of the compound synthesized in Example 1 (1.0 g) and the compound synthesized in Example 2 (370 mg), sodium triacetoxyborohydride (539 mg) was added. The reaction solution was stirred at room temperature for 3 hours. To the reaction solution, an aqeuous sodium hydrogencarbonate solution (20 mL) was added. The aqueous layer was extracted twice with ethyl acetate. The organic layers were combined, washed with saturated brine and then dried over anhydrous sodium sulfate. Anhydrous sodium sulfate was removed by filtration and the organic solvent was conentrated under reduced pressure. The residue was purified by silica gel chromatography (n-hexane : ethyl acetate = 4 : 1 → 0 : 1) to obtain the title compound having the following physical properties (554 mg).
TLC: Rf 0.26 (dichloromethane : methanol : 28% ammonia water = 90 : 10 : 1); NMR($CDCl_3$): δ 1.26(m, 3H), 2.27(s, 3H), 2.84(s, 6H), 3.79(s, 2H), 3.81(s, 2H), 4.15(m, 2H), 5.01(s, 2H), 6.87(d, J = 1.5 Hz, 1H), 6.95(d, J = 1.5 Hz, 1H), 6.99(m, 1H), 7.24(m, 1H).

Example 4: (2-{[({1-[(dimethylamino)sulfonyl]-1H-imidazol-2-yl}methyl)(methyl)amino]methyl}-1H-imidazol-1-yl)acetic acid

To a methanol (3 mL) solution of the compound (550 mg) synthesized in Example 3, an aqueous 2N-sodium hydroxide solution (1.5 mL) was added and the reaction solution was stirred at room temperature for one hour. To the reaction solution, 2N-hydrochloric acid (1.5 mL) was added. The reaction solution was conentrated under reduced pressure. The residue was dissolved in ethanol and an insoluble substance was removed by filtration. The filtrate was conentrated under reduced pressure to obtain the title compound having the following physical properties (522 mg).
TLC: Rf 0.01 (dichloromethane : methanol : 28% ammonia water = 90 : 10 : 1); NMR($CD_3OD$): δ 2.36(s, 3H), 291(s, 6H), 4.04(s, 2H), 4.05(s, 2H), 4.86(s, 2H), 7.08(d, J = 2.1 Hz, 1H), 7.38(d, J = 2.1 Hz, 1H), 7.42(d, J = 1.5 Hz, 1H), 7.47 (d, J = 1.5 Hz, 1H).

Example 4(1): 4-[(2-{[({1-[(dimethylamino)sulfonyl]-1H-imidazol-2-yl}methyl)(methyl)amino]methyl}-1H-imidazol-1-yl) methyl]benzoic acid

The same procedure as in Example 1 → Example 3 → Example 4 was carried out, except that 4-methoxycarbonyl-benzylbromide was used in place of ethyl chloroacetate in Example 1, the title compound having the following physical properties was obtained. TLC: Rf 0.1 (chloroform : methanol : 28% ammonia water = 80 : 10 : 1);
NMR($CD_3OD$): δ -2.21(s, 3H), 2.88(s, 6H), 3.73(s, 2H), 3.86(s, 2H), 5.41(s, 2H), 6.98(t, J = 1.8Hz, 1H), 7.03(d, J = 1.8Hz, 1H), 7.15(d, J = 8.4Hz, 2H), 7.17(t, J = 1.8Hz, 1H), 7.45(d, J = 1.8Hz, 1H), 7.94(d, J = 8.4Hz, 2H).

Example 5: tert-butyl 2-[(2-{[({1-[(dimethylamino)sulfonyl]-1H-imidazol-2-yl}rnethyl)(methyl)amino]methyl}-1H-imidazol-1-yl)acetyl]-2,8-diazaspiro[4.5]decane-8-carboxylate

To an N,N-dimethylformamide (5 mL) solution of the compound (522 mg) synthesized in Example 4 and tert-butyl 2,8-diazaspiro[4.5]decane-8-carboxylate (346 mg), N,N-diisopropylethylamine (0.38 mL) and N,N,N',N'-tetramethyluroniumhexafluorophosphate (HATU) (674 mg) were added. The reaction solution was stirred at room temperature for one hour. To the reaction solution, an aqeuous sodium hydrogencarbonate solution(20 mL) was added. The aqueous layer was extracted twice with ethyl acetate. The organic layers were combined, washed with saturated brine and then dried over anhydrous sodium sulfate. Anhydrous sodium sulfate was removed by filtration, the organic solvent was conentrated under reduced pressure. The residue was purified by silica gel chromatography (n-hexane : ethyl acetate = 4 : 1 → 0 : 1) to obtain the title compound (656 mg) having the following physical properties. TLC: Rf 0.13 (dichloromethane : methanol : 28% ammonia water = 90 : 10 : 1);
NMR($CDCl_3$): δ 1.46(s, 9H), 1.54(m, 4H), 1.78(t, J = 7.2 Hz, 1H), 1.89(t, J = 7.2 Hz, 1H), 2.24(s, 3H), 2.86(s, 6H), 3.37 (m, 5H), 3.59(m, 3H), 3.73(s, 2H), 3.80(s, 2H), 4.96(s, 2H), 6.88(m, 1H), 6.93(m, 1H), 6.95(m, 1H), 7.23(m, 1H).

Example 6: 1-{1-[2-(2,8-diazaspiro[4.5]deca-2-yl)-2-oxoethyl]-1H-imidazol-2-yl}-N-(1H-imidazol-2-ylmethyl)-N-methylmethaneamine

A 2N-hydrochloric acid (5 mL) solution of the compound (656 mg) synthesized in Example 5 was stirred at 80°C for 2 hours. After cooling to room temperature, the reaction solution was conentrated under reduced pressure. The residue was purified by silica gel chromatography (NH silica, dichloromethane : methanol = 10 : 1 → 5 : 1) to obtain the title compound (404 mg) having the following physical properties.
TLC: Rf 0.18 (methanol : 28% ammonia water = 10 : 1);
NMR(CDCl3): δ 1.52(m, 4H), 1.79(t, J = 6.9 Hz, 1H), 1.90(t, J = 6.9 Hz, 1H), 2.28(s, 3H), 2.81(m, 4H), 3.30(m, 1H), 3.38 (m, 1H), 3.43(m, 1H), 3.48(s, 2H), 3.57(m, 3H), 4.75(m, 2H), 6.98(m, 3H), 7.04(m, 1H).

Example 7: 1-(1H-imidazol-2-yl)-N-({1-[2-(8-isobutyl-2,8-diazaspiro[4.5]deca-2-yl)-2-oxoethyl]-1H-imidazol-2-yl}methyl)-N-methylmethaneamine

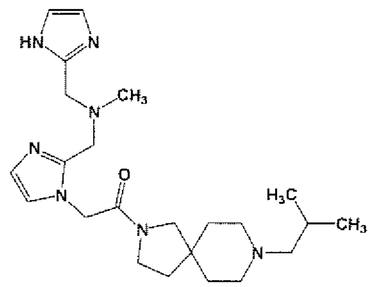

The same procedure as in Example 3 was carried out, except that the compound (80 mg) synthesized in Example 6 and 2-methylprorpanal (39 μL) were used, the title compound having the following physical properties (78 mg) was obtained.
TLC: Rf 0.53 (dichloromethane : methanol : 28% ammonia water = 80 : 10 : 1);
NMR($CDCl_3$): δ 0.85-0.92(m, 6H), 1.50-1.95(m, 7H), 2.04-2.10(m, 2H), 2.14-2.50(m, 4H), 2.28(s, 3H), 3.25-3.61(m, 8H), 4.74(s, 2H), 6.92-7.05(m, 4H).

Example 7(1) to Example 7(6):

The same procedure as in Example 3 → Example 4 → Example 5 → Example 6 → Example 7 was carried out, except that corresponding aldehyde compound was used in place of the compound synthesized in Example 1 in Example 3 and corresponding carbonyl compound was used in place of 2-methylpropanal in Example 7, the following compounds of the present invention were obtained.

Example 7(1): 1-{1-[2-(8-cyclohexyl-2,8-diazaspiro[4.5]deca-2-yl)-2-oxoethyl]-1H-imidazol-2-yl}-N-(1H-imidazol-2-ylmethyl)-N-methylmethaneamine

TLC: Rf 0.45 (dichloromethane : methanol : 28% ammonia water = 80 : 10 : 1); NMR($CDCl_3$): δ 1.00-1.34(m, 6H), 1.50-1.94(m, 10H), 2.28(s, 3H), 2.21-2.68(m, 5H), 3.23-3.60(m, 8H), 4.70-4.77(m, 2H), 6.93-7.05(m, 4H).

Example 7(2): 1-(1H-benzoimidazol-2-yl)-N-({1-[3-(8-isobutyl-2,8-diazaspiro[4.5]deca-2-yl)-3-oxopropyl]-1H-imidazol-2-yl}methyl)-N-methylmethaneamine

TLC: Rf 0.29 (dichloromethane : methanol: 28% ammonia water = 90 : 10 : 1);
NMR($CDCl_3$): δ 0.85-0.95(m, 6H), 1.50-1.90(m, 7H), 2.06(d, J = 7.2 Hz, 2H), 2.30(s, 3H), 2.18-2.50(m, 4H), 2.76-2.85 (m, 2H), 3.21(s, 1H), 3.38(s, 1H), 3.46(t, J = 7.2 Hz, 1H), 3.59(t, J = 7.2 Hz, 1H), 3.70-3.75(m, 2H), 3.80-3.85(m, 2H), 4.38-4.46(m, 2H), 6.96-7.05(m, 2H), 7.18-7.26(m, 2H), 7.54(m, 1H), 7.73(m, 1H).

Example 7(3): 1-(1H-benzoimidazol-2-yl)-N-[(1-{3-[8-(1-ethylpropyl)-2,8-diazaspiro[4.5]deca-2-yl]-3-oxopropyl}-1H-imidazol-2-yl)methyl]-N-methylmethaneamine

TLC: Rf 0.29 (dichloromethane : methanol: 28% ammonia water = 90 : 10 : 1);
NMR($CDCl_3$): δ 0.89(t, J = 7.2 Hz, 6H), 1.20-1.85(m, 10H), 2.15(m, 1H), 2.30(s, 3H), 2.34-2.58(m, 4H), 2.81(t, J = 7.2 Hz, 2H), 3.20(s, 1H), 3.38(s, 1H), 3.45(t, J = 7.2 Hz, 1H), 3.58(t, J = 7.2 Hz, 1H), 3.70-3.74(m, 2H), 3.80-3.85(m, 2H), 4.38-4.46(m, 2H), 6.97-7.04(m, 2H), 7.18-7.25(m, 2H), 7.54(m, 1H), 7.73(m, 1H).

Example 7(4): 1-(1H-imidazol-2-yl)-N-({1-[3-(8-isobutyl-2,8-diazaspiro[4.5]deca-2-yl)-3-oxopropyl]-1H-imidazol-2-yl}methyl)-N-methylmethaneamine

TLC: Rf 0.30 (dichloromethane : methanol : 28% ammonia water = 90 : 10 : 1);
NMR($CDCl_3$): δ 0.88-0.92(m, 6H), 1.50-1.84(m, 7H), 2.03-2.10(m, 2H), 2.16-2.30(m, 5H), 2.35-2.51(m, 2H), 2.78(t, J = 7.2Hz, 2H), 3.17(s, 1H), 3.33(s, 1H), 3.42(t, J = 7.2Hz, 1H), 3.53(t, J = 7.2Hz, 1H), 3.58-3.63(m, 4H), 4.36(t, J = 7.2Hz, 2H), 6.96-7.10(m, 4H).

Example 7(5): 1-(1H-imidazol-2-yl)-N-({1-[4-(8-isobutyl-2,8-diazaspiro[4.5]deca-2-yl)-4-oxobutyl]-1H-imidazol-2-yl}methyl)-N-methylmethaneamine

TLC: Rf 0.30 (dichloromethane : methanol : 28% ammonia water = 90 : 10 : 1); NMR($CDCl_3$): δ 0.88-0.92(m, 6H), 1.50-1.85(m, 7H), 2.02-2.10(m, 2H), 2.12-2.50(m, 11H), 3.17(s, 1H), 3.32(s, 1H), 3.41(t, J = 7.2Hz, 1H), 3.46-3.62 (m, 5H), 4.10(t, J = 7.2Hz, 2H), 6.92(m, 1H), 6.95-7.08(m, 3H).

Example 7(6): 1-(1H-imidazol-2-yl)-N-[(1-{4-[(8-isobutyl-2,8-diazaspiro[4.5]deca-2-yl)carbonyl]benzyl}-1H-imidazol-2-yl)methyl]-N-methylmethaneamine

TLC: Rf 0.35 (chloroform: methanol : 28% ammonia water = 80 : 10 : 1);
NMR(DMSO-$D_6$): δ 0.81(dd, J = 13.8, 6.6Hz, 6H), 1.34-1.59(m, 4H), 1.59-1.82(m, 3H), 1.84-1.98(m, 2H), 1.98-2.17(m, 4H), 2.16-2.44(m, 3H), 3.17(s, 2H), 3.34-3.58(m, 6H), 5.17-5.31(m, 2H), 6.67-7.14(m, 5H), 7.15-7.24(m, 1H), 7.34-7.51 (m, 2H), 11.59-12.20(m, 1H).

Example 8: 1-[3-(5,5-dimethyl-1,3-dioxane-2-yl)propyl]-1H-imidazole-2-carbaldehyde

The same procedure as in Example 1 was carried out, except that 1H-imidazole-2-carbaldehyde (1.0 g) and 2-(3-bromopropyl)-5,5-dimethyl-1,3-dioxane (5.8 mL) were used to obtain the title compound having the following physical properties (2.53 g). TLC: Rf 0.56 (ethyl acetate);
NMR($CDCl_3$): δ 0.72(s, 3H), 1.24(s, 3H), 1.68(m, 4H), 1.92(m, 2H), 3.41(m, 2H), 3.58(m, 2H), 4.42(m, 3H), 7.17(d, J = 0.9 Hz, 1H), 7.26(d, J = 0.9 Hz, 1H), 9.80(s, 1H).

Example 9: 2-{[({1-[3-(5,5-dimethyl-1,3-dioxane-2-yl)propyl]-1H-imidazol-2-yl}methyl)(methyl)amino]methyl}-N,N-dimethyl-1H-imidazole-1-sulfonamide

The same procedure as in Example 3 was carried out, except that the compound (500 mg) synthesized in Example 2 and the compound (867 mg) synthesized in Example 8 were used, the title compound (924 mg) having the following physical properties was obtained.
TLC: Rf 0.43 (dichloromethane : methanol: 28% ammonia water = 90 : 10 : 1); NMR($CDCl_3$): δ 0.71(s, 3H), 1.17(s, 3H), 1.62(m, 2H), 1.82(m, 2H), 2.29(s, 3H), 2.85(s, 6H), 3.39(m, 2H), 3.58(m, 2H), 3.76(s, 2H), 3.85(s, 2H), 4.01(t, J = 7.5 Hz, 1H), 4.40(t, J = 5.4 Hz, 1H), 6.87(d, J = 1.8 Hz, 1H), 6.91(d, J = 1.8 Hz, 1H), 7.01(d, J = 1.8 Hz, 1H), 7.23(d, J = 1.8 Hz, 1H).

Example 10: 4-(2-{[(1H-imidazol-2-ylmethyl)(methyl)amino]methyl}-1H-imidazol-1-yl)butanal

To an acetone/water (1.5 mL/1.5 mL) solution of the compound (300 mg) synthesized in Example 9, p-toluenesulfonic acid monohydrate (34 mg) was added. The reaction solution was stirred under reflux for 4 hours. The reaction solution was cooled to room temperature and then water (20 mL) was added. The aqueous layer was extracted twice with ethyl acetate and an aqeuous sodium hydrogencarbonate solution (20 mL) was aded to the aqueous layer. The aqueous layer was extracted twice with dichloromethane. The dichloromethane layers ware washed with saturated brine and then dried over anhydrous sodium sulfate. Ahydrous sodium sulfate was removed by filtration and then the organic

solvent was conentrated under reduced pressure. Without purifying the residue, the title compound (30 mg) having the following physical properties was obtained. TLC: Rf 0.11 (dichloromethane : methanol : 28% ammonia water = 90 : 10 : 1); NMR($CDCl_3$): δ 2.14(m, 2H), 2.34(s, 3H), 2.52(t, J = 6.6 Hz, 2H), 4.06(m, 2H), 5.52(m, 4H), 6.94(s, 1H), 7.04(m, 3H), 9.76(s, 1H).

Example 11: tert-butyl 2-[4-(2-{[(1H-imidazol-2-ylmethyl)(methyl)ainmo]methyl}-1H-imidazol-1-yl)butyl]-2,8-diazaspiro [4.5]decane-8-carboxylate

The same procedure as in Example 3 was carried out, except that the compound (250 mg) synthesize in Example 10 and tert-butyl 2,8-diazaspiro[4.5]decane-8-carboxylate (230 mg) were used, the title compound (183 mg) having the following physical properties were obtained.
TLC: Rf 0.35 (dichloromethane : methanol : 28% ammonia water = 50 : 10 : 1); NMR($CDCl_3$): δ 1.45(s, 9H), 1.53(m, 4H), 1.63(t, J = 6.6 Hz, 2H), 1.80(m, 4H), 2.37(m, 5H), 2.55(m, 2H), 3.31(m, 2H), 3.39(m, 2H), 3.51(m, 4H), 4.00(t, J = 7.2 Hz, 2H), 6.94(d, J = 1.2 Hz, 1H), 7.03(m, 3H).

Example 12: 1-{1-[4-(2,8-diazaspiro[4.5]deca-2-yl)butyl]-1H-imidazol-2-yl}-N-(1H-imidazol-2-ylmethyl)-N-methylmethaneamine

To a methanol (1 mL) solution of the compound (183 mg) synthesized in Example 11, 4N-hydrogen chloride : 1,4-dioxane (1 mL) was added. The reaction solution was stirred at room temperature for 2 hours. The reaction solution was conentrated under reduced pressure. The residue was purified by silica gel chromatography (NH silica, ethyl acetate : methanol = 10 : 1 → 5 : 1) to obtain the title compound (150 mg) having the following physical properties.
TLC: Rf 0.02 (dichloromethane : methanol : 28% ammonia water = 50 : 10 : 1);
NMR($CDCl_3$): δ 1.50(m, 4H), 1.60(m, 2H), 1.84(m, 4H), 2.36(m, 5H), 2.42(m, 2H), 2.56(t, J = 6.9 Hz, 2H), 2.78(t, J = 5.4 Hz, 4H), 3.52(m, 4H), 3.99(t, J = 7.5 Hz, 2H), 6.93(d, J = 1.2 Hz, 1H), 7. 02(d, J = 1.2 Hz, 1H), 7.04(s, 2H).

Example 13: 1-(1H-imidazol-2-yl)-N-({1-[4-(8-isobutyl-2,8-diazaspiro[4.5]deca-2-yl)butyl]-1H-imidazol-2-yl}methyl)-N-methylmethaneamine

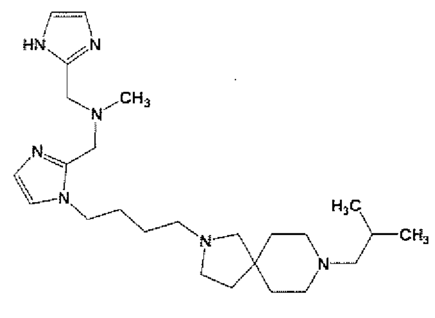

The same procedure as in Example 3 was carried out, except that the compound (75 mg) synthesized in Example 12 and 2-methylpropanal (36 μL) were used, the title compound (66 mg) having the following physical properties were obtained.
TLC: Rf 0.60 (dichloromethane : methanol : 28% ammonia water = 50 : 10 : 1); NMR($CDCl_3$): δ 0.88(d, J = 6.6 Hz, 6H), 1.46-1.90(m, 11H), 2.03(d, J = 7.2 Hz, 2H), 2.20-2.46(m, 11H), 2.52(t, J = 6.9 Hz, 2H), 3.51(s, 2H), 3.52(s, 2H), 3.99(t, J = 7.2 Hz, 2H), 6.93(d, J = 1.5 Hz, 1H), 7.01(d, J = 1.5 Hz, 1H), 6.98-7.10(m, 2H).

Example 13(1) to Example 13(7):

The same procesure as in Example 9 → Example 10 → Example 11 → Example 12 → Example 13 was carried out, except that corresponding aldehyde compound was used in place of the compound synthesized in Example 2 and corresponding amine compound was used in place of the compound synthesized in Example 8 in Example 9 and that corresponding carbonyl compound was used in place of 2-methylpropanal in Example 13, the following compounds of the present invention were obtained.

Example 13(1): 1-{1-[4-(8-cyclohexyl-1,8-diazaspiro[4.5]deca-2-yl)butyl]-1H-imidazol-2-yl}-N-(1H-imidazol-2-ylmethyl)-N-methylmethaneamine

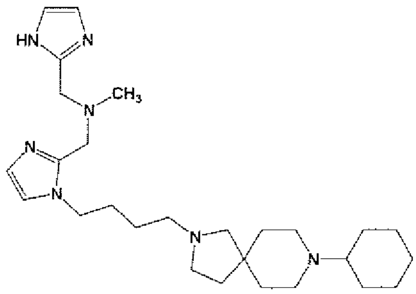

TLC: Rf 0.58 (dichloromethane : methanol: 28% ammonia water = 50 : 10 : 1); NMR($CDCl_3$): δ 1.00-1.32(m, 6H), 1.46-1.68 (m, 10H), 1.70-1.92(m, 4H), 2.18-2.55(m, 14H), 3.51(s, 2H), 3.52(s, 2H), 4.00(t, J = 7.2 Hz, 2H), 6.93(d, J = 1.5 Hz, 1H), 7.01(d, J = 1.5 Hz, 1H), 6.98-7.10(m,2H).

Example 13(2): 1-(1H-benzoimidazol-2-yl)-N-({1-[3-(8-isobutyl-2,8-diazaspiro[4.5]deca-2-yl)propyl]-1H-imidazol-2-yl} methyl)-N-methylmethaneamine

TLC: Rf 0.26 (dichloromethane : methanol: 28% ammonia water = 90 : 10 : 1); NMR($CDCl_3$): δ 0.86(d, J = 6.6 Hz, 6H), 1.45-1.82(m, 7H), 1.93-2.04(m, 4H), 2.20-2.32(m, 4H), 2.40-2.50(m, 7H), 2.66(t, J = 7.2 Hz, 2H), 3.57(s, 2H), 3.78(s, 2H), 4.00(t, J = 7.2 Hz, 2H), 6.92(d, J = 1.2 Hz, 1H), 7.03(d, J = 1.2 Hz, 1H), 7.20-7.30(m, 2H), 7.48(m, 1H), 7.77 (m, 1H).

Example 13(3): 1-(1H-benzoimidazol-2-yl)-N-[(1-{3-[8-(1-ethylpropyl)-2,8-diazaspiro[4.5]deca-2-yl]propyl}-1H-imidazol-2-yl)methyl]-N-methylmethaneamine

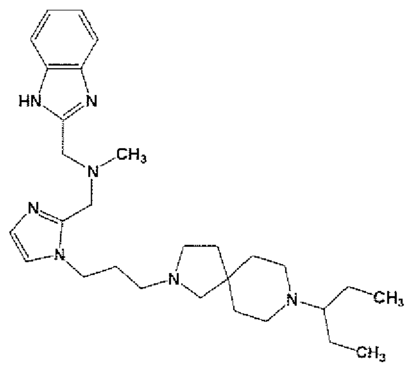

TLC: Rf 0.22 (dichloromethane : methanol : 28% ammonia water = 90 : 10 : 1);
NMR($CDCl_3$): δ 0.87(t, J = 7.2 Hz, 6H), 1.18-1.80(m, 10H), 1.92-2.15(m, 3H), 2.30-2.50(m, 11H), 2.66(t, J = 7.2 Hz, 2H), 3.57(s, 2H), 3.78(s, 2 H), 3.99(t, J = 7.2 Hz, 2H), 6.92(d, J = 1.2 Hz, 1H), 7.03(d, J = 1.2 Hz, 1H), 7.18-7.30(m, 2H), 7.47 (m, 1H), 7.75(m, 1H).

Example 13(4): N-benzyl-1-(1H-imidazol-2-yl)-N-({1-[3-(8-isobutyl-2,8-diazaspiro[4.5]deca-2-yl)propyl]-1H-imidazol-2-yl}methyl)methaneamine

TLC: Rf 0.24 (dichloromethane : methanol : 28% ammonia water = 90 : 10 : 1); NMR($CDCl_3$): δ 0.88(d, J = 6.6 Hz, 6H), 1.46-1.92(m, 9H), 2.03(d, J = 7.5 Hz, 2H), 2.20-2.35(m, 8H), 2.47(t, J = 6.9 Hz, 2H), 3.48(s, 2H), 3.63(s, 2H),

3.67(s, 2H), 3.90(t, J = 6.9 Hz, 2H), 6.92(d, J = 1.2 Hz, 1H), 7.00(d, J = 1.2 Hz, 1H), 7.05-7.14(m, 2H), 7.25-7.42(m, 5H).

Example 13(5): N-benzyl-1-(1-{3-[8-(1-ethylpropyl)-2,8-diazaspiro[4.5]deca-2-yl]propyl}-1H-imidazol-2-yl)-N-(1H-imidazol-2-ylmethyl)methaneamine

TLC: Rf 0.26 (dichloromethane : methanol: 28% ammonia water = 90 : 10 : 1); NMR($CDCl_3$): δ 0.88(t, J = 7.2 Hz, 6H), 1.20-1.90(m, 12H), 2.11(m, 1H), 2.20-2.30(m, 4H), 2.32-2.45(m, 4H), 2.47(t, J = 6.9 Hz, 2H), 3.48(s, 2H), 3.62 (s, 2H), 3.67(s, 2H), 3.90(t, J = 6.9 Hz, 2H), 6.92(d, J = 1.2 Hz, 1H), 7.00(d, J = 1.2 Hz, 1H), 7.04-7.14(m, 2H), 7.22-7.42 (m, 5H).

Example 13(6): 1-(1H-imidazol-2-yl)-N-({1-[2-(8-isobutyl-2,8-diazaspiro[4.5]deca-2-yl)ethyl]-1H-imidazol-2-yl}methyl)-N-methylmethaneamine

TLC: Rf 0.19 (dichloromethane : methanol : 28% ammonia water = 90 : 10 : 1); NMR($CDCl_3$): δ 0.88(d, J = 6.6Hz, 6H), 1.50-1.70(m, 6H), 1.78(m, 1H), 2.03(d, J = 7.2Hz, 2H), 2.22-2.38(m, 4H), 2.31 (s, 3H), 2.43(s, 2H), 2.62(t, J = 7.2Hz, 2H), 2.81(t, J = 7.2Hz, 2H), 3.57(s, 2H), 3.61(s, 2H), 4.07(t, J = 7.2Hz, 2H), 6.96(d, J = 1.2Hz, 1H), 6.98(d, J = 1.2Hz, 1H), 7.02(s, 2H).

Example 13(7): 1-(1H-imidazol-2-yl)-N-({1-[3-(8-isobutyl-2,8-diazaspiro[4.5]deca-2-yl)propyl]-1H-imidazol-2-yl}methyl)-N-methylmethaneamine

TLC: Rf 0.18 (dichloromethane : methanol : 28% ammonia water = 90 : 10 : 1); NMR(CDCl3): δ 0.88(d, J = 6.6Hz, 6H), 1.50-1.83(m, 7H), 1.70-2.06(m, 2H), 2.03(d, J = 7.2Hz, 2H), 2.20-2.38(m, 4H), 2.34(s, 3H), 2.38-2.45(m, 4H), 2.58(t, J = 6.9Hz, 2H), 3.51(s, 2H), 3.56(s, 2H), 4.00(t, J = 7.2Hz, 2H), 6.91(d, J = 1.5Hz, 1H), 6.99(d, J = 1.5Hz, 1H), 7.00-7.08(m, 2H).

Example 14: 1-(3-{[tert-butyl(dimethyl)silyl]oxy}propyl)-1H-imidazole-2-carbaldehyde

The same procedure as in Example 1 was carried out, except that 1-tert-butyldimethylsiloxy-3-bromopropane was used in place of ethyl chloroacetate in Example 1, the title compound having the following physical properties was obtained.
TLC: Rf 0.81 (ethyl acetate : methanol : 28% ammonia water = 90 : 10 : 1); NMR($CDCl_3$): δ 0.06(s, 9H), 0.91(s, 6H), 1.90-2.02(m, 2H), 3.57(t, J = 5.7Hz, 2H), 4.49(t, J = 6.9Hz, 2H), 7.16(s, 1H), 7.26(s, 1H), 9.79(s, 1H).

Example 15: Methyl {4-[({[1-(3-{[tert-butyl(dimethyl)silyl]oxy}propyl)-1H-imidazol-2-yl]methyl}amino)methyl]phenyl}acetate

Under an argon atmosphere, the compound synthesized in Example 14 (18.9 g) and methyl [4-(aminomethyl) phenyl]acetate hydrochloride (15.2 g) were dissolved in methanol (200 mL) and trimethyl orthoformate (23.2 mL) was added, followed by stirring at room temperature for 16 hours. The reaction solution was cooled to 0°C and sodium borohydride (5.33 g) was added, followed by stirring for 3 hours while heating to room temperature. To the reaction solution, water (100 mL) was added, followed by extraction twice with ethyl acetate (200 mL). The organic layers were combined, washed with saturated brine and then dried over anhydrous magnesium sulfate. Anhydrous magnesium sulfate was removed by filtration and then the filtrate was concentrated. The residue was purified by silica gel chromatography (manufactured by FUJI SILYSIA CHEMICAL LTD., CHROMATOREX NH (trade name)) (n-hexane : ethyl acetate = 5 : 1 → 1 : 1) to obtain the title compound having the following physical properties (13.3 g). TLC: Rf 0.83 (ethyl acetate : methanol : 28% ammonia water = 80 : 20 : 2);
NMR($CDCl_3$): δ 0.05(s, 9H), 0.91(s, 6H), 1.85-1.98(m, 2H), 3.57(t, J = 5.7Hz, 2H), 3.61(s, 2H), 3.68(s, 3H), 3.80(s, 2H), 3.85(s, 2H), 4.06(t, J = 7.2Hz, 2H), 6.86(d, J = 1.2Hz, 1H), 6.95(d, J = 1.2Hz, 1H), 7.22(d, J = 8.4Hz, 2H), 7.29(d, J = 8.4Hz, 2H).

Example 16: Methyl [4-({{[1-(3-{[tert-butyl(dimethyl)silyl]oxy}propyl)-1H-imidazol-2-yl]methyl}[(1-{[2-(trimethylsilyl) ethoxy]methyl}-1H-imidazol-2-yl)methyl] amino}methyl)phenyl]acetate

The same procedure as in Example 3 was carried out, except that 1-{[2-(trimethylsilyl)ethoxy]methyl}-1H-imidazole-2-carbaldehyde (CAS Registry Number: 101226-42-0) was used in place of the compound synthesized in Example 1 and Example 15 was used in place of the compound synthesized in Example 2 in Example 3, the title compound having the following physical properties was obtained.

TLC: Rf 0.62 (ethyl acetate : methanol : 28% ammonia water = 90 : 10 : 2);
NMR($CDCl_3$): δ 0.85-1.00(m, 15H), 0.76(t, J = 8.4Hz, 2H), 0.90(s, 9H), 1.90-2.00(m, 2H), 3.20(t, J = 8.4Hz, 2H), 3.39(t, J = 5.7Hz, 2H), 3.58-3.80(m, 11H), 4.08-4.18(m, 2H), 4.97(s, 2H), 6.84(d, J = 1.5Hz, 1H), 6.94-7.00(m, 3H), 7.15-7.22 (m, 4H).

Example 17: Methyl {4-[({[1-(3-hydroxypropyl)-1H-imidazol-2-yl]methyl}[(1-{[2-(trimethylsilyl)ethoxy]methyl}-1H-imidazol-2-yl)methyl]amino)methyl]phenyl}acetate

Under an argon atmosphere, the compound (10.3 g) synthesized in Example 16 was dissolved in tetrahydrofuran (100 mL) and tetrahydrofuran solution (40 mL) of 1M tetrabutylammonium fluoride was added, followed by stirring at room temperature for 2 hours. To the reaction solution, water (50 mL) was added, followed by extraction twice with ethyl acetate (100 mL). The organic layers were combined, washed with saturated brine and then dried over anhydrous magnesium sulfate. The anhydrous magnesium sulfate was removed by filtration and the filtrate was concentrated. The residue was purified by a preparative medium-pressure liquid chromatograph W-prep 2XY (trade name: manufactured by YAMAZEN CORPORATION, column: main column 3L, inject column 3L, (n-hexane : ethyl acetate = 1:1 → 0 : 1 → ethyl acetate : methanol : 28% ammonia water = 100 : 0 : 0 → 80 : 2 : 2), preparative mode GR) to obtain the title compound having the following physical properties (5.85 g).
TLC: Rf 0.33 (ethyl acetate : methanol : 28% ammonia water = 90 : 10: 2);
NMR($CDCl_3$): δ 0.04(s, 9H), 0.81(t, J = 8.4Hz, 2H), 0.80-0.85(m, 2H), 3.31(t, J = 8.4Hz, 2H), 3.45(t, J = 5.4Hz, 2H), 3.61 (s, 2H), 3.63(s, 2H), 3.70(s, 3H), 3.72(s, 2H), 3.73(s, 2H), 3.97(t, J = 6.9Hz, 2H), 4.60(brs, 1H), 5.04(s, 2H), 6.87(d, J = 1.5Hz, 1H), 6.93(d, J = 1.5Hz, 1H), 6.95(d, J = 1.5Hz, 1H), 7.00(d, J = 1.5Hz, 1H), 7.2 1 (d, J = 8.4Hz, 2H), 7.31(d, J = 8.1Hz, 2H).

Example 18: Methyl [4-({[(1-{3-[(methylsulfonyl)oxy]propyl}-1H-imidazol-2-yl)methyl][(1-{[2-(trimethylsilyl)ethoxy]methyl}-1H-imidazol-2-yl)methyl]amino}methyl)phenyl]acetate

Under an argon atmosphere, the compound (7.70 g) synthesized in Example 17 was dissolved in tetrahydrofuran (60 mL) and cooled to 0°C. Triethylamine (4.04 mL) was added and then methanesulfonyl chloride (1.46 mL) was added, followed by stirring at 0°C for one hour. To the reaction solution, ice water (100 mL) was added, followed by extraction twice with ethyl acetate (100 mL). The organic layers were combined, washed in turn with water and saturated brine, and then dried over anhydrous magnesium sulfate. Anhydrous magnesium sulfate was removed by filtration and then the filtrate was concentrated. Without purifying the residue, the title compound (7.70 g) having the following physical properties was obtained.
TLC: Rf 0.80 (ethyl acetate : methanol: 28% ammonia water = 80 : 20 : 2); NMR($CDCl_3$): δ 0.05(s, 9H), 0.77(t, J = 8.4Hz, 2H), 1.76-1.88(m, 2H), 2.99(s, 3H), 3.24(t, J = 8.4Hz, 2H), 3.61(s, 2H), 3.62(s, 2H), 3.69(s, 3H), 3.70(s, 2H), 3.76(s, 2H), 3.85(t, J = 6.0Hz, 2H), 3.99(t, J = 6.9Hz, 2H), 4.96(s, 2H), 6.87(m, 1H), 6.95(m, 1H), 6.99(s, 2H), 7.16-7.24(m, 4H).

Example 19: tert-butyl 2-{3-[2-({[4-(2-methoxy-2-oxoethyl)benzyl][(1-{[2-(trimethylsilyl)ethoxy]methyl}-1H-imidazol-2-yl)methyl]amino}methyl)-1H-imidazol-1-yl]propyl}-2,8-diazaspiro[4.5]decane-8-carboxylate

Under an argon atmosphere, the compound (7.70 g) synthesized in Example 18 was dissolved in acetonitrile (80 mL) and then diisopropylethylamine (5.08 mL) was added. Then, tert-butyl 2,8-diazaspiro[4.5]decane-8-carboxylate (5.26 g) was added, followed by stirring at 60°C for 16 hours. To the reaction solution, water (100 mL) was added, followed by extraction twice with ethyl acetate (100 mL). The organic layers were combined, washed with saturated brine and then dried over anhydrous magnesium sulfate. Anhydrous magnesium sulfate was removed by filtration and then the filtrate was concentrated. The residue was purified by a preparative medium-pressure liquid chromatograph W-prep 2XY (trade name: manufactured by YAMAZEN CORPORATION, column: main column 3L, inject column L, (n-hexane : ethyl acetate = 80 : 20 → 0 : 100 → ethyl acetate : methanol : 28% ammonia water = 100 : 0 : 0 → 80 : 20 : 2), preparative mode GR) to obtain the title compound (5.48 g) having the following physical properties. TLC: Rf 0.66 (ethyl acetate : methanol : 28% ammonia water = 90 : 10 : 2);
NMR($CDCl_3$): δ 0.06(s, 9H), 0.75(t, J = 8.1Hz, 2H), 1.35-1.80(m, 8H), 1.45(s, 9H), 2.11(t, J = 6.9Hz, 2H), 2.24(s, 2H), 2.43(t, J = 6.9Hz, 2H), 3.18(t, J = 8.1Hz, 2H), 3.22-3.50(m, 4H), 3.59(s, 2H), 3.61(s, 2H), 3.66(s, 2H), 3.67(s, 3H), 3.72 (t, J = 6.6Hz, 2H), 3.75(s, 2H), 4.97(s, 2H), 6.84(d, J = 0.9Hz, 1H), 6.90-7.00(m, 3H), 7.14-7.22(m, 4H).

Example 19(1) to Example 19(2):

The same procedure as in Example 15 → Example 16 → Example 17 → Example 18 → Example 19 was carried out, except that corresponding compound was used in place of methyl [4-(aminomethyl)phenyl]acetate hydrochloride in Example 15, the title compounds having the following physical properties were obtained.

Example 19(1): tert-butyl 2-{3-[2-({(4-ethoxy-4-oxobutyl)[(1-{[2-(trimethylsilyl)ethoxy]methyl}-1H-imidazol-2-yl)methyl] amino}methyl)-1H-imidazol-1-yl]propyl}-2,8-diazaspiro[4.5]decane-8-carboxylate

TLC: Rf 0.47 (ethyl acetate : methanol: 28% ammonia water = 90 : 10 : 2);
NMR($CDCl_3$): δ -0.03(s, 9H), 0.83(t, J = 8.4Hz, 2H), 1.23(t, J = 7.2Hz, 3H), 1.38-1.88(m, 10H), 1.46(s, 9H), 2.17(t, J = 7.5Hz, 2H), 2.22-2.38(m, 4H), 2.45-2.62(m, 4H), 3.22-3.50(m, 6H), 3.70(s, 2H), 3.75(s, 2H), 3.90(t, J = 6.6Hz, 2H), 4.07 (q, J = 7.2Hz, 2H), 5.16(s, 2H), 6.86(d, J = 1.2Hz, 1H), 6.92-6.98(m, 3H).

Example 19(2): tert-butyl 2-{3-[2-({(5-ethoxy-5-oxopentyl)[(1-{[2-(trimethylsilyl)ethoxy]methyl}-1H-imidazol-2-yl)methyl] amino}methyl)-1H-imidazol-1-yl]propyl}-2,8-diazaspiro[4.5]decane-8-carboxylate

TLC: Rf 0.60 (ethyl acetate : methanol : 28% ammonia water = 80 : 20 : 2);
NMR($CDCl_3$): δ -0.02(s, 9H), 0.83(t, J = 8.4Hz, 2H), 1.27(t, J = 7.2Hz, 3H), 1.40-1.58(m, 8H), 1.46(s, 9H), 1.64(t, J = 6.9Hz, 2H), 1.70-1.82(m,2H), 2.00-2.62(m, 10H), 3.24-3.50(m, 6H), 3.68(s, 2H), 3.73(s, 2H), 3.89(t, J = 6.6Hz, 2H), 4.12 (q, J = 7.2Hz, 2H), 5.18(s, 2H), 6.85(d, J = 1.2Hz, 1H), 6.94(d. J = 1.2Hz, 1H), 6.95(s, 2H).

Example 20: Methyl (4-{[({1-[3-(2,8-diazaspiro[4.5]deca-2-yl)propyl]-1H-imidazol-2-yl}methyl)(1H-imidazol-2-ylmethyl) amino]methyl}phenyl)acetate

Under an argon atmosphere, trifluoroacetic acid (20 mL) was added to the compound (5.48 g) synthesized in Example 19, followed by stirring at 60°C for 2 hours. The reaction solution was concentrated and the residue was purified by silica gel chromatography (manufactured by FUJI SILYSIA CHEMICAL LTD., CHROMATOREX NH (trade name)) (n-hexane : ethyl acetate = 4 : 1 → 1 : 1 → 0 : 1 → ethyl acetate : methanol = 100 : 0 → 80 : 20) to obtain the title compound (3.18 g) having the following physical properties.
TLC: Rf 0.40 (dichloromethane : methanol : 28% ammonia water = 80 : 20 : 2);
NMR($CDCl_3$): δ 1.60-2.00(m, 8H), 2.38-2.55(m, 4H), 2.62-2.76(m, 2H), 3.00-3.20(m, 4H), 3.60(s, 2H), 3.62-3.74(m, 7H), 3.90(t, J = 5.7Hz, 2H), 4.03(s, 2H), 6.92(d, J = 1.5Hz, 1H), 7-10-7.32(m, 7H).

Example 21: methyl (4-{[({1-[3-(8-cyclohexyl-2,8-diazaspiro[4.5]deca-2-yl)propyl]-1H-imidazol-2-yl}methyl)(1H-imidazol-2-ylmethyl)amino]methyl}phenyl)acetate

The same procedure as in Example 3 was carried out, except that cyclohexanone was used in place of the compound synthesized in Example 1 and the compound synthesized in Example 20 was used in place of the compound synthesized in Example in Example 3, the title compound having the following physical properties was obtained. TLC: Rf 0.29 (ethyl acetate : methanol : 28% ammonia water = 80 : 20 : 2);
NMR($CDCl_3$): δ 1.00-1.98(m, 18H), 2.18-2.60(m, 11H), 3.48(s, 2H), 3.59(s, 2H), 3.61(s, 2H), 3.64(s, 2H), 3.69(s, 3H), 3.92(t, J = 6.9Hz, 2H), 6.92(d, J = 1.2Hz, 1H), 7.00(d, J = 1.2Hz, 1H), 7.02-7.15(m, 2H), 7.24(d, J = 8.4Hz, 2H), 7.35(d, J = 8.4Hz, 2H), 12.5(brs, 1H).

Example 21(1) to Example 21(30):

The same procedure as in Example 14 → Example 15 → Example 16 → Example 17 → Example 18 → Example 19 → Example 20 → Example 21 was carried out, except that corresponding halide was used in place of 1-tert-butyldimethylsiloxy-3-bromopropane in Example 14, corresponding amine compound was used in place of methyl 4-aminomethylphenylacetate in Example 15, and corresponding ketone or aldehyde was used in place of cyclohexanone in Example 21, the title compounds having the following physical properties were obtained.

Example 21(1): Ethyl N-(1H-imidazol-2-ylmethyl)-N-({1-[3-(8-isobutyl-2,8-diazaspiro[4.5]deca-2-yl)propyl]-1H-imidazol-2-yl}methyl)glycinate

TLC: Rf 0.32 (dichloromethane : methanol : 28% ammonia water = 90 : 10 : 1); NMR(CDCl3): δ 0.88(d, J = 6.6Hz, 6H), 1.26(t, J = 7.2Hz, 3H), 1.50-1.65(m, 6H), 1.77(m, 1H), 1.90-2.03(m, 2H), 2.03(d, J = 7.2Hz, 2H), 2.22-2.40 (m, 8H), 2.54(t, J = 6.9Hz, 2H), 3.39(s, 2H), 3.67(s, 2H), 3.73(s, 2H), 4.10-4.20(m, 4H), 6.95(d, J = 1.2Hz, 1H), 7.00(d, J = 1.2Hz, 1H), 6.98-7.10(m, 2H).

Example 21(2): Methyl N-(1H-imidazol-2-ylmethyl)-N-({1-[3-(8-isobutyl-2,8-diazaspiro[4.5]deca-2-yl)propyl]-1H-imidazol-2-yl}methyl)-β-alaninate

TLC: Rf 0.41 (dichloromethane : methanol : 28% ammonia water = 80 : 10 : 2); NMR($CDCl_3$): δ 0.88(d, J = 6.6Hz, 6H), 1.48-1.65(m, 6H), 1.68-1.85(m, 1H), 1.85-1.98(m, 2H), 2.03(d, J = 7.3Hz, 2H), 2.18-2.40(m, 8H), 2.53(t, J = 7.0Hz, 2H), 2.61(t, J = 6.6Hz, 2H), 2.85(t, J = 6.6Hz, 2H), 3.50(s, 2H), 3.65(s, 2H), 3.67(s, 3H), 4.02(t, J = 6.9Hz, 2H), 6.94(d, J = 1.3Hz, 1H), 6.99(d, J = 1.3Hz, 1H), 7.04(s, 2H), 12.37(br-s, 1H).

Example 21(3): Ethyl 4-[(1H-imidazol-2-ylmethyl)({1-[3-(8-isobutyl-2,8-diazaspiro[4.5]deca-2-yl)propyl]-1H-imidazol-2-yl}methyl)amino]butanoate

TLC: Rf 0.60 (ethyl acetate : methanol : 28% ammonia water = 80 : 20 : 4);
NMR($CDCl_3$): δ 0.88(d, J = 6.6Hz, 6H), 1.24(t, J = 7.2Hz, 3H), 1.48-2.10(m, 13H), 2.20-2.62(m, 14H), 3.52(s, 2H), 3.61 (s, 2H), 4.05(t, J = 6.9Hz, 2H), 4.12(q, J = 7.2Hz, 2H), 6.95(s, 1H), 7.01(s, 1H), 6.98-7.12(m, 2H), 12.3(brs, 1H).

Example 21(4): Methyl (4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)acetate

TLC: Rf 0.66 (ethyl acetate : methanol: 28% ammonia water = 40 : 10 : 2);
NMR($CDCl_3$): δ 0.88(d, J = 6.6Hz, 6H), 1.47-1.64(m, 6H), 1.69-1.93(m, 3H), 2.03(d, J = 7.5Hz, 2H), 2.20-2.36(m, 8H), 2.48(t, J = 6.9Hz, 2H), 3.49(s, 2H), 3.60(s, 2H), 3.62(s, 2H), 3.65(s, 2H), 3.70(s, 3H), 3.92(t, J = 6.9Hz, 2H), 6.93(d, J = 1.3Hz, 1H), 7.01(d, J = 1.3Hz, 1H), 7.07(s, 1H), 7.11(s, 1H), 7.25(d, J = 8.4Hz, 2H), 7.37(d, J = 8.4Hz, 2H), 12.49(br-s, 1H).

Example 21(5): Ethyl 5-[(1H-imidazol-2-ylmethyl)({1-[3-(8-isobutyl-2,8-diazaspiro[4.5]deca-2-yl)propyl]-1H-imidazol-2-yl}methyl)amino]pentanoate

TLC: Rf 0.36 (dichloromethane : methanol : 28% ammonia water = 80 : 10 : 2); NMR(CDCl3): δ 0.88(d, J = 6.6Hz, 6H), 1.25(t, J = 7.1Hz, 3H), 1.48-1.84(m, 11H), 1.88-2.01(m, 2H), 2.03(d, J = 7.2Hz, 2H), 2.18-2.41(m, 10H), 2.46-2.58(m, 4H), 3.51(s, 2H), 3.59(s, 2H), 4.04(t, J = 7.0Hz, 2H), 4.12(q, J = 7.1Hz, 2H), 6.94(d, J = 1.3Hz, 1H), 6.99 (d, J = 1.3Hz, 1H), 6.99-7.08(m, 2H).

Example 21(6): Ethyl 3-(4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)propanoate

TLC: Rf 0.44 (dichloromethane : methanol: 28% ammonia water = 80 : 10 : 2); NMR($CDCl_3$): δ 0.88(d, J = 6.6Hz, 6H), 1.24(t, J = 7.1Hz, 3H), 1.46-1.63(m, 6H), 1.68-1.92(m, 3H), 2.03(d, J = 7.1Hz, 2H), 2.11-2.37(m, 8H), 2.48 (t, J = 6.9Hz, 2H), 2.60(t, J = 7.8Hz, 2H), 2.94(t, J = 7.8Hz, 2H), 3.47(s, 2H), 3.59(s, 2H), 3.63(s, 2H), 3.91(t, J = 7.0Hz, 2H), 4.12(q, J = 7.1Hz, 2H), 6.91(d, J = 1.3Hz, 1H), 6.99(d, J = 1.3Hz, 1H), 7.06(s, 1H), 7.10(s, 1H), 7.15(d, J = 8.1Hz, 2H), 7.31(d, J = 8.1Hz, 2H), 12.47(brs, 1H).

Example 21(7): Methyl 4-(4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)butanoate

TLC: Rf 0.34 (dichloromethane : methanol: 28% ammonia water = 80 : 10 : 2); NMR($CDCl_3$): δ 0.88(d, J = 6.6Hz, 6H), 1.46-1.66(m, 6H), 1.70-2.01(m, 5H), 2.03(d, J = 7.1Hz, 2H), 2.23-2.38(m, 10H), 2.48(t, J = 6.9Hz, 2H), 2.64 (t, J = 7.6Hz, 2H), 3.48(s, 2H), 3.61(s, 2H), 3.63(s, 2H), 3.67(s, 3H), 3.91(t, J = 6.9Hz, 2H), 6.92(d, J = 1.3Hz, 1H), 6.99 (d, J = 1.3Hz, 1H), 7.06(s, 1H), 7.10(s, 1H), 7.13(d, J = 8.1Hz, 2H), 7.30(d, J = 8.1Hz, 2H), 12.46(s, 1H).

Example 21(8): Ethyl 4-[({1-[3-(8-cyclohexyl-2,8-diazaspiro[4.5]deca-2-yl)propyl]-1H-imidazol-2-yl}methyl)(1H-imidazol-2-ylmethyl)amino]butanoate

TLC: Rf 0.78 (ethyl acetate : methanol : 28% ammonia water = 80 : 20 : 4);
NMR($CDCl_3$): δ 1.00-2.02(m, 20H), 1.25(t, J = 7.2Hz, 3H), 2.15-2.60(m, 15H), 3.51(s, 2H), 3.60(s, 2H), 4.04(t, J = 6.9Hz, 2H), 4.11(q, J = 7.2Hz, 2H), 6.94(d, J = 1.2Hz, 1H), 7.00(d, J = 1.2Hz, 1H), 6.95-7.10(m, 2H), 12.3(s, 1H).

Example 21(9): Ethyl 4-{[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}butanoate

TLC: Rf 0.55 (ethyl acetate : methanol : 28% ammonia water = 80 : 20 : 4);
NMR($CDCl_3$): δ 0.85(s, 9H), 1.08-2.62(m, 29H), 3.53(s, 2H), 3.60(s, 2H), 4.03(t, J = 6.9Hz, 2H), 4.11(q, J = 7.2Hz, 2H), 6.94(s, 1H), 6.99(s, 1H), 7.03(s, 2H).

Example 21(10): Methyl 4-[(1H-imidazol-2-ylmethyl)({1-[3-(8-isobutyl-2,8-diazaspiro[4.5]deca-2-yl)propyl]-1H-imidazol-2-yl}methyl)amino]butanoate

TLC: Rf 0.88 (dichloromethane : methanol : 28% ammonia water = 80 : 20 : 4); NMR($CDCl_3$): δ 0.88(d, J = 6.6Hz, 6H), 1.45-2.02(m, 11H), 2.03(d, J = 6.6Hz, 2H), 2.20-2.42(m, 10H), 2.45-2.60(m, 4H), 3.51(s, 2H), 3.60(s, 2H), 3.66(s, 3H), 4.04(t, J = 7.2Hz, 2H), 6.94(d, J = 1.2Hz, 1H), 7.00(d, J = 1.2Hz, 1H), 7.00-7.08(m, 2H), 12.3(brs, 1H).

Example 21(11): Ethyl 4-[(1H-imidazol-2-ylmethyl)({1-[2-(8-isobutyl-2,8-diazaspiro[4.5]deca-2-yl)ethyl]-1H-imidazol-2-yl}methyl)amino]butanoate

TLC: Rf 0.38 (dichloromethane : methanol: 28% ammonia water = 90 : 10 : 1); NMR($CDCl_3$): δ 0.88(d, J = 6.6Hz, 6H),1.25(t, J = 7.1Hz, 3H), 1.46-1.69(m, 6H), 1.70-1.84(m, 1H), 1.85-1.99(m, 2H), 2.04(d, J = 8.2Hz, 2H), 2.16-2.65(m, 12H), 2.79(t, J = 6.7Hz, 2H), 3.53(s, 2H), 3.61(s, 2H), 4.00-4.17(m, 4H), 6.97-7.02(m, 1H), 6.98(d, J = 1.3Hz, 1H), 7.00(d, J = 1.3Hz, 1H), 7.03-7.09(m, 1H), 12.16-12.39(m, 1H).

Example 21(12): Ethyl 4-[(1H-imidazol-2-ylmethyl)({1-[4-(8-isobutyl-2,8-diazaspiro[4.5]deca-2-yl)butyl]-1H-imidazol-2-yl}methyl)amino]butanoate

TLC: Rf 0.31 (dichloromethane : methanol : 28% ammonia water = 90 : 10 : 1); NMR($CDCl_3$): δ 0.88(d, J = 6.6Hz, 6H), 1.26(t, J = 7.1Hz, 3H), 1.45-1.99(m, 13H), 2.03(d, J = 7.3Hz, 2H), 2.18-2.58(m, 14H), 3.49(s, 2H), 3.59(s, 2H), 4.00(t, J = 7.3Hz, 2H), 4.12(q, J= 7.1Hz, 2H), 6.95(d, J = 1.3Hz, 1H), 7.00-7.04(m, 1H), 7.02(d, J = 1.3Hz, 1H), 7.06-7.10(m, 1H), 12.24-12.36(m, 1H).

Example 21(13): Ethyl (4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)acetate

TLC: Rf 0.46 (dichloromethane : methanol : 28% ammonia water = 80 : 20 : 2);
NMR($CDCl_3$): δ 0.88(d, J = 6.6Hz, 6H), 1.26(t, J = 7.2Hz, 3H), 1.45-1.95(m, 9H), 2.03(d, J = 7.2Hz, 2H), 2.18-2.42(m, 8H), 2.48(t, J = 6.9Hz, 2H), 3.48(s, 2H), 3.59(s, 4H), 3.64(s, 2H), 3.91(t, J = 6.9Hz, 2H), 4.14(q, J = 7.2Hz, 2H), 6.92(d, J = 1.2Hz, 1H), 7.00(d, J = 1.2Hz, 1H), 7.02-7.15(m, 2H), 7.24(d, J = 7.8Hz, 2H), 7.35(d, J = 7.8Hz, 2H), 12.5(brs, 1H).

Example 21(14): Ethyl (4-{[({1-[3-(8-cyclohexyl-2,8-diazaspiro[4.5]deca-2-yl)propyl]-1H-imidazol-2-yl}methyl)(1H-imidazol-2-ylmethyl)amino]methyl}phenyl)acetate

TLC: Rf 0.73 (dichloromethane : methanol: 28% ammonia water = 80 : 20 : 4);
NMR($CDCl_3$): δ 1.00-1.98(m, 21H), 2.20-2.62(m, 11H), 3.48(s, 2H), 3.59(s, 4H), 3.64(s, 2H), 3.91(t, J = 6.9Hz, 2H), 4.14(q, J = 7.2Hz, 2H), 6.92(s, 1H), 6.99(s, 1H), 7.02-7.15(m, 2H), 7.24(d, J = 8.1Hz, 2H), 7.35(d, J = 8.1Hz, 2H), 12.5(brs, 1H).

Example 21(15): Methyl [4-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenyl]acetate

TLC: Rf 0.55 (dichloromethane : methanol : 28% ammonia water = 80 : 20 : 2);
NMR($CDCl_3$): δ 0.84(s, 9H), 1.40-1.96(m, 8H), 1.99(s, 2H), 2.22-2.48(m, 8H), 2.48(t, J = 6.9Hz, 2H), 3.48(s, 2H), 3.59(s, 2H), 3.61(s, 2H), 3.64(s, 2H), 3.69(s, 3H), 3.91(t, J = 6.9Hz, 2H), 6.92(d, J = 1.5Hz, 1H), 7.00(d, J = 1.5Hz, 1H), 7.02-7.15(m, 2H), 7.24(d, J = 8.1Hz, 2H), 7.35(d, J = 8.1Hz, 2H), 12.5(brs, 1H).

Example 21(16): Ethyl [4-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenyl]acetate

TLC: Rf 0.79 (dichloromethane : methanol: 28% ammonia water = 80 : 20 : 4);
NMR($CDCl_3$): δ 0.84(s, 9H), 1.26(t, J = 7.2Hz, 3H), 1.42-1.80(m, 6H), 1.80-1.95(m, 2H), 1.99(s, 2H), 2.22-2.46(m, 8H),

2.48(t, J = 6.9Hz, 2H), 3.48(s, 2H), 3.60(s, 4H), 3.64(s, 2H), 3.91 (t, J = 7.2Hz, 2H), 4.14(q, J = 7.2Hz, 2H), 6.92(d, J = 1.2Hz, 1H), 6.99(d, J = 1.2Hz, 1H), 7.02-7.15(m, 2H), 7.24(d, J = 8.1Hz, 2H), 7.35(d, J = 8.1Hz, 2H), 12.5(brs, 1H).

Example 21(17): Methyl (3-{[(1H-imidazol-1-ylmethyl)({1-[3-(8-isobutyl-2,8-diazaspiro[4.5]deca-2-yl)propyl]-1H-imidazol-2-yl}methyl)amino]methyl}phenyl)acetate

TLC: Rf 0.55 (dichloromethane : methanol: 28% ammonia water = 80 : 10 : 2);
NMR($CDCl_3$): δ 0.88(d, J = 6.6Hz, 6H), 1.45-1.64(m, 6H), 1.69-1.91(m, 3H), 2.03(d, J = 7.3Hz, 2H), 2.19-2.37(m, 8H), 2.47(t, J = 6.8Hz, 2H), 3.49(s, 2H), 3.62(s, 4H), 3.67(s, 2H), 3.69(s, 3H), 3.90(t, J = 6.9Hz, 2H), 6.93(d, J = 1.3Hz, 1H), 7.00(d, J = 1.3Hz, 1H), 7.07(s, 1H), 7.11(s, 1H), 7.16-7.22(m, 1H), 7.23-7.37(m, 3H), 12.34-12.52(m, 1H).

Example 21(18): Methyl [3-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenyl]acetate

TLC: Rf 0.55 (dichloromethane : methanol : 28% ammonia water = 80 : 10 : 2);
NMR($CDCl_3$): δ 0.85(s, 9H), 1.42-1.61(m, 6H), 1.78-1.91(m, 2H), 1.99(s, 2H), 2.23-2.33(m, 4H), 2.40(t, J = 4.8Hz, 4H), 2.47(t, J = 7.0Hz, 2H), 3.49(s, 2H), 3.63(s, 4H), 3.67(s, 2H), 3.70(s, 3H), 3.90(t, J = 6.8Hz, 2H), 6.93(d, J = 1.3Hz, 1H), 7.00(d, J = 1.3Hz, 1H), 7.05-7.13(m, 2H), 7.15-7.23(m, 1H), 7.27-7.33(m, 3H).

Example 21(19): Ethyl (2E)-3-(4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)acrylate

TLC: Rf 0.34 (dichloromethane : methanol : 28% ammonia water = 90 : 10 : 1);
NMR($CDCl_3$): δ 0.88(d, J = 6.5Hz, 6H), 1.34(t, J = 7.1Hz, 3H), 1.46-1.60(m, 4H), 1.64-1.92(m, 5H), 2.02(d, J = 7.2Hz, 2H), 2.19-2.33(m, 8H), 2.46(t, J = 6.9Hz, 2H),3.49(s, 2H), 3.59(s, 2H), 3.67(s, 2H), 3.93(t, J = 6.9Hz, 2H), 4.26(q, J = 7.1Hz, 2H), 6.42(d, J = 15.9Hz, 1H), 6.93(d, J = 1.2Hz, 1H), 7.01(d, J = 1.2Hz, 1H), 7.06(s, 1H), 7.11(s, 1H), 7.42(d, J = 8.2Hz, 2H), 7.49(d, J = 8.2Hz, 2H), 7.67(d, J = 15.9Hz, 1H), 12.50(s, 1H).

Example 21(20): Ethyl 1-{4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)cyclopropanecarboxylate

TLC: Rf 0.67 (chloroform : methanol: 28% ammonia water = 40 : 10: 2);
NMR($CDCl_3$): δ 0.88(d, J = 6.6Hz, 6H), 1.11-1.22(m, 5H), 1.48-1.92(m, 12H), 1.98-2.10(m, 2H), 2.17-2.42(m, 7H), 2.49 (t, J = 6.7Hz, 2H), 3.51(s, 2H), 3.60(s, 2H), 3.65(s, 2H), 3.86-3.98(m, 2H), 4.09(q, J = 7.1Hz, 2H), 6.93(d, J = 1.1Hz, 1H), 7.01(d, J = 1.1Hz, 1H), 7.05-7.43(m, 6H).

Example 21(21): Ethyl (2E)-3-[4-({[(1-{3-[8-(2,2-dimethylpropyl)-2,8-diazaspiro[4.5]deca-2-yl]propyl}-1H-imidazol-2-yl) methyl](1H-imidazol-2-ylmethyl)amino}methyl)phenyl]acrylate

TLC: Rf 0.73 (ethyl acetate : methanol : 28% ammonia water = 80 : 20 : 4);
NMR($CDCl_3$): δ 0.84(s, 9H), 1.26(t, J = 7.2Hz, 3H), 1.40-1.60(m, 6H), 1.78-1.95(m, 2H), 1.98(s, 2H), 2.20-2.42(m, 8H), 2.45(t, J = 6.9Hz, 2H), 3.49(s, 2H), 3.60(s, 2H), 3.67(s, 2H), 3.92(t, J = 6.9Hz, 2H), 4.26(q, J = 7.2Hz, 2H), 6.42(d, J = 15.9Hz, 1H), 6.92(d, J = 1.8Hz, 1 H), 7.00(d, J = 1.8Hz, 1H), 7.02-7.18(m, 2H), 7.42(d, J = 8.1Hz, 2H), 7.49(d, J = 8.1Hz, 2H), 7.66(d, J = 15.9Hz, 1H), 12.5(s, 1H).

Example 21(22): Ethyl (2E)-3-(4-{[({1-[3-(8-cyclohexyl-2,8-diazaspiro[4.5]deca-2-yl)propyl]-1H-imidazol-2-yl}methyl) (1H-imidazol-2-ylmethyl)amino]methyl}phenyl)acrylate

TLC: Rf 0.48 (ethyl acetate : methanol : 28% ammonia water = 80 : 20 : 4);
NMR($CDCl_3$): δ 1.00-1.95(m, 18H), 1.34(t, J = 7.2Hz, 3H), 2.16-2.58(m, 11H), 3.50(s, 2H), 3.59(s, 2H), 3.67(s, 2H), 3.93 (t, J = 7.2Hz, 2H), 4.26(q, J = 7.2Hz, 2H), 6.42(d, J = 15.9Hz, 1H), 6.93(d, J = 1.5Hz, 1H), 7.01(d, J = 1.5Hz, 1H), 7.06 (s, 1H), 7.11(s, 1H), 7.42(d, J = 8.4Hz, 2H), 7.49(d, J = 8.4Hz, 2H), 7.67(d, J = 15.9Hz, 1H), 12.5(brs, 1H).

Example 21(23): Ethyl 2-(4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)-2-methylpropanoate

TLC: Rf 0.83 (chloroform : methanol : 28% ammonia water = 40 : 10 : 2);
NMR($CDCl_3$): δ 0.88(d, J = 6.6Hz, 6H), 1.19(t, J = 7.0Hz, 3H), 1.47-1.91(m, 16H), 2.03(d, J = 7.3Hz, 2H), 2.20-2.35(m,

7H), 2.47(t, J = 7.0Hz, 2H), 3.49(s, 2H), 3.59(s, 2H), 3.64(s, 2H), 3.92(t, J = 7.0Hz, 2H), 4.11(q, J = 7.0Hz, 2H), 6.92(d, J = 1.3Hz, 1H), 6.99(d, J = 1.3Hz, 1H), 7.02-7.17(m, 2H), 7.21-7.47(m, 4H).

Example 21(24): Ethyl 3-[4-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenyl]propanoate

TLC: Rf 0.60 (ethyl acetate : methanol : 28% ammonia water = 80 : 20 : 2);
NMR($CDCl_3$): δ 1.00-1.95(m, 20H), 2.16-2.56(m, 12H), 2.60(t, J = 8.1Hz, 2H), 2.93(t, J = 8.1Hz, 2H), 3.47(s, 2H), 3.59 (s, 2H), 3.63(s, 2H), 3.91(t, J = 7.2Hz, 2H), 4.12(q, J = 7.2Hz, 2H), 6.91(d, J = 1.2Hz, 1H), 6.99(d, J = 1.2Hz, 1H), 7.05 (s, 1H), 7.10(s, 1H), 7.15(d, J = 8.4Hz, 2H), 7.31(d, J = 8.4Hz, 2H), 12.5(brs, 1H).

Example 21(25): Ethyl 3-(4-{[({1-[3-(8-cyclohexyl-2,8-diazaspiro[4.5]deca-2-yl)propyl]-1 H-imidazol-2-yl}methyl)(1H-imidazol-2-ylmethyl)amino]methyl}phenyl)propanoate

TLC: Rf 0.22 (ethyl acetate : methanol : 28% ammonia water = 80 : 20 : 2);
NMR($CDCl_3$): δ 1.00-1.95(m, 21H), 2.16-2.58(m, 11H), 2.60(t, J = 7.8Hz, 2H), 2.93(t, J = 7.8Hz, 2H), 3.47(s, 2H), 3.59 (s, 2H), 3.62(s, 2H), 3.91(t, J = 6.9Hz, 2H), 4.12(q, J = 7.2Hz, 2H), 6.91(d, J = 1.2Hz, 1H), 6.99(d, J = 1.2Hz, 1H), 7.05 (s, 1H), 7.10(s, 1H), 7.15(d, J = 8.1Hz, 2H), 7.31(d, J = 8.1Hz, 2H), 12.4(brs, 1H).

Example 21(26): Ethyl (2E)-3-[3-({[(1-{3-[8-(2,2-dimethylpropyl)-2,8-diazaspiro[4.5]deca-2-yl]propyl}-1H-imidazol-2-yl) methyl](1H-imidazol-2-ylmethyl)amino}methyl)phenyl]acrylate

TLC: Rf 0.44 (chloroform : methanol : 28% ammonia water = 100 : 10 : 2);
NMR($CDCl_3$): δ 0.84(s, 9H), 1.34(t, J = 7.0Hz, 3H), 1.41-1.57(m, 8H), 1.76-1.91(m, 2H), 2.03(s, 2H), 2.18-2.32(m, 2H), 2.37(t, J = 4.7Hz, 4H), 2.44(t, J = 6.7Hz, 2H), 3.49(s, 2H), 3.63(s, 2H), 3.69(s, 2H), 3.92(t, J = 7.0Hz, 2H), 4.27(q, J = 7.0Hz, 2H), 6.45(d, J = 16.0Hz, 1H), 6.94(d, J = 1.3Hz, 1H), 7.02(d, J = 1.3Hz, 1H), 7.05-7.17(m, 2H), 7.31-7.50(m, 3H), 7.58(s, 1H), 7.69(d, J = 16.0Hz, 1H), 12.44-12.66(m, 1H).

Example 21(27): Ethyl (2E)-3-(3-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)acrylate

TLC: Rf 0.43 (chloroform : methanol: 28% ammonia water = 100 : 10 : 2);
NMR($CDCl_3$): δ 0.88(t, J = 6.6Hz, 6H), 1.34(t, J = 7.1Hz, 3H), 1.43-1.61(m, 9H), 1.80-1.89(m, 2H), 1.95-2.04(m, 2H), 2.18-2.33(m, 6H), 2.44(t, J = 7.0Hz, 2H), 3.49(s, 2H), 3.62(s, 2H), 3.69(s, 2H), 3.92(t, J = 7.0Hz, 2H), 4.27(q, J = 7.1Hz, 2H), 6.45(d, J = 16.1Hz, 1H), 6.94(d, J = 1.3Hz, 1H), 7.02(d, J = 1.3Hz, 1H), 7.05-7.17(m, 2H), 7.32-7.49(m, 3H), 7.58 (s, 1H), 7.69(d, J = 16.1Hz, 1H), 12.46-12.59(m, 1H).

Example 21(28):ethyl 3-(3-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)propanoate

TLC: Rf 0.62 (dichloromethane : methanol : 28% ammonia water = 80 : 20 : 2);
NMR($CDCl_3$): δ 0.88(d, J = 6.4Hz, 6H), 1.23(t, J = 7.2Hz, 3H), 1.44-1.63(m, 6H), 1.67-1.91(m, 3H), 2.02(d, J = 7.3Hz, 2H), 2.17-2.37(m, 8H), 2.46(t, J = 6.9Hz, 2H), 2.55-2.67(m, 2H), 2.94(t, J = 7.9Hz, 2H), 3.47(s, 2H), 3.62(s, 2H), 3.64 (s, 2H), 3.89(t, J = 7.0Hz, 2H), 4.11(q, J = 7.1Hz, 2H), 6.91(d, J = 1.3Hz, 1H), 6.99(d, J = 1.3Hz, 1H), 7.03-7.15(m, 3H), 7.17-7.29(m, 3H), 12.43(s, 1H).

Example 21(29): Ethyl 3-[3-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenyl]propanoate

TLC: Rf 0.64 (dichloromethane : methanol : 28% ammonia water = 80 : 20 : 2);
NMR($CDCl_3$): δ 0.84(s, 9H), 1.23(t, J = 7.1Hz, 3H), 1.42-1.61(m, 6H), 1.80-1.90(m, 2H), 1.99(s, 2H), 2.20-2.31(m, 4H), 2.34-2.43(m, 4H), 2.46(t, J = 6.8Hz, 2H), 2.61 (t, J = 7.9Hz, 2H), 2.94(t, J = 7.9Hz, 2H), 3.47(s, 2H), 3.63(s, 2H), 3.64 (s, 2H), 3.89(t, J = 7.0Hz, 2H), 4.12(q, J = 7.1Hz, 2H), 6.92(d, J = 1.3Hz, 1H), 7.00(d, J = 1.3Hz, 1H), 7.03-7.16(m, 3H), 7.18-7.26(m, 3H), 12.34-12.58(m, 1H).

Example 21(30): Ethyl 3-(3-{[({1-[3-(8-cyclohexyl-2,8-diazaspiro[4.5]deca-2-yl)propyl]-1H-imidazol-2-yl}methyl)(1H-imidazol-2-ylmethyl)amino]methyl}phenyl)propanoate

TLC: Rf 0.51 (ethyl acetate : methanol : 28% ammonia water = 80 : 20 : 4);
NMR($CDCl_3$): δ 1.00-1.92(m, 21 H), 2.16-2.55(m, 11H), 2.61(t, J = 7.8Hz, 2H), 2.94(t, J = 7.8Hz, 2H), 3.47(s, 2H), 3.62 (s, 2H), 3.64(s, 2H), 3.90(t, J = 7.2Hz, 2H), 4.11(q, J = 7.2Hz, 2H), 6.92(s, 1H), 7.00(s, 1H), 7.02-7.35(m, 6H), 12.4(brs, 1H).

Example 22: (4-{[({1-[3-(8-cyclohexyl-2,8-diazaspiro[4.5]deca-2-yl)propyl]-1H-imidazol-2-yl}methyl)(1H-imidazol-2-ylmethyl)amino]methyl}phenyl)acetic acid

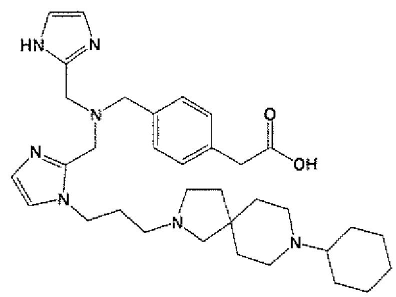

The same procedure as in Example 4 was carried out, except that the compound synthesized in Example 21 was used in place of the compound synthesized in Example 3 in Example 4, the title compound having the following physical properties were obtained.
TLC: Rf 0.52 (dichloromethane : methanol : 28% ammonia water = 80 : 20 : 4);
NMR($CD_3OD$): δ 1.10-3.20(m, 29H), 3.42(s, 2H), 3.45(s, 2H), 3.53(s, 2H), 3.63(s, 2H), 3.85(t, J = 7.8Hz, 2H), 6.91(d, J = 1.5Hz, 1H), 7.04(s, 2H), 7.13(d, J = 1.5Hz, 1H), 7.17(d, J = 8.1Hz, 2H), 7.30(d, J = 8.1Hz, 2H).

Example 22(1) to Example 22(26):

The same procedure as in Example 22 was carried out, except that corresponding compounds synthesized in Example 21(1) to Example 21(30) were used in place of the compound synthesized in Example 21 in Example 22, the title compounds having the following physical properties were obtained.

Example 22(1): N-(1H-imidazol-2-ylmethyl)-N-({1-[3-(8-isobutyl-2,8-diazaspiro[4.5]deca-2-yl)propyl]-1H-imidazol-2-yl}methyl)glycine

TLC: Rf 0.11 (dichloromethane : methanol : 28% ammonia water = 50 : 10 : 1); NMR($CD_3OD$): δ 0.91(d, J = 6.6Hz, 6H), 1.51-1.70(m, 4H), 1.71(t, J = 6.6Hz, 2H), 1.86(m, 1H), 1.97-2.10(m, 2H), 2.16(d, J = 7.2Hz, 2H), 2.32-2.48 (m, 4H), 2.54(s, 2H), 2.64(t, J = 6.6Hz, 2H), 2.82(t, J = 6.6Hz, 2H), 3.09(s, 2H), 3.66(s, 2H), 3.70(s, 2H), 4.20(t, J = 6.6Hz, 2H), 6.91(d, J = 1.2Hz, 1H), 6.99(s, 2H), 7.11(d, J = 1.2Hz, 1H).

Example 22(2): N-(1H-imidazol-2-ylmethyl)-N-({1-[3-(8-isobutyl-2,8-diazaspiro[4.5]deca-2-yl)propyl]-1H-imidazol-2-yl}methyl)-β-alanine

TLC: Rf 0.20 (chloroform : methanol : 28% ammonia water = 40 : 10:2);
NMR($CD_3OD$): δ 0.92(d, J = 6.6Hz, 6H), 1.52-1.90(m, 8H), 1.95-2.08(m, 1H), 2.20(d, J = 7.1Hz, 2H), 2.36-2.90(m, 12H), 3.05(t, J = 7.0Hz, 2H), 3.66(s, 2H), 3.69(s, 2H), 3.85(t, J = 7.0Hz, 2H), 6.85(d, J = 1.3Hz, 1H), 6.97-7.04(m, 3H).

Example 22(3): 4-[(1H-imidazol-2-ylmethyl)({1-[3-(8-isobutyl-2,8-diazaspiro[4.5]deca-2-yl)propyl]-1H-imidazol-2-yl}methyl)amino]butanoic acid

TLC: Rf 0.32 (dichloromethane : methanol : 28% ammonia water = 80 : 20 : 4);

NMR($CD_3OD$): δ 0.98(d, J = 6.6Hz, 6H), 1.68-1.92(m, 11H), 2.11(t, J = 6.9Hz, 2H), 2.49(t, J = 6.6Hz, 2H), 2.56(d, J = 7.5Hz, 2H), 2.72(t, J = 7.5Hz, 2H), 2.70-2.95(m, 6H), 2.97(d, J = 7.5Hz, 2H), 3.62(s, 2H), 3.67(s, 2H), 3.97(t, J = 7.5Hz, 2H), 6.89(d, J = 1.5Hz, 1H), 7.02(s, 2H), 7.07(d, J = 1.5Hz, 1H).

Example 22(4): (4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)acetic acid

TLC: Rf 0.33 (chloroform : methanol : 28% ammonia water = 40 : 10 : 2);
NMR($CD_3OD$): δ 0.99(d, J = 6.6Hz, 6H), 1.39-1.64(m, 4H), 1.68(t, J = 7.0Hz, 2H), 1.84-2.10(m, 3H), 2.40(s, 2H), 2.47 (t, J = 69Hz, 2H), 2.51-2.69(m, 4H), 2.81(t, J = 6.9Hz, 2H), 2.86-2.99(m, 2H), 3.43(s, 2H), 3.45(s, 2H), 3.53(s, 2H), 3.64 (s, 2H), 3.85(t, J = 7.8Hz, 2H), 6.90(d, J = 1.3Hz, 1H), 7.03(s, 2H), 7.10(d, J = 1.3Hz, 1H), 7.17(d, J = 7.9Hz, 2H), 7.29 (d, J = 7.9Hz, 2H).

Example 22(5): 5-[(1H-imidazol-2-ylmethyl)({1-[3-(8-isobutyl-2,8-diazaspiro[4.5]deca-2-yl)propyl]-1H-imidazol-2-yl}methyl)amino]pentanoic acid

TLC: Rf 0.25 (chloroform : methanol : 28% ammonia water = 40 : 10 : 2);
NMR($CD_3OD$): δ 0.95(d, J = 6.6Hz, 6H), 1.46-1.56(m, 4H), 1.61-1.81(m, 6H), 1.85-1.98(m, 3H), 2.07-2.16(m, 2H), 2.37 (d, J = 7.3Hz, 2H), 2.39-2.45(m, 2H), 2.54(t, J = 8.4Hz, 2H), 2.58-2.71(m, 6H), 2.82(t, J = 7.0Hz, 2H), 3.64(s, 2H), 3.64 (s, 2H), 3.95(t, J = 7.0Hz, 2H), 6.86(d, J = 1.3Hz, 1H), 7.01 (s, 2H), 7.06(d, J = 1.3Hz, 1H).

Example 22(6): 3-(4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)propanoic acid

TLC: Rf 0.37 (chloroform : methanol : 28% ammonia water = 40 : 10 : 2);
NMR($CD_3OD$): δ 0.96(d, J = 6.6Hz, 6H), 1.50-1.86(m, 8H), 1.90-2.06(m, 1H), 2.43-2.86(m, 12H), 2.90(t, J = 7.2Hz, 2H), 2.95(t, J = 7.2Hz, 2H), 3.45(s, 2H), 3.52(s, 2H), 3.63(s, 2H), 3.88(t, J = 6.9Hz, 2H), 6.88(d, J = 1.5Hz, 1H), 7.03(s, 2H), 7.08(d, J = 1.5Hz, 1H), 7.15(d, J = 8.1Hz, 2H), 7.22(d, J = 8.1 Hz, 2H).

Example 22(7): 4-(4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)butanoic acid

TLC: Rf 0.31 (chloroform : methanol : 28% ammonia water = 40 : 10 : 2);
NMR($CD_3OD$): δ 0.95-1.04(d, J = 6.6Hz, 6H), 1.56-1.97(m, 10H), 1.96-2.10(m, 1H), 2.20(t, J = 7.3Hz, 2H), 2.30(t, J = 7.5Hz, 2H), 2.36(s, 2H), 2.57-2.67(m, 6H), 2.68-2.81(m, 2H), 2.90-3.05(m, 2H), 3.47(s, 2H), 3.58(s, 2H), 3.65(s, 2H), 3.90(t, J = 6.7Hz, 2H), 6.87(d, J = 1.3Hz, 1H), 7.02(s, 2H), 7.05(d, J = 1.3Hz, 1H), 7.16(d, J = 9.0Hz, 2H), 7.18(d, J = 9.0Hz, 2H).

Example 22(8): 4-[({1-[3-(8-cyclohexyl-2,8-diazaspiro[4.5]deca-2-yl)propyl]-1H-imidazol-2-yl}methyl)(1H-imidazol-2-ylmethyl)amino]butanoic acid

TLC: Rf 0.16 (dichloromethane : methanol : 28% ammonia water = 80 : 20 : 4); NMR($CD_3OD$): δ 1.00-2.15(m, 22H), 2.40-2.55(m, 4H), 2.58(s, 2H), 2.78(t, J = 6.6Hz, 2H), 2.86-3.20(m, 5H), 3.63(s, 2H), 3.66(s, 2H), 3.95(t, J = 7.2Hz, 2H), 6.87(d, J = 1.2Hz, 1H), 7.01(s, 2H), 7.06(s, J = 1.2Hz, 1H).

Example 22(9): 4-{[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}butanoic acid

TLC: Rf 0.16 (dichloromethane : methanol : 28% ammonia water = 80 : 20 : 4);
NMR($CD_3OD$): δ 0.92(s, 9H), 1.60-2.18(m, 12H), 2.26(s, 2H), 2.50(t, J = 6.6Hz, 2H), 2.60-2.75(m, 4H), 2.90(t, J = 7.2Hz, 2H), 2.95(s, 2H), 3.16(t, J = 7.2Hz, 2H), 3.60(s, 2H), 3.66(s, 2H), 3.97(t, J = 7.2Hz, 2H), 6.88(d, J = 1.5Hz, 1H), 7.02(s, 2H), 7.07(s, J = 1.5Hz, 1H).

Example 22(10): 4-[(1H-imidazol-2-ylmethyl)({1-[2-(8-isobutyl-2,8-diazaspiro[4.5]deca-2-yl)ethyl]-1H-imidazol-2-yl}methyl)amino]butanoic acid

TLC: Rf 0.14 (dichloromethane : methanol : 28% ammonia water = 70 : 30 : 3);
NMR($CD_3OD$): δ 0.97(d, J = 6.6Hz, 6H), 1.58-2.12(m, 11H), 2.41-2.54(m, 6H), 2.61-2.86(m, 8H), 3.62(s, 2H), 3.70(s,

2H), 4.07-4.15(m, 2H), 6.85(d, J = 1.5Hz, 1H), 6.99(s, 2H), 7.10(d, J = 1. 5Hz, 1H).

Example 22(11): 4-[(1H-imidazol-2-ylmethyl)({1-[4-(8-isobutyl-2,8-diazaspiro[4.5]deca-2-yl)butyl]-1H-imidazol-2-yl}methyl)amino]butanoic acid

TLC: Rf 0.20 (dichloromethane : methanol: 28% ammonia water = 70 : 30 : 3);
NMR($CD_3OD$): δ 0.94(d, J = 6.6Hz, 6H), 1.38-1.96(m, 13H), 2.00-2.09(m, 2H), 2.31(d, J = 7.1Hz, 2H), 2.40-2.78(m, 10H), 2.91-3.00(m, 2H), 3.63(s, 2H), 3.65(s, 2H), 3.83-3.91(m, 2H), 6.85(d, J = 1.5Hz, 1H), 7.01(s, 2H), 7.06(d, J = 1.5Hz, 1H).

Example 22(12): [4-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenyl]acetic acid

TLC: Rf 0.43 (dichloromethane : methanol: 28% ammonia water = 80 : 20 : 4);
NMR($CD_3OD$): δ 0.95(s, 9H), 1.45-1.82(m, 8H), 2.28-2.38(m, 2H), 2.47(t, J = 7.8Hz, 2H), 2.52-2.82(m, 6H), 2.96(t, J = 6.9Hz, 2H), 3.40-3.48(s, 4H), 3.52(s, 2H), 3.67(s, 2H), 3.84(t, J = 6.6Hz, 2H), 6.88(d, J = 1.5Hz, 1H), 7.03(s, 2H), 7.07 (d, J = 1.5Hz, 1H), 7.18(d, J = 8.1Hz, 2H), 7.29(d, J = 8.1Hz, 2H).

Example 22(13): (3-{[(1H-imidazol-2-ylmethyl)({1-[3-(8-isobutyl-2,8-diazaspiro[4.5]deca-2-yl)propyl]-1H-irnidazol-2-yl} methyl)amino]methyl}phenyl)acetic acid

TLC: Rf 0.31 (chloroform: methanol: 28% ammonia water = 40 : 10 : 2);
NMR($CD_3OD$): δ 0.93(d, J = 6.6Hz, 6H), 1.55-1.71(m, 4H), 1.76(t, J = 7.0Hz, 2H), 1.81-1.97(m, 3H), 2.25(d, J = 7.1Hz, 2H), 2.42-2.58(m, 6H), 2.64(s, 2H), 2.91(t, J = 7.3Hz, 2H), 3.48(s, 4H), 3.61(s, 2H), 3.66(s, 2H), 3.75(t, J = 7.2Hz, 2H), 6.87(d, J = 1.3Hz, 1H), 6.99-7.11(m, 1H), 7.03(s, 2H), 7.08(d, J = 1.3Hz, 1H), 7.13-7.24(m, 2H), 7.29(s, 1H).

Example 22(14): [3-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenyl]acetic acid

TLC: Rf 0.39 (chloroform : methanol : 28% ammonia water = 40 : 10: 2);
NMR($CD_3OD$): δ 0.89(s, 9H), 1.54-1.74(m, 4H), 1.85(t, J = 7.1Hz, 2H), 1.91-2.05(m, 2H), 2.13(s, 2H), 2.48-2.59(m, 4H), 2.67-2.78(m, 2H), 2.92(s, 2H), 3.17(t, J = 7.0Hz, 2H), 3.49(s, 2H), 3.50(s, 2H), 3.59(s, 2H), 3.62-3.68(m, 2H), 3.67(s, 2H), 6.88(d, J = 1.3Hz, 1H), 7.02-7.25(m, 3H), 7.04(s, 2H), 7.09(d, J = 1.3Hz, 1H), 7.27(s, 1H).

Example 22(15): (2E)-3-(4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)acrylic acid

TLC: Rf 0.40 (dichloromethane : methanol: 28% ammonia water = 80 : 20 : 4);
NMR($CD_3OD$): δ 0.91(d, J = 6.5Hz, 6H), 1.48-1.64(m, 6H), 1.64-1.88(m, 3H), 2.05-2.16(m, 4H), 2.26(s, 2H), 2.29-2.40 (m, 4H), 2.45(t, J = 6.8Hz, 2H), 3.51(s, 2H), 3.61(s, 2H), 3.65(s, 2H), 3.90(t, J = 6.9Hz, 2H), 6.48(d, J = 15.9Hz, 1H), 6.87(d, J = 1.3Hz, 1H), 7.01(s, 2H), 7.05(d, J = 1.3Hz, 1H), 7.29(d, J = 8.1Hz, 2H), 7.35(d, J = 15.9Hz, 1H), 7.47(d, J = 8.1 Hz, 2H).

Example 22(16): 1-(4-{[(1H-imidazol-2-ylmethyl)({1-[3-(8-isobutyl-2,8-diazaspiro [4.5]deca-2-yl)propyl]-1H-imidazol-2-yl}methyl)amino]methyl}phenyl)cyclopropanecarboxylic acid

TLC: Rf 0.35 (chloroform : methanol : 28% ammonia water = 40 : 10: 2);
NMR($CD_3OD$): δ 0.89-0.95(m, 2H), 0.95(d, J = 6.6Hz, 6H), 1.33-1.42(m, 2H), 1.43-1.79(m, 6H), 1.79-2.00(m, 3H), 2.32-2.63(m, 8H), 2.66-2.81(m, 2H), 2.86(t, J = 6.0Hz, 2H), 3.44(s, 2H), 3.58(s. 2H), 3.65(s, 2H), 3.94(t, J = 7.2Hz, 2H), 6.89(d, J = 1.3Hz, 1H), 7.03(s, 2H), 7.10(d, J = 1.3 Hz, 1H), 7.16(d, J = 8.1Hz, 2H), 7.31(d, J = 8.1 Hz, 2H).

Example 22(17): (2E)-3-[4-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenyl]acrylic acid

TLC: Rf 0.19 (ethyl acetate : methanol : 28% ammonia water = 80 : 20 : 4);
NMR($CD_3OD$): δ 1.02(s, 9H), 1.60-1.84(m, 6H), 1.92-2.00(m, 2H), 2.50-3.08(m, 12H), 3.59(s, 4H), 3.75(s, 2H), 3.91(t, J = 7.2Hz, 2H), 6.49(d, J = 15.9Hz, 1H), 6.94(d, J = 1.5Hz, 1H), 7.05-7.18(m, 3H), 7.33(d, J = 8.4Hz, 2H), 7.52(d, J = 15.9Hz, 1H), 7.56(d, J = 8.4Hz, 2H).

Example 22(18): (2E)-3-(4-{[({1-[3-(8-cylohexyl-2,8-diazaspiro[4.5]deca-2-yl)propyl]-1H-imidazol-2-yl}methyl)(1H-imidazol-2-ylmethyl)amino]methyl}phenyl)acrylic acid

TLC: Rf 0.11 (ethyl acetate : methanol: 28% ammonia water = 80 : 20 : 4);
NMR($CD_3OD$): δ 1.15-1.90(m, 14H), 1.90-2.00(m, 2H), 2.05-2.18(m, 2H), 2.31(t, J = 7.8Hz, 2H), 2.46(s, 2H), 2.70(t, J = 7.8Hz, 2H), 3.04-3.30(m, 5H), 3.57(s, 2H), 3.60(s, 2H), 3.73(s, 2H), 3.90(t, J = 7.2Hz, 2H), 6.50(d, J = 15.9Hz, 1H), 6.92(d, J = 1.5Hz, 1H), 7.08(s, 3H), 7.34(d, J = 8.1Hz, 2H), 7.47(d, J = 15.9Hz, 1H), 7.54(d, J = 8.4Hz, 2H).

Example 22(19): 2-(4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)-2-methylpropanoic acid

TLC: Rf 0.50 (chloroform : methanol : 28% ammonia water = 40 : 10 : 2);
NMR($CD_3OD$): δ 0.98(d, J = 6.6Hz, 6H), 1.34-1.85(m, 10H), 1.82-2.13(m, 3H), 2.31-2.68(m, 8H), 2.77-3.12(m, 6H), 3.45 (s, 2H), 3.56(s, 2H), 3.60(s, 2H), 3.76-4.04(m, 2H), 6.90(d, J = 1.1Hz, 1H), 7.04(s, 2H), 7.11(d, J = 0.9Hz, 1H), 7.18(d, J = 8.1Hz, 2H), 7.41 (d, J = 8.1Hz, 2H).

Example 22(20): 3-[4-({[(1-{3-[8-(2,2-dimethylpropyl)-2,8-diazaspiro[4.5]deca-2-yl]propyl}-1H-imidazol-2-yl)methyl](1H-imidazol-2 ylmethyl)amino}methyl)phenyl]propanoic acid

TLC: Rf 0.26 (dichloromethane : methanol: 28% ammonia water = 80 : 20 : 4);
NMR($CD_3O0D$): δ 1.10(s, 9H), 1.75-2.05(m, 8H), 2.58(t, J = 6.9Hz, 2H), 2.90(t, J = 6.9Hz, 2H), 2.90-3.40(m, 12H), 3.60 (s, 2H), 3.64(s, 2H), 3.89(s, 2H), 3.94(t, J = 6.9Hz, 2H), 7.02(d, J = 1.2Hz, 1H), 7.12-7.28(m, 7H).

Example 22(21): 3-(4-{[({2-[3-(8-cyclohexyl-2,8-diazaspiro[4.5]deca-2-yl)propyl]-1H-imidazol-2-yl}methyl)(1H-imidazol-2-ylmethyl)amino]methyl}phenyl)propanoic acid

TLC: Rf 0.29 (dichloromethane : methanol : 28% ammonia water = 80 : 20 : 4);
NMR($CD_3OD$): δ 1.10-2.14(m, 18H), 2.40-2.60(m, 4H), 2.80-3.20(m, 11H), 3.46(s, 2H), 3.54(s, 2H), 3.61(s, 2H), 3.88(t, J = 7.2Hz, 2H), 6.89(d, J = 1.2Hz, 1H), 7.03(s, 2H), 7.10(d, J = 1.2Hz, 1H), 7.16(d, J = 8.1Hz, 2H), 7.22(d, J = 8.1Hz, 2H).

Example 22(22): (2E)-3-[3-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenyl]acrylic acid

TLC: Rf 0.15 (chloroform : methanol: 28% ammonia water = 40 : 10 : 2);
NMR($CD_3OD$): δ 1.11(s, 9H), 1.88-2.20(m, 8H), 2.99(s, 2H), 2.99-3.07(m, 2H), 3.15-3.20(m, 2H), 3.30(s, 6H), 3.70(s, 2H), 3.79(t, 2H), 3.92(s, 2H), 4.01(t, J = 7.6Hz, 2H), 6.54(d, J = 15.9Hz, 1H), 7.03(d, J = 1.1Hz, 1H), 7.19-7.22(m, 1H), 7.22(s, 2H), 7.32-7.44(m, 2H), 7.46-7.56(m, 1H), 7.61(d, J = 15.9Hz, 1H), 7.59-7.63 (m, 1H).

Example 22(23): (2E)-3-(3-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)acrylic acid

TLC: Rf 0.14 (chloroform : methanol : 28% ammonia water = 40 : 10 : 2);
NMR($CD_3OD$): δ 1.05(d, J = 6.6Hz, 6H), 1.86-2.23(m, 10H), 2.86-2.96(m, 2H), 2.96(d, J = 7.3Hz, 2H), 3.07(s, 2H), 3.18-3.36(m, 6H), 3.67(s, 2H), 3.74(s, 2H), 3.87(s, 2H), 3.97(t, J = 7.4Hz, 1H), 6.54(d, J = 15.8Hz, 1H), 7.00(d, J = 1.3Hz, 1H), 7.19(s, 3H), 7.29-7.43(m, 2H), 7.48-7.53(m, 1H), 7.58(d, J = 15.8Hz, 1H), 7.57(s, 1H).

Example 22(24): 3-(3-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenyl)propanoic acid

TLC: Rf 0.46 (dichloromethane : methanol: 28% ammonia water =30:10:2);
NMR($CD_3OD$): δ 0.93(d, J = 6.6Hz, 6H), 1.55-1.73(m, 4H), 1.73-1.95(m, 5H), 2.26(d, J = 7.3Hz, 2H), 2.41-2.62(m, 8H), 2.71(s, 2H), 2.83-3.00(m, 4H), 3.46(s, 2H), 3.59(s, 2H), 3.64(s, 2H), 3.82(t, J = 7.3Hz, 2H), 6.87(d, J = 1.5Hz, 1H), 6.99-7.06(m, 3H), 7.07(d, J = 1.5Hz, 1H), 7.08-7.24(m, 3H).

Example 22(25): 3-[3-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenyl]propanoic acid

TLC: Rf 0.53 (dichloromethane : methanol : 28% ammonia water = 30 : 10 : 2);

NMR($CD_3OD$): δ 0.88(s, 9H), 1.51-1.74(m, 4H), 1.76-1.97(m, 4H), 2.12(s, 2H), 2.40-2.58(m, 6H), 2.63-2.76(m, 2H), 2.83-2.98(m, 4H), 3.11(t, J = 7.2Hz, 2H), 3.46(s, 2H), 3.57(s, 2H), 3.63(s, 2H), 3.72-3.83(m, 2H), 6.87(d, J = 1.5Hz, 1H), 7.02(d, J = 7.3Hz, 1H), 7.05(s, 2H), 7.07(d, J = 1.5Hz, 1H), 7.08-7.14(m, 1H), 7.14-7.26(m, 2H).

Example 22(26): 3-(3-{[({1-[3-(8-cyclohexyl-2,8-diazaspiro[4.5]deca-2-yl)propyl]-1H-imidazol-2-yl}methyl)(1H-imidazol-2-ylmethyl)amino]methyl}phenyl)propanoic acid

TLC: Rf 0.49 (dichloromethane : methanol : 28% ammonia water = 80 : 20 : 5);
NMR($CD_3OD$): δ 1.08-2.10(m, 18H), 2.40-3.06(m, 15H), 3.47(s, 2H), 3.61(s, 2H), 3.63(s, 2H), 3.86(t, J = 7.5Hz, 2H), 6.89(d, J = 1.5Hz, 1H), 6.99-7.06(m, 3H), 7.08(d, J = 1.5Hz, 1H), 7.11-7.23(m, 3H).

Example 23: [(2,2-dimethylpropanoyl)oxy]methyl 4-[(1H-imidazol-2-ylmethyl)({1-[3-(8-isobutyl-2,8-diazaspiro[4.5]deca-2-yl)propyl]-1H-imidazol-2-yl}methyl)amino]butanloate

Under an argon atmosphere, the compound (600 mg) synthesized in Example 22(3) was dissolved in N,N-dimethylformamide (DMF) (6 mL) and then potassium hydrogencarbonate (40 mg), sodium iodide (60 mg), chloromethyl pivalate (58 μL) were added, followed by stirring at 40°C for 15 hours. To the reaction solution, water (50 mL) was added, followed by extraction twice with dichloromethane (50 mL). The organic layers were combined, washed in turn with water and saturated brine, and then dried over anhydrous magnesium sulfate. Anhydrous magnesium sulfate was removed by filtration and then the filtrate was concentrated. The residue was purified by silica gel chromatography (manufactured by FUJI SILYSIA CHEMICAL LTD., CHROMATOREX NH (trade name)) (n-hexane : ethyl acetate = 4 : 1 → 0 : 1) to obtain the title compound (50 mg) having the following physical properties.
TLC: Rf 0.73 (dichloromethane : methanol : 28% ammonia water = 80 : 20 : 4); NMR($CDCl_3$): δ 0.88(d, J = 6.6Hz, 6H), 1.21(s, 9H), 1.45-2.02(m, 13H), 2.03(d, J = 6.9Hz, 2H), 2.20-2.60(m, 12H), 3.50(s, 2H), 3.60(s, 2H), 4.04(t, J = 7.2Hz, 2H), 5.73(s, 2H), 6.95(d, J = 0.9Hz, 1H), 6.98-7.10(m, 3H), 12.4(brs, 1H).

Example 23(1): 1-{[(cyclohexyloxy)carbonyl]oxy}ethyl 4-[(1H-imidazol-2-ylmethyl)({1-[3-(8-isobutyl-2,8-diazaspiro[4.5] deca-2-yl)propyl]-1H-imidazol-2-yl }methyl)amino]butanoate

The same procedure as in Example 23 was carried out, except that 1-chloroethylcyclohexyl carbonate was used in place of chloromethyl pivalate in Example 23, the title compound having the following physical properties was obtained.
TLC: Rf 0.33 (ethyl acetate : methanol : 28% ammonia water = 80 : 20 : 2);
NMR($CDCl_3$): δ 0.89(d, J = 6.3Hz, 6H), 1.20-2.02(m, 28H),(s, 9H), 2.20-2.60(m, 12H), 3.51(s, 2H), 3.61(s, 2H), 4.05(t, J = 6.9Hz, 2H), 4.62(m, 1H), 6.76(q, J = 5.4Hz, 1H), 6.96(d, J = 0.9Hz, 1H), 6.98-7.10(m, 3H), 12.4(brs, 1H).

Example 24: Methyl [4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]acetate

Under an argon atmosphere, the compound (800 mg) synthesized in Example 20 was dissolved in 3-pentanone (1.3 mL) and tetraisopropyl orthotitanate (657 mg) were added, followed by stirring at room temperature for 2 hours. To the reaction solution, ethanol (5 mL) was added, cooled to 0°C, followed by the addition of sodium borohydride (75 mg) and further stirring at 0°C for one hour. To the reaction solution, an aqueous sodium hydrogencarbonate solution (20 mL) was added, followed by extraction twice with ethyl acetate (50 mL). The organic layers were combined, washed with saturated brine and then dried anhydrous magnesium sulfate. Anhydrous magnesium sulfate was removed by filtration and then the filtrate was concentrated. The residue was purified by silica gel chromatography (manufactured by FUJI SILYSIA CHEMICAL LTD., CHROMATOREX NH(trade name)) (n-hexane : ethyl acetate = 1 : 1 → 0 : 1 → ethyl acetate : methanol = 20 : 1 → 10 : 1 to obtain the title compound (285 mg) having the following physical properties.
TLC: Rf 0.81 (dichloromethane : methanol : 28% ammonia water = 80 : 20 : 3);
NMR($CDCl_3$): δ 0.88(t, J = 7.2Hz, 6H), 1.08-1.80(m, 10H), 1.80-1.94(m, 2H), 2.10(m, 1H), 2.20-2.46(m, 8H), 2.48(t, J = 6.9Hz, 2H), 3.49(s, 2H), 3.59(s, 2H), 3.61(s, 2H), 3.64(s, 2H), 3.69(s, 3H), 3.92(t, J = 6.9Hz, 2H), 6.92(d, J = 1.2Hz, 1H), 7.00(d, J = 1.2Hz, 1H), 7.02-7.155(m, 2H), 7.24(d, J = 8.1Hz, 2H), 7.35(d, J = 8.1Hz, 2H), 12.5(brs, 1H).

Example 24(1) to Example 24(11):

The same procedure as in Example 15 → Example 16 → Example 17 → Example 18 → Example 19 → Example 20 → Example 24 was carried out, except that corresponding amine compound was used in place of methyl 4-aminomethylphenylacetate in Example 15, the following compounds of the present invention were obtained.

Example 24(1): Ethyl 4-{[(1-{3-[8-(1-ethylpropyl)-2,8-diazaspiro[4.5]deca-2-yl]propyl}-1H-imidazol-2-yl)methyl](1H-imidazol-2-ylmethyl)amino}butanoate

TLC: Rf 0.35 (dichloromethane : methanol : 28% ammonia water = 90 : 10 : 1); NMR($CDCl_3$): δ 0.88(t, J = 7.4Hz, 6H), 1.19-1.64(m, 10H), 1.25(t, J = 7.1Hz, 3H), 1.83-2.02(m, 4H), 2.05-2.16(m, 1H), 2.28-2.58(m, 14H), 3.52(s, 2H), 3.60(s, 2H), 4.04(t, J = 7.0Hz, 2H), 4.11(q, J = 7.1Hz, 2H), 6.94(d, J = 13Hz, 1H), 6.98-7.02(m, 1H), 7.00(d, J = 1.3Hz, 1H), 7.03-7.08(m, 1H), 12.30-12.42(m, 1H).

Example 24(2): Ethyl [4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]acetate

TLC: Rf 0.77 (dichloromethane : methanol : 28% ammonia water = 80 : 20 : 3); NMR($CDCl_3$): δ 0.88(t, J = 7.2Hz, 6H), 1.20-1.80(m, 13H), 1.80-1.95(m, 2H), 2.14(m, 1H), 2.22-2.46(m, 8H), 2.48(t, J = 6.9Hz, 2H), 3.48(s, 2H), 3.59(s, 4H), 3.64(s, 2H), 3.91(t, J = 6.9Hz, 2H), 4.14(q, J = 7.2Hz, 2H), 6.92(d, J = 1.5Hz, 1H), 6.99(d, J = 1.5Hz, 1H), 7.02-7.15(m, 2H), 7.24(d, J = 7.8Hz, 2H), 7.35(d, J = 7.8Hz, 2H), 12.5(brs, 1H).

Example 24(3): Methyl [3-({[(1-{3-[8-(1-ethylpropyl)-2,8-diazaspiro[4.5]deca-2-yl]propyl}-1H-imidazol-2-yl)methy](1H-imidazol-2-ylmethyl)amino}methyl)phenyl]acetate

TLC: Rf 0.44 (dichloromethane : methanol : 28% ammonia water = 80 : 10 : 2); NMR($CDCl_3$): δ 0.88(t, J = 7.4Hz, 6H), 1.19-1.36(m, 2H), 1.38-1.62(m, 8H), 1.78-1.91(m, 2H), 2.05-2.15(m, 1H), 2.21-2.31 (m, 2H), 2.27(s, 2H), 2.39(t, J = 4.6Hz, 4H), 2.46(t, J = 6.9Hz, 2H), 3.49(s, 2H), 3.62(s, 4H), 3.66(s, 2H), 3.69(s, 3H), 3.89(t, J = 6.9Hz, 2H), 6.91(d, J = 1.3Hz, 1H), 6.99(d, J = 1.3Hz, 1H), 7.05(s, 1H), 7.09(s, 1H), 7.14-7.22(m, 1H), 7.24-7.34 (m, 3H), 12.32-12.52(m, 1H).

Example 24(4): Ethyl [3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]acetate

TLC: Ref 0.44 (dichloromethane : methanol: 28% ammonia water = 80 : 10: 2); NMR($CDCl_3$): δ 0.88(t, J = 7.4Hz, 6H), 1.17-1.36(m, 2H), 1.25(t, J = 7.1Hz, 3H), 1.38-1.60(m, 8H), 1.75-1.92(m, 2H), 2.04-2.17(m, 1H), 2.21-2.30(m, 2H), 2.27(s, 2H), 2.39(t, J = 4.5Hz, 4H), 2.46(t, J = 6.9Hz, 2H), 3.50(s, 2H), 3.60(s, 2H), 3.62(s, 2H), 3.65(s, 2H), 3.88(t, J = 6.9Hz, 2H), 4.14(q, J = 7.1Hz, 2H), 6.91(d, J = 1.3Hz, 1H), 6.98(d, J = 1.3Hz, 1H), 7.07(s, 2H), 7.14-7.22(m, 1H), 7.23-7.35(m, 3H), 12.22-12.71(m, 1H).

Example 24(5): Ethyl 3-[3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]propanoate

TLC: Rf 0.41 (dichloromethane : methanol : 28% ammonia water = 90 : 10 : 1); NMR($CDCl_3$); δ 0.88(t, J = 7.4Hz, 6H), 1.16-1.61(m, 10H), 1.23(t, J = 7.0Hz, 3H), 1.76-1.91(m, 2H), 2.05-2.17(m, 1H), 2.21-2.31(m, 4H), 2.33-2.51(m, 6H), 2.61 (t, J = 7.8Hz, 2H), 2.94(t, J = 7.8Hz, 2H), 3.47(s, 2H), 3.62(s, 2H), 3.64(s, 2H), 3.89(t, J = 7.0Hz, 2H), 4.11(q, J = 7.0Hz, 2H), 6.91(d, J = 1.3Hz, 1H), 6.99(d, J = 1.3Hz, 1H), 7.02-7.14(m, 3H), 7.17-7.29 (m, 3H), 12.29-12.58(m, 1H).

Example 24(6): Ethyl 1-[4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]cyclopropanecarboxylate

TLC: Rf 0.45 (chloroform : methanol: 28% ammonia water = 90 : 10 : 1); NMR($CDCl_3$): δ 0.88(t, J = 7.4Hz, 6H), 1.11-1.22(m, 5H), 1.38-1.64(m, 6H), 1.74-1.98(m, 2H), 2.03-2.17(m, 1H), 2.26-2.34 (m, 2H), 2.40(t, J = 5.5Hz, 2H), 2.48(t, J = 7.0Hz, 2H), 3.51(s, 2H), 3.61(s, 2H), 3.65(s, 2H), 3.88-3.96(m, 2H), 4.09(q, J = 7.1Hz, 2H), 6.93(d, J = 1.1Hz, 1H), 7.00(d, J = 1.1Hz, 1H), 7.03-7.19(m, 2H), 7.18-7.44(m, 4H).

Example 24(7): Ethyl 2-[4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]-2-methylpropanoate

TLC: Rf 0.45 (chloroform : methanol : 28% ammonia water = 90 : 10 : 1); NMR($CDCl_3$): δ 0.89(t, J = 7.4Hz, 6H), 1.19(t, J = 7.1Hz, 3H), 1.24-1.40(m, 2H), 1.39-1.63(m, 14H), 1.68-1.99(m, 3H), 2.06-2.22(m, 1H), 2.24-2.35(m, 4H), 2.37-2.56(m, 5H), 3.49(s, 2H), 3.60(s, 2H), 3.63(s, 2H), 3.91(t, J = 6.9Hz, 2H), 4.11 (q, J = 7.1Hz, 2H), 6.88-6.95(m, 1H), 6.95-7.02(m, 1H), 7.07(s, 2H), 7.20-7.48(m, 4H).

Example 24(8): Ethyl (2E)-3-[3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]acrylate

TLC: Rf 0.40 (chloroform : methanol: 28% ammonia water = 100 : 10 : 2);
NMR($CDCl_3$): δ 0.88(t, J = 7.3Hz, 6H), 1.34(t, J = 7.2Hz, 3H), 1.39-1.58(m, 12H), 1.77-1.95(m, 2H), 2.05-2.16(m, 1H), 2.18-2.29(m, 2H), 2.33-2.41(m, 4H), 2.44(t, J = 6.8Hz, 2H), 3.49(s, 2H), 3.62(s, 2H), 3.69(s, 2H), 3.92(t, J = 6.9Hz, 2H), 4.27(q, J = 7.2Hz, 2H), 6.45(d, J = 16.0Hz, 1H), 6.94(s, 1H), 7.02(d, J = 0.9Hz, 1H), 7.10(d, J = 13.7Hz, 2H), 7.31-7.49 (m, 3H), 7.58(s, 1H), 7.69(d, J = 16.0Hz, 1H), 12.45-12.60(m, 1H).

Example 24(9): Ethyl (2E)-3-[4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]acrylate

TLC: Rf 0.54 (dichloromethane : methanol: 28% ammonia water = 90 : 10 : 1);
NMR($CDCl_3$): δ 0.88(t, J = 7.4Hz, 6H), 1.18-1.39(m, 6H), 1.39-1.59(m, 7H), 1.80-1.93(m, 2H), 2.05-2.16(m, 1H), 2.22-2.31 (m, 4H), 2.33-2.53(m, 6H), 3.50(s, 2H), 3.59(s, 2H), 3.67(s, 2H), 3.92(t, J = 7.1Hz, 2H), 4.26(q, J = 7.1Hz, 2H), 6.42(d, J = 16.1Hz, 1H), 6.93(d, J = 1.2Hz, 1H), 7.00(d, J = 1.2Hz, 1H), 7.04-7.14(m, 2H), 7.42(d, J = 8.2Hz, 2H), 7.49(d, J = 8.2Hz, 2H), 7.67(d, J = 16.1Hz, 1H), 12.49(s, 1H).

Example 24(10): Ethyl 3-[4-({[(1-{3-[8-(1-ethylpropyl)-2,8-diazaspiro[4.5]deca-2-yl]propyl}-1H-imidazol-2-yl)methyl](1H-imidazol-2-ylmethyl)ammo}methyl)phenyl]propanoate

TLC: Rf 0.54 (dichloromethane : methanol : 28% ammonia water = 90 : 10 : 1);
NMR($CDCl_3$): δ 0.88(t, J = 7.4Hz, 6H), 1.17-1.36(m, 6H), 1.38-1.61(m, 7H), 1.79-1.92(m, 2H), 2.05-2.16(m, 1H), 2.24-2.32 (m, 4H), 2.35-2.43(m, 4H), 2.47(t, J = 7.9Hz, 2H), 2.60(t, J = 7.9Hz, 2H), 2.93(t, J = 8.2Hz, 2H), 3.47(s, 2H), 3.59(s, 2H), 3.62(s, 2H), 3.90(t, J = 6.9Hz, 2H), 4.12(q, J = 7.1Hz, 2H), 6.92(d, J = 1.2Hz, 1H), 6.99(d, J = 1.2Hz, 1H), 7.03-7.11(m, 2H), 7.16(d, J = 8.1Hz, 2H), 7.27-7.34(m, J = 8.1 Hz, 2H), 12.46(s, 1H).

Example 24(11): Ethyl 5-{[(1-{3-[8-(1-ethylpropyl)-2,8-diazaspiro[4.5]deca-2-yl]propyl}-1H-imidazol-2-yl)methyl](1H-imidazol-2-ylmethyl)amino}pentanoate

TLC: Rf 0.69 (ethyl acetate : methanol : 28% ammonia water = 80 : 20 : 5);
NMR($CDCl_3$): δ 0.78-1.05(m, 6H), 1.20-2.08(m, 16H), 1.25(t, J = 7.2Hz, 3H), 2.20-2.75(m, 15H), 3.51(s, 2H), 3.59(s, 2H), 4.04(t, J = 6.9Hz, 2H), 4.11(q, J = 7.2Hz, 2H), 6.94(d, J = 1.2Hz, 1H), 7.00(d, J = 1.2Hz, 1H), 7.04(s, 2H).

Example 25: [4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]acetic acid

The same procedure as in Example 4 was carried out, except that the compound synthesized in Example 24 was used in place of the compound synthesized in Example 3 in Example 4, the title compound having the following physical properties was obtained.
TLC: Rf 0.38 (dichloromethane : methanol : 28% ammonia water = 80 : 20 : 4); NMR($CD_3OD$): δ 1.00(t, J = 7.5Hz, 6H), 1.40-2.02(m, 12H), 2.32(s, 2H), 2.44(t, J = 6.6Hz, 2H), 2.60-3.02(m, 7H), 3.43(s, 2H), 3.45(s, 2H), 3.54(s, 2H), 3.64(s, 2H), 3.84(t, J = 7.5Hz, 2H), 6.90(d, J = 1.5Hz, 1H), 7.03(s, 2H), 7.11(d, J = 1.5Hz, 1H), 7.17(d, J = 8.1Hz, 2H), 7.29(d, J = 8.1 Hz, 2H).

Example 25(1) to Example 25(9):

The same procedure as in Example 25 was carried out, except that corresponding compounds synthesized in Example 24(1) to Example 24(11) were used in place of the compound synthesized in Example 24 in Example 25, the following compound shaving the following physical properties were obtained.

Example 25(1): 4-{[(1-{3-[8-(1-ethylpropyl)-2,8-diazaspiro[4.5]deca-2-yl]propyl}-1H-imidazol-2-yl)methyl](1H-imidazol-2-ylmethyl)amino}butanoic acid

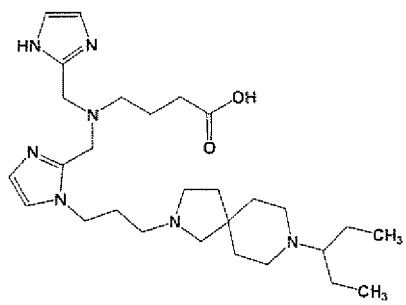

TLC: Rf 0.17 (dichloromethane : methanol: 28% ammonia water = 70 : 30 : 3);
NMR($CD_3OD$): δ 0.99(t, J = 7.5Hz, 6H), 1.43-2.12(m, 16H), 2.40-2.66(m, 7H), 2.76-2.91(m, 6H), 3.62(s, 2H), 3.65(s, 2H), 3.89-3.98(m, 2H), 6.86(d, J = 1.5Hz, 1H), 7.01(s, 2H), 7.06(d, J = 1. 5Hz, 1H).

Example 25(2): [3-({[(1-{3-[8-(1-ethylpropyl)-2,8-diazaspiro[4.5]deca-2-yl]propyl}-1H-imidazal-2-yl)methyl](1H-imidazol-2-ylmethyl)amino}methyl)phenyl]acetic acid

TLC: Rf 0.3 (chloroform: methanol: 28% ammonia water = 40 : 10 : 2);
NMR($CD_3OD$): δ 0.96(t, J = 7.4Hz, 6H), 1.36-1.72(m, 8H), 1.76(t, J = 7.1Hz, 2H), 1.85-1.98(m, 2H), 2.37-2.48(m, 1H), 2.49-2.77(m, 8H), 2.94(t, J = 6.9Hz, 2H), 3.48(s, 4H), 3.62(s, 2H), 3.66(s, 2H), 3.75(t, J = 7.3Hz, 2H), 6.88(d, J = 1.3Hz, 1H), 6.99-7.07(m, 1H), 7.03(s, 2H), 7.09(d, J = 1.3Hz, 1H), 7.14-7.26(m, 2H), 7.29(s, 1H).

Example 25(3): 1-[4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]cyclopropanecarboxylic acid

TLC: Rf 0.47 (chloroform : methanol: 28% ammonia water = 40 : 10 : 2);
NMR($CD_3OD$): δ 0.88-0.95(m, 2H), 0.99(t, J = 7.4Hz, 6H), 1.34-1.43(m, 2H), 1.42-1.83(m, 10H), 1.84-2.02(m, 2H), 2.36-2.54(m, 4H), 2.57-2.92(m, 7H), 3.44(s, 2H), 3.57(s, 2H), 3.65(s, 2H), 3.92(t, J = 7.3Hz, 2H), 6.89(d, J = 1.3Hz, 1H), 7.03(s, 2H), 7.11(d, J = 1.3Hz, 1H), 7.15(d, J = 8.1Hz, 2H), 7.31(d, J = 8.1Hz, 2H).

Example 25(4): 2-[4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]-2-methylpropanoic acid

TLC: Rf 0.58 (chloroform : methanol : 28% ammonia water = 40 : 10 : 2);
NMR($CD_3OD$): δ 1.01(t, J = 7.5Hz, 6H), 1.41-2.02(m, 18H), 2.30-2.53(m, 4H), 2.63-3.08(m, 7H), 3.39-3.50(m, 2H), 3.58 (s, 2H), 3.60(s, 2H), 3.75-3.93(m, 2H), 6.89(d, J = 1.3Hz, 1H), 7.02(s, 2H), 7.11(d, J = 1.3Hz, 1H), 7.17(d, J = 8.2Hz, 2H), 7.40(d, J = 8.2Hz, 2H).

Example 25(5): (2E)-3-[3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]acrylic acid

TLC: Rf 0.13 (chloroform : methanol : 28% ammonia water = 40 : 10: 2);
NMR($CD_3OD$): δ 1.06(t, J = 7.4Hz, 6H), 1.64-1.82(m, 2H), 1.80-2.18(m, 11H), 2.83 - 2.95(m, 2H), 3.04(s, 2H), 3.13-3.38 (m, 6H), 3.67(s, 2H), 3.73(s, 2H), 3.86(s, 2H), 3.97(t, J = 7.2Hz, 2H), 6.53(d, J = 15.9Hz, 1H), 7.00(d, J = 1.3Hz, 1H), 7.17-7.21(m, 3H), 7.28-7.44(m, 2H), 7.47-7.53(m, 1H), 7.58(d, J = 15.9Hz, 1H), 7.55-7.58(m, 1H).

Example 25(6): (2E)-3-[4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]acrylic acid

TLC: Rf 0.56 (dichloromethane : methanol: 28% ammonia water = 80: 20 : 4);
NMR($CD_3OD$): δ 0.92(t, J = 7.5Hz, 6H), 1.28-1.44(m, 2H), 1.49-1.62(m, 8H), 1.65-1.78(m, 2H), 2.05-2.28(m, 5H), 2.39-2.56(m, 6H), 3.51(s, 2H), 3.61 (s, 2H), 3.65(s, 2H), 3.90(t, J = 7.0Hz, 2H), 6.48(d, J = 16.0Hz, 1H), 6.87(d, J = 1.3Hz, 1H), 7.01(s, 2H), 7.05(d, J = 1.3Hz, 1H), 7.29(d, J = 8.2Hz, 2H), 7.35(d, J = 16.0Hz, 1H), 7.47(d, J = 8.2Hz, 2H).

Example 25(7): 3-[4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]propanoic acid

TLC: Rf 0.60 (dichloromethane : methanol: 28% ammonia water = 80 : 20 : 4);
NMR($CD_3OD$): δ 0.98(t, J = 7.5Hz, 6H), 1.44-1.62(m, 4H), 1.64-1.90(m, 8H), 2.41-2.64(m, 7H), 2.68-2.97(m, 8H), 3.45 (s, 2H), 3.54(s, 2H), 3.62(s, 2H), 3.87(t, J = 7.0Hz, 2H), 6.88(d, J = 1.3Hz, 1H), 7.02(s, 2H), 7.07(d, J = 1.3Hz, 1H), 7.16 (d, J = 8.1Hz, 2H), 7.22(d, J = 8.1Hz, 2H).

Example 25(8): 3-[3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]propanoic acid

TLC: Rf 0.15 (dichloromethane : methanol : 28% ammonia water = 80 : 20 : 2);
NMR($CD_3OD$): δ 0.96(t, J = 7.5Hz, 6H), 1.36-1.98(m, 12H), 2.42-2.53(m, 3H), 2.59-2.68(m, 2H), 2.69-2.81(m, 6H), 2.88-2.96(m, 2H), 2.97-3.07(m, 2H), 3.46(s, 2H), 3.59(s, 2H), 3.63(s, 2H), 3.81(t, J = 7.5Hz, 2H), 6.88(d, J = 1.3Hz, 1H), 6.99-7.04(m, 1H), 7.06(s, 2H), 7.07-7.09(m, J = 1.3Hz, 1H), 7.10-7.25(m, 3H).

Example 25(9): 5-{[(1-{3-[8-(1-ethylpropyl)-2,8-diazaspiro[4.5]deca-2-yl]propyl}-1H-imidazol-2-yl)methyl](1H-imidazol-2-ylmethyl)amino}pentanoic acid

TLC: Rf 0.33 (dichloromethane : methanol : 28% ammonia water = 80 : 20 : 5);
NMR($CD_3OD$): δ 0.96(t, J = 7.2Hz, 6H), 1.40-1.80(m, 14H), 1.80-1.98(m, 2H), 2.05-2.18(m, 2H), 2.30-2.80(m, 13H), 3.64(s, 4H), 3.95(t, J = 7.2Hz, 2H), 6.86(d, J = 1.5Hz, 1H), 7.02(s, 2H), 7.07(d, J = 1.5Hz, 1H).

Example 26: Ethyl 4-{({1-[3-(2,8-diazaspiro[4.5]deca-2-yl)propyl]-1H-imidazol-2-yl}methyl)[(1-{[2-(trimethylsilyl)ethoxy] methyl}-1H-imidazol-2-yl)methyl] amino }butanoate

Under an argon atmosphere, the compound synthesized in Example 19(1) (1.0 g) was dissolved in ethanol (10 mL) and a 4N hydrogen chloride-dioxane solution (5 mL) was added, followed by stirring at room temperature for 3 hours. The reaction solution was concentrated under reduced pressure and the residue was purified by silica gel chromatography (manufactured by FUJI SILYSIA CHEMICAL LTD., CHROMATOREX NH (trade name)) (n-hexane : ethyl acetate = 4 : 1 → 1 : 1 → 0 : 1 → ethyl acetate : methanol = 100 : 0 → 80 : 20) to obtain the title compound (1.0 g) having the following physical properties.
TLC: Rf 0.21 (dichloromethane : methanol : 28% ammonia water = 80 : 20 : 2);
NMR($CD_3OD$): δ 0.02(s, 9H), 0.97(t, J = 8.4Hz, 2H), 1.20(t, J = 7.2Hz, 3H), 1.70-1.88(m, 2H), 1.88-2.50(m, 8H), 2.58-2.72 (m, 2H), 3.15-3.88(m, 14H), 4.05(q, J = 7.2Hz, 2H), 4.33(s, 2H), 4.37(s, 2H), 4.44(t, J = 7.5Hz, 2H), 5.68(s, 2H), 7.58-7.65 (m, 2H), 7.70-7.80(m, 2H).

Example 27: Ethyl 4-{({1-[3-(8-isobutyl-2,8-diazaspiro[4.5]deca-2-yl)propyl]-1H-imidazol-2-yl}methyl)[(1-{[2-(trimethylsilyl)ethoxy]methyl}-1H-imidazol-2-yl)methyl]amino}butanoate

The same procedure as in Example 3 was carried out, except that isobutylaldehyde was used in place of the compound synthesized in Example 1 and the compound synthesized in Example 26 was used in place of the compound synthesized in Example 2 in Example 3, the title compound having the following physical properties was obtained. TLC: Rf 0.31 (ethyl acetate : methanol : 28% ammonia water = 90 : 10 : 2);
NMR($CDCl_3$): δ 0.03(s, 9H), 0.83(t, J = 8.4Hz, 2H), 0.89(d, J = 6.3Hz, 6H), 1.23(t, J = 7.2Hz, 3H), 1.45-1.90(m, 11H), 2.04(d, J = 7.2Hz, 2H), 2.17(t, J = 7.2Hz, 2H), 2.18-2.40(m, 8H), 2.48(t, J = 7.2Hz, 2H), 2.56(t, J = 7.2Hz, 2H), 3.34(t, J = 8.4Hz, 2H), 3.70(s, 2H), 3.75(s, 2H), 3.90(t, J = 6.9Hz, 2H), 4.07(q, J = 7.2Hz, 2H), 5.16(s, 2H), 6.87(d, J = 1.2Hz, 1H), 6.90-7.00(m, 3H).

Example 28: 4-{({1-[3-(8-isobutyl-2,8-diazaspiro[4.5]deca-2-yl)propyl]-1H-imidazol-2-yl}methyl)[(1-{[2-(trimethylsilyl) ethoxy]methyl}-1H-imidazol-2-yl)methyl]amino}butanoic acid

The same procedure as in Example 4 was carried out, except that the compound synthesized in Example 27 was used in place of the compound synthesized in Example 3 in Example 4, the title compound having the following physical properties was obtained. TLC: Rf 0.44 (dichloromethane : methanol : 28% ammonia water = 80 : 20 : 5);
NMR($CD_3OD$): δ 0.05(s, 9H), 1.52-1.92(m, 2H), 1.98(t, J = 7.2Hz, 2H), 2.10(d, J = 7.2Hz, 2H), 2.24-2.50(m, 10H), 2.55 (t, J = 6.9Hz, 2H), 3.30-3.52(m, 2H), 3.69(s, 2H), 3.71(s, 2H), 4.00(t, J = 6.9Hz, 2H), 5.28(s, 2H), 6.87(d, J = 1.5Hz, 1H), 6.89(d, J = 1.5Hz, 1H), 7.11(d, J = 1.5Hz, 1H), 7.19(d, J = 1.5Hz, 1H).

Example 29: 2-(dimethylamino)-2-oxoethyl 4-{({1-[3-(8-isobutyl-2,8-diazaspiro[4.5]deca-2-yl)propyl]-1H-imidazol-2-yl} methyl)[(1-{[2-(trimethylsilyl)ethoxy]methyl}-1H-imidazol-2-yl)methyl]amino}butanoate

Under an argon atmosphere, the compound synthesized in Example 28 (350 mg) and N,N-dimethyl-2-hydroxyacetoamide (71 mg) were dissolved in N,N-dimethylformamide (DMF) (5 mL). 1-hydroxybenzotriazole (HOBt) (124 mg) and 1-ethyl-3-(3-dimethylaminopropyl)carbodiimide hydrochloride (EDC)(176 mg) were sequentially added, followed by stirring at room temperature for 17 hours. To the reaction solution, an aqueous sodium hydrogencarbonate solution (20 mL) was added, followed by extraction twice with dichloromethane (50 mL). The organic layers were combined, washed with saturated brine and then dried over anhydrous magnesium sulfate. Anhydrous magnesium sulfate was removed by filtration and then the filtrate was concentrated. The residue was purified by silica gel chromatography (manufactured by FUJI SILYSIA CHEMICAL LTD., CHROMATOREX NH (trade name)) (ethyl acetate : 2-propanol = 100 : 0 → 20 : 1) to obtain the title compound 224 mg) having the following physical properties.
TLC: Rf 0.44 (dichloromethane : methanol : 28% ammonia water = 80 : 20 : 5); NMR($CD_3OD$): δ 0.05(s, 9H), 1.52-1.92 (m, 2H), 1.98(t, J = 7.2Hz, 2H), 2.10(d, J = 7.2Hz, 2H), 2.24-2.50(m, 10H), 2.55(t, J = 6.9Hz, 2H), 3.30-3.52(m, 2H), 3.69(s, 2H), 3.71(s, 2H), 4.00(t, J = 6.9Hz, 2H), 5.28(s, 2H), 6.87(d, J = 1.5Hz, 1H), 6.89(d, J = 1.5Hz, 1H), 7.11(d, J = 1.5Hz, 1H), 7.19(d, J = 1.5Hz, 1H).

Example 30: 2-(dimethylamino)-2-oxoethyl 4-[(1H-imidazol-2-ylmethyl)({1-[3-(8-isobutyl-2,8-diazaspiro[4.5]deca-2-yl) propyl]-1H-imidazol-2-yl}methyl) amino] butanoate

Under an argon atmosphere, trifluoroacetic acid (5 mL) was added to the compound (190 mg) synthesized in Example 29, followed by stirring at room temperature for 4 hours. The reaction solution was concentrated under reduced pressure. The residue was purified by silica gel chromatography (manufactured by FUJI SILYSIA CHEMICAL LTD., CHROMATOREX NH (trade name)) (n-hexane : ethyl acetate = 1 : 1 → 0 : 1 → ethyl acetate : 2-propanol = 100 : → 20 : 1) to obtain the title compound (155 mg) having the following physical properties.
TLC: Rf 0.53 (ethyl acetate : methanol : 28% ammonia water = 80 : 20 : 5);
NMR($CDCl_3$): δ 0.98(d, J = 6.3Hz, 6H), 1.70-2.12(m, 11H), 2.45-3.20(m, 16H), 2.96(s, 3H), 2.99(s, 3H), 3.68(s, 2H), 3.69(s, 2H), 4.05(t, J = 6.9Hz, 2H), 4.72(s, 2H), 6.95(d, J = 1.2Hz, 1H), 7.04(d, J = 1.2Hz, 1H), 7.10(s, 2H).

Example 30(1) to Example 30(6):

The same procedure as in Example 26 → Example 27 → Example 28 → Example 29 → Example 30 was carried out, except that the compound synthesized in Example 19(2) or the compound synthesized in Example 19(1) was used in place of the compound synthesized in Example 19(1) in Example 26 and corresponding compound was used in place of 2-hydroxy-N,N-dimethyl acetamide in Example 29, the following compounds were obtained.

Example 30(1): 2-morpholin-4-ylethyl 4-[(1H-imidazol-2-ylmethyl)({1-[3-(8-isobutyl-2,8-diazaspiro[4.5]deca-2-yl)propyl]-1H-imidazol-2-yl}methyl)amino]butanoate

TLC: Rf 0.24 (dichloromethane : methanol: 28% ammonia water = 80 : 20 : 1);
NMR($CDCl_3$) : δ 0.88(d, J = 6.6Hz, 6H), 1.52-1.65(m, 6H), 1.68-2.00(m, 7H), 2.04(d, J = 7.2Hz, 2H), 2.20-2.43(m, 10H), 2.45-2.58(m, 6H), 2.61(t, J = 6.0Hz, 2H), 3.52(s, 2H), 3.61(s, 2H), 3.70(t, J = 4.5Hz, 4H), 4.05(t, J = 6.9Hz, 2H), 4.20(t, J = 6.0Hz, 2H), 6.95(d, J = 1.2Hz, 1H), 7.01(d, J = 1.2Hz, 1H), 7.04(s, 2H).

Example 30(2): 2-(diethylamino)-2-oxoethyl 4-[(1H-imidazol-2-ylmethyl)({1-[3-(8-isobutyl-2,8-diazaspiro[4.5]deca-2-yl) propyl]-1H-imidazol-2-yl}methyl)amino]butanoate

TLC: Rf 0.51 (dichloromethane : methanol : 28% ammonia water = 80 : 20 : 2);
NMR($CDCl_3$): δ 0.88(d, J = 6.4Hz, 6H), 1.13(t, J = 7.1Hz, 3H), 1.23(t, J = 7.1Hz, 3H), 1.47-1.65(m, 6H), 1.68-2.00(m, 5H), 2.03(d, J = 7.5Hz, 2H), 2.20-2.40(m, 8H), 2.45-2.62(m, 6H), 3.25(q, J = 7.1Hz, 2H), 3.38(q, J = 7.1Hz, 2H), 3.53(s, 2H), 3.62(s, 2H), 4.02(t, J = 7.0Hz, 2H), 4.70(s, 2H), 6.93(d, J = 1.3Hz, 1H), 6.95-7.09(m, 2H), 6.98(d, J = 1.3Hz, 1H), 12.16-12.39(m, 1H).

Example 30(3): 2-oxo-2-pyrrolidin-1-ylethyl 4-[(1H-imidazol-2-ylmethyl)({1-[3-(8-isobutyl-2,8-diazaspiro[4.5]deca-2-yl) propyl]-1H-imidazol-2-yl}methyl)amino]butanoate

TLC: Rf 0.47 (dichloromethane : methanol: 28% ammonia water = 80 : 20 : 2);
NMR($CDCl_3$): δ 0.88(d, J = 6.6Hz, 6H), 1.47-2.07(m, 11H), 1.48-1.70(m, 6H), 2.21-2.40(m, 8H), 2.45-2.62(m, 6H), 3.39(t, J = 6.9Hz, 2H), 3.49(t, J = 6.9Hz, 2H), 3.53(s, 2H), 3.63(s, 2H), 4.03(t, J = 6.9Hz, 2H), 4.62(s, 2H), 6.93(d, J = 1.3Hz, 1H), 6.96-7.09(m, 2H), 6.98(d, J = 1.3Hz, 1H), 12.18-12.37(m, 1H).

Example 30(4): 2-(diethylamino)-2-oxoethyl 5-[(1H-imidazol-2-ylmethyl)({1-[3-(8-isobutyl-2,8-diazaspiro[4.5]deca-2-yl)propyl]-1H-imidazol-2-yl}methyl)amino]pentanoate

TLC: Rf 0.61 (ethyl acetate : methanol: 28% ammonia water = 80 :20 : 5);
NMR($CDCl_3$): δ 0.88(d, J = 6.6Hz, 6H), 1.13(t, J = 7.2Hz, 3H), 1.23(t, J = 7.2Hz, 3H), 1.45-2.00(m, 13H), 2.04(d, J = 7.8Hz, 2H), 2.18-2.60(m, 14H), 3.25(q, J = 7.2Hz, 2H), 3.38(q, J = 7.2Hz, 2H), 3.53(s, 2H), 3.62(s, 2H), 4.03(t, J = 6.9Hz, 2H), 4.72(s, 2H), 6.93(d, J = 1.2Hz, 1H), 6.99(d, J = 1.2Hz, 1H), 7.03(brs, 2H).

Example 30(5): 2-oxo-2-pyrrolidin-1-ylethyl 5-[(1H-imidazol-2-ylmethyl)({1-[3-(8-isobutyl-2,8-diazaspiro[4.5]deca-2-yl)propyl]-1H-imidazol-2-yl}methyl)amino]pentanoate

TLC: Rf 0.61 (ethyl acetate : methanol: 28% ammonia water = 80 : 20 : 5);
NMR($CDCl_3$): δ 0.88(d, J = 6.6Hz, 6H), 1.48-2.10(m, 17H), 2.20-2.60(m, 16H), 3.40(q, J = 6.9Hz, 2H), 3.49(q, J = 7.2Hz, 2H), 3.54(s, 2H), 3.63(s, 2H), 4.02(t, J = 6.9Hz, 2H), 4.63(s, 2H), 6.93(d, J = 1.2Hz, 1H), 6.99(d, J = 1.2Hz, 1H), 7.03 (brs, 2H), 12.3(brs, 1H).

Example 30(6): 2-(dimethylamino)-2-oxoethyl 5-[(1H-imidazol-2-ylmethyl)({1-[3-(8-isobutyl-2,8-diazaspiro[4.5]deca-2-yl)propyl]-1H-imidazol-2-yl}methyl)amino]pentanoate

TLC: Rf 0.58 (ethyl acetate : methanol : 28% ammonia water = 80 : 20 : 5);
NMR($CDCl_3$): δ 0.87(d, J = 6.6Hz, 6H), 1.48-1.85(m, 11H), 1.85-2.00(m, 2H), 2.03(d, J = 7.2Hz, 2H), 2.20-2.60(m, 14H), 2.96(s, 3H), 2.98(s, 3H), 3.52(s, 2H), 3.61(s, 2H), 4.02(t, J = 6.9Hz, 2H), 4.71(s, 2H), 6.92(d, J = 1.2Hz, 1H), 6.99(d, J = 1.2Hz, 1H), 6.99-7.06(m, 2H), 12.2(brs, 1H).

Example 31: 2-(dimethylamino)-2-oxoethyl 4-{[(1-{3-[8-(1-ethylpropyl)-2,8-diazaspiro[4.5]deca-2-yl]propyl}-1H-imidazol-2-yl)methyl](1H-imidazol-2-ylmethyl)amino}butanoate

The same procedure as in Example 24 → Example 28 → Example 29 → Example 30 was carried out, except that the compound synthesized in Example 26 was used in place of the compound synthesized in Example 20 in Example 24, the title compound having the following physical properties was obtained.
TLC: Rf 0.36 (dichloromethane : methanol : 28% ammonia water = 14 : 20 : 2);
NMR($CDCl_3$): δ 0.88(t, J = 7.4Hz, 6H), 1.18-1.37(m, 2H), 1.38-1.65(m, 8H), 1.85-2.18(m, 5H), 2.28-2.45(m, 8H), 2.46-2.62 (m, 6H), 2.96(s, 3H), 2.98(s, 3H), 3.53(s, 2H), 3.62(s, 2H), 4.03(t, J = 6.9Hz, 2H), 4.71(s, 2H), 6.93(d, J = 1.3Hz, 1H), 6.96-7.12(m, 3H), 11.98-12.56(m, 1H).

Example 31(1) to Example 31(5):

The same procedure as in Example 26 → Example 24 → Example 28 → Example 29 → Example 30 was carried out, except that the compound synthesized in Example 19(2) or the compound synthesized in Example 19(1) was used in place of the compound synthesized in Example 19(1) in Example 26 and corresponding compound was used in place of 2-hydroxy-N,N-dimethyl acetamide in Example 29, the following compounds of the present invention were obtained.

Example 31(1): 2-(diethylamino)-2-oxoethyl 4-{[(1-{3-[8-(1-ethylpropyl)-2,8-diazaspiro[4.5]deca-2-yl]propyl}-1H-imidazol-2-yl)methyl](1H-imidazol-2-ylmethyl)amino}butanoate

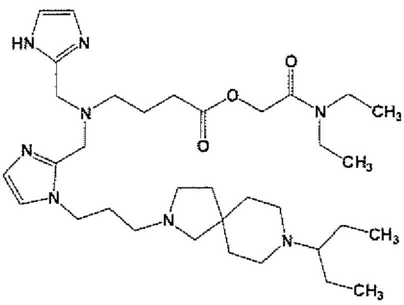

TLC: Rf 0.36 (dichloromethane : methanol: 28% ammonia water = 10 : 20 : 2); NMR($CDCl_3$): δ 0.88(t, J = 7.4Hz, 6H), 1.13(t, J = 7.1Hz, 3H), 1.18-1.37(m, 5H), 1.38-1.65(m, 8H), 1.82-2.20(m, 5H), 2.27-2.45(m, 8H), 2.46-2.64(m, 6H), 3.25(q, J = 7.1Hz, 2H), 3.38(q, J = 7.1Hz, 2H), 3.54(s, 2H), 3.62(s, 2H), 4.02(t, J = 6.9Hz, 2H), 4.70(s, 2H), 6.93(d, J = 1.3Hz, 1H), 695-7.12(m, 3H), 12.03-12.51(m, 1H).

Example 31(2): 2-oxo-2-(1-pyrrolidinyl)ethyl 4-{[(1-{3-[8-(1-ethylpropyl)-2,8-diazaspiro[4.5]deca-2-yl]propyl}-1H-imidazol-2-yl)methyl](1H-imidazol-2-ylmethyl)amino}butanoate

TLC: Rf 0.38 (dichloromethane: methanol: 28% ammonia water = 10 : 20 : 2);
NMR($CDCl_3$): δ 0.88(t, J = 7.3Hz, 6H), 1.18-1.37(m, 2H), 1.38-1.66(m, 8H), 1.76-2.18(m, 9H), 2.27-2.45(m, 8H), 2.46-2.63 (m, 6H), 3.39(t, J = 6.8Hz, 2H), 3.44-3.57(m, 4H), 3.63(s, 2H), 4.03(t, J = 6.9Hz, 2H), 4.62(s, 2H), 6.93(d, J = 1.3Hz, 1H), 6.96-7.09(m, 3H), 12.12-12.46(m, 1H).

Example 31(3): 2-(dimethylamino)-2-oxoethyl 5-{[(1-{3-[8-(1-ethylpropyl)-2,8-diazaspiro[4.5]deca-2-yl]propyl}-1H-imidazol-2-yl)methyl](1H-imidazol-2-ylmethyl)amino}pentanoate

TLC: Rf 0.56 (ethyl acetate : methanol: 28% ammonia water = 80 : 20 : 5);
NMR(CDCl3): δ 0.88(t, J = 7.5Hz, 6H), 1.20-1.80(m, 14H), 1.84-2.00(m, 2H), 2.12(m, 1H), 2.30-2.58(m, 14H), 2.96(s, 3H), 2.98(s, 3H), 3.52(s, 2H), 3.61(s, 2H), 4.02(t, J = 6.9Hz, 2H), 4.71(s, 2H), 6.93(m, 1H), 6.98(m, 1H), 6.98-7.06(m, 2H), 12.3(brs, 1H).

Example 31(4): 2-(diethylamino)-2-oxoethyl 5-{[(1-{3-[8-(1-ethylpropyl)-2,8-diazaspiro[4.5]deca-2-yl]propyl}-1H-imidazol-2-yl)methyl](1H-imidazol-2-ylmethyl)amino}pentanoate

TLC: Rf 0.63 (ethyl acetate : methanol: 28% ammonia water = 80 : 20 : 5);
NMR($CDCl_3$): δ 0.88(t, J = 7.2Hz, 6H), 1.12(t, J = 7.2Hz, 3H), 1.18-1.84(m, 17H), 1.84-2.00(m, 2H), 2.12(m, 1H), 2.30-2.58 (m, 14H), 3.25(q, J = 7.2Hz, 2H), 3.38(q, J = 7.2Hz, 2H), 3.53(s, 2H), 3.62(s, 2H), 4.03(t, J = 7.2Hz, 2H), 4.72(s, 2H), 6.94(m, 1H), 6.99(m, 1H), 6.99-7.06(m, 2H), 12.3(brs, 1H).

Example 31(5): 2-oxo-2-(1-pyrrolidinyl)ethyl 5-{[(1-{3-[8-(1-ethylpropyl)-2,8-diazaspiro[4.5]deca-2-yl]propyl}-1H-imidazol-2-yl)methyl](1H-imidazol-2-ylmethyl)amino}pentanoate

TLC: Rf 0.62 (ethyl acetate : methanol: 28% ammonia water = 80 : 20 : 5);
NMR($CDCl_3$): δ 0.88(t, J = 7.5Hz, 6H), 1.20-2.08(m, 20H), 2.12(m, 1H), 2.30-2.58(m, 14H), 3.40(t, J = 6.9Hz, 2H), 3.49 (t, J = 6.9Hz, 2H), 3.53(s, 2H), 3.62(s, 2H), 4.02(t, J = 6.6Hz, 2H), 4.63(s, 2H), 6.93(d, J = 1.2Hz, 1H), 6.99(d, J = 1.2Hz, 1H), 6.99-7.08(m, 2H), 12.3(brs, 1H).

Example 32: 2-[(benzylamino)methyl]-N,N-dimethyl-1H-imidazole-1-sulfonamide

The same procedure as in Example 15 was carried out, except that 2-formyl-N,N-dimethyl-1H-imidazole-1-sulfonamide was used in place of the compound synthesized in Example 14 and benzylamine was used in place of methyl 4-aminomethylphenylacetate in Example 15, the title compound having the following physical properties was obtained. TLC: Rf 0.50 (chloroform : methanol: 28% ammonia water = 90 : 10 : 1);
NMR($CDCl_3$): δ 2.85(s, 6H), 3.84(s, 2H), 4.07(s, 2H), 7.00(d, J = 1.8Hz, 1H), 7.24(d, J = 1.8Hz, 1H), 7.25-7.35(m, 6H).

Example 33: 2-[(benzyl{[1-(3-{[tert-butyl(dimethyl)silyl]oxy}propyl)-1H-imidazol-2-yl]methyl}amino)methyl]-N,N-dimethyl-1H-imidazole-1-sulfonamide

The same procedure as in Example 3 was carried out, except that the compound synthesized in Example 14 was used in place of the compound synthesized in Example 1 and the compound synthesized in Example 32 was used in place of the compound synthesized in Example 2 in Example 3, the title compound having the following physical properties was obtained.
TLC: Rf 0.40 (chloroform : methanol : 28% ammonia water = 90 : 10 : 1);
NMR($CDCl_3$); δ 0.03(s, 6H), 0.89(s, 9H), 1.65-1.80(m, 2H), 2.71(s, 6H), 3.47(t, J = 6.0Hz, 2H), 3.81(s, 2H), 3.88(s, 2H), 3.93(s, 2H), 4.02(t, J = 6.0Hz, 2H), 6.85(d, J = 1.2Hz, 1H), 6.88(d, J = 1.2Hz, 1H), 7.00(d, J = 1.2Hz, 1H), 7.20-7.35(m, 6H).

Example 34: 2-[(benzyl{[1-(3-hydroxypropyl)-1H-imidazol-2-yl]methyl}amino)methyl]-N,N-dimethyl-1H-imidazole-1-sulfonamide

The same procedure as in Example 17 was carried out, except that the compound synthesized in Example 33 was used in place of the compound synthesized in Example 16 in Example 17, the title compound having the following physical properties was obtained.
TLC: Rf 0.38 (chloroform : methanol : 28% ammonia water = 90 : 10 : 1);
NMR($CDCl_3$): δ 1.90-2.05(m, 2H), 2.75(s, 6H), 3.53(t, J = 5.4Hz, 2H), 3.76(s, 2H), 3.85(s, 2H), 3.91(s, 2H), 4.16(t, J = 6.6Hz, 2H), 6.88(d, J = 1.5Hz, 1H), 6.95(d, J = 1.5Hz, 1H), 7.03(d, J = 1.5Hz, 1H), 7.23(d, J = 1.5Hz, 1H), 7.20-7.35(m, 3H), 7.40-7.45(m, 2H).

Example 35: 3-(2-{[benzyl({1-[(dimethylamino)sulfonyl]-1H-imidazol-2-yl}methyl)amino]methyl}-1H-imidazol-1-yl)propyl methanesulfonate

The same procedure as in Example 18 was carried out, except that the compound synthesized in Example 34 was used in place of the compound synthesized in Example 17 in Example 18, the title compound having the following physical properties was obtained. TLC: Rf 0.40 (chloroform : methanol: 28% ammonia water = 90 : 10 : 1);
NMR($CDCl_3$): δ 1.96-2.08(m, 2H), 2.73(s, 6H), 2.98(s, 3H), 3.75(s, 2H), 3.86(s, 2H), 3.92(s, 2H), 4.06-4.17(m, 4H), 6.87 (d, J = 1.3Hz, 1H), 6.92(d, J = 1.3Hz, 1H), 7.02(d, J = 1.7Hz, 1H), 7.23(d, J = 1.7Hz, 1H), 7.25-7.33(m, 5H).

Example 36: tert-butyl 2-[3-(2-{[benzyl({1-[(dimethylamino)sulfonyl]-1H-imidazol-2-yl}methyl)amino]methyl}-1H-imidazol-1-yl)propyl]-2,8-diazaspiro[4.5]decane-8-carboxylate

The same procedure as in Example 19 was carried out, except that the compound synthesized in Example 35 was used in place of the compound synthesized in Example 18 in Example 19, the title compound having the following physical properties was obtained. TLC: Rf 0.37 (chloroform : methanol : 28% ammonia water = 90 : 10 : 1);
NMR($CDCl_3$): δ 1.43-1.74(m, 17H), 2.20(t, J = 6.2Hz, 2H), 2.27(s, 2H), 2.47(t, J = 6.2Hz, 2H), 2.71-2.72(m, 6H), 3.23-3.34 (m, 2H), 3.34-3.45(m, 2H), 3.80(s, 2H), 3.87(s, 2H), 3.92(s, 2H), 3.97(t, J = 7.0Hz, 2H), 6.84(d, J = 1.3Hz, 1H), 6.88(d, J = 1.3Hz, 1H), 7.00(d, J = 1.7Hz, 1H), 7.22(d, J = 1.7Hz, 1H), 7.24-7.35(m, 5H).

Example 37: N-benzyl-1-{1-[3-(2,8-diazaspiro[4.5]deca-2-yl)propyl]-1H-imidazol-2-yl}-N-(1H-imidazol-2-ylmethyl)methaneamine

The same procedure as in Example 6 was carried out, except that the compound synthesized in Example 36 was used in place of the compound synthesized in Example 5 in Example 6, the title compound having the following physical properties was obtained.
TLC: Rf 0.15 (chloroform: methanol: 28% ammonia water = 50 : 10 : 1);
NMR($CDCl_3$): δ 1.55-1.88(m, 8H), 2.26(t, J = 6.9Hz, 2H), 2.31(s, 2H), 2.50(t, J = 6.9Hz, 2H), 3.00-3.13(m, 4H), 3.47(s, 2H), 3.65(s, 2H), 3.67(s, 2H), 3.89(t, J = 6.9Hz, 2H), 6.90(d, J = 0.7Hz, 1H), 7.00(d, J = 0.7Hz, 1H), 7.09(s, 2H), 7.23-7.42 (m, 5H).

Example 38: N-benzyl-1-(1H-imidazol-2-yl)-N-({1-[3-(8-isobutyl-2,8-diazaspiro[4.5]deca-2-yl)propyl]-1H-imidazol-2-yl} methyl)methaneamine

The same procedure as in Example 3 was carried out, except that 2-methylpropanal was used in place of the compound synthesized in Example 1 and the compound synthesized in Example 37 was used in place of the compound synthesized in Example 2 in Example 3, the title compound having the following physical properties was obtained.

TLC: Rf 0.15 (chloroform : methanol : 28% ammonia water = 50 : 10 : 1);
NMR($CDCl_3$): δ 1.55-1.88(m, 8H), 2.26(t, J = 6.9Hz, 2H), 2.31(s, 2H), 2.50(t, J = 6.9Hz, 2H), 3.00-3.13(m, 4H), 3.47(s, 2H), 3.65(s, 2H), 3.67(s, 2H), 3.89(t, J = 6.9Hz, 2H), 6.90(d, J = 0.7Hz, 1H), 7.00(d, J = 0.7Hz, 1H), 7.09(s, 2H), 7.23-7.42 (m, 5H).

Example 39: 1-(1H-imidazol-2-yl)-N-({1-[3-(8-isobutyl-2,8-diazaspiro[4.5]deca-2-yl)propyl]-1H-imidazol-2-yl}methyl) methaneamine

To an acetic acid (6 mL) solution of the compound (700 mg) synthesized in Example 38, 20% palladium hydroxide on carbon (140 mg) was added and the atmosphere in the reaction system was replaced by hydrogen. The reaction solution was stirred at 50°C for 8 hours. The reaction solution was filtered through celite (trade name) to remove the palladium hydroxide on carbon, and then the filtrate was concentrated. The residue was purified by silica gel chromatography (manufactured by FUJI SILYSIA CHEMICAL LTD., CHROMATOREX NH (trade name)) (ethyl acetate : methanol = 30 : 1) to obtain the title compound (200 mg) having the following physical properties.
TLC: Rf 0.16 (dichloromethane : methanol : 28% ammonia water = 90 : 10 : 1);
NMR($CDCl_3$): δ 0.89(d, J = 6.6Hz, 6H), 1.50-1.66(m, 6H), 1.79(m, 1H), 1.90-2.02(m, 2H), 2.06(d, J = 7.2Hz, 2H), 2.24-2.42 (m, 8H), 2.55(t, J = 6.9Hz, 2H), 3.80(s, 2H), 3.85(s, 2H), 4.02(t, J = 6.9Hz, 2H), 6.91 (d, J = 1.2Hz, 1H), 6.99(d, J = 1.2Hz, 1H), 7.01 (s, 2H).

Example 40: Methyl 2-{[(1H-imidazol-2-ylmethyl)({1-[3-(8-isobutyl-2,8-diazaspiro[4.5]deca-2-yl)propyl]-1H-imidazol-2-yl}methyl)amino]methyl}benzonate

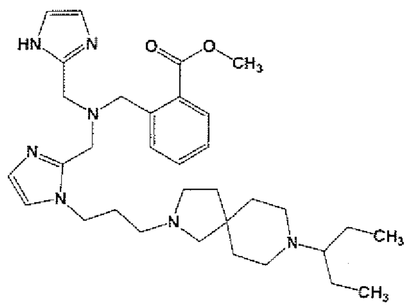

The same procedure as in Example 3 was carried out, except that methyl 2-formylbenzoate was used in place of the compound synthesized in Example 1 and the compound synthesized in Example 39 was used in place of the compound synthesized in Example 2 in Example 3, the title compound having the following physical properties. TLC: Rf 0.41 (dichloromethane : methanol : 28% ammonia water = 90: 10: 1);
NMR($CDCl_3$): δ 0.88(d, J = 6.6Hz, 6H), 1.45-1.84(m, 9H), 2.03(d, J = 7.2Hz, 2H), 2.16-2.32(m, 8H), 2.42(t, J = 6.9Hz, 2H), 3.51(s, 2H), 3.54(s, 2H), 3.84-3.90(m, 5H), 3.97(s, 2H), 6.90(d, J = 1.2Hz, 1H), 6.98(d, J = 1.2Hz, 1H), 7.03-7.14 (m, 2H), 7.35(dt, J = 1.2, 7.5Hz, 1H), 7.46(dt, J = 1.2, 7.5Hz, 1H), 7.52(dd, J = 7.5, 1.2Hz, 1H), 7.83(dd, J = 7.5, 1.2Hz, 1H).

Example 40(1) to Example 40(16):

The same procedure as in Example 38 → Example 39 → Example 40 was carried out, except that corresponding aldehyde or ketone was used in place of 2-methylpropanal in Example 38 and corresponding aldehyde or ketone was used in place of methyl 2-formylbenzoate in Example 40, the following compounds of the present invention were obtained.

Example 40(1): 2-[(1H-imidazol-2-ylmethyl)({1-[3-(8-isobutyl-2,8-diazaspiro[4.5]deca-2-yl)propyl]-1H-imidazol-2-yl}methyl)amino]ethanol

TLC: Rf 0.20 (dichloromethane : methanol : 28% ammonia water = 90 : 10 : 1);
NMR($CDCl_3$): δ 0.88(d, J = 6.6Hz, 6H), 1.50-1.66(m, 6H), 1.76(m, 1H), 1.88-2.05(m, 2H), 2.03(d, J = 7.2Hz, 2H), 2.23-2.40 (m, 8H), 2.52(t, J = 6.9Hz, 2H), 2.78(t, J = 5.1Hz, 2H), 3.64-3.78(m, 6H), 4.04(t, J = 6.9Hz, 2H), 6.93(d, J = 1.5Hz, 1H), 7.00-7.04(m, 3H).

Example 40(2): Methyl 3-{[(1H-imidazol-2-ylmethyl)({1-[3-(8-isobutyl-2,8-diazaspiro[4.5]deca-2-yl)propyl]-1H-imidazol-2-yl}methyl)amino]methyl}benzonate

TLC: Rf 0.35 (dichloromethane : methanol : 28% ammonia water = 90 : 10 : 1);
NMR($CDCl_3$): δ 0.88(d, J = 6.6Hz, 6H), 1.43-1.90(m, 9H), 2.02(d, J = 7.2Hz, 2H), 2.18-2.34(m, 8H), 2.45(t, J = 6.9Hz, 2H), 3.49(s, 2H), 3.62(s, 2H), 3.72(s, 2H), 3.88-3.96(m, 5H), 6.93(d, J = 1.2Hz, 1H), 7.00(d, J = 1.2Hz, 1H), 7.03-7.14 (m, 2H), 7.41(t, J = 7.8Hz, 1H), 7.60(d, J = 7.8Hz, 1H), 7.94(d, J = 7.8Hz, 1H), 8.09(s, 1H).

Example 40(3): Methyl 4-{[(1H-imidazol-2-ylmethyl)({1-[3-(8-isobutyl-2,8-diazaspiro [4.5]deca-2-yl)propyl]-1H-imidazol-2-yl}methyl)amino]methyl}benzonate

TLC: Rf 0.37 (dichloromethane : methanol : 28% ammonia water = 90 : 10 : 1);
NMR($CDCl_3$): δ 0.88(d, J = 6.6Hz, 6H), 1.48-1.92(m, 9H), 2.02(d, J = 7.2Hz, 2H), 2.20-2.34(m, 8H), 2.46(t, J = 6.9Hz, 2H), 3.49(s, 2H), 3.61(s, 2H), 3.71 (s, 2H), 3.88-3.95(m, 5H), 6.93(d, J = 1.2Hz, 1H), 7.01(d, J = 1.2Hz, 1H), 7.05-7.15 (m, 2H), 7.48(d, J = 8.1Hz, 2H), 8.01(d, J = 8.1Hz, 2H).

Example 40(4): 3-{[(1H-imidazol-2-ylmethyl)({1-[3-(8-isobutyl-2,8-diazaspiro[4.5]deca-2-yl)propyl]-1H-imidazol-2-yl} methyl)amino]methyl}phenol

TLC: Rf 0.30 (dichloromethane : methanol: 28% ammonia water = 90 : 10 : 1);
NMR($CDCl_3$): δ 0.88(d, J = 6.6Hz, 6H), 1.50-1.70(m, 6H), 1.77(m, 1H), 1.95-2.10(m, 4H), 2.20-2.40(m, 4H), 2.42(s, 2H), 2.46(t, J = 6.9Hz, 2H), 2.61(t, J = 6.9Hz, 2H), 3.48(s, 2H), 3.61(s, 2H), 3.64(s, 2H), 3.92(t, J = 7.5Hz, 2H), 6.73-6.82(m, 2H), 691(d, J = 1.5Hz, 1H), 6.97(m, 1H), 7.01 (d, J = 1.5Hz, 1H), 7.09(s, 2H), 7.14(t, J = 7.5Hz, 1H).

Example 40(5): 4-{[(1H-imidazol-2-ylmethyl)({1-[3-(8-isobutyl-2,8-diazaspiro[4.5]deca-2-yl)propyl]-1H-imidazol-2-yl} methyl)amino]methyl}phenol

TLC: Rf 0.30 (dichloromethane : methanol : 28% ammonia water = 90 : 10 : 1);
NMR($CDCl_3$): δ 0.88(d, J = 6.6Hz, 6H), 1.48-1.66(m, 6H), 1.70-1.98(m, 3H), 2.06(d, J = 7.2Hz, 2H), 2.20-2.40(m, 8H), 2.54(t, J = 6.9Hz, 2H), 3.52(s, 2H), 3.56(s, 2H), 3.61(s, 2H), 3.94(t, J = 6.9Hz, 2H), 6.72(d, J = 8.4Hz, 2H), 6.91(d, J = 1.2Hz, 1H), 7.01(d, J = 1.2Hz, 1H), 7.07(s, 2H), 7.13(d, J = 8.4Hz, 2H).

Example 40(6): 2-{[(1H-imidazol-2-ylmethyl)({1-[3-(8-isobutyl-2,8-diazaspiro[4.5]deca-2-yl)propyl]-1H-imidazol-2-yl} methyl)amino]methyl}phenol

TLC: Rf 0.31 (dichloromethane : methanol : 28% ammonia water = 90 : 10 : 1);
NMR($CDCl_3$): δ 0.89(d, J = 6.6Hz, 6H), 1.50-1.66(m, 6H), 1.72-1.96(m, 3H), 2.06(d, J = 7.2Hz, 2H), 2.20-2.40(m, 8H), 2.48(t, J = 6.9Hz, 2H), 3.63(s, 2H), 3.65(s, 2H), 3.78(s, 2H), 3.99(t, J = 6.9Hz, 2H), 6.82(dt, J = 1.5, 7.5Hz, 1H), 6.92 (dd, J = 7.5, 1.5Hz, 1H), 6.94(d, J = 1.5Hz, 1H), 7.04(d, J = 1.5Hz, 1H), 7.07(s, 2H), 7.10(dd, J = 7.5, 1.5Hz, 1H), 7.19 (m, 1H).

Example 40(7): N-(1H-imidazol-2-ylmethyl)-N-({1-[3-(8-isobutyl-2,8-diazaspiro[4.5]deca-2-yl)propyl]-1H-imidazol-2-yl} methyl)cyclopentaneamine

TLC: Rf 0.52 (ethyl acetate : methanol : 28% ammonia water = 80 : 10 : 2);
NMR($CDCl_3$): δ 0.89(d, J = 6.4Hz, 6H), 1.34-2.18(m, 19H), 2.23-2.34(m, 4H), 2.35(s, 2H), 2.39(t, J = 6.9Hz, 2H), 2.54 (t, J = 6.9Hz, 2H), 2.67-2.81(m, 1H), 3.55(s, 2H), 3.63-3.70(m, 2H), 4.09(t, J = 6.9Hz, 2H), 6.95(d, J = 1.3Hz, 1H), 7.01 (d, J = 1.3Hz, 1H), 7.05(s, 2H), 12.13-12.82(m, 1H).

Example 40(8): N-(1H-imidazol-2-ylmethyl)-N-({1-[3-(8-isobutyl-2,8-diazaspiro[4.5]deca-2-yl)propyl]-1H-imidazol-2-yl} methyl)cyclohexaneamine

TLC: Rf 0.52 (ethyl acetate : methanol : 28% ammonia water = 80 : 10 : 2);
NMR($CDCl_3$): δ 0.88(d, J = 6.6Hz, 6H), 1.01-1.42(m, 6H), 1.48-1.65(m, 6H), 1.68-2.10(m, 9H), 2.20-2.42(m, 8H), 2.46-2.61(m, 3H), 3.65(s, 2H), 3.78(s, 2H), 4.01 (t, J = 7.0Hz, 2H), 6.91(d, J = 0.9Hz, 1H), 6.99(s, 3H), 11.69-12.60(m, 1H).

Example 40(9): Ethyl (4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenoxy)acetate

TLC: Rf 0.20 (dichloromethane : methanol : 28% ammonia water = 90 : 10 : 1);
NMR($CDCl_3$): δ 0.88(d, J = 6.6Hz, 6H), 1.30(t, J = 7.1Hz, 3H), 1.45-1.65(m, 6H), 1.68-1.80(m, 1H), 1.80-1.95(m, 2H), 2.03(d, J = 7.3Hz, 2H), 2.14-2.39(m, 8H), 2.49(t, J = 6.9Hz, 2H), 3.47(s, 2H), 3.59(s, 2H), 3.60(s, 2H), 3.90(t, J = 6.9Hz, 2H), 4.28(q, J = 7.1Hz, 2H), 4.61(s, 2H), 6.88(d, J = 8.6Hz, 2H), 6.93(d, J = 1.3Hz, 1H), 7.00(d, J = 1.3Hz, 1H), 7.03-7.16 (m, 2H), 7.33(d, J = 8.7Hz, 2H), 12.39-12.60(m, 1H).

Example 40(10): Ethyl (3-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenoxy)acetate

TLC: Rf 0.28 (dichloromethane : methanol : 28% ammonia water = 90 : 10 : 1);
NMR($CDCl_3$): δ 0.88(d, J = 6.4Hz, 6H), 1.29(t, J = 7.1Hz, 3H), 1.41-1.92(m, 9H), 2.03(d, J = 7.3Hz, 2H), 2.16-2.37(m, 8H), 2.47(t, J = 6.9Hz, 2H), 3.47(s, 2H), 3.61(s, 2H), 3.64(s, 2H),3.91(t, J = 7.0Hz, 2H), 4.26(q, J = 7.1Hz, 1H), 4.61 (s, 2H), 6.76-6.83(m, 1H), 6.92(d, J = 1.3Hz, 1H), 6.95-7.13(m, 4H), 6.99(d, J = 1.3Hz, 1H), 7.24(dd, J = 7.9, 7.5Hz, 2H), 12.31-12.58(m, 1H).

Example 40(11): Ethyl [4-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenoxy]acetate

TLC: Rf 0.26 (dichloromethane : methanol : 28% ammonia water = 90 : 10 : 1);
NMR($CDCl_3$): δ 0.84(s, 9H), 1.30(t, J = 7.1Hz, 3H), 1.43-1.61(m, 6H), 1.79-1.93(m, 2H), 1.99(s, 2H), 2.24-2.34(m, 4H), 2.34-2.45(m, 4H), 2.49(t, J = 7.0Hz, 2H), 3.47(s, 2H), 3.59(s, 2H), 3.60(s, 2H), 3.90(t, J = 7.2Hz, 2H), 4.28(q, J = 7.1 Hz, 2H), 4.61 (s, 2H), 6.88(d, J = 8.6Hz, 2H), 6.93(d, J = 1.3Hz, 1H), 7.00(d, J = 1.3Hz, 1H), 7.03-7.16(m, J = 10.6Hz, 2H), 7.33(d, J = 8.6Hz, 2H), 12.38-12.61(m, 1H).

Example 40(12): Ethyl (4-{[({1-[3-(8-cyclohexyl-2,8-diazaspiro[4.5]deca-2-yl)propyl]-1H-imidazol-2-yl}methyl)(1H-imidazol-2-ylmethyl)amino]methyl}phenoxy)acetate

TLC: Rf 0.54 (dichloromethane : methanol : 28% ammonia water = 80 : 20 : 2);
NMR($CDCl_3$): δ 1.01-1.27(m, 6H), 1.30(t, J = 7.1Hz, 3H), 1.47-1.67(m, 8H), 1.80-1.94(m, 4H), 2.15-2.35(m, 5H), 2.39-2.57 (m, 6H), 3.47(s, 2H), 3.59(s, 2H), 3.60(s, 2H), 3.90(t, J = 6.9Hz, 2H), 4.28(q, J = 7.1Hz, 2H), 4.61 (s, 2H), 6.88(d, J = 8.8Hz, 2H), 6.93(d, J = 1.3Hz, 1H), 7.00(d, J = 1.3Hz, 1H), 7.07(s, 1H), 7.11(s, 1H), 7.33(d, J = 8.8Hz, 2H), 12.41-12.57 (m, 1H).

Example 40(13): Ethyl (3-{[({1-[3-(8-cyclohexyl-2,8-diazaspiro[4.5]deca-2-yl)propyl]-1H-imidazol-2-yl}methyl)(1H-imidazol-2-ylmethyl)amino]methyl}phenoxy)acetate

TLC: Rf 0.27 (dichloromethane : methanol : 28% ammonia water = 90 : 10 : 1);
NMR($CDCl_3$): δ 0.93-1.97(m, 18H), 1.29(t, J = 7.1Hz, 3H), 2.13-2.61(m, 11H), 3.48(s, 2H), 3.61(s, 2H),3.64(s, 2H), 3.91 (t, J = 6.8Hz, 2H), 4.26(q, J = 7.1Hz, 2H), 4.61(s, 2H), 6.76-6.82(m, 1H), 6.92(d, J = 1.1Hz, 1H), 6.94-7.15(m, 4H), 6.99 (d, J = 1.1Hz, 1H), 7.17-7.33(m, 1H), 12.34-12.56(m, 1H).

Example 40(14): Ethyl [3-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenoxy]acetate

TLC: Rf 0.42 (dichloromethane : methanol : 28% ammonia water = 90 : 10 : 1);
NMR($CDCl_3$): δ 0.84(s, 9H), 1.29(t, J = 7.1Hz, 3H), 1.43-1 .60(m, 6H), 1.79-1.92(m, 2H), 1.98(s, 2H), 2.22-2.33(m, 4H), 2.34-2.43(m, 4H), 2.47(t, J = 6.9Hz, 2H), 3.48(s, 2H), 3.61(s, 2H), 3.63(s, 2H), 3.91(t, J = 6.9Hz, 2H), 4.26(q, J = 7.1Hz, 2H), 4.61(s, 2H), 6.77-6.83(m, 1H), 6.92(d, J = 1.3Hz, 1H), 6.95-7.14(m, 4H), 6.99(d, J = 1.3Hz, 1H), 7.24(t, J = 7.9Hz, 1H), 12.34-12.58(m, 1H).

Example 40(15): Ethyl (2-fluoro-5-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenoxy)acetate

TLC: Rf 0.60 (ethyl acetate : methanol : 28% ammonia water = 80 : 20 : 4);
NMR($CDCl_3$): δ 0.88(d, J = 6.6Hz, 6H), 1.29(t, J = 7.2Hz, 3H), 1.44-1.95(m, 9H), 2.03(d, J = 6.6Hz, 2H), 2.15-2.40(m,

8H), 2.48(t, J = 6.9Hz, 2H), 3.48(s, 2H), 3.58(s, 4H), 3.91(t, J = 6.9Hz, 2H), 4.24(q, J = 7.2Hz, 2H), 4.70(s, 2H), 6.88-7.15 (m, 7H), 12.4(brs, 1H).

Example 40(16): Ethyl (5-{[(1H-imidazol-2-ylmethyl)({1-[3-(8-isobutyl-2,8-diazaspiro[4.5]deca-2-yl)propyl]-1H-imidazol-2-yl}methyl)amino]methyl}-2-methoxyphenoxy)acetate

TLC: Rf 0.48 (ethyl acetate : methanol : 28% ammonia water = 80 : 20 : 4);
NMR($CDCl_3$): δ 0.88(d, J = 6.6Hz, 6H), 1.27(t, J = 7.2Hz, 3H), 1.42-1.92(m, 9H), 2.03(d, J = 7.2Hz, 2H), 2.15-2.40(m, 8H), 2.47(t, J = 6.9Hz, 2H), 3.47(s, 2H), 3.57(s, 2H), 3.59(s, 2H), 3.88(s, 3H), 3.90(t, J = 6.9Hz, 2H), 4.24(q, J = 7.2Hz, 2H), 4.68(s, 2H), 6.84(d, J = 8.1Hz, 1H), 6.88-7.15(m, 6H), 12.4(brs, 1H).

Example 41: 2-{[(1H-imidazol-2-ylmethyl)({1-[3-(8-isobutyl-2,8-diazaspiro[4.5]deca-2-yl)propyl]-1H-imidazol-2-yl}methyl)amino]methyl}benzoic acid

The same procedure as in Example 4 was carried out, except that the compound synthesized in Example 40 was used in place of the compound synthesized in Example 3 in Example 4, the title compound having the following physical properties were obtained.
TLC: Rf 0.48 (dichloromethane : methanol : 28% ammonia water = 50 : 10 : 1);
NMR($CD_3OD$): δ 0.93(d, J = 6.6Hz, 6H), 1.68-2.08(m, 9H), 2.23(d, J = 7.2Hz, 2H), 2.45-2.62(m, 4H), 3.00(t, J = 6.9Hz, 2H), 3.07(s, 2H), 3.20-3.33(m, 2H), 3.40(s, 2H), 3.70(s, 2H), 3.86(t, J = 6.9Hz, 2H), 3.95(s, 2H), 6.86(d, J = 1.2Hz, 1H), 6.93(s, 2H), 6.96(d, J = 1.2Hz, 1H), 7.22-7.30(m, 3H), 7.43(m, 1H).

Example 41(1) to Example 41(10):

The same procedure as in Example 41 was carried out, except that the compounds synthesized in Example 40(1) to Example 40(16) were used in place of the compound synthesized in Example 40 in Example 41, the following compounds of the present invention were obtained.

Example 41(1): 3-{[(1H-imidazol-2-ylmethyl)({1-[3-(8-isobutyl-2,8-diazaspiro[4.5]deca-2-yl)propyl]-1H-imidazol-2-yl}methyl)amino]methyl}benzoic acid

TLC: Rf 0.33 (dichloromethane : methanol : 28% ammonia water = 50 : 10 : 1);
NMR($CD_3OD$): δ 0.96(d, J = 6.6Hz, 6H), 1.57-1.85(m, 8H), 1.94(m, 1H), 2.28(t, J = 6.9Hz, 2H), 2.37-2.48(m, 4H), 2.58-2.75(m, 6H), 3.54(s, 2H), 3.62(s, 2H), 3.65(s, 2H), 3.93(t, J = 6.9Hz, 2H), 6.86(d, J = 1.2Hz, 1H), 7.03(s, 2H), 7.05 (d, J = 1.2Hz, 1H), 7.28-7.34(m, 2H), 7.84(m, 1H), 7.94(s, 1H).

Example 41(2): 4-{[(1H-imidazol-2-ylmethyl)({1-[3-(8-isobutyl-2,8-diazaspiro[4.5]deca-2-yl)propyl]-1H-imidazol-2-yl}methyl)amino]methyl}benzoic acid

TLC: Rf 0.31 (dichloromethane : methanol : 28% ammonia water = 50 : 10 : 1);
NMR($CD_3OD$): δ 0.96(d, J = 6.6Hz, 6H), 1. 56-1.80(m, 8H), 1.96(m, 1H), 2.18(t, J = 6.9Hz, 2H), 2.32(s, 2H), 2.46(d, J = 7.2Hz, 2H), 2.54(t, J = 6.9Hz, 2H), 2.60-2.80(m, 4H), 3.54(s, 2H), 3.60(s, 2H), 3.67(s, 2H), 3.89(t, J = 6.9Hz, 2H), 6.88 (d, J = 1.2Hz, 1H), 7.02(s, 2H), 7.05(d, J = 1.2Hz, 1H), 7.29(d, J = 8.4Hz, 2H), 7.90(d, J = 8.4Hz, 2H).

Example 41(3): 4-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenoxy)acetic acid

TLC: Rf 0.43 (dichloromethane : methanol: 28% ammonia water = 30 : 10 : 2);
NMR($CD_3OD$): δ 0.94(d, J = 6.6Hz, 6H), 1.46-1.84(m, 8H), 1.84-2.00(m, 1H), 2.18-2.79(m, 12H), 3.42(s, 2H), 3.55(s, 2H), 3.62(s, 2H), 3.87(t, J = 7.0Hz, 2H), 4.38(s, 2H), 6.82-6.93(m, 3H), 7.02(s, 2H), 7.07(d, J = 1.5Hz, 1H), 7.19(d, J = 8.8Hz, 2H).

Example 41(4): (3-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenoxy)acetic acid

TLC: Rf 0.34 (dichloromethane : methanol : 28% ammonia water = 70 : 30 : 3);
NMR($CD_3OD$): δ 0.95(d, J = 6.4Hz, 6H), 1.51-2.01(m, 9H), 2.24-3.14(m, 12H), 3.46(s, 2H),3.61(s, 2H), 3.65(s, 2H), 3.78 (t, J = 7.3Hz, 2H), 4.39(s, 2H), 6.76-6.86(m, 3H), 6.88(d, J = 1.1Hz, 1H), 7.05(s, 2H), 7.08(d, J = 1.1Hz, 1H), 7.19(t, J

= 8.0Hz, 1H).

Example 41(5): [4-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenoxy]acetic acid

TLC: Rf 0.39 (dichloromethane : methanol : 28% ammonia water = 30 : 10 : 2);
NMR($CD_3OD$): δ 0.91(s, 9H), 1.47-1.81(m, 8H), 2.13-2.28(m, 2H), 2.28-2.41(m, 2H), 2.46-2.73(m, 6H), 2.80-2.98(m, 2H), 3.41(s, 2H), 3.51(s, 2H), 3.64(s, 2H), 3.88(t, J = 7.0Hz, 2H), 4.41(s, 2H), 6.84-6.93(m, 3H), 7.02(s, 2H), 7.07(d, J = 1.3Hz, 1H), 7.19(d, J= 8.8Hz, 2H).159

Example 41(6): (4-{[({1-[3-(8-cyclohexyl-2,8-diazaspiro[4.5]deca-2-yl)propyl]-1H-imidazol-2-yl}methyl)(1H-imidazol-2-ylmethyl)amino]methyl}phenoxy)acetic acid

TLC: Rf 0.40 (dichloromethane : methanol : 28% ammonia water = 30 : 10 : 2);
NMR($CD_3OD$): δ 1.08-1.46(m, 6H), 1.49-1.76(m, 6H), 1.76-1.92(m, 4H), 1.99-2.10(m, 2H), 2.20-2.39(m, 4H), 2.63(t, J = 6.5Hz, 2H), 2.70-2.87(m, 4H), 2.87-3.06(m, 1H), 3.42(s, 2H), 3.56(s, 2H), 3.61(s, 2H), 3.86(t, J = 7.1Hz, 2H), 4.33-4.43 (m, 2H), 6.81-6.92(m, 3H), 7.02(s, 2H), 7.08(d, J = 1.3Hz, 1H), 7.18(d, J = 8.6Hz, 2H).

Example 41(7): (3-{[{1-[3-(8-cyclohexyl-2,8-diazaspiro[4.5]deca-2-yl)propyl]-1H-imidazol-2-yl}methyl)(1H-imidazol-2-ylmethyl)amino]methyl}phenoxy)acetic acid

TLC: Rf 0.30 (dichloromethane : methanol : 28% ammonia water = 70 : 30 : 3);
NMR($CD_3OD$): δ 1.03-2.13(m, 18H), 2.23-3.13(m, 11H), 3.46(s, 2H), 3.62(s, 2H), 3.64(s, 2H), 3.86(t, J = 6.7Hz, 2H), 4.39(s, 2H), 6.74-6.87(m, 3H), 6.88(d, J = 1,1Hz, 1H), 7.04(s, 2H),7.08(d, J = 1.1Hz, 1H), 7.19(t, J = 7.8Hz, 1H).

Example 41(8): [3-({[(1-{3-[8-(2,2-dimethylpropyl)-2,8-diazaspiro[4.5]deca-2-yl]propyl}-1H-imidazol-2-yl)methyl](1H-imidazol-2-ylmethyl)amino}methyl)phenoxy]acetic acid

TLC: Rf 0.65 (dichloromethane : methanol : 28% ammonia water = 70 : 30 : 3);
NMR($CD_3OD$): δ 0.89(s, 9H), 1.53-1.77(m, 4H), 1.80-1.99(m, 4H), 2.10-2.24(m, 2H), 2.47-2.65(m, 4H), 2.67-2.86(m, 2H), 2.92-3.10(m, 2H), 3.16-3.37(m, 2H), 3.47(s, 2H), 3.61(s, 2H), 3.65(s, 2H), 3.67-3.76(m, 2H), 4.40(s, 2H), 6.73-6.88 (m, 3H), 6.89(d, J = 1.3Hz, 1H), 7.06(s, 2H), 7.09(d, J = 1.3Hz, 1H), 7.20(t, J = 7.8Hz, 1H).

Example 41(9): (2-fluoro-5-{[(1H-imidazol-2-ylmethyl)({1-[3-(8-isobutyl-2,8-diazaspiro[4.5]deca-2-yl)propyl]-1H-imidazol-2-yl}methyl)amino]methyl}phenoxy)acetic acid

TLC: Rf 0.41 (dichloromethane : methanol: 28% ammonia water = 80 : 20 : 5);
NMR($CD_3OD$): δ 0.94(d, J = 6.6Hz, 6H), 1.40-2.00(m, 9H), 2.18-3.18(m, 12H), 3.44(s, 2H), 3.55(s, 2H), 3.67(s, 2H), 3.77(t, J = 7.5Hz, 2H), 4.46(s, 2H), 6.72-6.79(m, 1H), 6.82-6.89(m, 2H), 7.00(d, J = 11.4, 8.4Hz, 1H), 7.05(s, 2H), 7.09 (d, J = 1.2Hz, 1H).

Example 41(10): (5-{[(1H-imidazol-2-ylmethyl)({1-[3-(8-isobutyl-2,8-diazaspiro[4.5]deca-2-yl)propyl]-1H-imidazol-2-yl}methyl)amino]methyl}-2-methoxyphenoxy)acetic acid

TLC: Rf 0.40 (dichloromethane : methanol : 28% ammonia water = 80 : 20 : 5);
NMR($CD_3OD$): δ 0.94(d, J = 6.6Hz, 6H), 1.60-1.98(m, 9H), 2.18-3.35(m, 12H), 3.39(s, 2H), 3.57(s, 2H), 3.65(s, 2H), 3.68(t, J = 8.1Hz, 2H), 3.83(s, 3H), 4.41(s, 2H), 6.71(d, J = 2.1Hz, 1H), 6.79(dd, J = 8.4,2.1Hz, 1H), 6.86-6.92(m, 2H), 7.04(s, 2H), 7.08(d, J = 1.5Hz, 1H).

Example 42: Ethyl [4-({[(1-{3-[8-(1-ethylpmpyl)-2,8-diazaspiro[4.5]deca-2-yl]propyl}-1H-imidazol-2-yl)methyl](1H-imidazol-2-ylmethyl)amino}methyl)phenoxy]acetate

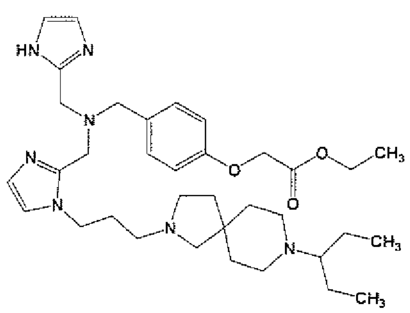

The same procedure as in Example 24 → Example 39 → Example 40, except that the compound synthesized in Example 37 was used in place of the compound synthesized in Example 20 in Example 24 and ethyl 4-formylphenoxyacetate was used in place of methyl 2-formylbenzoate in Example 40, the title compound having the following physical properties were obtained.
TLC: Rf 0.60 (ethyl acetate : methanol: 28% ammonia water = 100 : 30 : 3);
NMR($CDCl_3$): δ 0.88(t, J = 7.4Hz, 6H), 1.19-1.37(m, 5H), 1.38-1.63(m, 8H), 1.72-1.93(m, 2H), 2.05-2.17(m, 1H), 2.23-2.34 (m, 4H), 2.35-2.44(m, 4H), 2.48(t, J = 6.9Hz, 2H), 3.47(s, 2H), 3.55-3.63(m, 4H), 3.89(t, J = 6.9Hz, 2H), 4.27(q, J = 7.1Hz, 2H), 4.60(s, 2H), 6.83-6.90(m, 2H), 6.92(d, J = 1.3Hz, 1H), 6.99(d, J = 1.3Hz, 1H), 7.01-7.14(m, 2H), 7.27-7.35 (m, 2H), 12.47(s, 1H).

Example 42(1) to Example 42(8):

The same procedure as in Example 42 was carried out, except that corresponding aldehyde was used in place of ethyl 4-formylphenoxyacetate Example 42, the following compounds were obtained.

Example 42(1): Ethyl [3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenoxy]acetate

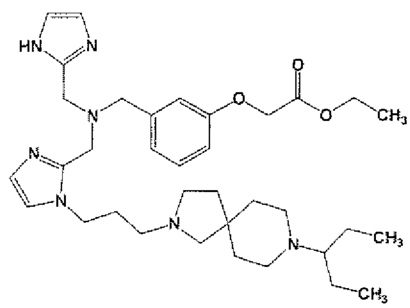

TLC: Rf 0.60 (ethyl acetate : methanol: 28% ammonia water = 100 : 30 : 3);
NMR($CDCl_3$): δ 0.88(t,J=7.4Hz,6H), 1.17-1.37(m, 5H), 1.37-1.61(m, 8H), 1.79-1.92(m, 2H), 2.05-2.17(m, 1H), 2.22-2.32 (m, 4H), 2.34-2.43(m, 4H), 2.47(t, J = 7.0Hz, 2H), 3.48(s, 2H), 3.61 (s, 2H), 3.63(s, 2H), 3.91(t, J = 7.0Hz, 2H), 4.26(q, J = 7.1Hz, 2H), 4.61(s, 2H), 6.79(m, 1H), 6.92(m, 1H), 6.95-7.13 (m, 5H), 7.19-7.29(m, 1H), 12.44(s, 1H).

Example 42(2): Ethyl {[3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]thio} acetate

TLC: Rf 0.55 (dichloromethane : methanol: 28% ammonia water = 80 : 10: 2);

NMR($CDCl_3$): δ 0.88(t, J = 7.3Hz, 6H), 1.15-1.35(m, 5H), 1.39-1.62(m, 8H), 1.78-1.92(m, 2H), 2.05-2.17(m, 1H), 2.24-2.32 (m, 4H), 2.39(t, J = 5.0Hz, 4H), 2.47(t, J = 6.9Hz, 2H), 3.48(s, 2H), 3.61(s, 2H), 3.63(s, 2H), 3.64(s, 2H), 3.91(t, J = 6.9Hz, 2H), 4.15(q, J = 7.2Hz, 2H), 6.92(s, 1H), 6.99(s, 1H), 7.05(s, 1H), 7.09(s, 1H), 7.22-7.33(m, 3H), 7.45(s, 1H), 12.24-12.58(m, 1H).

Example 42(3): Ethyl {[6-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)-2-naphthyl]oxy} acetate

TLC: Rf 0.55 (dichloromethane : methanol : 28% ammonia water = 80 : 10 : 2);
NMR(CDCl3): δ 0.88(t, J = 7.4Hz, 6H), 1.15-1.56(m, 13H), 1.74-1.86(m, 2H), 2.04-2.24(m, 5H), 2.31-2.43(m, 6H), 3.49 (s, 2H), 3.67(s, 2H), 3.79(s, 2H), 3.86(t, J = 7.0Hz, 2H), 4.29(q, J = 7.1Hz, 2H), 4.72(s, 2H), 6.90(d, J = 1.3Hz, 1H), 6.99 (d, J = 1.3Hz, 1H), 7.07(d, J = 2.7Hz, 1H), 7.08(s, 1H), 7.12(s, 1H), 7.19-7.26(m, 1H), 7.52(dd, J = 8.4, 1.7Hz, 1H), 7.66-7.75(m, 3H), 12.34-12.62(m, 1H).

Example 42(4): Ethyl 3-[5-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)-2-thienyl]propanoate

TLC: Rf 0.26 (dichloromethane : methanol : 28% ammonia water = 90 : 10 : 1);
NMR($CDCl_3$); δ 0.82-0.95(m, 6H), 1.20-1.37(m, 5H), 1.38-1.62(m, 8H), 1.87-1.99(m, 2H), 2.04-2.18(m, 1H), 2.28-2.45 (m, 6H), 2.51(t, J = 7.0Hz, 2H), 2.61-2.71(m, 2H), 3.11(t, J = 7.6Hz, 2H), 3.55(s, 2H), 3.61 (s, 2H), 3.80(s, 2H), 4.03(t, J = 6.9Hz, 2H), 4.15(q, J = 7.1Hz, 2H), 6.64(d, J = 3.4Hz, 2H), 6.77(d, J = 3.4Hz, 2H), 6.95(d, J = 1.4Hz, 1H), 7.01(d, J = 1.4Hz, 1H), 7.03-7.08(m, 1H), 7.08-7.15(m, 1H), 12.37-12.57(m, 1H).

Example 42(5): Ethyl 4-[5-({[(1-{3-[8-(1-etllylpropyl)-2,8-diazaspiro[4.5]deca-2-yl]propyl}-1H-imidazol-2-yl)methyl](1H-imidazol-2-ylmethyl)amino}methyl)-2-thienyl]butanoate

TLC: Rf0.26 (dichloromethane : methanol : 28% ammonia water = 90 : 10 : 1);
NMR($CDCl_3$): δ 0.88(t, J = 7.4Hz, 6H), 1.19-1.37(m, 5H), 1.38-1.63(m, 8H), 1.81-2.05(m, 4H), 2.05-2.18(m, 1H), 2.25-2.45 (m, 10H), 2.50(t, J = 6.9Hz, 2H), 2.83(t, J = 7.6Hz, 2H), 3.55(s, 2H), 3.62(s, 2H), 3.80(s, 2H), 4.02(t, J = 6.9Hz, 2H), 4.13(q, J = 7.1Hz, 2H), 6.61(d, J = 3.4Hz, 1H), 6.77(d, J = 3.4Hz, 1H), 6.95(d, J = 1.3Hz, 1H), 7.01(d, J = 1.3Hz, 1H), 7.02-7.17(m, 2H), 12.32-12.64(m, 1H).

Example 42(6): Ethyl [5-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)-2-methoxyphenoxy]acetate

TLC: Rf 0.76 (ethyl acetate : methanol : 28% ammonia water = 80 : 20 : 4);
NMR($CDCl_3$): δ 0.88(t, J = 7.8Hz, 6H), 1.27(t, J = 7.2Hz, 3H), 1.40-1.92(m, 12H), 2.12(m, 1H), 2.18-2.55(m, 10H), 3.47 (s, 2H), 3.57(s, 2H), 3.59(s, 2H), 3.87(s, 3H), 3.90(t, J = 6.3Hz, 2H), 4.23(q, J = 7.2Hz, 2H), 4.68(s, 2H), 6.84(d, J = 7.8Hz, 1H), 6.88-7.02(m, 4H), 7.02-7.16(m, 2H), 12.4(brs, 1H).

Example 42(7): Methyl [4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenoxy]acetate

TLC: Rf 0.64 (chloroform : methanol: 28% ammonia water = 90 : 10: 1);
NMR($CD_3OD$): δ 0.89(t, J = 7.4Hz, 6H), 1.20-1.40(m, 2H), 1.41-1.93(m, 10H), 2.07-2.21(m, 1H), 2.25-2.35(m, 4H), 2.37-2.56(m, 6H), 3.47(s, 2H), 3.48(s, 2H), 3.59(s, 3H), 3.81(s, 2H), 3.89(t, J = 7.0Hz, 2H), 4.62(s, 2H), 6.87(d, J = 8.4Hz, 2H), 6.91(d, J = 1.1Hz, 1H), 6.99(d, J = 1.1Hz, 1H), 7.07(s, 2H), 7.31(d, J = 8.4Hz, 2H).

Example 42(8): Methyl [3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenoxy]acetate

TLC: Rf 0.64 (chloroform : methanol : 28% ammonia water = 90 : 10 : 1);
NMR($CDCl_3$): δ 0.89(t, J = 7.4Hz, 6H), 1.18-1.39(m, 2H), 1.38-1.66(m, 4H), 1.70-2.05(m, 6H), 2.11-2.20(m, 1H), 2.25-2.34 (m, 4H), 2.36-2.55(m, 6H), 3.47-3.50(m, 4H), 3.63(s, 2H), 3.79(s, 3H), 3.90(t, J = 7.0Hz, 2H), 4.64(s, 2H), 6.74-7.04(m, 5H), 7.07(s, 2H), 7.16-7.32(m, 1H).

Example 43: [4-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenoxy]acetic acid

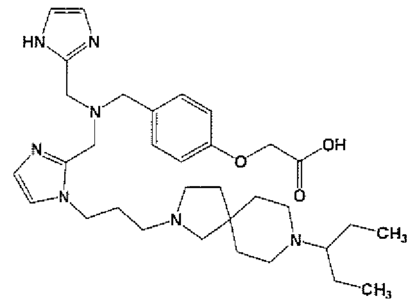

The same procedure as in Example 4, except that the compound synthesized in Example 42 was used in place of the compound synthesized in Example 3 in Example 4, the title compound having the following physical properties were obtained.
TLC: Rf 0.29 (dichloromethane : methanol: 28% ammonia water = 100 : 50 : 5);
NMR($DMSO-D_6$): δ 0.85(t, J = 7.4Hz, 6H), 1.20-1.45(m, 4H), 1.46-1.65(m, 6H), 1.65-1.82(m, 2H), 2.19-2.67(m, 11H), 3.40(s, 2H), 3.47(s, 2H), 3.50(s, 2H), 3.82(t, J = 7.0Hz, 2H), 4.21(s, 2H), 6.73(d, J = 8.6Hz, 2H), 6.81(d, J = 1.1Hz, 1H), 6.93-7.01(m, 2H), 7.11(d, J = 1.1Hz, 1H), 7.16(d, J = 8.6Hz, 2H).

Example 43(1) to Example 43(6):

The same procedure as in Example 43, except that corresponding compounds synthesized in Example 42(1) to Example 42(5) were used in place of the compound synthesized in Example 42 in Example 43, the following compounds of the present invention were obtained.

Example 43(1): [3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenoxy]acetic acid

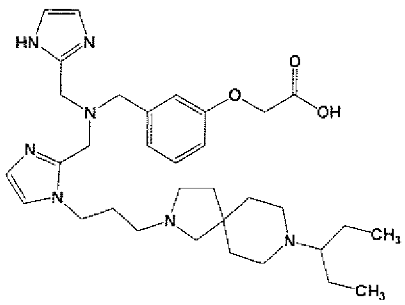

TLC: Rf 0.38 (dichloromethane: methanol : 28% ammonia water = 70 : 30 : 3);
NMR($CD_3OD$): δ 0.97(t, J = 7.4Hz, 6H), 1.37-1.94(m, 12H), 2.33-3.05(m, 11H), 3.46(s, 2H), 3.61(s, 2H), 3.65(s, 2H), 3.75-3.88(m, 2H), 4.39(s, 2H), 6.75-6.86(m, 3H), 6.86-6.90(m, 1H), 7.04(s, 2H), 7.06-7.10(m, 1H), 7.18(t, J = 8.1 Hz, 1H).

Example 43(2): ([3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenyl]thio}acetic acid

TLC: Rf 0.49 (chloroform : methanol: 28% ammonia water = 40 : 10 : 2);
NMR($CD_3OD$): δ 0.96(t, J = 7.5Hz, 6H), 1.35-1.93(m, 12H), 2.35-2.81(m, 9H), 2.85-3.01(m, 2H), 3.47(s, 2H), 3.56(s,

2H), 3.63(s, 2H), 3.65(s, 2H), 3.87(t, J = 7.4Hz, 2H), 6.86(d, J = 1.3Hz, 1H), 6.90-6.98(m, 1H), 7.02-7.08(m, 3H), 7.13-7.24 (m, 2H), 7.37(s, 1H).

Example 43(3): {[6-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)-2-naphthyl]oxy}acetic acid

TLC: Rf 0.42 (chloroform : methanol : 28% ammonia water = 40 : 10 : 2);
NMR($CD_3OD$): δ 0.99(t, J = 7.5Hz, 6H), 1.36-1.95(m, 16H), 2.28(t, J = 6.4Hz, 2H), 2.49-2.98(m, 5H), 3.61(s, 2H), 3.62 (s, 2H), 3.70(s, 2H), 3.88(t, J = 7.0Hz, 2H), 4.49(s, 2H), 6.88(d, J = 1.5Hz, 1H), 7.00-7.06(m, 3H), 7.15(d, J = 2.5Hz, 1H), 7.22(dd, J = 8.9, 2.5Hz, 1H), 7.36(dd, J = 8.2, 1.7Hz, 1H), 7.68-7.76(m, 3H).

Example 43(4): 3-[5-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)-2-thienyl]propanoic acid

TLC: Rf 0.59 (dichloromethane : methanol : 28% ammonia water = 30 : 10:2);
NMR($CD_3OD$): δ 0.96(t, J = 7.5Hz, 6H), 1.37-1.54(m, 2H), 1.55-1.85(m, 8H), 1.91-2.11(m, 2H), 2.37-2.54(m, 3H), 2.57-2.84(m, 8H), 2.96(t, J = 7.0Hz, 1H), 3.06(t, J = 6.9Hz, 2H), 3.59-3.71(m, 6H), 3.88(t, J = 7.2Hz, 2H), 6.66(d, J = 3.4Hz, 1H), 6.72(d, J = 3.4Hz, 2H), 6.89(d, J = 1.4Hz, 1H), 7.03(s, 2H), 7.12(d, J = 1.4Hz, 1H).173

Example 43(5): 4-[5-({[(1-{3-[8-(l-ethytpropyl)-2,8-diazaspiro[4.5]deca-2-yl]propyl}-1H-imidazol-2-yl)methyl](1H-imidazol-2-ylmethyl)amino}methyl)-2-thienyl]butanoic acid

TLC: Rf 0.59 (dichloromethane : methanol : 28% ammonia water = 30 : 10: 2);
NMR($CD_3OD$): δ 0.98(t, J = 7.5Hz, 6H), 1.41-1.82(m, 10H), 1.83-2.02(m, 4H), 2.22(t, J = 7.2Hz, 2H), 2.31-2.41(m, 2H), 2.43(s, 2H), 2.49-2.59(m, 1H), 2.65(t, J = 7.0Hz, 2H), 2.69-2.94(m, 6H), 3.56-3.74(m, 6H), 3.93(t, J = 7.2Hz, 2H), 6.64 (d, J = 3.4Hz, 1H), 6.76(d, J = 3.4Hz, 1H), 6.89(d, J = 1.4Hz, 1H), 7.02(s, 2H), 7.10(d, J = 1.4Hz, 1H).

Example 43(6): [5-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)-2-methoxyphenoxy]acetic acid

TLC: Rf 0.47 (dichloromethane : methanol : 28% ammonia water = 80 : 20 : 5);
NMR($CD_3OD$): δ 0.93(t, J = 7.5Hz, 6H), 1.24-1.83(m, 12H), 2.15-2.90(m, 11H), 3.38(s, 2H), 3.54(s, 2H), 3.64(s, 2H), 3.78-3,92(m, 2H), 3.82(s, 3H), 4.42(s, 2H), 6.76-6.91(m, 4H), 7.00-7.10(m, 2H), 7.07(d, J = 1.5Hz, 1H).

Example 44: N-benzyl-1-{-[3-(2,8-diazaspiro[4.5]deca-2-yl)propyl]-1H-imidazol-2-yl}-N-methylmethaneamine

The same procedure as in Example 9 → Example 10 → Example 11 → Example 26 was carried out, except that N-methylbenzylamine was used in place of the compound synthesized in Example 2 in Example 9, the title compound having the following physical properties was obtained.
TLC: Rf 0.05 (chloroform : methanol : 28% ammonia water = 90 : 10 : 1);
NMR($CDCl_3$): δ 1.50-1.65(m, 6H), 1.75-1.90(m, 2H), 2.16(s, 3H), 2.25-2.35(m, 4H), 2.51(t, J = 6.6Hz, 2H), 2.80-2.90(m, 4H), 3.51(s, 2H), 3.60(s, 2H), 4.04(t, J = 6.6Hz, 2H), 6.87(d, J = 1.2Hz, 1H), 6.92(d, J = 1.2Hz, 1H), 7.20-7.35(m, 5H).

Example 45: N-benzyl-1-{1-[3-(8-isobutyl-2,8-diazaspiro[4.5]deca-2-yl)propyl]-1H-imidazol-2-yl}-N-methylmethaneamine

The same procedure as in Example 3 was carried out, except that isobutylaldehyde was used in place of the compound synthesized in Example 1 and the compound synthesized in Example 44 was used in place of the compound synthesized in Example 2 in Example 3, the title compound having the following physical properties were obtained. TLC: Rf 0.37 (chloroform : methanol, 28% ammonia water = 90 : 10 : 1);
NMR($CDCl_3$): δ 0.88(d, J = 6.6Hz, 6H), 1.50-1.70(m, 6H), 1.70-1.90(m, 3H), 2.04(d, J = 7.2Hz, 2H), 2.16(s, 3H), 2.25-2.35 (m, 8H), 2.49(t, J = 6.9Hz, 2H), 3.51 (s, 2H), 3.60(s, 2H), 4.03(t, J = 6.9Hz, 2H), 6.87(d, J = 1.2Hz, 1H), 6.92(d, J = 1.2Hz, 1H), 7.20-7.3 5(m, 5H).

Example 46: 1-{1-[3-(8.isobutyl-2,8-diazaspiro[4.5]deca-2-yl)propyl]-1H-imidazol-2-yl}-N-methylmethaneamine

The same procedure as in Example 39 was carried out, except that the compound synthesized in Example 45 was used in place of the compound synthesized in Example 38 in Example 39, the title compound having the following

physical properties were obtained. TLC: Rf 0.28 (chloroform: methanol : 28% ammonia water = 90 : 10 : 1); NMR($CDCl_3$): δ 0.88(d, J = 6.6Hz, 6H), 1.55-1.65(m, 6H), 1.70-1.95(m, 3H), 2.04(d, J = 7.2Hz, 2H), 2.25-2.40(m, 8H), 2.45(s, 3H), 2.51(t, J = 7.2Hz, 2H), 3.81(s, 2H), 4.03(t, J = 6.9Hz, 2H), 6.86(d, J = 1.2Hz, 1H), 6.95(d, J = 1.2Hz, 1H).

Example 47: Ethyl (2-{[({1-[3-(8-isobutyl-2,8-diazaspiro[4.5]deca-2-yl)propyl]-1H-imidazol-2-yl}methyl)(methyl)amino] methyl}-1H-imidazol-1-yl)acetate

The same procedure as in Example 3 was carried out, except that the compound synthesized in Example 46 was used in place of the compound synthesized in Example 2 in Example 3, the title compound having the following physical properties was obtained. TLC: Rf 0.27 (dichloromethane : methanol : 28% ammonia water = 90 : 10 : 1); NMR ($CDCl_3$): δ 0.88(d, J = 6.6Hz, 6H), 1.24(t, J = 7.2Hz, 3H), 1.50-1.90(m, 9H), 2.05(d, J = 7.2Hz, 2H), 2.24(s, 3H), 2.12-2.38 (m, 8H), 2.50(t, J = 6.9Hz, 2H), 3.61(so 4H), 3.94(t, J = 6.9Hz, 2H), 4.15(q, J = 7.2Hz, 2H), 4.75(s, 2H), 6.85(d, J = 1.2Hz, 1H), 6.87(d, J = 1.2Hz, 1H), 6.94(d, J = 1.2Hz, 1H), 6.96(d, J = 1.2Hz, 1H).

Example 47(1) to Example 47(9):

The same procedure as in Example 47 was carried out, except that corresponding aldehyde was used in place of ethyl(2-formyl-1H-imidazol-1-yl)acetate synthesized in Example 1 in Example 47, the following compound of the present invention were obtained.

Example 47(1): 2-(2-{[({1-[3-(8-isobutyl-2,8-diazaspiro[4.5]deca-2-yl)propyl]-1H-imidazol-2-yl}methyl)(methyl)amino] methyl}-1H-imidazol-1-yl)ethanol

TLC: Rf 0.16 (dichloromethane : methanol : 28% ammonia water = 90 : 10 : 1); NMR($CDCl_3$): δ 0.88(d, J = 6.6Hz, 6H), 1.52-1.90(m, 9H), 2.03(d, J = 7.2Hz, 2H), 2.24-2.36(m, 11H), 2.50(t, J = 6.9Hz, 2H), 3.70(s, 2H), 3.72(s, 2H), 3.80(t, J = 5.4Hz, 2H), 3.92(t, J = 6.9Hz, 2H), 4.00(t, J = 5.4Hz, 2H), 6.86(d, J = 1.2Hz, 1 H), 6.90(d, J = 1.2Hz, 1H), 6.96(d, J = 1.2Hz, 1H), 6.97(d, J = 1.2Hz, 1H).

Example 47(2): 1-{1-[3-(8-isobutyl-2,8-diazaspiro[4.5]deca-2-yl)propyl]-1H-imidazol-2-yl}-N-{[1-(2-methoxyethyl)-1H-imidazol-2-yl]methyl}-N-methylmethaneamine

TLC: Rf 0.20 (dichloromethane : methanol : 28% ammonia water = 90 : 10 : 1); NMR($CDCl_3$): δ 0.88(d, J = 6.6Hz, 6H), 1.50-1.87(m, 9H), 2.03(d, J = 7.2Hz, 2H), 2.21-2.38(m, 11H), 2.48(t, J = 6.9Hz, 2H), 3.28(s, 3H), 3.49(t, J = 5.4Hz, 2H), 3.63(s, 2H), 3.64(s, 2H), 3.92(t, J = 6.9Hz, 2H), 4.00(t, J = 5.4Hz, 2H), 6.87(d, J = 1.2Hz, 1H), 6.93(s, 2H), 6.94(d, J = 1.2Hz, 1H).

Example 47(3): Ethyl 3-(2-{[({1-[3-(8-isobutyl-2,8-diazaspiro[4.5]deca-2-yl)propyl]-1H-imidazol-2-yl}methyl)(methyl)amino]methyl}-1H-imidazol-1-yl)propanoate

TLC: Rf 0.60 (dichloromethane : methanol : 28% ammonia water = 80 : 20 : 2); NMR($CDCl_3$): δ 0.88(d, J = 6.6Hz, 6H), 1.24(t, J = 7.1Hz, 3H), 1.47-1.66(m, 6H), 1.69-1.85(m, 3H), 2.04(d, J = 7.3Hz, 2H), 2.16-2.40(m, 11H), 2.49(t, J = 6.9Hz, 2H), 2.60(t, J = 6.9Hz, 2H), 3.64(s, 2H), 3.65(s, 2H), 3.95(t, J = 6.9Hz, 2H), 4.07-4.19(m, 4H), 6.87(d, J = 1.3Hz, 1H), 6.88(d, J = 1.3Hz, 1H), 6.92(d, J = 1.3Hz, 1H), 6.95(d, J = 1.3Hz, 1H).

Example 47(4): Ethyl 4-(2-{[({1-[3-(8-isobutyl-2,8-diazaspiro[4.5]deca-2-yl)propyl]-1H-imidazol-2-yl}methyl)(methyl)amino]methyl}-1H-imidazol-1-yl)butanoate

TLC: Rf 0.57 (dichloromethane : methanol: 28% ammonia water = 80 : 20 : 2); NMR($CDCl_3$): δ 0.88(d, J = 6.6Hz, 6H), 1.26(t, J = 7.1Hz, 3H), 1.48-1.65(m, 6H), 1.70-1.86(m, 3H), 1.86-1.99(m, 2H), 2.04(d, J = 7.3Hz, 2H), 2.17-2.39(m, 13H), 2.48(t, J = 7.0Hz, 2H), 3.62(s, 2H), 3.64(s, 2H), 3.86(t, J = 7.1Hz, 2H), 3.93 (t, J = 6.9Hz, 2H), 4.12(q, J = 7.1Hz, 2H), 6.86(d, J = 1.3Hz, 1H), 6.89(d, J = 1.3Hz, 1H), 6.95(d, J = 1.3Hz, 1H), 6.96 (d, J = 1.3Hz, 1H).

Example 47(5): Ethyl 5-(2-{[({1-[3-(8-isobutyl-2,8-diazaspiro[4.5]deca-2-yl)propyl]-1H-imidazol-2-yl}methyl)(methyl)amino]methyl}-1H-imidazol-1-yl)pentanoate

TLC: Rf 0.56 (dichloromethane : methanol : 28% ammonia water = 80 : 20 : 2); NMR($CDCl_3$): δ 0.89(d, J = 6.6Hz, 6H), 1.25(t, J = 7.1 Hz, 3H), 1.46-1.72(m, 10H), 1.72-1.90(m, 3H), 2.06(d, J = 7.1Hz,

2H), 2.20-2.36(m, 9H), 2.36-2.46(m, 4H), 2.50(t, J = 6.9Hz, 2H), 3.61(s, 2H), 3.64(s, 2H), 3.80(t, J = 7.1Hz, 2H), 3.94(t, J = 6.9Hz, 2H), 4.12(q, J = 7.1Hz, 2H), 6.85(d, J = 1.3Hz, 1H), 6.89(d, J = 1.3Hz, 1H), 6.94(d, J = 1.3Hz, 1H), 6.96(d, J = 1.3Hz, 1H).

Example 47(6): Methyl 2-[(2-{[({1-[3-(8-isobutyl-2,8-diazaspiro[4.5]deca-2-yl)propyl]-1H-imidazol-2-yl}methyl)(methyl) amino]methyl}-1H-imidazol-1-yl)methyl]benzonate

TLC: Rf 0.66 (dichloromethane ; methanol : 28% ammonia water = 80 : 20 : 2);
NMR($CDCl_3$): δ 0.88(d, J = 6.6Hz, 6H), 1.45-1.64(m, 6H), 1.66-1.85(m, 3H), 2.03(d, J = 7.3Hz, 2H), 2.12-2.37(m, 11H), 2.44(t, J = 6.9Hz, 2H), 3.55(s, 2H), 3.61(s, 2H), 3.82(t, J = 6.9Hz, 2H), 3.93(s, 3H), 5.50(s, 2H), 6.47(dd, J = 7.7, 0.9Hz, 1H), 6.76(d, J = 1.3Hz, 1H), 6.82(d, J = 1.3Hz, 2H), 7.04(d, J = 1.3Hz, 1H), 7.29-7.45(m, 2H), 8.03(dd, J = 7.7, 1.5Hz, 1H).

Example 47(7): Methyl 3-[(2-{[({1-[3-(8-isobutyl-2,8-diazaspiro[4.5]deca-2-yl)propyl]-1H-imidazol-2-yl}methyl)(methyl) amino]methyl}-1H-imidazol-1-yl)methyl]benzonate

TLC: Rf 0.66 (dichloromethane : methanol: 28% ammonia water = 80 : 20 : 2);
NMR($CDCl_3$): δ 0.88(d, J = 6.6Hz, 6H), 1.46-1.64(m, 6H), 1.66-1.87(m, 3H), 2.03(d, J = 7.3Hz, 2H), 2.12-2.37(m, 11H), 2.44(t, J = 6.9Hz, 2H), 3.62(s, 2H), 3.64(s, 2H), 3.79(t, J = 6.9Hz, 2H), 3.91(s, 3H), 5.06(s, 2H), 6.76-6.87(m, 2H), 6.92 (d, J = 1.3Hz, 1H), 6,99(d, J = 1.3Hz, 1H), 7.06-7.19(m, 1H), 7.38(t, J = 7.7Hz, 1H), 7.70-7.82(m, 1H), 7.89-8.02(m, 1H).181

Example 47(8): Methyl 4-[(2-{[({1-[3-(8-isobutyl-2,8-diazaspiro[4.5]deca-2-yl)propyl]-1H-imidazol-2-yl}methyl)(methyl) amino]methyl}-1H-imidazol-1-yl)methyl]benzonate

TLC: Rf 0.61 (dichloromethane : methanol : 28% ammonia water = 80 : 20 : 2);
NMR($CDCl_3$): δ 0.88(d, J = 6.6Hz, 6H), 1.46-1.64(m, 6H), 1.66-1.86(m, 3H), 2.04(d, J = 7.1Hz, 2H), 2.14-2.38(m, 11H), 2.45(t, J = 69Hz, 2H), 3.59(s, 2H), 3.62(s, 2H), 3.79(t, J = 7.0Hz, 2H), 3.91(s, 3H), 5.10(s, 2H), 6.82(d, J = 1.3Hz, 1H), 6.83(d, J = 1.3Hz, 1H), 6.93(d, J = 1.3Hz, 1H), 6.96-7.08(m, 3H), 7.97(d, J = 8.4Hz, 2H).

Example 47(9): Methyl 5-[(2-{[({1-[3-(8-isobutyl-2,8-diazaspiro[4.5]deca-2-yl)propyl]-1H-imidazol-2-yl}methyl)(methyl) amino]methyl}-1H-imidazol-1-yl)methyl]-2-furoate

TLC: Rf 0.63 (dichloromethane : methanol : 28% ammonia water = 80 : 20 : 2);
NMR($CDCl_3$): δ 0.88(d, J = 6.4Hz, 6H), 1.47-1.65(m, 6H), 1.69-1.89(m, 3H), 2.04(d, J = 7.1Hz, 2H), 2.17-2.38(m, 11H), 2.47(t, J = 7.0Hz, 2H), 3.65(s, 2H), 3.67(s, 2H), 3.88(s, 3H), 3.93(t, J = 7.0Hz, 2H), 5.14(s, 2H), 6.09(d, J = 3.5Hz, 1H), 6.87(d, J = 1.3Hz, 1H), 6.92-6.99(m, 3H), 7.05(d, J = 3.5Hz, 1H).

Example 48: (2-{[({1-[3-(8-isobutyl-2,8-diazaspiro[4.5]deca-2-yl)propyl]-1H-imidazol-2-yl}methyl)(methyl)amino]methyl}-1H-imidazol-1-yl)acetic acid

The same procedure as in Example 4 was carried out, except that the compound synthesized in Example 47 was used in place of the compound synthesized in Example 3 in Example 4, the title compound having the following physical properties was obtained.
TLC: Rf 0.14 (dichloromethane : methanol : 28% ammonia water = 50 : 10 : 1);
NMR($CD_3OD$): δ 0.94(d, J = 6.6Hz, 6H), 1.60-1.98(m, 7H), 1.98-2.10(m, 2H), 2.10(s, 3H), 2.32(d, J = 6.6Hz, 2H), 2.50-2.68(m, 4H), 2.74(t, J = 6.9Hz, 2H), 2.81(s, 2H), 3.00(t, J = 6.9Hz, 2H), 3.60(s, 2H), 3.65(s, 2H), 4.03(t, J = 6.9Hz, 2H), 4.59(s, 2H), 6.88(d, J = 1.2Hz, 1H), 6.90(d, J = 1.2Hz, 1H), 7.07(d, J = 1.2Hz, 1H), 7.11(d, J = 1.2Hz, 1H).

Example 48(1) to Example 48(7):

The same procedure as in Example 48 was carried out, except that corresponding compounds synthesized in Example 47(1) to Example 47(9) were used in place of the compound synthesized in Example 47 in Example 48, the following compounds of the present invention were obtained.

Example 48(1): 4-(2-{[({1-[3-(8-isobutyl-2,8-diazaspiro[4.5]deca-2-yl)propyl]-1H-imidazol-2-yl}methyl)(methyl)amino] methyl}-1H-imidazol-1-yl)butanoic acid

TLC: Rf 0.38 (dichloromethane : methanol : 28% ammonia water = 30 : 10 : 2);
NMR($CD_3OD$): δ 0.94(d, J = 6.6Hz, 6H), 1.56-1.80(m, 6H), 1.82-2.05(m, 5H), 2.11(s, 3H), 2.16(t, J = 7.0Hz, 2H), 2.30(d, J = 7.1Hz, 2H), 2.48-2.62(m, 6H), 2.64(s, 2H), 2.83(t, J = 7.0Hz, 2H), 3.63(s, 2H), 3.67(s, 2H), 3.91-4.11(m, 4H), 6.88(d, J = 1.5Hz, 1H), 6.89(d, J = 1.3Hz, 1H), 7.13(d, J = 1.5Hz, 1H), 7.15(d, J = 1.3Hz, 1H).

Example 48(2): 5-(2-{[({1-[3-(8-isobutyl-2,8-diazaspiro[4.5]deca-2-yl)propyl]-1H-imidazol-2-yl}methyl)(methyl)amino]methyl}-1H-imidazol-1-yl)pentanonic acid

TLC: Rf 0.24 (dichloromethane : methanol : 28% ammonia water = 30 : 10 : 2);
NMR($CD_3OD$): δ 0.93(d, J = 6.6Hz, 6H), 1.47-1.80(m, 10H), 1.80-2.00(m, 3H), 2.11-2.21(m, 5H), 2.26(d, J = 7.3Hz, 2H), 2.37-2.46(m, 2H), 2.46-2.61(m, 6H), 2.68(t, J = 7.0Hz, 2H), 3.55-3.68(m, 4H), 3.91(t, J = 7.5Hz, 2H), 4.00(t, J = 6.9Hz, 2H), 6.84-6.93(m, 2H), 7.09-7.19(m, 2H).

Example 48(3): 3-{2-{[({1-[3-(8-isobutyl-2,8-diazaspiro[4.5]deca-2-yl)propyl]-1H-imidazol-2-yl}methyl)(methyl)amino]methyl}-1H-imidazol-1-yl)propanoic acid

TLC: Rf 0.20 (dichloromethane : methanol : 28% ammonia water = 30 : 10 : 2);
NMR($CD_3OD$): δ 0.93(d, J = 6.6Hz, 6H), 1.55-1.77(m, 6H), 1.79-2.01(m, 3H), 2.13(s, 3H), 2.26(d, J = 7.0Hz, 2H), 2.39-2.63(m, 10H 2.73(t, J = 7.0Hz, 2H), 3.63(s, 2H), 3.66(s, 2H), 4.01(t, J = 6.9Hz, 2H), 4.21(t, J = 7.3Hz, 2H), 6.88(m, 2H), 7.11(d, J = 1.3Hz, 1 H), 7.18(d, J = 1.5Hz, 1H).

Example 48(4): 2-[(2-{[({1-[3-(8-isobutyl-2,8-diazaspiro[4.5]deca-2-yl)propyl]-1H-imidazol-2-yl}methyl)(methyl)amino]methyl}-1H-imidazol-1-yl)methyl]benzoic acid

TLC: Rf 0.34 (dichloromethane : methanol : 28% ammonia water = 30 : 10 : 2);
NMR($CD_3OD$): δ 0.93(d, J = 6.6Hz, 6H), 1.53-2.06(m, 9H), 2.08(s, 3H), 2.26(d, J = 6.4Hz, 2H), 2.36-2.77(m, 8H), 2.78-3.00(m, 2H), 3.56(s, 2H), 3.60(s, 2H), 3.96(t, J = 7.1 Hz, 2H), 5.59(s, 2H), 6.60(dd, J = 7.3, 1.7Hz, 1H), 6.84(d, J = 1.5Hz, 1H), 6.96(d, J = 1.5Hz, 1H), 7.09(d, J = 1.5Hz, 1H), 7.15(d, J= 1.5Hz, 1H), 7.18-7.35(m, 2H), 7.62-7.76(m, 1H).

Example 48(5): 3-[(2-{[({1-[3-(8-isobutyl-2,8-diazaspiro[4.5]deca-2-yl)propyl]-1H-imidazol-2-yl}methyl)(methyl)amino]methyl}-1H-imidazol-1-yl)methyl]benzoic acid

TLC: Rf 0.27 (dichloromethane : methanol: 28% ammonia water = 30 : 10 : 2);
NMR($CD_3OD$): δ 0.96(d, J = 6.6Hz, 6H), 1.51-1.75(m, 6H), 1.75-2.04(m, 3H), 2.18(s, 3H), 2.31(t, J = 7.3Hz, 2H), 2.36-2.47 (m, 4H), 2.51-2.81(m, 6H), 3.60(s, 2H), 3.63(s, 2H), 3.85(t, J = 6.9Hz, 2H), 5.20(s, 2H), 6.85(d, J = 1.5Hz, 1H), 6.94(d, J = 1.5Hz, 1H), 7.00-7.08(m, 2H), 7.10(d, J = 1.5Hz, 1H), 7.31(t, J = 7.6Hz, 1H), 7.74-7.82(m, 1H), 7.82-7.91(m, 1H).

Example 48(6): 4-[(2-{[({1-[3-(8-isobutyl-2,8-diazaspiro[4.5]deca-2-yl)propyl]-1H-imidazol-2-yl}methyl)(methyl)anino]methyl}-1H-imidazol-1-yl)methyl]benzoic acid

TLC: Rf 0.31 (dichloromethane : methanol: 28% ammonia water = 30 : 10 : 2);
NMR($CD_3OD$): δ 1.00(d, J = 6.6Hz, 6H), 1.57-1.86(m, 8H), 1.94-2.09(m, 1H), 2.18(s, 3H), 2.28(t, J = 6.9Hz, 2H), 2.39 (s, 2H), 2.53-2.69(m, 4H), 2.71-3.05(m, 4H), 3.59(s, 2H), 3.61(s, 2H), 3.77(t, J = 7.0Hz, 2H), 5.17(s, 2H), 6.88(d, J = 1.5Hz, 1H), 6.97(d, J = 1.5Hz, 1H), 7.01-7,08(m, 3H), 7.10(d, J = 1.3Hz, 1H), 7.90(d, J = 8.4Hz, 2H).

Example 48(7): 5-[(2-{[({1-[3-(8-isobutyl-2,8-diazaspiro[4.5]deca-2-yl)propyl]-1H-imidazol-2-yl}methyl)(methyl)amino]methyl}-1H-imidazol-1-yl)methyl]-2-furancarboxylic acid

TLC: Rf 0.27 (dichloromethane : methanol: 28% ammonia water = 30 : 10 : 2);
NMR($CD_3OD$): δ 0.95(d, J = 6.6Hz, 6H), 1.55-1.78(m, 6H), 1.81-2.00(m, 3H), 2.19(s, 3H), 2.27-2.85(m, 13H), 3.64(s, 2H), 3.72(s, 2H), 3.93(t, J = 6.8Hz, 2H), 5.20(s, 2H), 6.26(d, J = 3.2Hz, 1H), 6.84(d, J = 3.2Hz, 1H), 6.87(d, J = 1.3Hz, 1H), 6.91(d, J = 1.3Hz, 1H), 7.10(d, J = 1.3Hz, 1H), 7.17(d, J = 1.3Hz, 1H).

Example 49: 2-{[({-[4-(hydroxymethyl)benzyl]-1H-imidazol-2-yl}methyl)(methyl)amino]methyl}-N,N-dimethyl-1H-imidazole-1-sulfonamide

Example 4(1) (618 mg), triethylamine (0.80 mL) and tetrahydrofuran (5 mL) were stirred at 0°C and isobutyl chloroformate (0.39 mL) was added dropwise. The reaction solution was stirred at 0°C for 2 hours and the precipitated salt was filtered through celite (trade name). The filtrate was cooled to 0°C and sodium borohydride (108 mg) was added while suspending in water. The reaction solution was stirred at 0°C for one hour, diluted with an aqueous 1N sodium

hydroxide solution and then extracted with dichloromethane. The organic layer was washed with saturated brine and then dried over anhydrous magnesium sulfate. Anhydrous sodium sulfate was removed by filtration and the filtrate was concentrated. The residue was purified by silica gel chromatography (ethyl acetate : methanol : 28% ammonia water = 1 : 0 : 0 → 70 : 27 : 3) to obtain the title compound (220 mg) having the following physical properties.
TLC: Rf 0.16 (chloroform : methanol : 28% ammonia water = 80 : 20 : 1);
NMR($CDCl_3$): δ 2.30(s, 3H), 2.83(s, 6H), 3.71(s, 2H), 3.83(s, 2H), 4.67(s, 2H), 5.27(s, 2H), 6.85(d, J = 1.2Hz, 1H), 6.95 (d, J = 1.2Hz, 1H), 6.97(d, J = 1.5Hz, 1H), 7.05(d, J = 8.4Hz, 2H), 7.21(d, J = 1.5Hz, 1H), 7.30(d, J = 8.4Hz, 2H).

Example 50: 2-{[{[1-(4-formylbenzyl)-1H-imidazol-2-yl]methyl}(methyl)amino]methyl}-N,N-dimethyl-1H-imidazole-1-sulfonamide

To a dimethyl sulfoxide (3 mL) solution of the compound (215 mg) synthesized in Example 49 and triethylamine (0.29 mL), a sulfur trioxide-pyridine complex (164 mg) was added. The reaction solution was stirred at room temperature for one hour. To the reaction solution, 1N sodium hydroxide water was added. The aqueous layer was extracted with dichloromethane. The organic layer was washed with saturated brine and then dried over anhydrous magnesium sulfate. Anhydrous magnesium sulfate was removed by filtration and then concentrated under reduced pressure. The residue was purified by silica gel chromatography (ethyl acetate : methanol : 28% ammonia water = 1 : 0 : 0 → 70 : 27 : 3) to obtain the title compound (1.21 g) having the following physical properties.
TLC: Rf 0.31 (chloroform : methanol : 28% ammonia water = 80 : 20 : 1);
NMR($CDCl_3$): δ 2.04(s, 3H), 2.82(s, 6H), 3.70(s, 2H), 3.82(s, 2H), 5.43(s, 2H), 6.85-7.80(m, 6H), 7.82(d, J = 8.1Hz, 2H), 9.98(s, 1H).

Example 51: tert-butyl 2-{4-[(2-{[({1-[(dimethylamino)sulfonyl]-1H-imidazol-2-yl}methyl)(methyl)amino]methyl}-1H-imidazol-1-yl)methyl]benzyl}-2,8-diazaspiro[4.5]decane-8-carboxylate

The same procedure as in Example 11, except that the compound synthesized in Example 50 was used in place of the compound synthesized in Example 10 in Example 11, the title compound having the following physical properties was obtained.
TLC: Rf 0.43 (chloroform : methanol : 28% ammonia water = 80 : 20 : 1);
NMR($CDCl_3$): δ 1.41-1.67(m, 15H), 2.31(s, 3H), 2.35(s, 2H), 2.57(t, J = 6.7Hz, 2H), 2.83(s, 6H), 3.22-3.43(m, 4H), 3.54 (s, 2H), 3.72(s, 2H), 3.84(s, 2H), 5.25(s, 2H), 6.86(d, J = 1.3Hz, 1H), 6.95(d, J = 1.3Hz, 1H), 6.97(d, J = 1.3Hz, 1H), 7.00 (d, J = 7.7Hz, 2H), 7.22(d, J = 1.3Hz, 1H), 7.24(d, J = 7.7Hz, 2H).

Example 52: 1-{1-[4-(2,8-diazaspiro[4.5]deca-2-ylmethyl)benzyl]-1H-imidazol-2-yl}-N-(1H-imidazol-2-ylmethyl)-N-methylmethaneamine

The same procedure as in Example 6, except that the compound synthesized in Example 51 was used in place of the compound synthesized in Example 5 in Example 6, the title compound having the following physical properties was obtained.
TLC: Rf 0.56 (chloroform : methanol: 28% ammonia water = 80 : 20 : 4);
NMR(DMSO-$D_6$): δ 1.36(t, J = 5.4Hz, 4H), 1.51(t, J = 6.9Hz, 2H), 2.06(s, 3H), 2.25(s, 2H), 2.37-2.47(m, 2H), 2.51-2.64 (m, 4H), 3.46(s, 4H), 3.52(s, 2H), 5.15(s, 2H), 6.74-6.97(m, 3H), 7.02(d, J = 7.8Hz, 2H), 7.13(d, J = 1.5Hz, 1H), 7.20(d, J = 7.8Hz, 2H), 11.63-12.25(m, 1H).

Example 53: 1-(1H-imidazol-2-yl)-N-[(1-{4-[(8-isobutyl-2,8-diazaspiro[4.5]deca-2-yl)methyl]benzyl}-1H-imidazol-2-yl) methyl]-N-methylmethaneamine

The same procedure as in Example 3, except that isobutylaldehyde was used in place of the compound synthesized in Example 1 and the compound synthesized in Example 52 was used in place of the compound synthesized in Example 2 in Example 3, the title compound having the following physical properties was obtained.
TLC: Rf 0.50 (chloroform : methanol : 28% ammonia water = 80 : 10 : 1);
NMR(DMSO-$D_6$): δ 0.81(d, J = 6.6Hz, 6H), 1.36-1.57(m, 6H), 1.63-1.76(m, 1H), 1.89-2.00(m, 2H), 2.06(s, 3H), 2.14-2.32 (m, 6H), 2.36-2.47(m, 2H), 3.41-3.50(m, 4H), 3.52(s, 2H), 5.16(s, 2H), 6.80(d, J = 1.2Hz, 1H), 6.82-6.98(m, 2H), 7.02 (d, J = 8.1Hz, 2H), 7.09-7.17(m, 1H), 7.21(d, J = 8.1Hz, 2H), 11.09-12.56(m, 1H).

Example 54: 1-[1-(4-{[8-(1-ethylpropyl)-2,8-diazaspiro[4.5]deca-2-yl]carbonyl}benzyl)-1H-imidazol-2-yl]-N-(1H-imidazol-2-ylmethyl)-N-methylmethaneamine

The same procedure as in Example 5 → Example 6 → Example 24 was carried out, except that the compound synthesized in Example 4(1) was used in place of the compound synthesized in Example 4 in Example 5, the title compound having the following physical properties was obtained.
TLC: Rf 0.35 (chloroform : methanol: 28% ammonia water = 80 : 10 : 1);
NMR(DMSO-$D_6$): δ 0.72-0.94(m, 6H), 1.05-1.59(m, 8H), 1.60-1.84(m, 2H), 2.00-2.09(m, 3H), 2.09-2.46(m, 5H), 3.15(s, 2H), 3.33-3.58(m, 6H), 5.19-5.30(m, 2H), 6.61-6.90(m, 2H), 6.92-7.14(m, 3H), 7.14-7.24(m, 1H), 7.34-7.53(m, 2H), 11.64-12.08(m, 1H).

Example 54(1) to Example 54(2):

The same procedure as in Example 54, except that corresponding compound was used in place of the compound synthesized in Example 4(1) in Example 54, the following compounds were obtained.

Example 54(1): 1-(1-{3-[8-(1-ethylpropyl)-2,8-diazaspiro[4.5]deca-2-yl]-3-oxopropyl}-1H-imidazol-2-yl)-N-(1H-imidazol-2-ylmethyl)-N-methylmethaneamine

TLC: Rf 0.30 (dichloromethane : methanol : 28% ammonia water = 90 : 10 : 1);
NMR($CDCl_3$): δ 0.88(t, J = 7.2Hz, 6H), 1.20-1.58(m, 8H), 1.62-1.82(m, 2H), 2.14(m, 1H), 2.27(s, 3H), 2.32-2.57(m, 4H), 2.77(t, J = 7.2Hz, 2H), 3.15(s, 1H), 3.32(s, 1H), 3.41(t, J = 7.2Hz, 1H), 3.52(t, J = 7.2Hz, 1H), 3.58-3.63(m, 4H), 4.36(t, J = 7.2Hz, 2H), 6.96-7.10(m, 4H).

Example 54(2): 1-(1-{4-[8-(1-ethylpropyl)-2,8-diazaspiro[4.5]deca-2-yl]-4-oxobutyl}-1H-imidazol-2-yl)-N-(1H-imidazol-2-ylmethyl)-N-methylmethaneamine

TLC: Rf 0.30 (dichloromethane : methanol : 28% ammonia water = 90 : 10 : 1);
NMR($CDCl_3$): δ 0.88-0.95(m, 6H), 1.20-1.58(m, 8H), 1.60-1.85(m, 2H), 2.10-2.58(m, 12H), 3.15(s, 1H), 3.32(s, 1H), 3.40 (t, J = 7.2Hz, 1H), 3.48-3.63(m, 5H), 4.10(t, J = 7.2Hz, 2H), 6.93(m, 1H), 6.96-7.10(m, 3H).

BIOLOGICAL EXAMPLES

Efficacy of the compound of the present invention, for example, the fact that the compound of the present invention has CXCR4 antagonistic activity, the compound having a group capable of reducing basicity of a compound, namely, a hydroxyl group, a sulfo group or a carboxyl group of the present invention has low toxicity (for example, low risk of phospholipidosis, small distribution volume as a causative that enhances a risk of toxicity peculiar to a compound having basicity), and the compound of the present invention obtaind by converting the compound into a prodrug has high maximum blood concentration and is also conberted in vivo, has been demonstrated by the following experiment.

A measuring method of the present invention was modified to improve accuracy and/or sensitivity of the measurement for evaluating the compound of the present invention. The detailed experimental methods are shown bellow.

As mentioned above, a more direct procedure is a screening a compound that prevents for HIV from binding to CXCR4, which is a receptor on CD4+ cell, on an assay system using HIV viruses. However, using HIV viruses for a large-scale screening is not practical due to its difficult handling. On the other hand, both of T cell-directed (X4) HIV-1 and SDF-1 bind to CXCR4 and therefore CXCR4 binding sites at both of HIV-side and SDF-1-side as well as SDF-1- and HIV-binding sites at the CXCR4 side may presumably have any common characteristics. Thus, in order to find a compound inhibiting absorption of HIV viruses to a cell that is a different mechanism from those of pre-existing anti-AIDS drugs (reverse transcriptase inhibitors and protease inhibitors), an assay system using an endogenous ligand for CXCR4, SDF-1 instead of HIV may be available.

Specifically, as a system of screening a compound that inhibits the binding between SDF-1 and CXCR4, for example a system of measuring the binding between iodine-labeled SDF-1 and a human T cell strain in which CXCR4 is known to be expressed is operable.

TEST METHODS

Test Example 1

Study for Inhibition of Binding Human SDF-1 to CEM Cells

To human T cell strain CEM cells in a binding buffer (containing HEPES and BSA), the test compound and $^{125}$I-SDF-1 (PerkinElmer) were added and the mixture was incubated at 4°C for 60 minutes. The reacted CEM cells were rapidly filtrated with a GF/B membrane filter plate (Packard) to adsorb. The plate was washed with phosphate buffered saline (hereinafter abbreviated to PBS) three times and then dried. Microscint+20 (Packard) was added thereto. An amount of the radioactivity bound to the CEM cells was measured using Top Count (Packard) and inhibition (%) of the test compound was calculated according to the following equation:

[Equation 1]

$$\text{Inhibition} = \{(Et - Ea)/(Et - Ec)\} \times 100$$

wherein

Et: Amount of radioactivity when the test compound is not added,
Ec: Amount of radioactivity when non-radioactive SDF-1 (Pepro Tech) is added in an amount of 1,000 times as much as $^{125}$I-SDF-1 as a test compound, and
Ea: Amount of radioactivity when the test compound is added.

All compounds of the present invention shown in Examples exhibited inhibition of 50% or more in a concentration of 10 μM. For example, $IC_{50}$ values for the compounds synthesized in Example 21(4), Example 22(4), Example 25(1), Example 40(12), Example 41(6), Example 42, Example 43, Example 40(13), Example 41(7), Example 42(1), Example 42(4), Example 43(1), Example 43(2), Example 43(3) and Example 47(4) were respectively 7.4 nM, 20.7 nM, 6.6 nM, 3.0 nM or less, 4.4 nM, 3.0 nM, 4.0 nM, 3.0 nM or less, 4.5 nM, 3.0 nM or less, 4.4 nM, 6.3 nM, 9.3 mM, 13.6 nM, and 4.3 nM.

Test Example 2

Investigation with Phospholipidosis Detecting System using Fluorescent Labeling Phospholipidosis Analog under Severity Condition

(1) Phospholipid Accumulation Measurement

100 μL/well as required (1 dose 2 wells) of cell suspension of CHL/IU (cell line derived from a Chinese hamster lung) (7 × $10^4$ cells/mL) prepared by MEM (minimum essential medium) culture medium was added to a 96-well plate (96-well clear-bottom plate), and cultured for about 24 hours. After culture, the supernatant of the 96-well plate was removed, and the 100 μL/well compounds of each concentration dissolved and suspended in an MEM including 25 μmol/L nitrobenzoxadiazole dipalmitoyl phosphatidylethanolamine (NBD-PE) (hereinafter abbreviated as a NPD-PE medium) were added and treated for about 48 hours. The treatment concentrations of each compound were set to be 5, 50, 100 and 200 μM. The positive control substance was set to be amiodarone hydrochloride, and the treatment concentrations were set to be 1.25, 2.5, 5, 10 and 20 μM. In addition, 5-well untreated controls (only MEM) and 5-well NBD-PE controls (made by means of adding a 1/100 amount of DMSO to the NBD-PE culture medium) were set per compound, and cultured in the same manner. After finishing the culture, the cultures were washed twice with phosphate buffered saline (hereinafter abbreviated as PBS) (-) 100 μL/well, and the MEM (100 μL) was added to all of the treatment wells including two empty wells for WST-1 background controls and cultured for about a half hour. The fluorescence intensities of each well were measured by using a microplate reader (manufactured by Molecular Devices Inc., SPECTRA max M2; the excitation wavelength 485 nm/fluorescence wavelength 535 nm).

(2) Analysis

Using the average values of each dose × 2 wells, a phospholipid increase rate (%) to the NBD-PE control was calculated by using the following calculating formula:

[Equation 2]

Rate of increase of phospholipid accumulation (%) = 100 × {(test compound fluorescence intensity – non-treated control fluorescence intensity)/(NBD-PE control fluorescence intensity - non-treated control fluorescence intensity)}

(3) Cytotoxicity Assay

96-well plate measured in the phospholipid accumulation measurement was measured by means of Plate Reader (manufactured by Molecular Devices Inc., SPECTRA max M2) with the main wavelength of 450 nm and the correct wavelength of 690 nm to calculate a Pre value. An amount of 5 μL/well of Premix WS T-1 was added to each of the 96-hole plates by which Pre measurement was conducted. After culture for 2 to 4 hours, the 96-well plate was measured as well as the Pre measurement to calculate an Aft value. Then, the background control value was subtracted from the each measured value. A value which was calculated by subtracting the Pre value from the Aft value was used, then the cell growth rate (%) was calculated by using the following calculating formula:

[Equation 3]

Cell growth rate (%) = 100 × [(test substance OD)/(NBD-PE control OD)]

(4) Determination

A test dose that indicated value of 25% or more of the maximum phospholipid accumulation increase rate of amiodarone which was the positive control was determined as positive. In addition, the dose whose cell growth rate was equal to or less than 50% in the cytotoxicity assay was not used for determination of existence or nonexistence of a phospholipidosis inductive effect.

As a result, it was found that the compound having a hydroxyl group in a prodrug modification, a sulfo group in a prodrug modification or a carboxyl group in a prodrug modification of the present invention, which was converted as a result of the reaction with an enzyme or gastric acid in vivo, had a low phospholipidosis inductive effect in phospholipidosis experiment system using fluorescent labeling phospholipidosis analog. For example, the rates of increase of phospholipid accumulation (%) of the compounds synthesized in Example 22(3), Example 22(4), Example 25(1), Example 41(6), Example 43, Example 41(7) and Example 43(1) are as described in the following table.

Table 1

| Compound | Rate of increase of phospholipid accumulation (%) (compound dose: 200 μM) | Determination |
|---|---|---|
| Example 22 (3) | 20 | Negative |
| Example 22(4) | 16 | Negative |
| Example 25(1) | 16 | Negative |
| Example 41(6) | 16 | Negative |
| Example 43 | 16 | Negative |
| Example 41(7) | 21 | Negative |
| Example 43(1) | 14 | Negative |

Test Example 3

Artificial Lipid Membrane Binding Account by using Biacore® S51 System

(1) Liposome Preparation

10 mM of 1,2-dioleoyl-sn-glycero-3-phosphate monosodium salt (hereinafter abbreviated as DOPA) chloroform solution was evaporated to dryness by means of an aspirator, and 0.6 mL ofPBS/5% dimethyl sulfoxide (hereinafter abbreviated as DMSO) was added. The chloroform solution was fully suspended by means of a vortex mixer, and the freezing and thawing were repeated for 5 times. Liposome was created by means of a liposome preparation instrument (manufactured by Avestin Inc.) and two syringes, and was diluted to 0.5 mM with PBS/5% DMSO just prior to immobilization.

(2) Measurement compound preparation

38 $\mu$l of 1 $\times$ PBS is added to 2 $\mu$l of a DMSO solution (10 mM), and further the 360 $\mu$l of 1 $\times$ PBS/5% DMSO was added and the 50 $\mu$M of final concentration in PBS/5% DMSO was prepared and measured.

(3) Analysis

All of the following analysis used Biacore® S51 system and the measurement conditions were set by Biacore S51 Control Soft.

The measurement temperature was set to be 37°C, and PBS/5% DMSO (pH 7.4 or 6.0) was used as a buffer. Series S Sensor Chip L1 was used for a sensor chip. DOPA was immobilized in one of the measuring spots on the sensor surface and the central spot was used as a reference.

Immobilization of liposome was conducted at a flow rate of 10 $\mu$L/min for about 3 minutes and then the compound was added at a flow rate of 30 $\mu$L/min and the interaction was measured. The measurement conditions are as follows:

Assay buffer: PBS/5% DMSO (pH 7.4)
Measurement temperature: 37°C
Sensor chip: Series S Sensor Chip L1
Flow rate: 10 $\mu$L/min at the time of the liposome immobilization; 30 $\mu$L/min at the time of the measurement of interaction with the compound

Regeneration: 20 mM CHAPS (3-[(3-cholamidopropyl)dimethylammonio]-1-propanesulfonate), isopropylalcohol/aqueous 50 mM sodium hydroxide solution = 40/60 (volume ratio) mixture solution (60 seconds)

(4) Data processing

The data processing was conducted by means of method ofAbdiche et al., (Analytical biochemistry, 328, 233-243 (2004)) by using Biacore S51 Evaluation Soft.

Regarding the value of binding response (RU) in which the value of reference was subtracted, it was divided by a sample molecular weight after minute error of the DMSO concentration included in the sample solution was corrected. In addition, the value obtained here was divided by an amount of capture at the time of the cycle because the value obtained here was proportional to the amount of capture of the liposome and the value was multiplied by a million to be considered as a corrected value (Corrected value = 1,000,000 x RU (test compound)/molecular quantity (test compound) RU (liposome)).

Propranolol, amiodarone, desipramine, imipramine and procaine were added as the controls, and it was recognized that the variation of the binding response was within about 10 to 15%.

(5) Determination

The compound wherein a value of the binding response after the correction was 150 or more was determined as positive.

As a result, it was found that the compound having a hydroxyl group in a prodrug modification, a sulfo group in a prodrug modification or a carboxyl group in a prodrug modification of the present invention, which was converted as a result of the reaction with an enzyme or gastric acid in vivo, had a low phospholipidosis inductive effect in artificial lipid membrane binding experiment system using Biacore® S51 system.

For example, the values of binding response (RU) of the compounds synthesized in Example 22(3), Example 22(4), Example 25(1), Example 41(6), Example 43, Example 41 (7) and Example 43(1) are as described in the following table.

Table 2

| Compound | Value of binding response (RU) | Determination |
|---|---|---|
| Example 22(3) | 29.9 | Negative |
| Example 22(4) | 23.4 | Negative |
| Example 25(1) | 28.3 | Negative |
| Example 41(6) | 72.9 | Negative |
| Example 43 | 36.1 | Negative |
| Example 41(7) | 63.6 | Negative |
| Example 43(1) | 41.2 | Negative |

Test Example 4

Test on Stability in Serum (Conversion into Carboxylic Acid)

After preincubating 495 μL of serum at 37°C for 5 minutes, 5 μL of a preparation solution (10 g/mL acetonitrile solution) of a substance to be evaluated was added and incubation was initiated at 37°C (final concentration: 100 ng/mL). Immediately after initiation of incubation and 30 minutes after initiation of incubation, 100 μL of the reaction solution was collected and then mixed with 1 mL of acetonitrile thereby terminating the reaction. After termination of the reaction, the reaction solution was centrifuge at 3,000 rpm for 10 minutes. After the supernatant was dried by a centrifugal concentrator, the residue was redissolved in water/acetonitrile (1/1) and then analyzed by C/MS/MS (API5000). Measuring conditions of the column suited for the compound were selected.

When the compound to be evaluated is the compound of the present invention such as a compound having a hydroxyl group in a prodrug modification, a sulfo group in a prodrug modification, or a carboxyl group in a prodrug modification, the remaining amount of the compound to be evaluated, and the amount of the compound produced by converting the compound as a result of the reaction with an enzyme or gastric acid in vivo were calculated.

As a result, it was found that the compound having a hydroxyl group in a prodrug modification, a sulfo group in a prodrug modification, or a carboxyl group in a prodrug modification to be evaluated as the compound of the present invention is converted nearly completely in serum. For example, the amounts of compounds Example 25(1), Example 25(1), Example 41(6), Example 41(7), Example 43 and Example 43(1) produced by in vivo conversion of compounds having a carboxyl group in a prodrug modification, namely, compounds synthesized in Example 31, Example 31(1), Example 40(12), Example 40(13), Example 42 and Example 42(1) are as shown in the following table.

[Table 3]

| Compound to be evaluated | Compound to be converted in vivo | Produced amount of converted compound (%) |
|---|---|---|
| Example 31 | Example 25(1) | 80 |
| Example 31(1) | Example 25(1) | 98 |
| Example 40(12) | Example 41(6) | 76 |
| Example 40(13) | Example 41(7) | 96 |
| Example 42 | Example 43 | 99 |
| Example 42(1) | Example 43(1) | 97 |

Test Example 5

Measurement of Maximum Plasma Level (Cmax) and Stationary State Distribution Volume (Vss)

A compound to be evaluated is weighed, dissolved in WellSolve (trade name; manufactured by Celeste Corporation) which is heated to 60°C and adjusted to 20 mg/mL; thereafter, the test compound is diluted by 10 times with distilled water for injection, and further diluted by 2 times with saline solution, by which intravenously administered solution is eventually made. A compound to be evaluated is dissolved in WellSolve which is heated to 60°C and adjusted to 50 mg/mL; thereafter, the compound to be evaluated is diluted with distilled water for injection and adjusted to 1 mg/mL. Five compounds (one set) were mixed in the same amount and adjusted to final concentration of 0.2 mg/mL per one compound, by which orally administered solution is eventually made. Intravenous administration is performed by rapid single administration (n = 3) of intravenously administered solution (1 mg/kg) from the tail vein of Crl:CD(SD) Rat (male, manufactured by CHARLES RIVER LABORATORIES JAPAN, INC). Oral administration is performed by forced administration (n = 3) of orally administered solution (1 mg/kg) into the stomach of Crl:CD(SD) Rat (male, manufactured by CHARLES RIVER LABORATORIES JAPAN, INC.) by using a sonde. The administration is conducted under fasting conditions; water is freely ingested. In the case of intravenous administration, a 0.35 mL of blood sample is taken from cervical vein by using Heparinized Syringe at 5 minutes, 15 minutes, 30 minutes, 1 hour, 2 hours, 4 hours, 6 hours, 8 hours, and 24 hours after administration. In the case of oral administration, a 0.35 mL of blood sample is taken from cervical vein by using Heparinized Syringe at 15 minutes, 30 minutes and 1 hour after administration. The obtained blood is stored in ice, and after centrifugation at 12,000 rpm for 3 minutes, blood plasma is fractionated. In the case of intravenous administration, the blood plasma sample preserved at a temperature of -80°C is dissolved; inner standard solution (100 μL) and acetonitrile (2 mL) are added to the blood plasma sample (100 μL) and stirred, and the sample is centrifuged at 2,500 rpm for 10 minutes.

In the case of oral administration, the blood plasma sample (100 μL) and inner standard solution (μL) are mixed immediately after recovering the blood serum. After the supernatant centrifuged is evaporated to dryness by means of a centrifugal concentrator, 67% dimethyl sulfoxide solution (150 μL) is added to the residue and dissolves them again and 20 μL of the solution is analyzed by means of LC/MS/MS.

[LC conditions]

Measurement device: Waters 2795 (manufactured by Waters Corporation)
Analytical column: Unison UK-C18, 3.0 μm particle size, 2.0 mm × 30 mm (manufactured by Imtakt Corporation)
Analytical column temperature: Room temperature
Flow rate: 200 μL/min
Moving bed: 5 mM IPC-PFAA-7 solution/acetonitrile (9/1 → 1/9)

[MS/MS conditions]

Measurement device: Quatro micro API (manufactured by Micromass Communications Inc.)
Scan type: MPM
Polarity: Positive
Ion Source: ESI
The monitor ion which was suitable for each sample was selected.
A portion of the compound to be intravenously administered was subjected to the following treatment. The blood plasma sample preserved at a temperature of -80°C is dissolved; inner standard solution (50 μL) and acetonitrile (1 mL) are added to the blood plasma sample (50 μL) and stirred, and the sample is centrifuged at 3,000 rpm for 10 minutes. After the supernatant is evaporated to dryness by means of a centrifugal concentrator, the residue was redissolved in water/acetonitrile (1/1) and the solution is analyzed by means of LC/MS/MS under the following conditions.

[LC conditions]

Measurement device: 1200 series system (manufactured by Agilent Technologies) Analytical column: Asahipak OOD-50 2D, 2 × 150 mm (manufactured by Showa Denko K.K.)
Analytical column temperature: Room temperature
Moving bed: 5 mM IPC-PFAA-7 solution/acetonitrile (gradient)
Flow rate: 200 μL/min

[MS/MS conditions]

Measurement device: API5000 (manufactured by Applied Biosystems)
Scan type: MRM
Polarity: Positive
Ion Source: Turbo Spray

The monitor ion which was suitable for each sample was selected.

The concentration transition in the blood plasma of the test compound is analyzed by using WinNonlin 4.0.1 and stationary state distribution volume (Vss) was calculated. Regarding the maximum blood concentration Cmax, an average of the compound concentrations in three individuals was determined and an average at the time of the highest value was taken as maximum blood concentration.

As a result, the maximum blood concentration Cmax of the compound having a group having an acidic group in a prodrug modification, a sulfo group in a prodrug modification or a carboxyl group in a prodrug modification of the present invention indicated good values. When the compound to be evaluated is a prodrug to be metabolized after administration, the maximum blood concentration Cmax of the compound converted as a result of subjection to the reaction with an enzyme or gastric acid in vivo was measured. For example, when the compound of Example 21 (4) as a prodrug of Example 22 (4), the compound of Example 24(1) as a prodrug of Example 25(1), the compound of Example 42 as a prodrug of Example 43 and the compound of Example 42(1) as aprodrug of Example 43(1) are administered, the maximum blood concentration Cmax of the compounds obtained by in vivo conversion, namely, compounds synthesized in Example 22(4), Example 25(1), Example 43 and Example 43(1) were measured.

Table 4

| Compound to be evaluated | Compound to be converted in vivo | Produced amount of converted compound (%) |
|---|---|---|
| Example 21(4) | Example 22(4) | 93.4 |
| Example 24(1) | Example 25(1) | 184 |
| Example 42 | Example 43 | 134 |
| Example 42(1) | Example 43(1) | 103 |

As a result, the compound having a hydroxyl group in a prodrug modification, a sulfo group in a prodrug modification, or a carboxyl group in a prodrug modification of the present invention converted as a result of subjection to the reaction with an enzyme or gastric acid in vivo, namely, the compound of th present invention such as the compound having a hydroxyl group, a carboxyl group, or sulfo group showed a low stationary state distribution volume (Vss). For example, the stationary state distribution volume of the compounds synthesized in Example 22(3), Example 22(4), Example 25(1), Example 41(6), Example 41(7), Example 43 and Example 43(1) are as shown in the following table.

Table 5

| Compound to be evaluated | Distribution volume (L/kg) |
|---|---|
| Example 22(3) | 0.90 |
| Example 22(4) | 1.10 |
| Example 25(1) | 0.36 |
| Example 43 | 0.65 |
| Example 43(1) | 0.42 |

FORMULATION EXAMPLES

Formulation Example 1

1-(1H-imidazol-2-yl)-N-({1-[4-(8-isobutyl-2,8-diazaspiro[4.5]deea-2-yl)butyl]-1H-imidazol-2-yl}methyl)-N-methylmethaneamine (100 g), calcium carboxymethyl cellulose (disintegrant, 20.0 g), magnesium stearate (lubricant, 10.0 g) and microcrystalline cellulose (870 g) were mixed by a conventional method and then compressed to obtain 10,000 tablets each containing 20 mg of an active ingredient.

Formulation Example 2

1-(1H-imidazol-2-yl)-N-({1-[4-(8-isobutyl-2,8-diazaspiro[4.5]deca-2-yl)butyl]-1H-imidazol-2-yl}methyl)-N-methylmethaneamine (100 g), mannitol (2 kg)and distilled water (50 L) were mixed by a conventional method and filtered with a dust filter, and then each ampoule was filled with 5 mL of the obtained mixture and subjected to heat sterilization in an autoclave to obtain 10,000 ampoules each containing 10 mg of an active ingredient.

INDUSTRIAL APPLICABILITY

The compound of the present invention has an antagonistic activity against CXCR4 and is therefore useful as a preventive and/or therapeutic agent for CXCR4-mediated diseases. For example, the compound of the present invention is useful as a preventive and/or therapeutic agent for inflammatory and immune diseases (for example, rheumatoid arthritis, arthritis, retinopathy, pulmonary fibrosis, acute lung injury, transplanted organ rejection, etc.), allergic diseases, infections (for example, human immunodeficiency virus infection, acquired immunodeficiency syndrome, etc.), psychoneurotic diseases, cerebral diseases, cardiovascular disease, metabolic diseases, and cancerous diseases (for example, cancer, cancer metastasis, etc.), or an agent for regeneration therapy.

## Claims

**1.** A compound represented by formula (I):

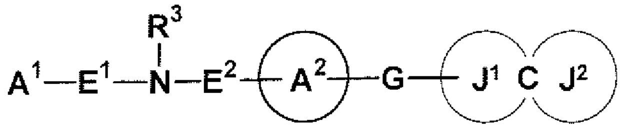

(I)

wherein $A^1$ represent a nitrogen-containing heterocyclic ring which may have a substituent(s) or $-NR^1R^2$ wherein $R^1$ and $R^2$ each independently represents a hydrogen atom or a substituent(s);
ring $A^2$ represents a divalent monocyclic cyclic group which may have a substituent(s);
$E^1$ represents a divalent C1-4 aliphatic hydrocarbon group which may have a substituent(s);
$E^2$ represents a methylene group or a carbonyl group;
$R^3$ represents (1) a hydrogen atom, (2) a C1-4 aliphatic hydrocarbon group which may be substituted with a hydroxyl group which may be protected by a protective group, a carboxyl group which may be protected by a protective group, or a sulfo group which may be protected by a protective group, and also may have a substituent(s), (3) a carbocyclic ring group which may be substituted with a hydroxyl group which may be protected by a protective group, a carboxyl group which may be protected by a protective group, or a sulfo group which may be protected by a protective group, and also may have a substituent(s), (4) a heterocyclic group which may be substituted with a hydroxyl group which may be protected by a protective group, a carboxyl group which may be protected by a protective group, or a sulfo group which may be protected by a protective group, and also may have a substituent(s), (5) a C1-4 aliphatic hydrocarbon group which may be substituted with a hydroxyl group which may be protected by a protective group, a carboxyl group which may be protected by a protective group, or a sulfo group which may be protected by a protective group, and is also substituted with a carbocyclic ring group which may have a substituent(s) and may have a substituent(s), (6) a C1-4 aliphatic hydrocarbon group which may be substituted with a hydroxyl group which may be protected by a protective group, a carboxyl group which may be protected by a protective group, or a sulfo group which may be protected by a protective group, and is also substituted with a heterocyclic group which may have a substituent(s) and may have a substituent(s), (7) -(a divalent C1-4 aliphatic hydrocarbon group which may have a substituent(s))-(a divalent carbocyclic ring which may have a substituent(s))-$(Y^3)_t$-Z, or (8)-(a divalent C1-4 aliphatic hydrocarbon group which may have a substituent(s))-(a divalent heterocyclic ring which may have a substituent(s))-$(Y^3)_t$-Z wherein $Y^3$ represents a methylene group which may have a substituent(s), an ethenylene group which may have a substituent(s), an ethynylene group, a divalent nitrogen atom which may have a substituent(s), -C(O)-, -O-, -S-, -S(O)-, or $-SO_2-$, Z represents a hydroxyl group which may be protected by a protective group, a carboxyl group which may be protected by a protective group, or a sulfo group which may be protected by a protective group, t represents an integer of 1 to 4, provided that when t represents an integer of 2 or more, a plurality of $Y^3$ may be the same or different;
G represents

$-(G^1)_p-$ , or (a)

(b) 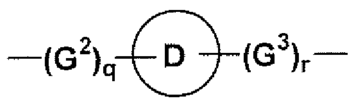

wherein $G^1$, $G^2$ and $G^3$ each independently represents a methylene group which may have a substituent(s), an ethenylene group which may have a substituent(s), an ethynylene group, a divalent nitrogen atom which may have a substituent(s), -C(O)-, -O-, -S-, -S(O)-, or - $SO_2$-, ring D represents a divalent monocyclic cyclic group which may have a substituent(s), p represents an integer of 1 to 8, q and r each independently represents 0 or an integer of 1 to 6, provided that when p represents an integer of 2 or more, a plurality of $G^1$ may be the same or different, when q represents an integer of 2 or more, a plurality of $G^2$ may be the same or different, and when r represents an integer of 2 or more, a plurality of $G^3$ may be the same or different, and the sum of q and r represents 6 or less;
ring $J^1$ represents a 3- to 10-membered monocyclic or bicyclic heterocyclic ring which has at least one nitrogen atom and also may have an oxygen atom and/or an optionally oxidized sulfur atom; ring $J^2$ represents (i) a C3-10 monocyclic or bicyclic carbocyclic ring substituted with a group having a basic group, (ii) a 3- to 10-membered monocyclic or bicyclic heterocyclic ring consisting of a carbon atom, an oxygen atom and/or an optionally oxidized sulfur atom which is substituted with a group having a basic group, or (iii) a 3- to 10-membered monocyclic or bicyclic heterocyclic ring which may be substituted with a group having a basic group, and also has at least one nitrogen atom and may have an oxygen atom and/or an optionally oxidized sulfur atom,
ring $J^1$ and ring $J^2$ may have 1 to 8 substituent(s) on the substitutable position and, when the number of substituents is 2 or more, a plurality of substituents may be the same or different,
a salt thereof, an N-oxide thereof or a solvate thereof, or a prodrug thereof.

**2.** The compound according to claim 1, wherein $R^3$ is:

(a) -$Y^1$-Z-, or

(b) 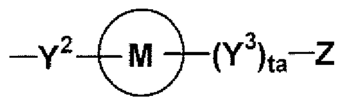

wherein $Y^1$ represents a divalent C1-4 aliphatic hydrocarbon group which may have a substituent(s);
Z represents (1) a hydroxyl group which may be protected by a protective group, (2) a carboxyl group which may be protected by a protective group, or (3) a sulfo group which may be protected by a protective group;
ring M represents a divalent C3-15 carbocyclic ring which may have a substituent(s), or a divalent 5- to 6-membered heterocyclic ring consisting of carbon atom, oxygen atom and/or an optionally oxidized sulfur atom which may have a substituent(s);
$Y^2$ represents a bond or a divalent C1-4 aliphatic hydrocarbon group which may have a substituent(s);
$Y^3$ has the same meaning as described in claim 1; and
ta represents 0 or an integer of 1 to 4 and, provided that when ta represents an integer of 2 or more, a plurality of $Y^3$ may be the same or different and, when ta represents an integer of 1 or more, $Y^2$ represent a divalent C1-4 aliphatic hydrocarbon group which may have a substituent(s).

**3.** The compound according to claim 2, wherein Z is

(1) a hydroxyl group protected by a protective group,
(2) a carboxyl group protected by a protective group, or
(3) a sulfo group protected by a protective group.

**4.** The compound according to claim 2, wherein (1) the hydroxyl group which may be protected by a protective group, (2) the carboxyl group which may be protected by a protective group, or (3) the sulfo group which may be protected by a protective group are respectively (1) a hydroxyl group in a prodrug modification, (2) a carboxyl group in a prodrug modification, or (3) a sulfo group in a prodrug modification.

**5.** The compound according to claim 1, wherein $A^1$ is a nitrogen-containing heterocyclic ring which may have a substituent(s).

**6.** The compound according to claim 5, wherein the nitrogen-containing heterocyclic ring is imidazole, benzoimidazole, 6,7-dihydro-5H-cyclopenta[b]pyridine, 5,6,7,8-tetrahydroquinoline, 6,7,8,9-tetrahydro-5H-cyclohepta[b]pyridine, or imidazo[1,2-a]pyridine ring.

**7.** The compound according to claim 1, wherein the ring $A^2$ is a divalent 3- to 8-membered monocyclic heterocyclic ring which may have a substituent(s), and also have 1 to 3 nitrogen atom(s) and may have an oxygen atom and/or an optionally oxidized sulfur atom.

**8.** The compound according to claim 7, wherein the 3- to 8-membered monocyclic heterocyclic ring is a 5- or 6-membered monocyclic aromatic heterocyclic ring.

**9.** The compound according to claim 8, wherein 5- or 6-membered monocyclic aromatic heterocyclic ring is:

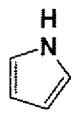 , 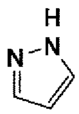 , 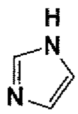 , 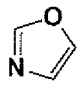 , 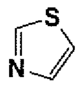 , 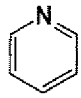 ,

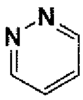 , 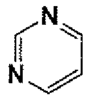 , 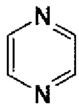 or 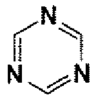

wherein a nitrogen atom of -NH- may be combined with $E^2$ or G, and also a nitrogen atom of -NH- may have a substituent(s).

**10.** The compound according to claim 9, wherein the 5- or 6-membered monocyclic aromatic heterocyclic ring is:

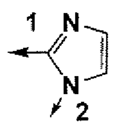 or 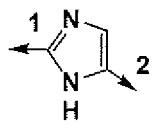

wherein arrow 1 is bonded to $E^2$, arrow 2 is bonded to G, and a nitrogen atom of -NH-represents a substituent(s).

**11.** The compound according to claim 1, wherein $E^2$ is a methylene group.

**12.** The compound according to claim 1, wherein $G^1$ represents a methylene group which may have a substituent(s) and p is an integer of 1 to 4.

**13.** The compound according to claim 1, wherein

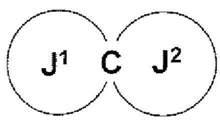 is 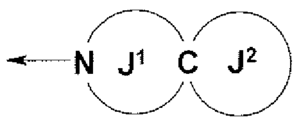

wherein arrow is bonded to G and other symbols have the same meanings as described in claim 1.

**14.** The compound according to claim 13, wherein

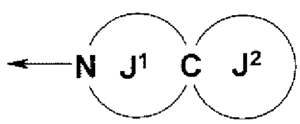

is:

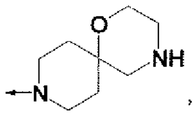, 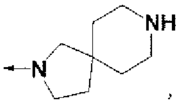, 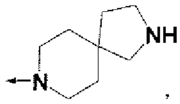, 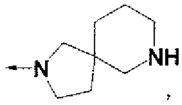, 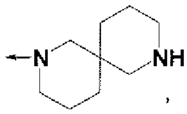,

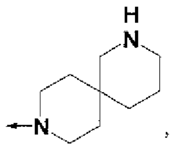, 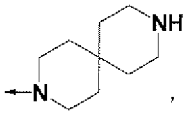, 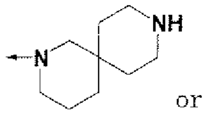 or 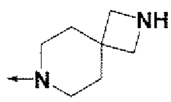

which may have a substituent(s) (wherein nitrogen atom of - NH- may have a substituent(s), which may have a substituent(s)).

**15.** The compound according to claim 1, which is represented by formula (I-1):

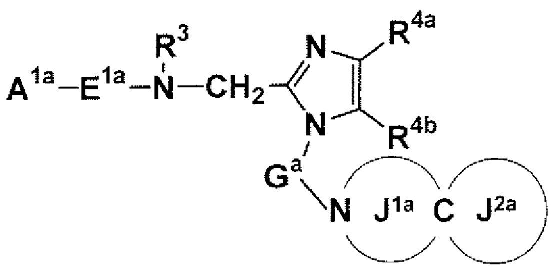

**(I-1)**

wherein $A^{1a}$ represents an imidazole ring which may have a substituent(s), a benzoimidazole ring which may have a substituent(s), a 6,7-dihydro-5H-cyclopenta[b]pyridine ring which may have a substituent(s), a 5,6,7,8-tetrahydroquinoline ring which may have a substituent(s), a 6,7,8,9-tetrahydro-5H-cyclohepta[b]pyridine ring which may have a substituent(s), or an imidazo[1,2-a]pyridine ring which may have a substituent(s);
$E^{1a}$ represents a methylene group which may have a substituent(s);
$R^{4a}$ and $R^{4b}$ each independently represents a hydrogen atom or a substituent(s);
$G^a$ represents:

(a) $-(G^1)_{pa}-$ , or

**(b)** 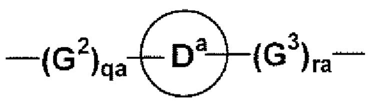

wherein ring $D^a$ represents a divalent C3-8 monocyclic carbocyclic ring which may have a substituent(s), pa represents an integer of 1 to 4, qa and ra each independently represents 0 or an integer of 1 to 4, and other symbols have the same meanings as described in claim 1, provided that when pa represents an integer of 2 or more, a plurality of $G^1$ may be the same or different, when qa represents an integer of 2 or more, a plurality of $G^2$ may be the same or different, and when ra represents an integer of 2 or more, a plurality of $G^3$ may be the same or different, and the sum of qa and ra represents 4 or less; and

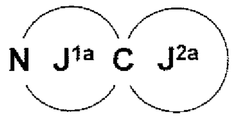

represents:

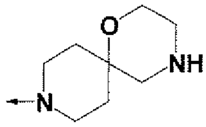, 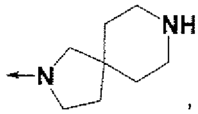, 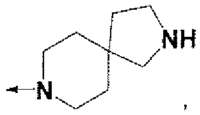, 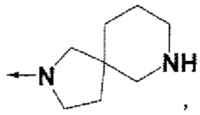, 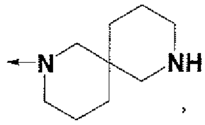,

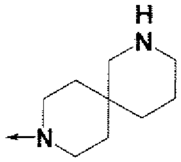, 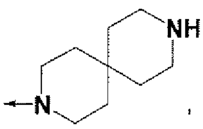, 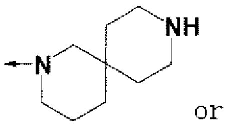 or 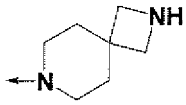

which may have a substituent(s)wherein arrow is bonded to $G^a$, a nitrogen atom of -NH-may have a substituent(s) which may have a substituent(s); and other symbols have the same meanings as described in claim 1.

**16.** The compound according to claim 15, which is represented by formula (I-1-1):

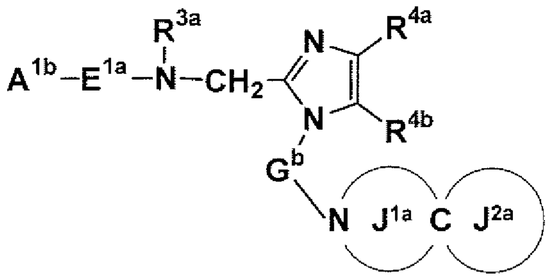

**(I-1-1)**

wherein $A^{1b}$ represents an imidazole ring which may have a substituent(s);
$G^b$ represents a divalent C1-4 aliphatic hydrocarbon group which may have a substituent(s);
$R^{3a}$ represents:

**(a) $-Y^1-Z^a$** , or

**(b)** 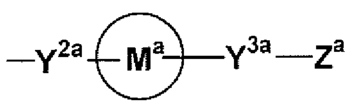

wherein $Z^a$ represents a carboxyl group which may be protected by a protective group, ring $M^a$ represents a divalent C5-10 aromatic carbocyclic ring which may have a substituent(s), a thiophene ring which may have a substituent (s), or a furan ring which may have a substituent(s), $Y^{2a}$ represents a divalent C1-4 aliphatic hydrocarbon group which may have a substituent(s), $Y^{3a}$ represents a bond, a divalent C1-4 aliphatic hydrocarbon group which may have a substituent(s), or ← O- (a divalent C1-3 alkylene group which may have a substituent(s)) → wherein arrow directing the left is bonded to ring $M^a$, and other symbols have the same meanings as described in claim 2; and

other symbols have the same meanings as described in claim 15.

**17.** The compound according to claim 16, wherein the protective group is a C1-4 aliphatic hydrocarbon group or a C1-4 aliphatic hydrocarbon group substituted with a mono- or di-substituted carbamoyl group.

**18.** The compound according to claim 1, which is represented by formula (I-2):

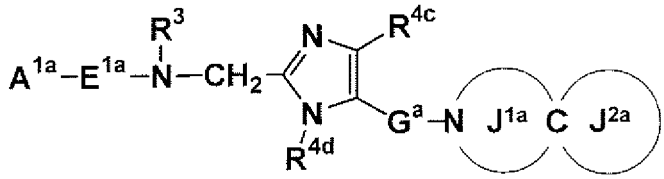

(I-2)

wherein $R^{4c}$ and $R^{4d}$ each independently represents a hydrogen atom or a substituent(s) and other symbols have the same meanings as described in claim 1 or claim 15.

**19.** The compound according to claim 16, which is
4-{[(1-{3-[8-(1-ethylpropyl)-2,8-diazaspiro[4.5]deca-2-yl]propyl}-1H-imidazol-2-yl)methyl](1H-imidazol-2-ylmethyl) amino}butanoic acid, ethyl
[4-({[(1-{3-[8-(1-ethylpropyl)-2,8-diazaspiro[4.5]deca-2-yl]propyl}-1H-imidazol-2-yl)methyl](1H-imidazol-2-ylmethyl) amino}methyl)phenoxy]acetate,
[4-({[(1-{3-[8-(1-ethylpropyl)-2,8-diazaspiro[4.5]deca-2-yl]propyl}-1H-imidazol-2-yl)methyl](1H-imidazol-2-ylmethyl) amino}methyl)phenoxy]acetic acid,
ethyl[3-({[(1-{3-[8-(1-ethylpropyl)-2,8-diazaspiro[4.5]deca-2-yl]propyl}-1H-imidazol-2-yl)methyl](1H-imidazol-2-ylmethyl)amino}methyl)phenoxy]acetate,
[3-({[(1-{3-[8-(1-ethylpropyl)-2,8-diazaspiro[4.5]deca-2-yl]propyl}-1H-imidazol-2-yl)methyl](1H-imidazol-2-ylmethyl) amino}methyl)phenoxy]acetic acid,
2-(dimethylamino)-2-oxoethyl 4-{[(1-{3-[8-(1-ethylpropyl)-2,8-diazaspiro[4.5]deca-2-yl]propyl}-1H-imidazol-2-yl) methyl](1H-imidazol-2-ylmethyl)amino}butanoate, or
2-(diethylamino)-2-oxoethyl 4-{[(1-{3-[8-(1-ethylpropyl)-2,8-diazaspiro[4.5]deca-2-yl]propyl}-1H-imidazol-2-yl)methyl](1H-imidazol-2-ylmethyl)amino}butanoate.

**20.** A pharmaceutical composition comprising a compound represented by formula (I) according to claim 1, a salt thereof, an N-oxide thereof or a solvate thereof, or a prodrug thereof.

**21.** The pharmaceutical composition according to claim 20, which is a CXCR4 antagonist.

**22.** The pharmaceutical composition according to claim 20, which is preventive and/or therapeutic agent for CXCR4-mediated diseases or cancerous diseases, or an agent for regeneration therapy.

**23.** The pharmaceutical composition according to claim 22, wherein CXCR4mediated disease is asthma, human immunodeficiency virus infection, acquired immunological deficiency syndrome, cancer, cancer metastasis, rheumatoid arthritis, arthritis, retinopathy, macular degeneration, pulmonary fibrosis, acute lung injury, ischemic diseases, systemic lupus erythematosus, or transplanted organ rejection, or the agent for regeneration therapy is an agent for a transplantation medical treatment, or an agent for a mobilization to peripheral blood of hematopoietic stem cells.

**24.** The pharmaceutical composition according to claim 22, wherein the CXCR4-mediated disease is human immunodeficiency virus infection.

**25.** A pharmaceutical comprising a compound represented by formula (I) according to claim 1, a salt thereof, an N-oxide thereof or a solvate thereof, or a prodrug thereof, and one or more kinds selected from reverse transcriptase inhibitor, protease inhibitor, CCR2 antagonist, CCR3 antagonist, CCR4 antagonist, CCR5 antagonist, CXCR4 antagonist, HIV integrase inhibitor, fusion inhibitor, CD4 antagonist, antibody against surface antigen of HIV, short interfering RNA of HIV, and vaccine of HIV.

**26.** A pharmaceutical comprising a compound represented by formula (I) according to claim 1, a salt thereof, an N-oxide thereof

thereof or a solvate thereof, or a prodrug thereof, and one or more kinds selected from anticancer drug, antimetabolite, antibiotic, antimitotic drug, platinum complex, plant-derived antineoplastic drug, anticancerous hormone, immunopotentiator, interferon, biologics capable of performing T cell activation, and neovascularisation inhibitor.

**27.** A method for antagonizing CXCR4 in a mammal, which comprises administering an effective dosage of a compound represented by formula (I) according to claim 1, a salt thereof, an N-oxide thereof or a solvate thereof, or a prodrug thereof to the mammal.

**28.** A method for preventing and/or treating CXCR4-mediated diseases in a mammal, which comprises administering an effective dosage of a compound represented by formula (I) according to claim 1, a salt thereof, an N-oxide thereof or a solvate thereof, or a prodrug thereof to the mammal.

**29.** Use of a compound represented by formula (I) according to claim 1, a salt thereof, an N-oxide thereof or a solvate thereof, or a prodrug thereof for the preparation of a CXCR4 antagonist.

**30.** Use of a compound represented by formula (I) according to claim 1, a salt thereof, an N-oxide thereof or a solvate thereof, or a prodrug thereof for the preparation of preventive and/or therapeutic agent for CXCR4-mediated diseases.

## INTERNATIONAL SEARCH REPORT

International application No.
PCT/JP2007/064893

A. CLASSIFICATION OF SUBJECT MATTER
*C07D487/10*(2006.01)i, *A61K31/438*(2006.01)i, *A61K45/00*(2006.01)i, *A61P9/10*(2006.01)i, *A61P11/00*(2006.01)i, *A61P11/06*(2006.01)i, *A61P19/02*(2006.01)i, *A61P27/02*(2006.01)i, *A61P29/00*(2006.01)i, *A61P31/18*(2006.01)i,
According to International Patent Classification (IPC) or to both national classification and IPC

B. FIELDS SEARCHED

Minimum documentation searched (classification system followed by classification symbols)
C07D487/10, A61K31/438, A61K45/00, A61P9/10, A61P11/00, A61P11/06, A61P19/02, A61P27/02, A61P29/00, A61P31/18, A61P35/00, A61P35/04, A61P37/02, A61P37/06, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN), REGISTRY(STN), MEDLINE(STN), BIOSIS(STN), EMBASE(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2007/058322 A1 (Ono Pharmaceutical Co., Ltd.), 24 May, 2007 (24.05.07), Full text; particularly, Claims; examples (Family: none) | 1,5-14, 20-26,29,30 |
| X<br>A | WO 2006/022454 A1 (Ono Pharmaceutical Co., Ltd.), 02 March, 2006 (02.03.06), Full text; particularly, page 25, line 18 to page 32, line 24; page 131, line 24 to page 146, line 12; examples; Claims & EP 1790639 A1 | 1,5-14, 20-26,29,30<br>2-4,15-19 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>14 September, 2007 (14.09.07) | Date of mailing of the international search report<br>25 September, 2007 (25.09.07) |
| Name and mailing address of the ISA/<br>Japanese Patent Office<br><br>Facsimile No. | Authorized officer<br><br><br>Telephone No. |

Form PCT/ISA/210 (second sheet) (April 2005)

**INTERNATIONAL SEARCH REPORT**

International application No.
PCT/JP2007/064893

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | WO 2006/006490 A1 (Ono Pharmaceutical Co., Ltd.),<br>19 January, 2006 (19.01.06),<br>Full text; particularly, page 11, line 5 to page 61, 5th line from the bottom; pages 100 to 101, Par. Nos. [0179] to [0182]; page 105, lines 1 to 14; examples; Claims<br>(Family: none) | 1-14,20,22,<br>23,26,30<br>15-19,21,24,<br>25,29 |
| A | WO 2005/115398 A2 (BRISTOL-MYERS SQUIBB CO.),<br>08 December, 2005 (08.12.05),<br>Full text<br>& US 7115601 B2 & EP 1756069 A2 | 1-26,29,30 |
| A | WO 2000/31037 A1 (SMITHKLINE BEECHAM S.P.A.),<br>02 June, 2000 (02.06.00),<br>Full text<br>& US 6780875 B2 & EP 1131295 A1<br>& JP 2002-530377 A & NO 200102473 A<br>& BR 9915475 A & KR 2001075726 A<br>& HU 200104959 A2 & ZA 200104071 A<br>& CN 1406225 A & MX 2001005095 A1<br>& NZ 511777 A & AU 768708 B | 1-26,29,30 |
| A | WO 2005/007656 A1 (VIROCHEM PHARMA INC.),<br>27 January, 2005 (27.01.05),<br>Full text<br>& US 2005/075326 A1 & EP 1776362 A1 | 1-26,29,30 |

Form PCT/ISA/210 (continuation of second sheet) (April 2005)

INTERNATIONAL SEARCH REPORT

International application No.
PCT/JP2007/064893

Continuation of A. CLASSIFICATION OF SUBJECT MATTER
(International Patent Classification (IPC))

*A61P35/00*(2006.01)i, *A61P35/04*(2006.01)i, *A61P37/02*(2006.01)i, *A61P37/06*(2006.01)i, *A61P43/00*(2006.01)i

(According to International Patent Classification (IPC) or to both national classification and IPC)

Form PCT/ISA/210 (extra sheet) (April 2005)

**INTERNATIONAL SEARCH REPORT**

International application No.
PCT/JP2007/064893

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 27, 28
because they relate to subject matter not required to be searched by this Authority, namely:
Claims 27 and 28 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.
PCT/JP2007/064893

<Subject of search>

Claims 1-14, 20-26, 29 and 30 include great many compounds having various combinations of substituents within their scopes. However, among these compounds, those which are supported by the description in the meaning within PCT Article 6 and disclosed in the meaning within PCT Article 5 are limited to an extremely small part of the compounds as claimed.

Further, it is unclear as to what types of compounds the "prodrug" recited in claims 1, 20, 25, 26, 29 and 30 includes within its scope. Therefore, claims 1, 20-26, 29 and 30 do not comply with the requirement of clearness in the meaning within PCT Article 6, too.

Such being the case, the search was made within a reasonable scope of burden based on the scope supported by and disclosed in the description.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007058322 A
- WO 2004024697 A
- WO 2005023247 A
- WO 2006022454 A
- WO 2006020415 A
- WO 2006023400 A
- WO 2006026703 A
- WO 2006036816 A
- WO 2003055876 A
- WO 9907351 A
- WO 9940913 A
- WO 0046195 A
- WO 0046196 A
- WO 0046197 A
- WO 0046198 A
- WO 0046199 A
- WO 0069432 A
- WO 0069815 A
- DE 19837386
- WO 9955324 A
- WO 9955330 A
- WO 0004003 A
- WO 0027800 A
- WO 0027835 A
- WO 0027843 A
- WO 0029377 A
- WO 0031032 A
- WO 0031033 A
- WO 0034278 A
- WO 0035449 A
- WO 0035451 A
- WO 0035452 A
- WO 0035453 A
- WO 0035454 A
- WO 0035876 A
- WO 0035877 A
- WO 0041685 A
- WO 0051607 A
- WO 0051608 A
- WO 0051609 A
- WO 0051610 A
- WO 0053172 A
- WO 0053600 A
- WO 0058305 A
- WO 0059497 A
- WO 0059498 A
- WO 0059502 A
- WO 0059503 A
- WO 0062814 A
- WO 0073327 A
- WO 0109088 A
- WO 02030357 A
- WO 02030358 A
- WO 9917773 A
- WO 9932100 A
- WO 0006085 A
- WO 0006146 A
- WO 0010965 A
- WO 0006153 A
- WO 0021916 A
- WO 0037455 A
- EP 1013276 A
- WO 0038680 A
- WO 0039125 A
- WO 0040239 A
- WO 0042045 A
- WO 0053175 A
- WO 0042852 A
- WO 0066551 A
- WO 0066558 A
- WO 0066559 A
- WO 0066141 A
- WO 0068203 A
- JP 2000309598 B
- WO 0056729 A
- WO 0076933 A
- WO 9825605 A
- WO 9904794 A
- WO 9938514 A
- WO 0116114 A
- WO 02083143 A
- WO 02085862 A
- US 6469002 B
- WO 03101970 A
- WO 0066112 A
- WO 2004052862 A
- WO 2006020891 A
- US 2006069122 A
- WO 2006034001 A
- WO 2006028896 A
- WO 2006048862 A
- WO 2006074426 A
- US 2006160860 A
- WO 2006076131 A
- JP 2006188445 A
- WO 2006090853 A
- WO 2006096444 A
- US 2006281712 A

- WO 2007008539 A
- US 20060293324 A
- WO 2006117011 A
- WO 2007022385 A
- WO 2007027999 A


**Non-patent literature cited in the description**


- *J. Immunol.,* 2000, vol. 165, 6590-6598
- *J. Immunol.,* 2001, vol. 167, 4648-4692
- *J. Immunol.,* 2000, vol. 165, 499-508
- *J. Clin. Invest.,* 2004, vol. 114, 438-446
- *J. Clin. Invest.,* 2005, vol. 115, 86-93
- *J. Exp. Med.,* 1997, vol. 185, 111-120
- *Blood,* 2001, vol. 97, 3354-3360
- *Nature,* 2001, vol. 410, 50-56
- *Cancer Res.,* vol. 62, 1832-1837
- *Cancer Res.,* 2002, vol. 62, 5930-5938
- *Nat. Med.,* 2001, vol. 12, 1339
- *Cancer Res.,* 2002, vol. 62, 3106-3112
- *Proc. Nat. Acad. Sci. USA,* 2003, vol. 100, 13513-13518
- *Mol Cancer Ther.,* 2006, vol. 5, 2592-9
- *Development,* 2002, vol. 129, 4249-4260
- *Trends in Neuroscience,* vol. 25, 548-549
- *Immunology,* 2002, vol. 107, 222-232
- *Circ. Res.,* 2000, vol. 86, 131-138
- *Journal Experimental Medicine,* 2005, vol. 2001, 1307-1318
- *Blood,* 2003, vol. 102, 2728-2730
- *Nature,* 1996, vol. 382, 635-639
- *Nature,* 1998, vol. 393, 591-594
- *Nature,* 1998, vol. 393, 595-599
- *Cell,* 1985, vol. 52, 631
- *Science,* 1996, vol. 272, 872
- *Nature,* vol. 382, 829
- *Nature,* 1996, vol. 382, 833
- *Science,* 1996, vol. 272, 1955
- *Nature Medicine,* 1998, vol. 4, 72
- *YAKUGAKU ZASSHI,* 2001, vol. 121, 557-565
- Development of Drug. Hirokawa Shoten, 1990, vol. 7
- *Molecular Design,* 163-198
- The Merck Manual of Diagnosis and Therapy. Merck & Co,
- **RICHARD C. LAROCK.** Comprehensive Organic Transformations: A Guide to Functional Group Preparations. John Wiley & Sons Inc, 1999
- **T. W. GREENE.** Protective Groups in Organic Synthesis. Wiley, 1999
- *Synthesis,* 2001, 1546-1550
- **GENDAI IRYOSHA.** *Drug Bioavailability (Science of Evaluation and Improvement),* 06 July 1998
- **SATO et al.** *Pharmacokinetic, Xenobio. Metabol. and Dispos.,* 2001, vol. 16 (2), 115-126
- *Drug Metabolism and Disposition,* 2000, vol. 28 (12), 1440-1448
- **ZOU et al.** *Biophys. J.,* 1998, vol. 74, 230-241
- **KASAHARA et al.** *Toxicol. Sci.,* 2006, vol. 90, 1330-141
- **NARITA et al.** *in vitro Phospholipidosis Detection System using Fluorescent-Labeled Phospholipids Analogue*
- *Bioorg. Med. Chem. Lett.,* 2000, vol. 10, 1803
- **ABDICHE et al.** *Analytical biochemistry,* 2004, vol. 328, 233-243